# EUROPEAN PATENT APPLICATION

(11) **EP 4 851 142 A1**
(43) Date of publication of application: **09.12.2026**
(21) Application number: 25748535.9
(22) Date of filing: 27.01.2025
(51) Int. Cl.: C07D 277/54, A61K 31/426, A61P 37/08, C07D 241/20, C07D 417/12, A61K 31/427, C07D 277/48, C07D 417/14, C07D 233/88, C07D 487/04

(54) **NOVEL COMPOUND AS MAS-RELATED G-PROTEIN COUPLED RECEPTOR MEMBER X2 (MRGPRX2) ANTAGONISTS AND PHARMACEUTICAL COMPOSITION COMPRISING SAME**

(30) Priority: 02.02.2024 KR 20240016807; 28.06.2024 KR 20240085749; 06.12.2024 KR 20240180647; 24.12.2024 KR 20240196154
(71) Applicant: Samjin Pharmaceutical Co., Ltd., Seoul 04054 (KR)
(72) Inventor: NAM, Eunhye, Bucheon-si Gyeonggi-do 14508 (KR); LEE, Soojin, Gimpo-si Gyeonggi-do 10099 (KR); JO, Seongman, Seoul 07711 (KR); KIM, Yu Ro, Incheon 21939 (KR); SON, Yeongnam, Seoul 05410 (KR); SONG, Eunsun, Seoul 07649 (KR); JEONG, Seunghwarn, Gimpo-si Gyeonggi-do 10068 (KR); YEON, Seung Ju, Seoul 07678 (KR); LEE, Soo Min, Seoul 06277 (KR); KIM, Jincheul, Seoul 07982 (KR); SOHN, Te-ik, Seoul 07794 (KR); WOO, Juhyun, Goyang-si Gyeonggi-do 10595 (KR); LEE, Jeongmin, Seoul 07204 (KR); HA, Haechan, Seoul 06262 (KR); JEONG, Dong Koo, Seoul 07643 (KR); LEE, Hyemi, Bucheon-si Gyeonggi-do 14544 (KR); LEE, Je Heon, Seoul 07717 (KR); CHO, Hyoung Min, Bucheon-si Gyeonggi-do 14693 (KR); PARK, Seunghee, Seoul 07794 (KR); KIM, Jong In, Seoul 07053 (KR)
(74) Representative: Witthoff Jaekel Steinecke Patentanwälte PartG mbB
(86) International application number: PCT/IB2025/050871
(87) International publication number: WO 2025/163461

(57) **Abstract**

The present invention relates to a compound having a novel structure represented by chemical formula I or chemical formula II, a stereoisomer thereof, or a pharmaceutically acceptable salt thereof as an MRGPRX2 antagonist, a pharmaceutical composition comprising the same, and a method and a use for preventing or treating diseases using the same.

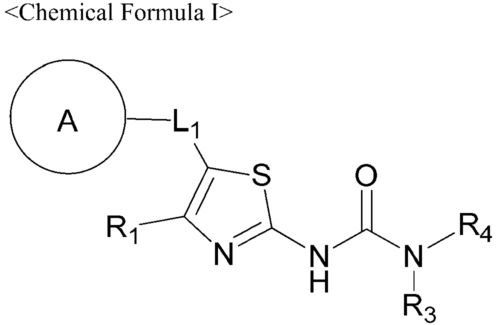

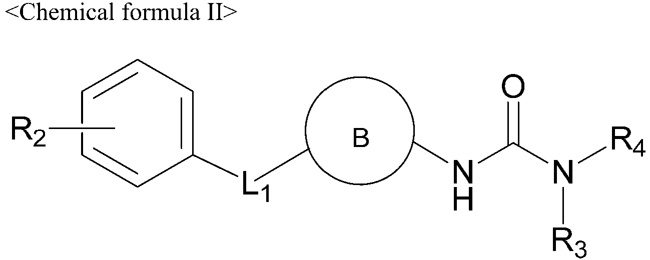

## Description

### Technical Field

The present invention relates to a compound having a novel structure as an MRGPRX2 antagonist, a stereoisomer thereof, or a pharmaceutically acceptable salt thereof, a pharmaceutical composition comprising the same, and a method and a use for preventing or treating diseases using the same.

### Background

Most drugs developed for treating allergic diseases are treatments for relieving symptoms, which act in the last stage of the allergic mechanism, but allergic diseases are refractory diseases that have not yet been conquered due to persistent symptoms, frequent recurrence, and side effects of the treatments, and the prevalence of chronic urticaria, atopic dermatitis, and the like continues to increase.

MRGPRX2 (Mas-related G-protein coupled receptor member X2) protein is a mast cell receptor, where degranulation occurs independent of immunoglobulin E (Ig E), is functionally expressed in the skin, and is known to exhibit activity against multiple agonists, such as various endogenous peptides, allergens, infectious agents, toxins, FDA-approved drugs, and the like, comprising receptors of basophils and eosinophils.

MRGPRX2-related indications comprise neurogenic inflammation, pain, itching, pruritic skin diseases, and the like, comprising chronic spontaneous urticaria (CSU) and atopic dermatitis (AD).

The number of positive cells for the protein MRGPRX2 has increased in the skin of patients with chronic spontaneous urticaria in light of molecular mechanisms and pathophysiological roles, and further studies have shown that CSU patients exhibit wheal reactions due to the intradermal application of the agonist of the protein (Allergy Asthma Immunol Res. 2021 May;13(3): 498-506). In addition, when the murine homolog of MRGPRX2 was knocked out (KO), it has been confirmed that house dust mite-induced allergic reactions were decreased, and the recruitment of immune cells and inflammation-induced hypersensitivity were lowered in a mouse model of inflammatory pain after surgery (Neuron. 2019 Feb 6;101(3):412-420).

Thus, there is a need to develop a compound capable of modulating allergic reaction disorders and allergic diseases, comprising anaphylaxis, chronic itching, inflammatory disorders, pain disorders, and the like, as an antagonist capable of blocking various pruritic mediators which activate MRGPRX2.

### Related Art Reference

### Non-Patent Documents

(0001) Allergy Asthma Immunol Res. 2021 May;13(3): 498-506

(0002) Neuron. 2019 Feb 6;101(3):412-420

### Detailed Description of the Invention

### Technical Problem

The present invention provides a compound having a novel structure as an MRGPRX2 antagonist, a stereoisomer thereof, or a pharmaceutically acceptable salt thereof.

The present invention provides a pharmaceutical composition comprising a compound having a novel structure as an MRGPRX2 antagonist, a stereoisomer thereof, or a pharmaceutically acceptable salt thereof.

The present invention provides a pharmaceutical composition for preventing or treating allergic diseases, inflammatory bowel disease, arthritis, or migraine, comprising a compound having a novel structure as an MRGPRX2 antagonist, a stereoisomer thereof, or a pharmaceutically acceptable salt thereof.

The present invention provides a method for preventing or treating allergic diseases, inflammatory bowel disease, arthritis, or migraine, comprising administering a compound having a novel structure as an MRGPRX2 antagonist, a stereoisomer thereof, or a pharmaceutically acceptable salt thereof, or a pharmaceutical composition comprising the same into a subject.

The present invention provides a use of a compound having a novel structure as an MRGPRX2 antagonist, a stereoisomer thereof, or a pharmaceutically acceptable salt thereof for preventing or treating allergic diseases, inflammatory bowel disease, arthritis, or migraine.

The present invention provides a use of a compound having a novel structure as an MRGPRX2 antagonist, a stereoisomer thereof, or a pharmaceutically acceptable salt thereof in the manufacture of a medicament for preventing or treating allergic diseases, inflammatory bowel disease, arthritis, or migraine.

### Technical Solution

Hereinafter, the present invention will be described in more detail.

### Compound, stereoisomer thereof, or pharmaceutically acceptable salt thereof

(1) The present invention provides a compound represented by chemical formula I or chemical formula II below, a stereoisomer thereof, or a pharmaceutically acceptable salt thereof: 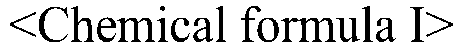 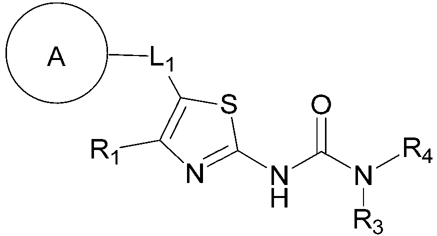 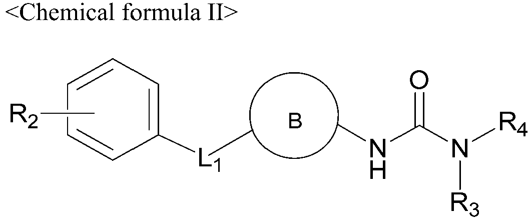 in which in chemical formula I,
   A is 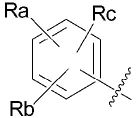 (Ra, Rb, and Rc are each independently H, halogen, CN, -NO₂, C1-C6 alkyl, C1-C6 haloalkyl, -O-(C1-C6 alkyl), -C(=O)-OH, -C(=O)-(C1-C6 alkyl), -C(=O)-O-(C1-C6 alkyl), -N(C1-C6 alkyl)(C1-C6 alkyl), or C3-C12 cycloalkyl), 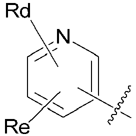 (Rd and Re are each independently H or halogen), 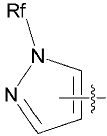 (Rf is H or C1-C6 alkyl), or 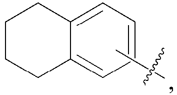
   R₁ is H, C1-C6 alkyl, or -C(=O)-O-(C1-C6 alkyl);
   in which in chemical formula II, B is 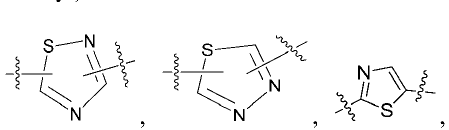 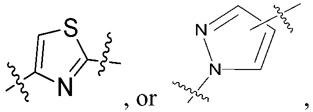
   R₂ is H or halogen;
   and in which in each of chemical formulas I and II,
   Li is -(CH₂)x- (x is an integer of 1 to 3), -O-, -N(Rg)- (Rg is H or C1-C6 alkyl), or - O-(CH₂)y- (y is an integer of 1 to 3),
   R₃ and R₄ are each a functional group as defined below, or are linked to each other to form a cyclic group comprising at least one N,
   R₃ is H, C1-C6 alkyl, or 3- to 12-membered heterocycloalkyl comprising at least one heteroatom selected from N, O and S, in which at least one H of C1-C6 alkyl may be each independently substituted with OH, CN, -O-(C1-C6 alkyl), or C3-C12 cycloalkyl,
   R₄ is 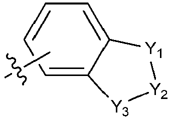 (Y₁ to Y₃ each independently represent CH₂, O, S, or NH), 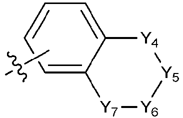 (Y₄ to Y₆ each independently represent CH₂, O, S, C(=O), or NH), 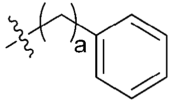 (a is an integer of 0 to 3), 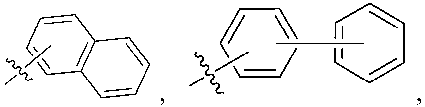 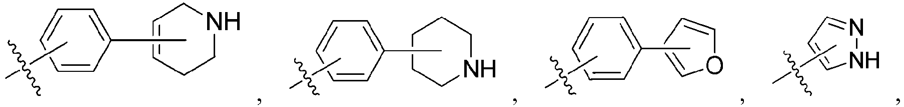 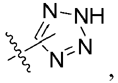 or 5- to 12-membered heteroaryl comprising at least one heteroatom selected from N, O and S,
   in which at least one H of R₄ may be each independently substituted with halogen, - CN, -NO₂, -OH, C1-C6 alkyl (in which at least one H of C1-C6 alkyl may be each independently substituted with -O-(C1-C6 alkyl)), C1-C6 haloalkyl, C2-C6 alkynyl, C3-C6 cycloalkyl, -O-(C1-C6 alkyl), -O-(C1-C6 haloalkyl), -S-(C1-C6 haloalkyl), -NH₂, -N(C1-C6 alkyl)(C1-C6 alkyl), -C(=O)-Rg (Rg is C1-C6 alkyl, -NH₂, -O-(C1-C6 alkyl), -OH, 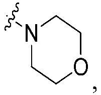 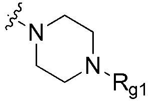 (R_{g1} is H or C1-C6 alkyl), or 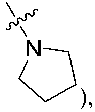 -[(C1-C6 alkylene)]z-NH-S(=O)₂-Rₕ (Rₕ is C1-C6 alkyl, C3-C6 cycloalkyl, -N(C1-C6 alkyl)(C1-C6 alkyl), and z is 0 or 1), -O-S(=O)₂-Rᵢ (Rᵢ is C1-C6 alkyl or C6-C12 aryl, in which at least one H of C6-C12 aryl may be each independently substituted with C1-C6 alkyl), -S(=O)₂-Rⱼ (Rⱼ is C1-C6 alkyl or 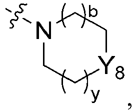 Y₈ is CH₂, O, or NH, b is 0 or 1, and y is 0 or 1, in which at least one H of Rⱼ may be each independently substituted with halogen, C1-C6 alkyl, or C1-C6 haloalkyl), or -S(=O)₂-N(Rₘ)Rₙ (Rm and Rn are each independently H, C1-C6 alkyl, C1-C6 haloalkyl, C3-C12 cycloalkyl, 3- to 12-membered heterocycloalkyl comprising at least one heteroatom selected from N, O and S, or C6-C12 aryl, and at least one H of each of Rm and Rn may be each independently substituted with OH or -O-(C1-C6 alkyl));
   a cyclic group formed by linking R₃ and R₄ to each other is 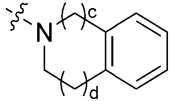 (c and d each independently represent any one integer of 0 to 3), 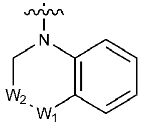 (W₁ and W₂ each represent CH₂, NH, or O, and W₁ and W₂ are not simultaneously CH₂), 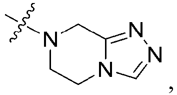 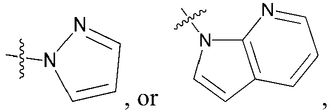 and
   at least one H of the cyclic group may be each independently substituted with halogen, CN, OH, NO₂, C1-C6 alkyl, C1-C6 haloalkyl, C3-C12 cycloalkyl, 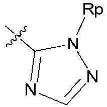 (Rp is H or C1-C6 alkyl), or -S(=O)₂-N(C1-C6 alkyl)(C1-C6 alkyl).

In the present invention, "Cm-Cn" (in which m and n are each independently an integer of 1 or more, and m < n) means the number of carbons and, for example, "C1-C6 alkyl" represents an alkyl having one to six carbon atoms.

In the present invention, "may be substituted" refers to being substituted or unsubstituted.

In the present invention, "a- to b-membered" (in which a and b are each independently an integer of 1 or more, and a < b) refers to the number of atoms which form a ring, and for example, "4- to 12-membered ring" refers to a ring having a shape ranging from a quadrangle to a dodecagon. As one example, 4- to 12-membered heterocycloalkyl is a ring which forms a shape ranging from a quadrangle to a dodecagon, and the atoms which form a ring may comprise not only carbon, but also one or more heteroatoms such as N, O, S and the like.

In the present invention, "alkyl" means a linear or branched saturated hydrocarbon group represented by -CₙH₂ₙ₊₁ (in which n is an integer of 1 or more), unless otherwise stated. Examples of alkyl include methyl, ethyl, n-propyl, isopropyl, n-butyl, sec-butyl, tert-butyl and the like, but are not limited thereto.

In the present invention, "alkylene" means a divalent functional group which is derived from the alkyl as defined above, unless otherwise stated.

In the present invention, "alkenyl" means an unsaturated hydrocarbon group comprising at least one double bond between carbons in the alkyl, unless otherwise stated. Examples of alkenyl include -CH=CH₂, -CH₂CH=CH₂, -CH=CH-CH₃ and the like.

In the present invention, "alkenylene" means a divalent functional group which is derived from the alkenyl as defined above, unless otherwise stated.

In the present invention, "alkynylene" means an unsaturated hydrocarbon group comprising at least one triple bond between carbons in the alkyl, unless otherwise stated. Examples of alkynylene include -CH≡CH, -CH₂C≡CH, -C≡CH-CH₃ and the like.

In the present invention, "cycloalkyl" means a saturated hydrocarbon ring having three or more carbon atoms, and the saturated hydrocarbon ring comprises both monocyclic and polycyclic structures, unless otherwise stated. In addition, it is meant to comprise all of bicyclic ring structures such as a bridged ring or a spiro structure. Examples of cycloalkyl include at least one selected from cyclopropyl, cyclobutyl, cyclopentyl, cyclohexyl, cycloheptyl, cyclooctyl, decahydronaphthalenyl, spiro[4.4]nonanyl, spiro[3.3]heptanyl, bicyclo[3.1.0]hexanyl, bicyclo[3.1.1]heptanyl, bicyclo[2.2.1]heptanyl and the like, but are not limited thereto.

In the present invention, "cycloalkylene" means a divalent functional group which is derived from the cycloalkyl as defined above, unless otherwise stated.

In the present invention, "heterocycloalkyl" means a cyclic group in which at least one carbon atom constituting a ring in the cycloalkyl is each independently substituted with a heteroatom selected from the group consisting of N, O, and S, unless otherwise stated. In the heterocycloalkyl, the heteroatom may comprise at least one heteroatom of the same kind, and two or more heteroatoms may be each independently comprised therein. Examples of heterocycloalkyl include at least one selected from oxiranyl, oxetanyl, tetrahydrofuranyl 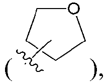 tetrahydropyranyl 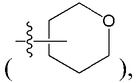 piperidinyl 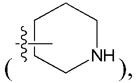 piperazinyl 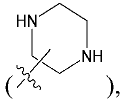 morpholinyl 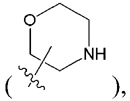 thiomorpholinyl 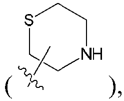 pyrrolidinyl 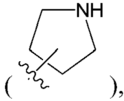 azetidinyl 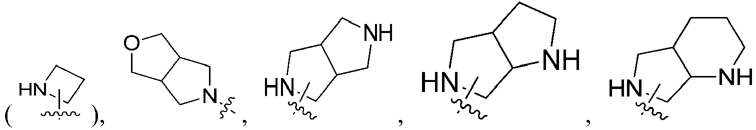 and the like, but are not limited thereto.

In the present invention, "heterocycloalkylene" means a divalent functional group which is derived from the heterocycloalkyl as defined above, unless otherwise stated.

In the present invention, "aryl" means an aromatic monocyclic or polycyclic cyclic group, unless otherwise stated. Examples of aryl include phenyl, naphthyl, tetrahydronaphthyl, indenyl, anthracenyl, and the like, but are not limited thereto.

In the present invention, "heteroaryl" means an aromatic monocyclic or polycyclic cyclic group comprising one to three heteroatoms selected from the group consisting of N, O, and S, unless otherwise stated. Examples of heteroaryl include thiazolyl, oxazolyl, thiophenyl, furanyl, pyrrolyl, imidazolyl, isoxazolyl, pyrazolyl, triazolyl, pyridinyl, indolyl, 2H-pyrazolo[3,4-b]pyridinyl, 2H-pyrazolo[3,4-c]pyridinyl, 1H-pyrrolo[2,3-b]pyridinyl, 1H-pyrrolo[2,3-c]pyridinyl, benzothiazolyl, benzoxazolyl, benzothiophenyl, benzofuranyl, benzopyrrolyl, benzoimidazolyl, benzoisoxazolyl, benzopyrazolyl, benzotriazolyl, benzopyridinyl, 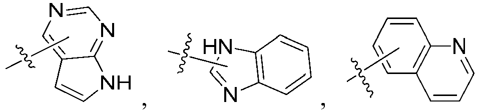 and the like, but are not limited thereto.

In the present invention, "haloalkyl" means a functional group in which at least one H of the alkyl as defined above is each independently substituted with halogen, unless otherwise stated. Examples of haloalkyl include -CF₃, -CH₂-CF₃, -CH₂CF₂H, -CF₂H, -CCl₃, -CH₂-CCl₃, -CCl₂H, -CF₂Cl, -CFH-CH₃, -CF₂-CH₃, -CFCl₂ and the like, but are not limited thereto.

In the present invention, "halogen" may be F, Cl, Br, or I.

Besides, the terms and abbreviations used in the present specification have their original meanings, unless otherwise defined.

(2) In above (1), in each of chemical formula I and chemical formula II according to one embodiment of the present invention, it may be provided that R₄ is 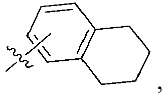 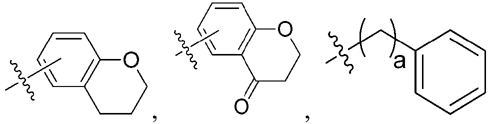 (a is an integer of 0 to 3), 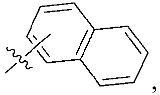 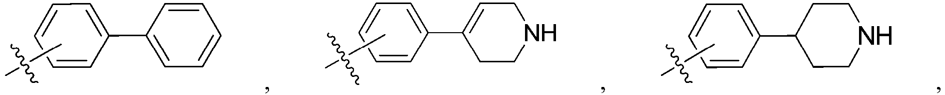 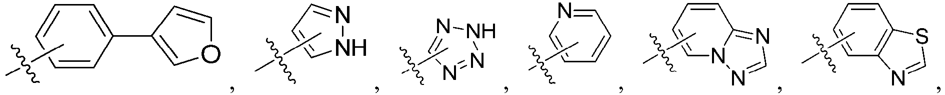 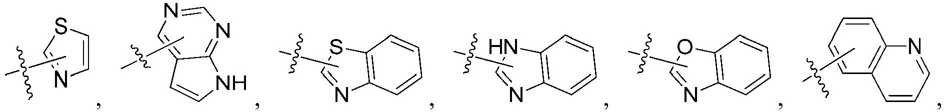 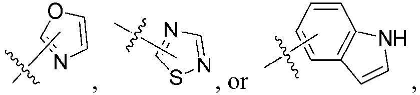
at least one H of R₄ is each independently halogen, -CN, -NO₂, -OH, C1-C6 alkyl (in which at least one H of C1-C6 alkyl may be each independently substituted with -O-(C1-C6 alkyl)), C1-C6 haloalkyl, C2-C6 alkynyl, C3-C6 cycloalkyl, -O-(C1-C6 alkyl), -O-(C1-C6 haloalkyl), -S-(C1-C6 haloalkyl), -NH₂, -N(C1-C6 alkyl)(C1-C6 alkyl), -C(=O)-Rg (Rg is C1-C6 alkyl, -NH₂, -O-(C1-C6 alkyl), -OH, 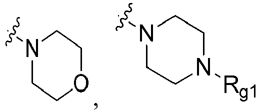 (R_{g1} is H or C1-C6 alkyl), or 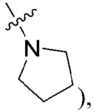 -[(C1-C6 alkylene)]z-NH-S(=O)₂-Rₕ (Rₕ is C1-C6 alkyl, C3-C6 cycloalkyl, - N(C1-C6 alkyl)(C1-C6 alkyl), and z is 0 or 1), -O-S(=O)₂-Rᵢ (Rᵢ is C1-C6 alkyl or C6-C12 aryl, in which at least one H of C6-C12 aryl may be each independently substituted with C1-C6 alkyl), -S(=O)₂-Rⱼ (Rⱼ is C1-C6 alkyl or 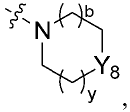 Y₈ is CH₂, O, or NH, b is 0 or 1, and y is 0 or 1, in which at least one H of Rⱼ may be each independently substituted with halogen, C1-C6 alkyl, or C1-C6 haloalkyl), or -S(=O)₂-N(Rₘ)Rₙ (Rm and Rn are each independently H, C1-C6 alkyl, C1-C6 haloalkyl, C3-C12 cycloalkyl, 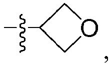 or C6-C12 aryl, and at least one H of each of Rm and Rn may be each independently substituted with OH or -O-(C1-C6 alkyl));
a cyclic group formed by linking R₃ and R₄ to each other is 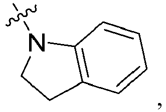 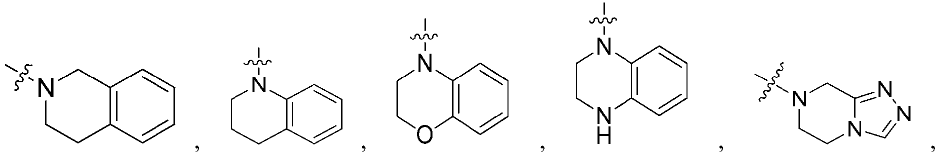 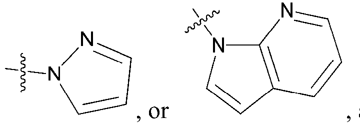 and
at least one H of the cyclic group may be each independently substituted with halogen, CN, OH, NO₂, C1-C6 alkyl, C1-C6 haloalkyl, C3-C12 cycloalkyl, 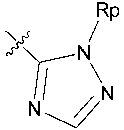 (Rp is H or C1-C6 alkyl), or -S(=O)₂-N(C1-C6 alkyl)(C1-C6 alkyl).

(3) In above (1) or (2), in the compound represented by chemical formula I according to the present invention, it may be provided that a compound comprising a cyclic group formed by R₃ and R₄ is represented by chemical formula III below.

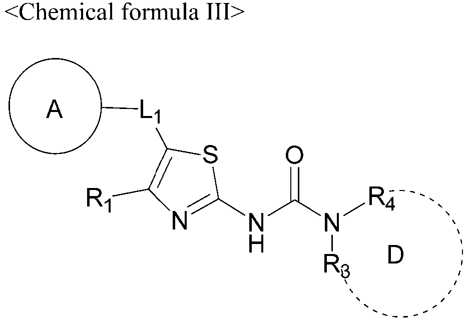

In above chemical formula III, it may be provided that 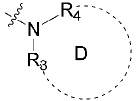 represents a cyclic group formed by linking R₃ and R₄ to each other in chemical formula I, and 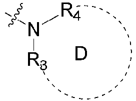 is 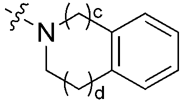 (c and d each independently represent any one integer of 0 to 3), 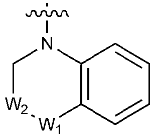 (W₁ and W₂ each independently represent CH₂, NH, or O, and W₁ and W₂ are not simultaneously CH₂), 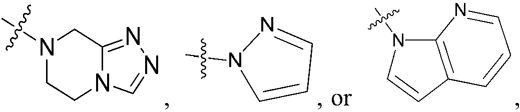 and at least one H of above 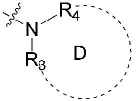 may be each independently substituted with halogen, CN, OH, NO₂, C1-C6 alkyl, C1-C6 haloalkyl, C3-C12 cycloalkyl, 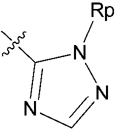 (Rp is H or C1-C6 alkyl), or -S(=O)₂-N(C1-C6 alkyl)(C1-C6 alkyl).

(4) In above (1) or (2), in the compound represented by chemical formula II according to the present invention, it may be provided that a compound comprising a cyclic group formed by R₃ and R₄ is represented by chemical formula IV below.

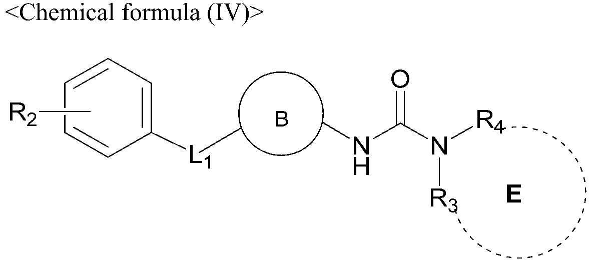

In above chemical formula IV, it may be provided that 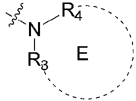 represents a cyclic group formed by linking R₃ and R₄ to each other in chemical formula II, and 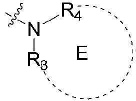 is 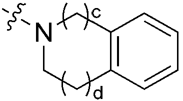 (c and d each independently represent any one integer of 0 to 3), 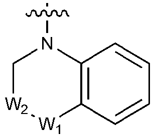 (W₁ and W₂ each independently represent CH₂, NH, or O, and W₁ and W₂ are not simultaneously CH₂), 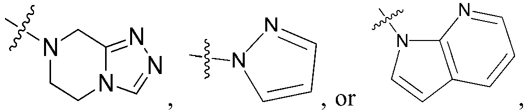 and at least one H of above 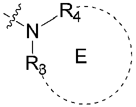 may be each independently substituted with halogen, CN, OH, NO₂, C1-C6 alkyl, C1-C6 haloalkyl, C3-C12 cycloalkyl, 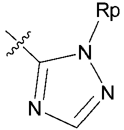 (Rp is H or C1-C6 alkyl), or -S(=O)₂-N(C1-C6 alkyl)(C1-C6 alkyl).

(5) In any one of above (1) to (3), it may be provided that the compound represented by chemical formula I according to the present invention comprises a compound represented by chemical formula Ia below.

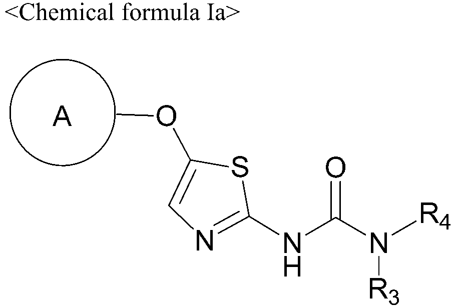

In chemical formula Ia, it may be provided that
A is 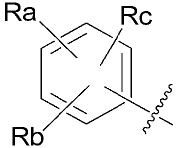 (Ra, Rb, and Rc are each independently H, halogen, CN, C1-C6 alkyl, C1-C6 haloalkyl, -CF₃, -O-(C1-C6 alkyl), -C(=O)-OH, -C(=O)-(C1-C6 alkyl), - C(=O)-O-(C1-C6 alkyl), -N(C1-C6 alkyl)(C1-C6 alkyl), or C6-C12 cycloalkyl), 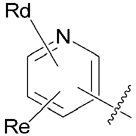 (Rd is H and Re is halogen), 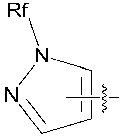 (Rf is C1-C6 alkyl), or 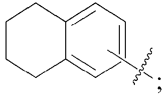
R₃ and R₄ are each a functional group defined below, or are linked to each other to form a cyclic group comprising at least one N,
R₃ is H or C1-C6 alkyl, in which at least one H of C1-C6 alkyl may be each independently substituted with CN,
R₄ is 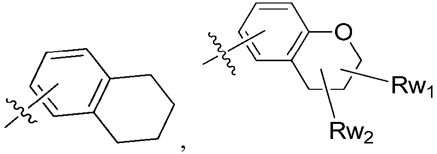 (Rw₁ and Rw₂ each independently represent H, halogen, or OH), 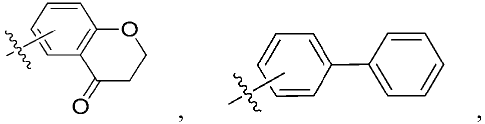 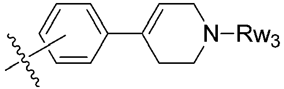 (Rw₃ represents H or C1-C6 alkyl), 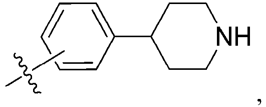 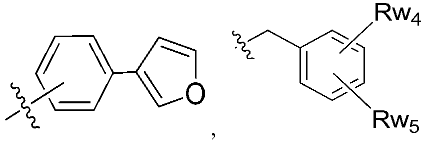 (Rw₄ and Rw₅ each independently represent H, C1-C6 haloalkyl, or -CF₃), 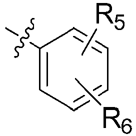 (R₅ and R₆ are each independently H, halogen, -CN, - NO₂, -OH, C1-C6 alkyl (in which at least one H of C1-C6 alkyl may be each independently substituted with -O-(C1-C6 alkyl), -CH₃, -CH(CH₃)(CH₃), -CH(CH₃)-O-CH₃, C1-C6 haloalkyl, -CF₃, -C≡C-CH₃, C3-C6 cycloalkyl, -O-(C1-C6 alkyl), -O-CH₃, -O-(C1-C6 haloalkyl), -O-CF₃, -O-CHF₂, -S-(C1-C6 haloalkyl), -S-CF₃, -NH₂, -N(CH₃)(CH₃), or -C(=O)-Rg (Rg is C1-C6 alkyl, -NH₂, -O-(C1-C6 alkyl), -OH, 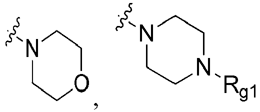 (R_{g1} is H or C1-C6 alkyl), or 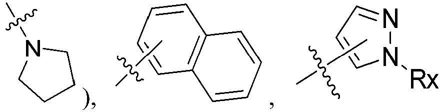 (Rx is H or C1-C6 alkyl), 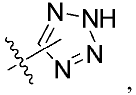 or heteroaryl, which is any one selected from 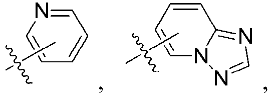 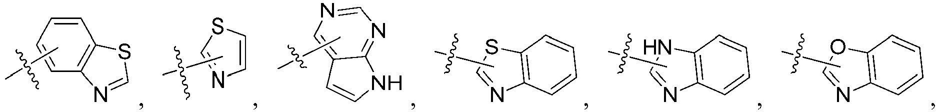 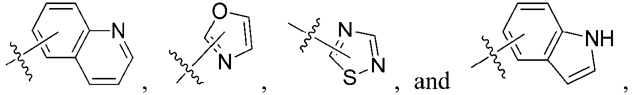 in which at least one H of heteroaryl may be substituted with C1-C6 alkyl, C1-C6 haloalkyl, or -CF₃;
a cyclic group formed by linking R₃ and R₄ to each other is 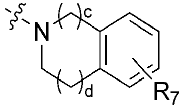 (c and d each independently represent any one integer of 0 to 3, and R₇ is H, halogen, OH, NO₂, C1-C6 alkyl, C1-C6 haloalkyl, -CF₃, or -S(=O)₂-N(C1-C6 alkyl)(C1-C6 alkyl)), 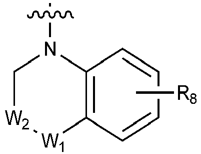 (W₁ and W₂ each independently represent CH₂, NH, N(C1-C6 alkyl), or O, and W₁ and W₂ are not simultaneously CH₂, and R₈ is H, halogen, CN, OH, NO₂, C1-C6 alkyl, C1-C6 haloalkyl, or -CF₃), 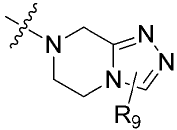 (R₉ is H, C1-C6 alkyl, or C3-C12 cycloalkyl), 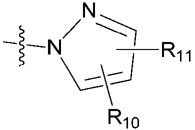 (R₁₀ and R₁₁ are each independently H, C1-C6 alkyl, C1-C6 haloalkyl, -CF₃, or 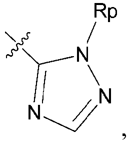 and Rp is H or C1-C6 alkyl), or

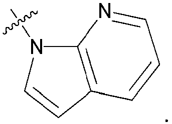

(6) In any one of above (1) to (3), it may be provided that the compound according to the present invention comprises a compound represented by chemical formula Ib, chemical formula Ic, or chemical formula Id below.

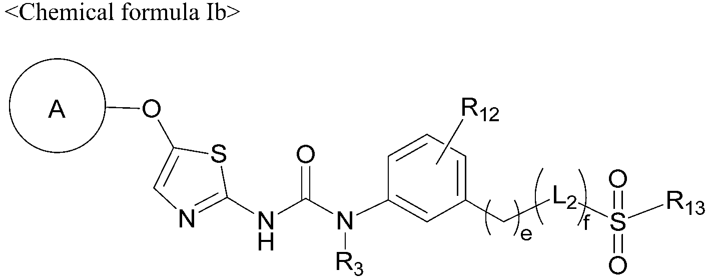

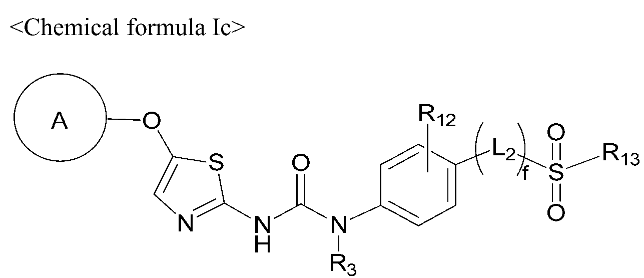

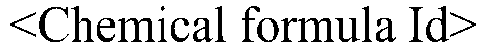

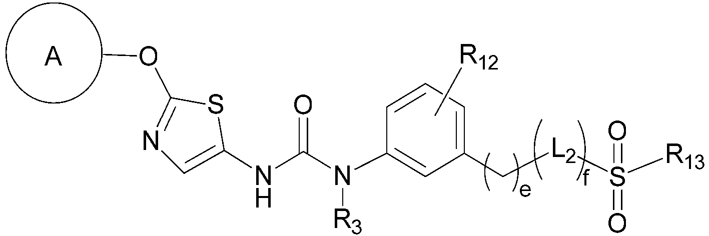

In each of chemical formula Ib, chemical formula Ic and chemical formula Id, it may be provided that
A is 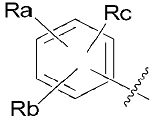 (Ra, Rb, and Rc are each independently H, halogen, CN, NO₂, C1-C6 alkyl, C1-C6 haloalkyl, -CF₃, -O-(C1-C6 alkyl), -C(=O)-(C1-C6 alkyl), or -C(=O)-O-(C1-C6 alkyl), 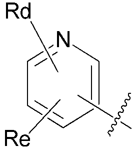 (Rd and Re are each independently H or halogen), or 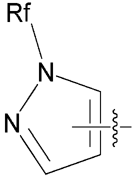 (in which Rf is H or C1-C6 alkyl);
R₃ is H or C1-C6 alkyl;
L₂ is -NH- or -O-, and e and f are each independently 0 or 1;
R₁₂ is H, halogen, C1-C6 alkyl, or -O-(C1-C6 alkyl);
R₁₃ is C1-C6 alkyl, C3-C6 cycloalkyl, C6-C12 aryl (in which at least one H of C6-C12 aryl may be each independently substituted with C1-C6 alkyl), 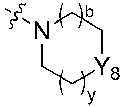 (Y₈ is CH₂ or O, b is 0 or 1, and y is 0 or 1, of which at least one H may be each independently substituted with halogen, C1-C6 alkyl, C1-C6 haloalkyl, or -CH₂F), or -N(Rₘ)Rₙ (in which Rm and Rn are each independently H, C1-C6 alkyl (in which at least one H of C1-C6 alkyl may be each independently substituted with -O-(C1-C6 alkyl) or OH), -CH₃, -CH₂CH₃, -CH(CH₃)(CH₃), - CH₂CH₂-O-CH₃, -CH₂-C(CH₃)₂(OH), C1-C6 haloalkyl, -CH₂CH₂F, -CH₂-CF₂H, C3-C12 cycloalkyl, 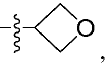 or C6-C12 aryl).

**(7)** The novel compound according to the present invention, the stereoisomer thereof, or the pharmaceutically acceptable salt thereof may be a compound represented in Table 1 below, a stereoisomer thereof, or a pharmaceutically acceptable salt thereof.

| **Compound No.** | **Structure** | **IUPAC compound name (English)** |
|---|---|---|
| **1** | 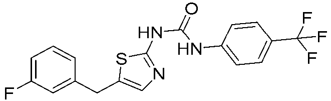 | 1-(5-(3-fluorobenzyl)thiazol-2-yl)-3-(4-(trifluoromethyl)phenyl)urea |
| **2** | 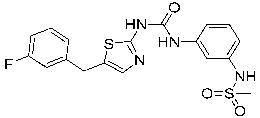 | N-(3-(3-(5-(3-fluorobenzyl)thiazol-2-yl)ureido)phenyl)methanesulfonamide |
| **3** | 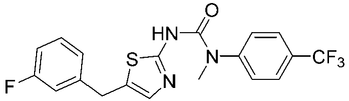 | 3-(5-(3-fluorobenzyl)thiazol-2-yl)-1-methyl-1-(4-(trifluoromethyl)phenyl)urea |
| **4** | 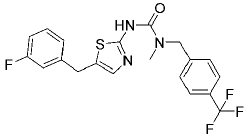 | 3-(5-(3-fluorobenzyl)thiazol-2-yl)-1-methyl-1-(4-(trifluoromethyl)benzyl)urea |
| **5** | 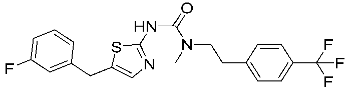 | 3-(5-(3-fluorobenzyl)thiazol-2-yl)-1-methyl-1-(4-(trifluoromethyl)phenethyl)urea |
| **6** | 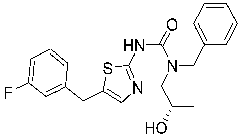 | (S)-1-benzyl-3-(5-(3-fluorobenzyl)thiazol-2-yl)-1-(2-hydroxypropyl)urea |
| **7** | 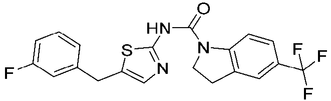 | N-(5-(3-fluorobenzyl)thiazol-2-yl)-5-(trifluoromethyl)indoline-1-carboxamide |
| **8** | 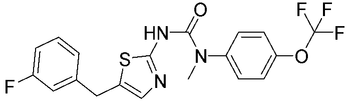 | 3-(5-(3-fluorobenzyl)thiazol-2-yl)-1-methyl-1-(4-(trifluoromethoxy)phenyl)urea |
| **9** | 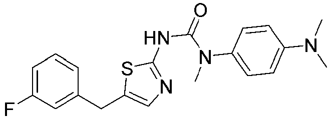 | 1-(4-(dimethylamino)phenyl)-3-(5-(3-fluorobenzyl)thiazol-2-yl)-1-methylurea |
| **10** | 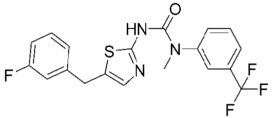 | 3-(5-(3-fluorobenzyl)thiazol-2-yl)-1-methyl-1-(3-(trifluoromethyl)phenyl)urea |
| **11** | 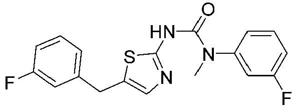 | 3-(5-(3-fluorobenzyl)thiazol-2-yl)-1-(3-fluorophenyl)-1-methylurea |
| **12** | 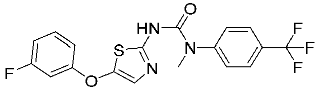 | 3-(5-(3-fluorophenoxy)thiazol-2-yl)-1-methyl-1-(4-(trifluoromethyl)phenyl)urea |
| **13** | 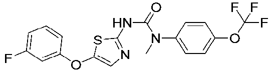 | 3-(5-(3-fluorophenoxy)thiazol-2-yl)-1-methyl-1-(4-(trifluoromethoxy)phenyl)urea |
| **14** | 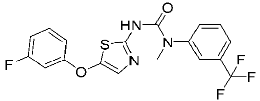 | 3-(5-(3-fluorophenoxy)thiazol-2-yl)-1-methyl-1-(3-(trifluoromethyl)phenyl)urea |
| **15** | 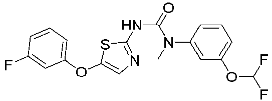 | 1-(3-(difluoromethoxy)phenyl)-3-(5-(3-fluorophenoxy)thiazol-2-yl)-1-methylurea |
| **16** | 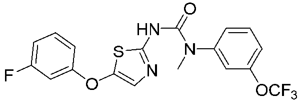 | 3-(5-(3-fluorophenoxy)thiazol-2-yl)-1-methyl-1-(3-(trifluoromethoxy)phenyl)urea |
| **17** | 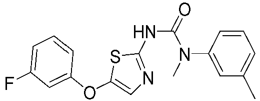 | 3-(5-(3-fluorophenoxy)thiazol-2-yl)-1-methyl-1-(m-tolyl)urea |
| **18** | 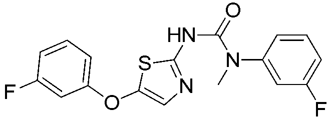 | 3-(5-(3-fluorophenoxy)thiazol-2-yl)-1-(3-fluorophenyl)-1-methylurea |
| **19** | 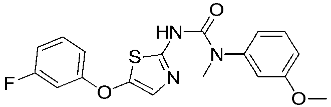 | 3-(5-(3-fluorophenoxy)thiazol-2-yl)-1-(3-methoxyphenyl)-1-methylurea |
| **20** | 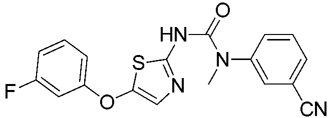 | 1-(3-cyanophenyl)-3-(5-(3-fluorophenoxy)thiazol-2-yl)-1-methylurea |
| **21** | 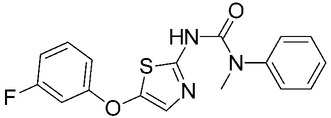 | 3-(5-(3-fluorophenoxy)thiazol-2-yl)-1-methyl-1-phenylurea |
| **22** | 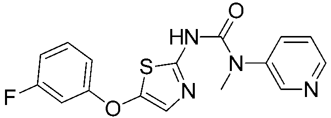 | 3-(5-(3-fluorophenoxy)thiazol-2-yl)-1-methyl-1-(pyridin-3-yl)urea |
| **23** | 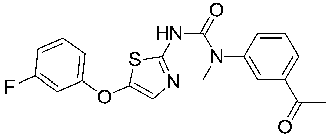 | 1-(3-acetylphenyl)-3-(5-(3-fluorophenoxy)thiazol-2-yl)-1-methylurea |
| **24** | 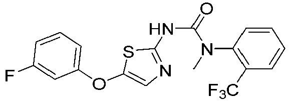 | 3-(5-(3-fluorophenoxy)thiazol-2-yl)-1-methyl-1-(2-(trifluoromethyl)phenyl)urea |
| **25** | 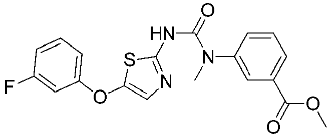 | Methyl 3-(3-(5-(3-fluorophenoxy)thiazol-2-yl)-1-methylureido)benzoate |
| **26** | 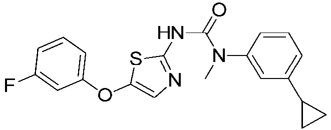 | 1-(3-cyclopropylphenyl)-3-(5-(3-fluorophenoxy)thiazol-2-yl)-1-methylurea |
| **27** | 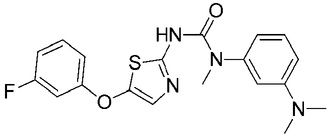 | 1-(3-(dimethylamino)phenyl)-3-(5-(3-fluorophenoxy)thiazol-2-yl)-1-methylurea |
| **28** | 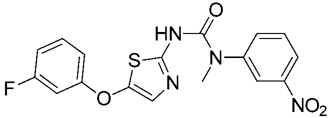 | 3-(5-(3-fluorophenoxy)thiazol-2-yl)-1-methyl-1-(3-nitrophenyl)urea |
| **29** | 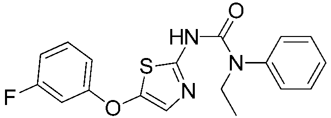 | 1-ethyl-3-(5-(3-fluorophenoxy)thiazol-2-yl)-1-phenylurea |
| **30** | 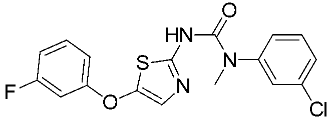 | 1-(3-chlorophenyl)-3-(5-(3-fluorophenoxy)thiazol-2-yl)-1-methylurea |
| **31** | 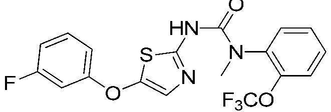 | 3-(5-(3-fluorophenoxy)thiazol-2-yl)-1-methyl-1-(2-(trifluoromethoxy)phenyl)urea |
| **32** | 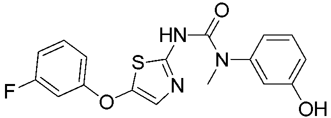 | 3-(5-(3-fluorophenoxy)thiazol-2-yl)-1-(3-hydroxyphenyl)-1-methylurea |
| **33** | 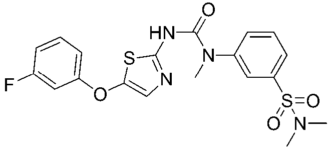 | 3-(3-(5-(3-fluorophenoxy)thiazol-2-yl)-1-methylureido)-N,N-dimethylbenzenesulfonamide |
| **34** | 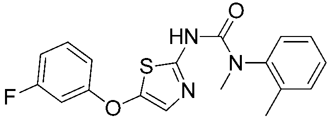 | 3-(5-(3-fluorophenoxy)thiazol-2-yl)-1-methyl-1-(o-tolyl)urea |
| **35** | 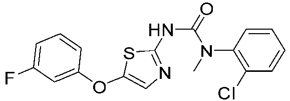 | 1-(2-chlorophenyl)-3-(5-(3-fluorophenoxy)thiazol-2-yl)-1-methylurea |
| **36** | 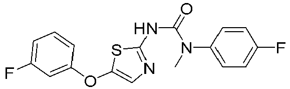 | 3-(5-(3-fluorophenoxy)thiazol-2-yl)-1-(4-fluorophenyl)-1-methylurea |
| **37** | 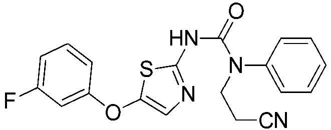 | 1-(2-cyanoethyl)-3-(5-(3-fluorophenoxy)thiazol-2-yl)-1-phenylurea |
| **38** | 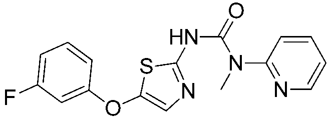 | 3-(5-(3-fluorophenoxy)thiazol-2-yl)-1-methyl-1-(pyridin-2-yl)urea |
| **39** | 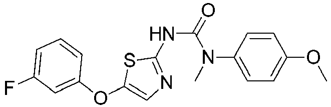 | 3-(5-(3-fluorophenoxy)thiazol-2-yl)-1-(4-methoxyphenyl)-1-methylurea |
| **40** | 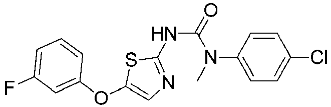 | 1-(4-chlorophenyl)-3-(5-(3-fluorophenoxy)thiazol-2-yl)-1-methylurea |
| **41** | 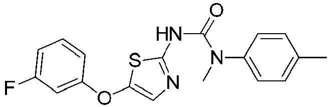 | 3-(5-(3-fluorophenoxy)thiazol-2-yl)-1-methyl-1-(p-tolyl)urea |
| **42** | 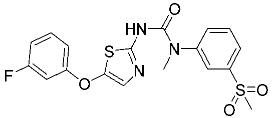 | 3-(5-(3-fluorophenoxy)thiazol-2-yl)-1-methyl-1-(3-(methylsulfonyl)phenyl)urea |
| **43** | 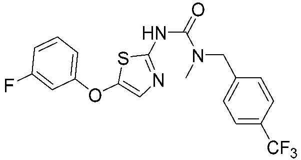 | 3-(5-(3-fluorophenoxy)thiazol-2-yl)-1-methyl-1-(4-(trifluoromethyl)benzyl)urea |
| **44** | 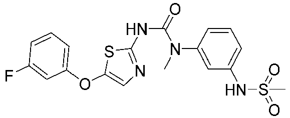 | N-(3-(3-(5-(3-fluorophenoxy)thiazol-2-yl)-1-methylureido)phenyl)methanesulfonamide |
| **45** | 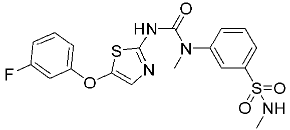 | 3-(3-(5-(3-fluorophenoxy)thiazol-2-yl)-1-methylureido)-N-methylbenzenesulfonamide |
| **46** | 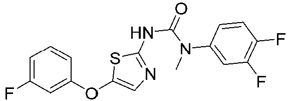 | 1-(3,4-difluorophenyl)-3-(5-(3-fluorophenoxy)thiazol-2-yl)-1-methylurea |
| **47** | 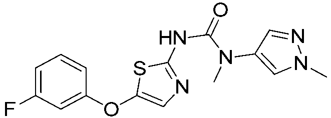 | 3-(5-(3-fluorophenoxy)thiazol-2-yl)-1-methyl-1-(1-methyl-1H-pyrazol-4-yl)urea |
| **48** | 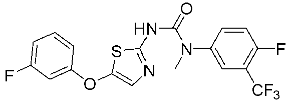 | 1-(4-fluoro-3-(trifluoromethyl)phenyl)-3-(5-(3-fluorophenoxy)thiazol-2-yl)-1-methylurea |
| **49** | 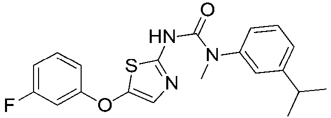 | 3-(5-(3-fluorophenoxy)thiazol-2-yl)-1-(3-isopropylphenyl)-1-methylurea |
| **50** | 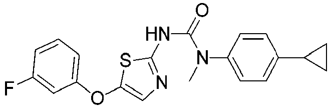 | 1-(4-cyclopropylphenyl)-3-(5-(3-fluorophenoxy)thiazol-2-yl)-1-methylurea |
| **51** | 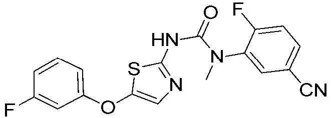 | 1-(5-cyano-2-fluorophenyl)-3-(5-(3-fluorophenoxy)thiazol-2-yl)-1-methylurea |
| **52** | 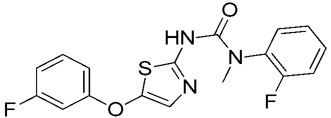 | 3-(5-(3-fluorophenoxy)thiazol-2-yl)-1-(2-fluorophenyl)-1-methylurea |
| **53** | 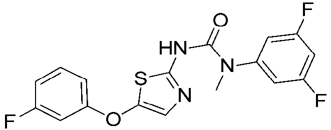 | 1-(3,5-difluorophenyl)-3-(5-(3-fluorophenoxy)thiazol-2-yl)-1-methylurea |
| **54** | 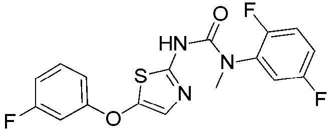 | 1-(2,5-difluorophenyl)-3-(5-(3-fluorophenoxy)thiazol-2-yl)-1-methylurea |
| **55** | 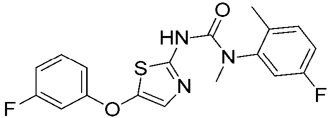 | 1-(5-fluoro-2-methylphenyl)-3-(5-(3-fluorophenoxy)thiazol-2-yl)-1-methylurea |
| **56** | 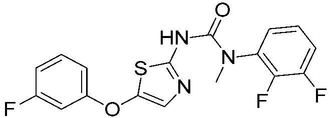 | 1-(2,3-difluorophenyl)-3-(5-(3-fluorophenoxy)thiazol-2-yl)-1-methylurea |
| **57** | 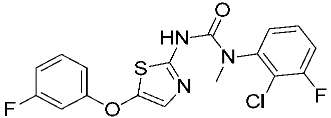 | 1-(2-chloro-3-fluorophenyl)-3-(5-(3-fluorophenoxy)thiazol-2-yl)-1-methylurea |
| **58** | 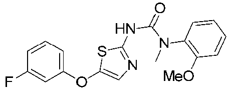 | 3-(5-(3-fluorophenoxy)thiazol-2-yl)-1-(2-methoxyphenyl)-1-methylurea |
| **59** | 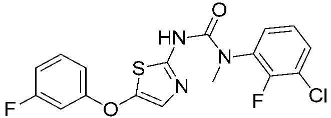 | 1-(3-chloro-2-fluorophenyl)-3-(5-(3-fluorophenoxy)thiazol-2-yl)-1-methylurea |
| **60** | 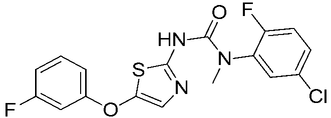 | 1-(5-chloro-2-fluorophenyl)-3-(5-(3-fluorophenoxy)thiazol-2-yl)-1-methylurea |
| **61** | 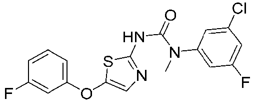 | 1-(3-chloro-5-fluorophenyl)-3-(5-(3-fluorophenoxy)thiazol-2-yl)-1-methylurea |
| **62** | 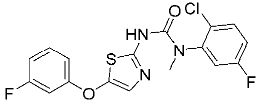 | 1-(2-chloro-5-fluorophenyl)-3-(5-(3-fluorophenoxy)thiazol-2-yl)-1-methylurea |
| **63** | 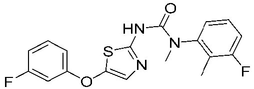 | 1-(3-fluoro-2-methylphenyl)-3-(5-(3-fluorophenoxy)thiazol-2-yl)-1-methylurea |
| **64** | 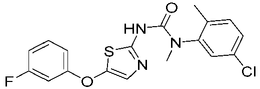 | 1-(5-chloro-2-methylphenyl)-3-(5-(3-fluorophenoxy)thiazol-2-yl)-1-methylurea |
| **65** | 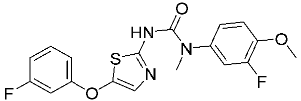 | 1-(3-fluoro-4-methoxyphenyl)-3-(5-(3-fluorophenoxy)thiazol-2-yl)-1-methylurea |
| **66** | 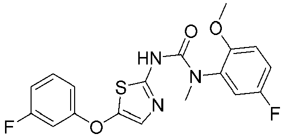 | 1-(5-fluoro-2-methoxyphenyl)-3-(5-(3-fluorophenoxy)thiazol-2-yl)-1-methylurea |
| **67** | 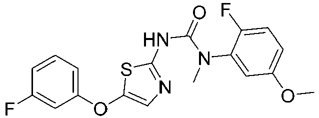 | 1-(2-fluoro-5-methoxyphenyl)-3-(5-(3-fluorophenoxy)thiazol-2-yl)-1-methylurea |
| **68** | 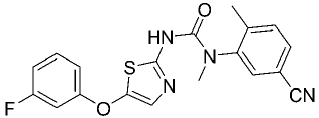 | 1-(5-cyano-2-methylphenyl)-3-(5-(3-fluorophenoxy)thiazol-2-yl)-1-methylurea |
| **69** | 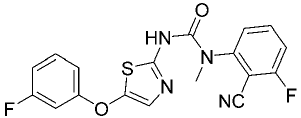 | 1-(2-cyano-3-fluorophenyl)-3-(5-(3-fluorophenoxy)thiazol-2-yl)-1-methylurea |
| **70** | 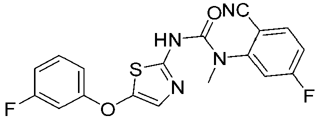 | 1-(2-cyano-5-fluorophenyl)-3-(5-(3-fluorophenoxy)thiazol-2-yl)-1-methylurea |
| **71** | 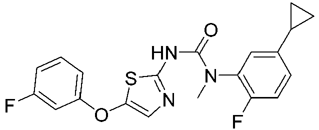 | 1-(5-cyclopropyl-2-fluorophenyl)-3-(5-(3-fluorophenoxy)thiazol-2-yl)-1-methylurea |
| **72** | 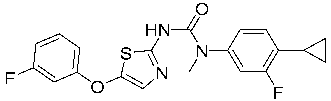 | 1-(4-cyclopropyl-3-fluorophenyl)-3-(5-(3-fluorophenoxy)thiazol-2-yl)-1-methylurea |
| **73** | 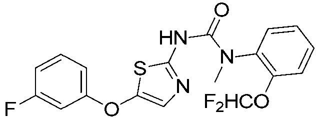 | 1-(2-(difluoromethoxy)phenyl)-3-(5-(3-fluorophenoxy)thiazol-2-yl)-1-methylurea |
| **74** | 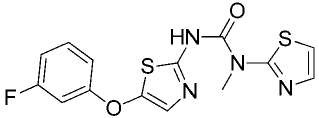 | 3-(5-(3-fluorophenoxy)thiazol-2-yl)-1-methyl-1-(thiazol-2-yl)urea |
| **75** | 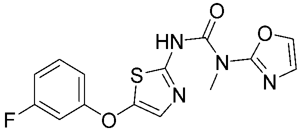 | 3-(5-(3-fluorophenoxy)thiazol-2-yl)-1-methyl-1-(oxazol-2-yl)urea |
| **76** | 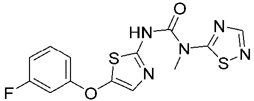 | 3-(5-(3-fluorophenoxy)thiazol-2-yl)-1-methyl-1-(1,2,4-thiadiazol-5-yl)urea |
| **77** | 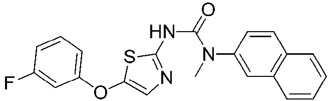 | 3-(5-(3-fluorophenoxy)thiazol-2-yl)-1-methyl-1-(naphthalen-2-yl)urea |
| **78** | 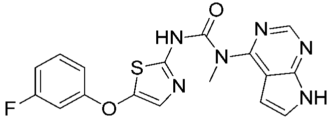 | 3-(5-(3-fluorophenoxy)thiazol-2-yl)-1-methyl-1-(7H-pyrrolo[2,3-d]pyrimidin-4-yl)urea |
| **79** | 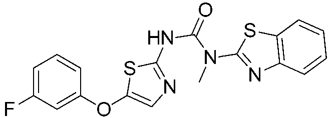 | 1-(benzo[d]thiazol-2-yl)-3-(5-(3-fluorophenoxy)thiazol-2-yl)-1-methylurea |
| **80** | 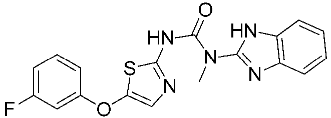 | 1-(1H-benzo[d]imidazol-2-yl)-3-(5-(3-fluorophenoxy)thiazol-2-yl)-1-methylurea |
| **81** | 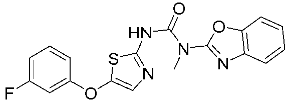 | 1-(benzo[d]oxazol-2-yl)-3-(5-(3-fluorophenoxy)thiazol-2-yl)-1-methylurea |
| **82** | 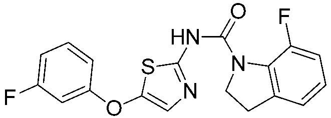 | 7-fluoro-N-(5-(3-fluorophenoxy)thiazol-2-yl)indoline-1-carboxamide |
| **83** | 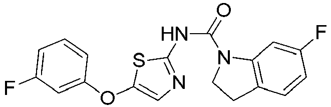 | 6-fluoro-N-(5-(3-fluorophenoxy)thiazol-2-yl)indoline-1-carboxamide |
| **84** | 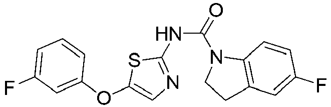 | 5-fluoro-N-(5-(3-fluorophenoxy)thiazol-2-yl)indoline-1-carboxamide |
| **85** | 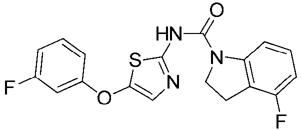 | 4-fluoro-N-(5-(3-fluorophenoxy)thiazol-2-yl)indoline-1-carboxamide |
| **86** | 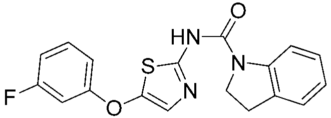 | N-(5-(3-fluorophenoxy)thiazol-2-yl)indoline-1-carboxamide |
| **87** | 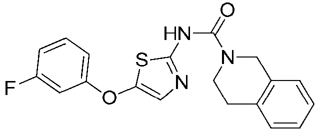 | N-(5-(3-fluorophenoxy)thiazol-2-yl)-3,4-dihydroisoquinoline-2(1H)-carboxamide |
| **88** | 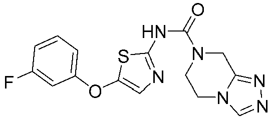 | N-(5-(3-fluorophenoxy)thiazol-2-yl)-5,6-dihydro-[1,2,4]triazolo[4,3-a]pyrazine-7(8H)-carboxamide |
| **89** | 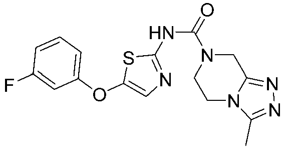 | N-(5-(3-fluorophenoxy)thiazol-2-yl)-3-methyl-5,6-dihydro-[1,2,4]triazolo[4,3-a]pyrazine-7(8H)-carboxamide |
| **90** | 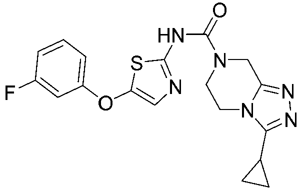 | 3-cyclopropyl-N-(5-(3-fluorophenoxy)thiazol-2-yl)-5,6-dihydro-[1,2,4]triazolo[4,3-a]pyrazine-7(8H)-carboxamide |
| **91** | 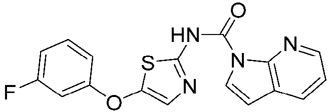 | N-(5-(3-fluorophenoxy)thiazol-2-yl)- 1H-pyrrolo[2,3-b]pyridine-1-carboxamide |
| **92** | 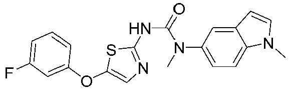 | 3-(5-(3-fluorophenoxy)thiazol-2-yl)-1-methyl-1-(1-methyl-1H-indol-5-yl)urea |
| **93** | 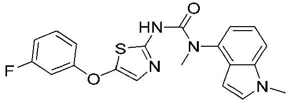 | 3-(5-(3-fluorophenoxy)thiazol-2-yl)-1-methyl-1-(1-methyl-1H-indol-4-yl)urea |
| **94** | 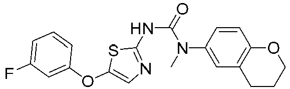 | 1-(chroman-6-yl)-3-(5-(3-fluorophenoxy)thiazol-2-yl)-1-methylurea |
| **95** | 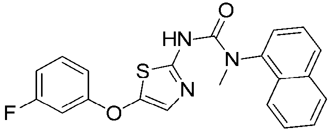 | 3-(5-(3-fluorophenoxy)thiazol-2-yl)-1-methyl-1-(naphthalen-1-yl)urea |
| **96** | 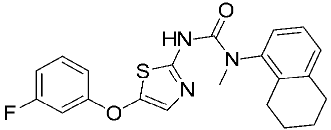 | 3-(5-(3-fluorophenoxy)thiazol-2-yl)-1-methyl-1-(5,6,7,8-tetrahydronaphthalen-1-yl)urea |
| **97** | 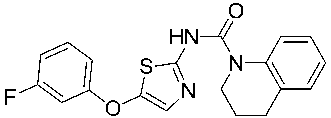 | N-(5-(3-fluorophenoxy)thiazol-2-yl)-3,4-dihydroquinoline-1 (2H)-carboxamide |
| **98** | 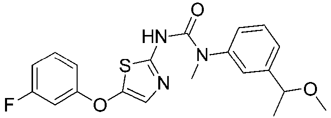 | 3-(5-(3-fluorophenoxy)thiazol-2-yl)-1-(3-(1-methoxyethyl)phenyl)-1-methylurea |
| **99** | 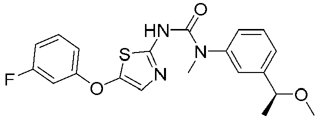 | (S)-3-(5-(3-fluorophenoxy)thiazol-2-yl)-1-(3-(1-methoxyethyl)phenyl)-1-methylurea |
| **100** | 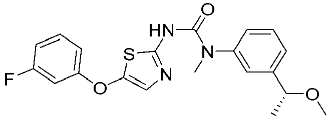 | (R)-3-(5-(3-fluorophenoxy)thiazol-2-yl)-1-(3-(1-methoxyethyl)phenyl)-1-methylurea |
| **101** | 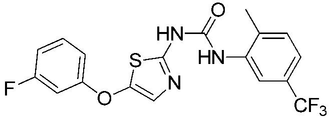 | 1-(5-(3-fluorophenoxy)thiazol-2-yl)-3-(2-methyl-5-(trifluoromethyl)phenyl)urea |
| **102** | 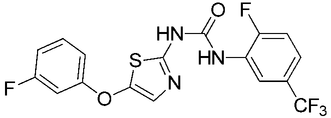 | 1-(2-fluoro-5-(trifluoromethyl)phenyl)-3-(5-(3-fluorophenoxy)thiazol-2-yl)urea |
| **103** | 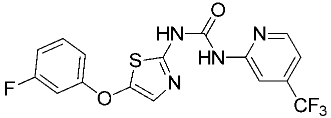 | 1-(5-(3-fluorophenoxy)thiazol-2-yl)-3-(4-(trifluoromethyl)pyridin-2-yl)urea |
| **104** | 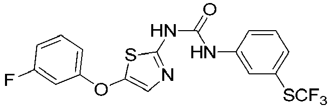 | 1-(5-(3-fluorophenoxy)thiazol-2-yl)-3-(3-((trifluoromethyl)thio)phenyl)urea |
| **105** | 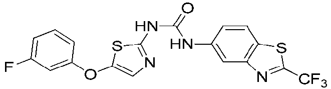 | 1-(5-(3-fluorophenoxy)thiazol-2-yl)-3-(2-(trifluoromethyl)benzo[d]thiazol-5-yl)urea |
| **106** | 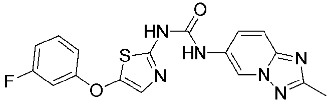 | 1-(5-(3-fluorophenoxy)thiazol-2-yl)-3-(2-methyl-[1,2,4]triazolo[1,5-a]pyridin-6-yl)urea |
| **107** | 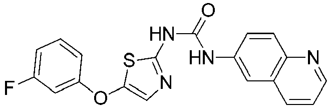 | 1-(5-(3-fluorophenoxy)thiazol-2-yl)-3-(quinolin-6-yl)urea |
| **108** | 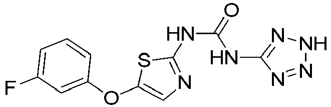 | 1-(5-(3-fluorophenoxy)thiazol-2-yl)-3-(2H-tetrazol-5-yl)urea |
| **109** | 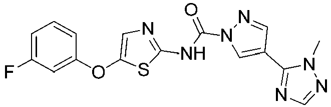 | N-(5-(3-fluorophenoxy)thiazol-2-yl)-4-(1-methyl-1H-1,2,4-triazol-5-yl)-1H-pyrazole-1-carboxamide |
| **110** | 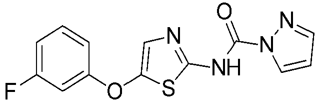 | N-(5-(3-fluorophenoxy)thiazol-2-yl)- 1H-pyrazole-1-carboxamide |
| **111** | 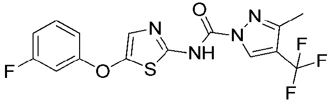 | N-(5-(3-fluorophenoxy)thiazol-2-yl)-3-methyl-4-(trifluoromethyl)-1H-pyrazole-1-carboxamide |
| **112** | 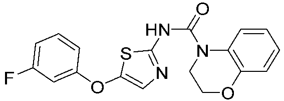 | N-(5-(3-fluorophenoxy)thiazol-2-yl)-2,3-dihydro-4H-benzo[b][1,4]oxazine-4-carboxamide |
| **113** | 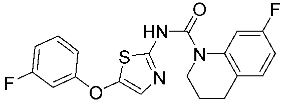 | 7-fluoro-N-(5-(3-fluorophenoxy)thiazol-2-yl)-3,4-dihydroquinoline-1(2H)-carboxamide |
| **114** | 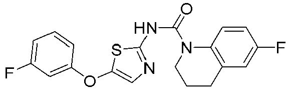 | 6-fluoro-N-(5-(3-fluorophenoxy)thiazol-2-yl)-3,4-dihydroquinoline-1(2H)-carboxamide |
| **115** | 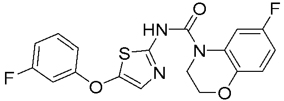 | 6-fluoro-N-(5-(3-fluorophenoxy)thiazol-2-yl)-2,3-dihydro-4H-benzo[b][1,4]oxazine-4-carboxamide |
| **116** | 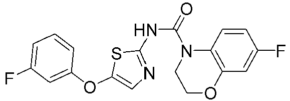 | 7-fluoro-N-(5-(3-fluorophenoxy)thiazol-2-yl)-2,3-dihydro-4H-benzo[b][1,4]oxazine-4-carboxamide |
| **117** | 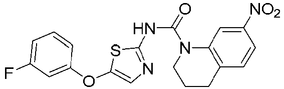 | N-(5-(3-fluorophenoxy)thiazol-2-yl)-7-nitro-3,4-dihydroquinoline-1(2H)-carboxamide |
| **118** | 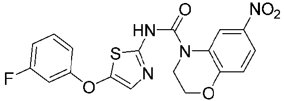 | N-(5-(3-fluorophenoxy)thiazol-2-yl)-6-nitro-2,3-dihydro-4H-benzo[b][1,4]oxazine-4-carboxamide |
| **119** | 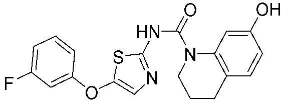 | N-(5-(3-fluorophenoxy)thiazol-2-yl)-7-hydroxy-3,4-dihydroquinoline-1(2H)-carboxamide |
| **120** | 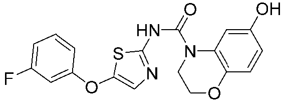 | N-(5-(3-fluorophenoxy)thiazol-2-yl)-6-hydroxy-2,3-dihydro-4H-benzo[b][1,4]oxazine-4-carboxamide |
| **121** | 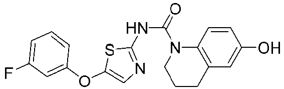 | N-(5-(3-fluorophenoxy)thiazol-2-yl)-6-hydroxy-3,4-dihydroquinoline-1(2H)-carboxamide |
| **122** | 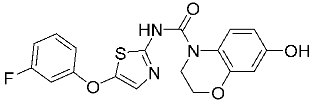 | N-(5-(3-fluorophenoxy)thiazol-2-yl)-7-hydroxy-2,3-dihydro-4H-benzo[b][1,4]oxazine-4-carboxamide |
| **123** | 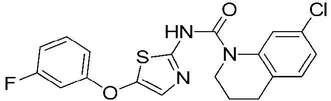 | 7-chloro-N-(5-(3-fluorophenoxy)thiazol-2-yl)-3,4-dihydroquinoline-1(2H)-carboxamide |
| **124** | 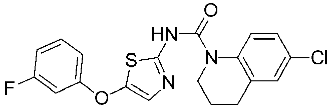 | 6-chloro-N-(5-(3-fluorophenoxy)thiazol-2-yl)-3,4-dihydroquinoline-1(2H)-carboxamide |
| **125** | 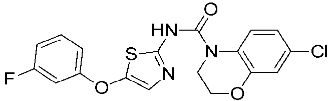 | 7-chloro-N-(5-(3-fluorophenoxy)thiazol-2-yl)-2,3-dihydro-4H-benzo[b][1,4]oxazine-4-carboxamide |
| **126** | 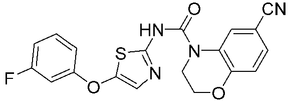 | 6-cyano-N-(5-(3-fluorophenoxy)thiazol-2-yl)-2,3-dihydro-4H-benzo[b][1,4]oxazine-4-carboxamide |
| **127** | 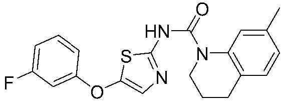 | N-(5-(3-fluorophenoxy)thiazol-2-yl)-7-methyl-3,4-dihydroquinoline-1(2H)-carboxamide |
| **128** | 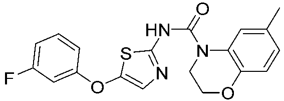 | N-(5-(3-fluorophenoxy)thiazol-2-yl)-6-methyl-2,3-dihydro-4H-benzo[b][1,4]oxazine-4-carboxamide |
| **129** | 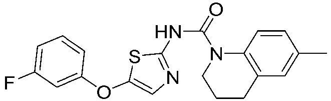 | N-(5-(3-fluorophenoxy)thiazol-2-yl)-6-methyl-3,4-dihydroquinoline-1(2H)-carboxamide |
| **130** | 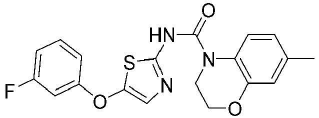 | N-(5-(3-fluorophenoxy)thiazol-2-yl)-7-methyl-2,3-dihydro-4H-benzo[b][1,4]oxazine-4-carboxamide |
| **131** | 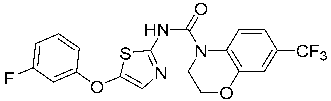 | N-(5-(3-fluorophenoxy)thiazol-2-yl)-7-(trifluoromethyl)-2,3-dihydro-4H-benzo[b][1,4]oxazine-4-carboxamide |
| **132** | 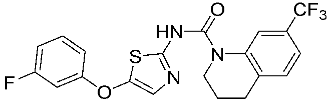 | N-(5-(3-fluorophenoxy)thiazol-2-yl)-7-(trifluoromethyl)-3,4-dihydroquinoline-1(2H)-carboxamide |
| **133** | 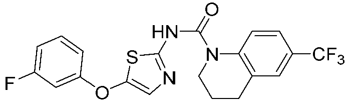 | N-(5-(3-fluorophenoxy)thiazol-2-yl)-6-(trifluoromethyl)-3,4-dihydroquinoline-1(2H)-carboxamide |
| **134** | 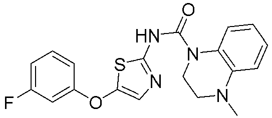 | N-(5-(3-fluorophenoxy)thiazol-2-yl)-4-methyl-3,4-dihydroquinoxaline-1(2H)-carboxamide |
| **135** | 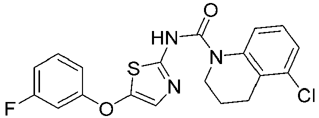 | 5-chloro-N-(5-(3-fluorophenoxy)thiazol-2-yl)-3,4-dihydroquinoline-1(2H)-carboxamide |
| **136** | 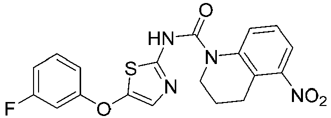 | N-(5-(3-fluorophenoxy)thiazol-2-yl)-5-nitro-3,4-dihydroquinoline-1(2H)-carboxamide |
| **137** | 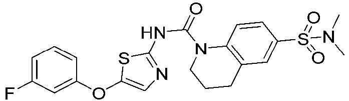 | 6-(N,N-dimethylsulfamoyl)-N-(5-(3-fluorophenoxy)thiazol-2-yl)-3,4-dihydroquinoline-1(2H)-carboxamide |
| **138** | 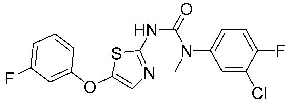 | 1-(3-chloro-4-fluorophenyl)-3-(5-(3-fluorophenoxy)thiazol-2-yl)-1-methylurea |
| **139** | 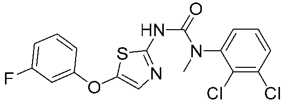 | 1-(2,3-dichlorophenyl)-3-(5-(3-fluorophenoxy)thiazol-2-yl)-1-methylurea |
| **140** | 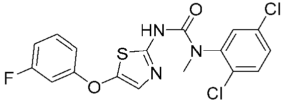 | 1-(2,5-dichlorophenyl)-3-(5-(3-fluorophenoxy)thiazol-2-yl)-1-methylurea |
| **141** | 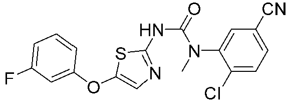 | 1-(2-chloro-5-cyanophenyl)-3-(5-(3-fluorophenoxy)thiazol-2-yl)-1-methylurea |
| **142** | 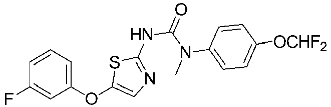 | 1-(4-(difluoromethoxy)phenyl)-3-(5-(3-fluorophenoxy)thiazol-2-yl)-1-methylurea |
| **143** | 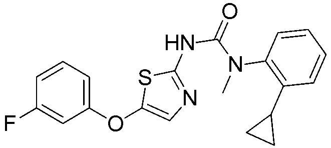 | 1-(2-cyclopropylphenyl)-3-(5-(3-fluorophenoxy)thiazol-2-yl)-1-methylurea |
| **144** | 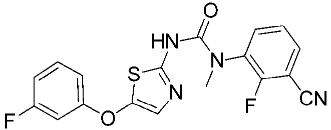 | 1-(3-cyano-2-fluorophenyl)-3-(5-(3-fluorophenoxy)thiazol-2-yl)-1-methylurea |
| **145** | 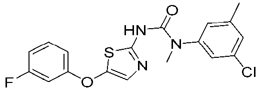 | 1-(3-chloro-5-methylphenyl)-3-(5-(3-fluorophenoxy)thiazol-2-yl)-1-methylurea |
| **146** | 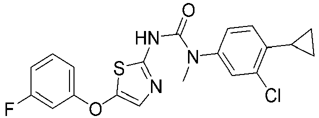 | 1-(3-chloro-4-cyclopropylphenyl)-3-(5-(3-fluorophenoxy)thiazol-2-yl)-1-methylurea |
| **147** | 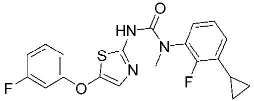 | 1-(3-cyclopropyl-2-fluorophenyl)-3-(5-(3-fluorophenoxy)thiazol-2-yl)-1-methylurea |
| **148** | 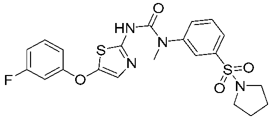 | 3-(5-(3-fluorophenoxy)thiazol-2-yl)-1-methyl-1-(3-(pyrrolidin-1-ylsulfonyl)phenyl)urea |
| **149** | 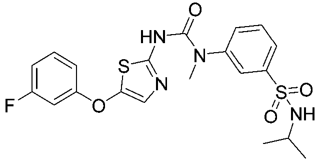 | 3-(3-(5-(3-fluorophenoxy)thiazol-2-yl)-1-methylureido)-N-isopropylbenzenesulfonamide |
| **150** | 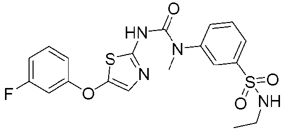 | N-ethyl-3-(3-(5-(3-fluorophenoxy)thiazol-2-yl)-1-methylureido)benzenesulfonamide |
| **151** | 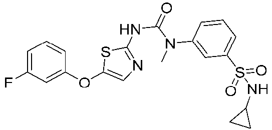 | N-cyclopropyl-3-(3-(5-(3-fluorophenoxy)thiazol-2-yl)-1-methylureido)benzenesulfonamide |
| **152** | 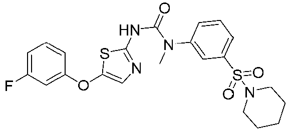 | 3-(5-(3-fluorophenoxy)thiazol-2-yl)-1-methyl-1-(3-(piperidin-1-ylsulfonyl)phenyl)urea |
| **153** | 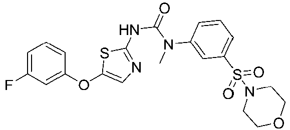 | 3-(5-(3-fluorophenoxy)thiazol-2-yl)-1-methyl-1-(3-(morpholinosulfonyl)phenyl)urea |
| **154** | 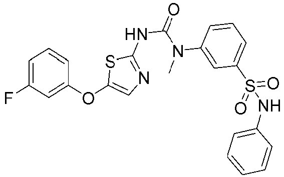 | 3-(3-(5-(3-fluorophenoxy)thiazol-2-yl)-1-methylureido)-N-phenylbenzenesulfonamide |
| **155** | 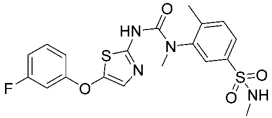 | 3-(3-(5-(3-fluorophenoxy)thiazol-2-yl)-1-methylureido)-N,4-dimethylbenzenesulfonamide |
| **156** | 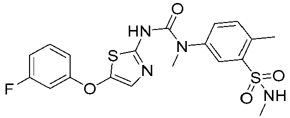 | 5-(3-(5-(3-fluorophenoxy)thiazol-2-yl)-1-methylureido)-N,2-dimethylbenzenesulfonamide |
| **157** | 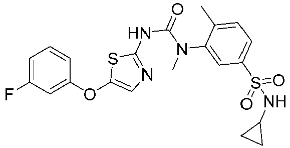 | N-cyclopropyl-3-(3-(5-(3-fluorophenoxy)thiazol-2-yl)-1-methylureido)-4-methylbenzenesulfonamide |
| **158** | 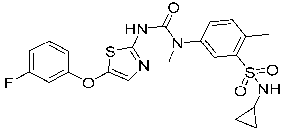 | N-cyclopropyl-5-(3-(5-(3-fluorophenoxy)thiazol-2-yl)-1-methylureido)-2-methylbenzenesulfonamide |
| **159** | 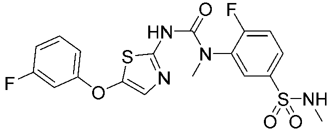 | 4-fluoro-3-(3-(5-(3-fluorophenoxy)thiazol-2-yl)-1-methylureido)-N-methylbenzenesulfonamide |
| **160** | 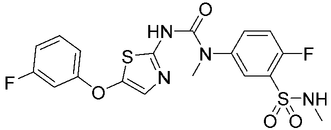 | 2-fluoro-5-(3-(5-(3-fluorophenoxy)thiazol-2-yl)-1-methylureido)-N-methylbenzenesulfonamide |
| **161** | 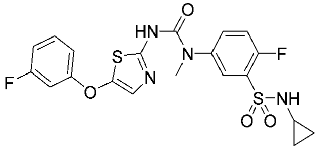 | N-cyclopropyl-2-fluoro-5-(3-(5-(3-fluorophenoxy)thiazol-2-yl)-1-methylureido)benzenesulfonamide |
| **162** | 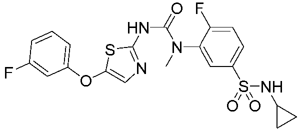 | N-cyclopropyl-4-fluoro-3-(3-(5-(3-fluorophenoxy)thiazol-2-yl)-1-methylureido)benzenesulfonamide |
| **163** | 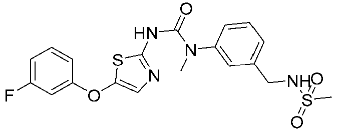 | N-(3-(3-(5-(3-fluorophenoxy)thiazol-2-yl)-1-methylureido)benzyl)methanesulfonamide |
| **164** | 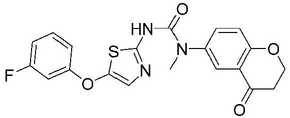 | 3-(5-(3-fluorophenoxy)thiazol-2-yl)-1-methyl-1-(4-oxochroman-6-yl)urea |
| **165** | 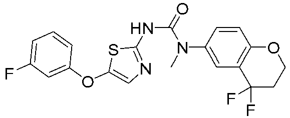 | 1-(4,4-difluorochroman-6-yl)-3-(5-(3-fluorophenoxy)thiazol-2-yl)-1-methylurea |
| **166** | 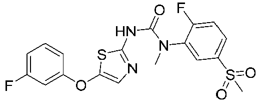 | 1-(2-fluoro-5-(methylsulfonyl)phenyl)-3-(5-(3-fluorophenoxy)thiazol-2-yl)-1-methylurea |
| **167** | 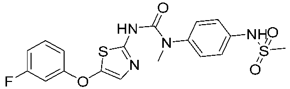 | N-(4-(3-(5-(3-fluorophenoxy)thiazol-2-yl)-1-methylureido)phenyl)methanesulfonamide |
| **168** | 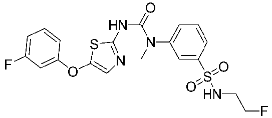 | N-(2-fluoroethyl)-3-(3-(5-(3-fluorophenoxy)thiazol-2-yl)-1-methylureido)benzenesulfonamide |
| **169** | 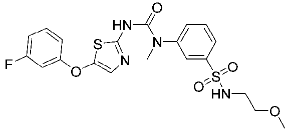 | 3-(3-(5-(3-fluorophenoxy)thiazol-2-yl)-1-methylureido)-N-(2-methoxyethyl)benzenesulfonamide |
| **170** | 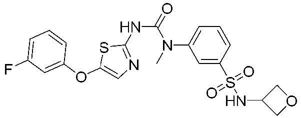 | 3-(3-(5-(3-fluorophenoxy)thiazol-2-yl)-1-methylureido)-N-(oxetan-3-yl)benzenesulfonamide |
| **171** | 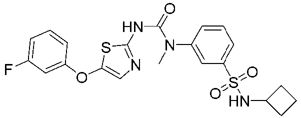 | N-cyclobutyl-3-(3-(5-(3-fluorophenoxy)thiazol-2-yl)-1-methylureido)benzenesulfonamide |
| **172** | 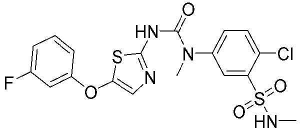 | 2-chloro-5-(3-(5-(3-fluorophenoxy)thiazol-2-yl)-1-methylureido)-N-methylbenzenesulfonamide |
| **173** | 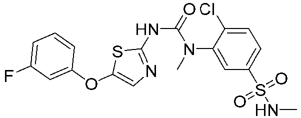 | 4-chloro-3-(3-(5-(3-fluorophenoxy)thiazol-2-yl)-1-methylureido)-N-methylbenzenesulfonamide |
| **174** | 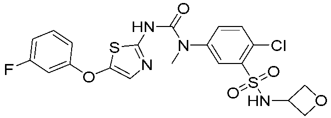 | 2-chloro-5-(3-(5-(3-fluorophenoxy)thiazol-2-yl)-1-methylureido)-N-(oxetan-3-yl)benzenesulfonamide |
| **175** | 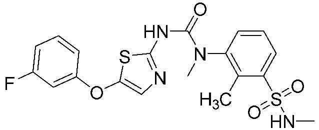 | 3-(3-(5-(3-fluorophenoxy)thiazol-2-yl)-1-methylureido)-N,2-dimethylbenzenesulfonamide |
| **176** | 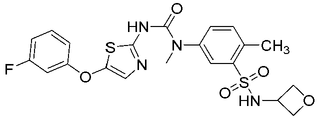 | 5-(3-(5-(3-fluorophenoxy)thiazol-2-yl)-1-methylureido)-2-methyl-N-(oxetan-3-yl)benzenesulfonamide |
| **177** | 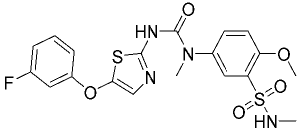 | 5-(3-(5-(3-fluorophenoxy)thiazol-2-yl)-1-methylureido)-2-methoxy-N-methylbenzenesulfonamide |
| **178** | 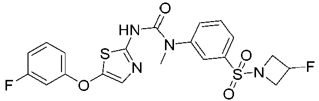 | 1-(3-((3-fluoroazetidin-1-yl)sulfonyl)phenyl)-3-(5-(3-fluorophenoxy)thiazol-2-yl)-1-methylurea |
| **179** | 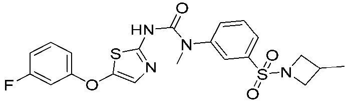 | 3-(5-(3-fluorophenoxy)thiazol-2-yl)-1-methyl-1-(3-((3-methylazetidin-1-yl)sulfonyl)phenyl)urea |
| **180** | 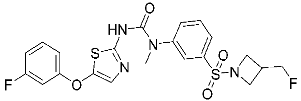 | 1-(3-((3-(fluoromethyl)azetidin-1-yl)sulfonyl)phenyl)-3-(5-(3-fluorophenoxy)thiazol-2-yl)-1-methylurea |
| **181** | 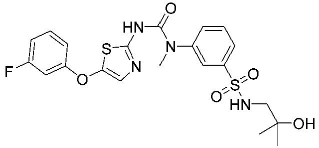 | 3-(3-(5-(3-fluorophenoxy)thiazol-2-yl)-1-methylureido)-N-(2-hydroxy-2-methylpropyl)benzenesulfonamide |
| **182** | 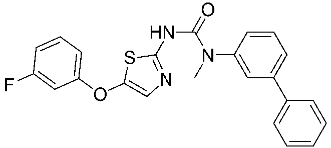 | 1-([1,1'-biphenyl]-3-yl)-3-(5-(3-fluorophenoxy)thiazol-2-yl)-1-methylurea |
| **183** | 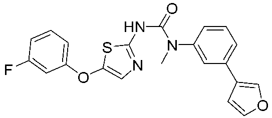 | 3-(5-(3-fluorophenoxy)thiazol-2-yl)-1-(3-(furan-3-yl)phenyl)-1-methylurea |
| **184** | 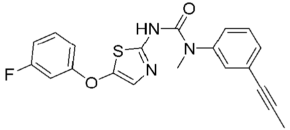 | 3-(5-(3-fluorophenoxy)thiazol-2-yl)-1-methyl-1-(3-(prop-1-yn-1-yl)phenyl)urea |
| **185** | 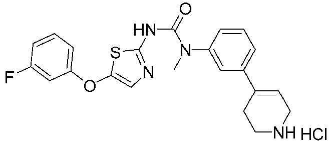 | 3-(5-(3-fluorophenoxy)thiazol-2-yl)-1-methyl-1-(3-(1,2,3,6-tetrahydropyridin-4-yl)phenyl)urea hydrochloride |
| **186** | 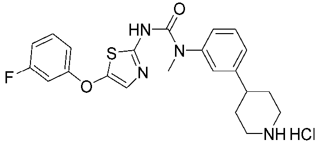 | 3-(5-(3-fluorophenoxy)thiazol-2-yl)-1-methyl-1-(3-(piperidin-4-yl)phenyl)urea hydrochloride |
| **187** | 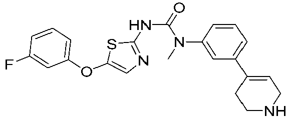 | 3-(5-(3-fluorophenoxy)thiazol-2-yl)-1-methyl-1-(3-(1,2,3,6-tetrahydropyridin-4-yl)phenyl)urea |
| **188** | 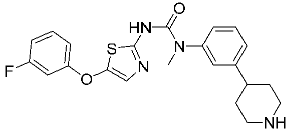 | 3-(5-(3-fluorophenoxy)thiazol-2-yl)-1-methyl-1-(3-(piperidin-4-yl)phenyl)urea |
| **189** | 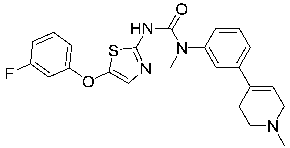 | 3-(5-(3-fluorophenoxy)thiazol-2-yl)-1-methyl-1-(3-(1-methyl-1,2,3,6-tetrahydropyridin-4-yl)phenyl)urea |
| **190** | 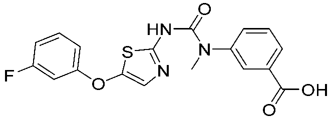 | 3-(3-(5-(3-fluorophenoxy)thiazol-2-yl)-1-methylureido)benzoic acid |
| **191** | 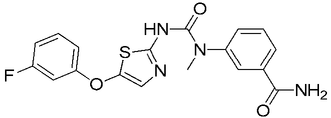 | 3-(3-(5-(3-fluorophenoxy)thiazol-2-yl)-1-methylureido)benzamide |
| **192** | 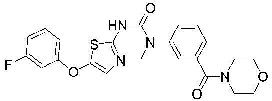 | 3-(5-(3-fluorophenoxy)thiazol-2-yl)-1-methyl-1-(3-(morpholine-4-carbonyl)phenyl)urea |
| **193** | 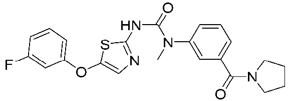 | 3-(5-(3-fluorophenoxy)thiazol-2-yl)-1-methyl-1-(3-(pyrrolidine-1-carbonyl)phenyl)urea |
| **194** | 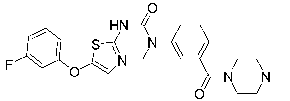 | 3-(5-(3-fluorophenoxy)thiazol-2-yl)-1-methyl-1-(3-(4-methylpiperazine-1-carbonyl)phenyl)urea |
| **195** | 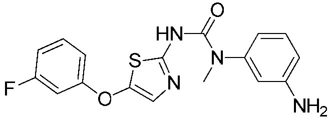 | 1-(3-aminophenyl)-3-(5-(3-fluorophenoxy)thiazol-2-yl)-1-methylurea |
| **196** | 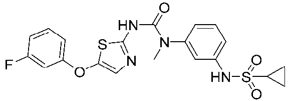 | N-(3-(3-(5-(3-fluorophenoxy)thiazol-2-yl)-1-methylureido)phenyl)cyclopropanesulfonamide |
| **197** | 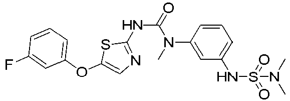 | N-(3-(3-(5-(3-fluorophenoxy)thiazol-2-yl)-1-methylureido)phenyl) N,N-dimethylsulfamide |
| **198** | 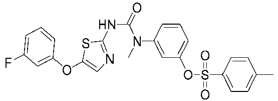 | 3-(3-(5-(3-fluorophenoxy)thiazol-2-yl)-1-methylureido)phenyl 4-methylbenzenesulfonate |
| **199** | 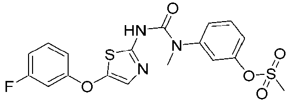 | 3-(3-(5-(3-fluorophenoxy)thiazol-2-yl)-1-methylureido)phenyl methanesulfonate |
| **200** | 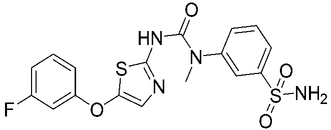 | 3-(3-(5-(3-fluorophenoxy)thiazol-2-yl)-1-methylureido)benzenesulfonamide |
| **201** | 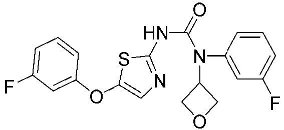 | 3-(5-(3-fluorophenoxy)thiazol-2-yl)-1-(3-fluorophenyl)-1-(oxetan-3-yl)urea |
| **202** | 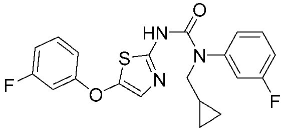 | 1-(cyclopropylmethyl)-3-(5-(3-fluorophenoxy)thiazol-2-yl)-1-(3-fluorophenyl)urea |
| **203** | 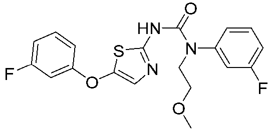 | 3-(5-(3-fluorophenoxy)thiazol-2-yl)-1-(3-fluorophenyl)-1-(2-methoxyethyl)urea |
| **204** | 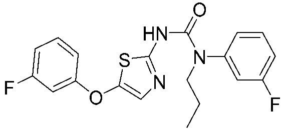 | 3-(5-(3-fluorophenoxy)thiazol-2-yl)-1-(3-fluorophenyl)-1-propylurea |
| **205** | 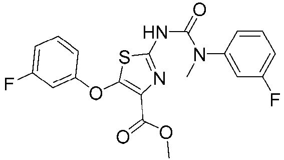 | Methyl 5-(3-fluorophenoxy)-2-(3-(3-fluorophenyl)-3-methylureido)thiazole-4-carboxylate |
| **206** | 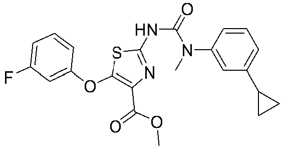 | Methyl 2-(3-(3-cyclopropylphenyl)-3-methylureido)-5-(3-fluorophenoxy)thiazole-4-carboxylate |
| **207** | 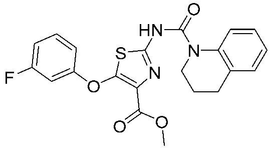 | Methyl 5-(3-fluorophenoxy)-2-(1,2,3,4-tetrahydroquinoline-1-carboxamido)thiazole-4-carboxylate |
| **208** | 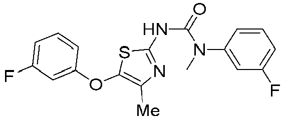 | 3-(5-(3-fluorophenoxy)-4-methylthiazol-2-yl)-1-(3-fluorophenyl)-1-methylurea |
| **209** | 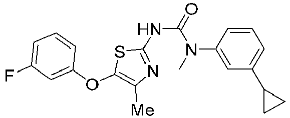 | 1-(3-cyclopropylphenyl)-3-(5-(3-fluorophenoxy)-4-methylthiazol-2-yl)-1-methylurea |
| **210** | 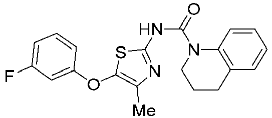 | N-(5-(3-fluorophenoxy)-4-methylthiazol-2-yl)-3,4-dihydroquinoline-1 (2H)-carboxamide |
| **211** | 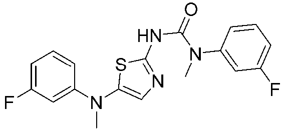 | 1-(3-fluorophenyl)-3-(5-((3-fluorophenyl)(methyl)amino)thiazol-2-yl)-1-methylurea |
| **212** | 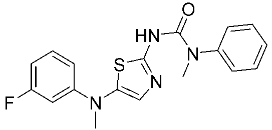 | 3-(5-((3-fluorophenyl)(methyl)amino)thiazol-2-yl)-1-methyl-1-phenylurea |
| **213** | 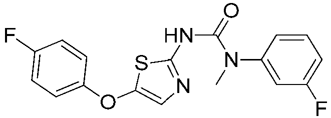 | 3-(5-(4-fluorophenoxy)thiazol-2-yl)-1-(3-fluorophenyl)-1-methylurea |
| **214** | 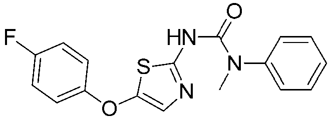 | 3-(5-(4-fluorophenoxy)thiazol-2-yl)-1-methyl-1-phenylurea |
| **215** | 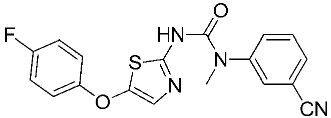 | 1-(3-cyanophenyl)-3-(5-(4-fluorophenoxy)thiazol-2-yl)-1-methylurea |
| **216** | 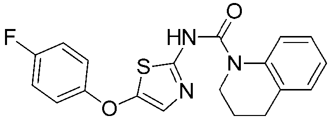 | N-(5-(4-fluorophenoxy)thiazol-2-yl)-3,4-dihydroquinoline-1(2H)-carboxamide |
| **217** | 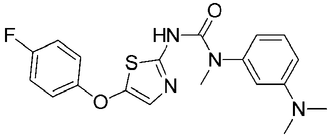 | 1-(3-(dimethylamino)phenyl)-3-(5-(4-fluorophenoxy)thiazol-2-yl)-1-methylurea |
| **218** | 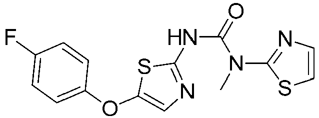 | 3-(5-(4-fluorophenoxy)thiazol-2-yl)-1-methyl-1-(thiazol-2-yl)urea |
| **219** | 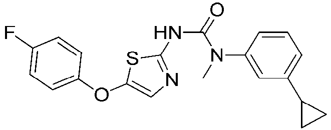 | 1-(3-cyclopropylphenyl)-3-(5-(4-fluorophenoxy)thiazol-2-yl)-1-methylurea |
| **220** | 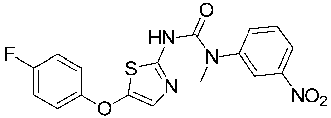 | 3-(5-(4-fluorophenoxy)thiazol-2-yl)-1-methyl-1-(3-nitrophenyl)urea |
| **221** | 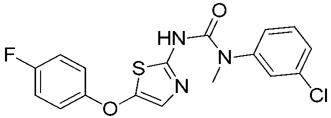 | 1-(3-chlorophenyl)-3-(5-(4-fluorophenoxy)thiazol-2-yl)-1-methylurea |
| **222** | 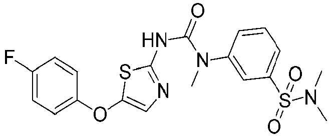 | 3-(3-(5-(4-fluorophenoxy)thiazol-2-yl)-1-methylureido)-N,N-dimethylbenzenesulfonamide |
| **223** | 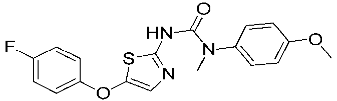 | 3-(5-(4-fluorophenoxy)thiazol-2-yl)-1-(4-methoxyphenyl)-1-methylurea |
| **224** | 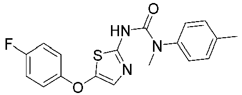 | 3-(5-(4-fluorophenoxy)thiazol-2-yl)-1-methyl-1-(p-tolyl)urea |
| **225** | 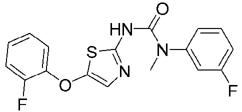 | 3-(5-(2-fluorophenoxy)thiazol-2-yl)-1-(3-fluorophenyl)-1-methylurea |
| **226** | 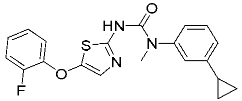 | 1-(3-cyclopropylphenyl)-3-(5-(2-fluorophenoxy)thiazol-2-yl)-1-methylurea |
| **227** | 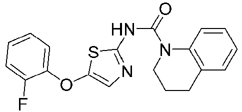 | N-(5-(2-fluorophenoxy)thiazol-2-yl)-3,4-dihydroquinoline-1 (2H)-carboxamide |
| **228** | 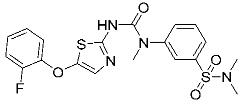 | 3-(3-(5-(2-fluorophenoxy)thiazol-2-yl)-1-methylureido)-N,N-dimethylbenzenesulfonamide |
| **229** | 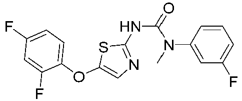 | 3-(5-(2,4-difluorophenoxy)thiazol-2-yl)-1-(3-fluorophenyl)-1-methylurea |
| **230** | 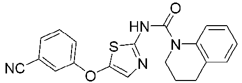 | N-(5-(3-cyanophenoxy)thiazol-2-yl)-3,4-dihydroquinoline-1(2H)-carboxamide |
| **231** | 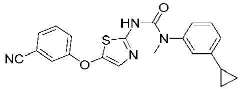 | 3-(5-(3-cyanophenoxy)thiazol-2-yl)-1-(3-cyclopropylphenyl)-1-methylurea |
| **232** | 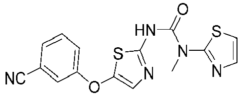 | 3-(5-(3-cyanophenoxy)thiazol-2-yl)-1-methyl-1-(thiazol-2-yl)urea |
| **233** | 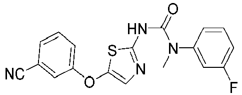 | 3-(5-(3-cyanophenoxy)thiazol-2-yl)-1-(3-fluorophenyl)-1-methylurea |
| **234** | 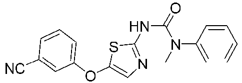 | 3-(5-(3-cyanophenoxy)thiazol-2-yl)-1-methyl-1-phenylurea |
| **235** | 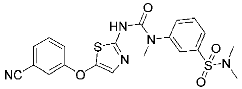 | 3-(3-(5-(3-cyanophenoxy)thiazol-2-yl)-1-methylureido)-N,N-dimethylbenzenesulfonamide |
| **236** | 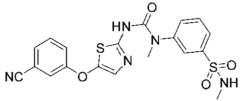 | 3-(3-(5-(3-cyanophenoxy)thiazol-2-yl)-1-methylureido)-N-methylbenzenesulfonamide |
| **237** | 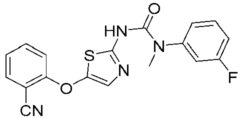 | 3-(5-(2-cyanophenoxy)thiazol-2-yl)-1-(3-fluorophenyl)-1-methylurea |
| **238** | 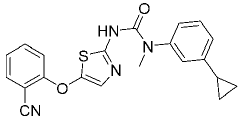 | 3-(5-(2-cyanophenoxy)thiazol-2-yl)-1-(3-cyclopropylphenyl)-1-methylurea |
| **239** | 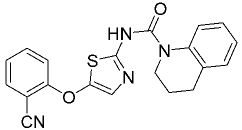 | N-(5-(2-cyanophenoxy)thiazol-2-yl)-3,4-dihydroquinoline-1(2H)-carboxamide |
| **240** | 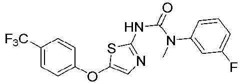 | 1-(3-fluorophenyl)-1-methyl-3-(5-(4-(trifluoromethyl)phenoxy)thiazol-2-yl)urea |
| **241** | 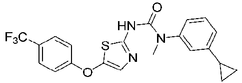 | 1-(3-cyclopropylphenyl)-1-methyl-3-(5-(4-(trifluoromethyl)phenoxy)thiazol-2-yl)urea |
| **242** | 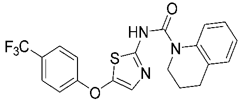 | N-(5-(3-fluorophenoxy)thiazol-2-yl)- 1H-pyrrolo[2,3-b]pyridine-1-carboxamide |
| **243** | 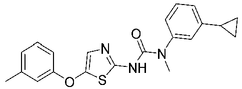 | 1-(3-cyclopropylphenyl)-1-methyl-3-(5-(m-tolyloxy)thiazol-2-yl)urea |
| **244** | 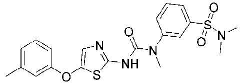 | N,N-dimethyl-3-(1-methyl-3-(5-(m-tolyloxy)thiazol-2-yl)ureido)benzenesulfonamide |
| **245** | 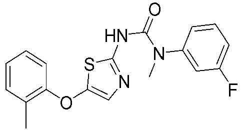 | 1-(3-fluorophenyl)-1-methyl-3-(5-(o-tolyloxy)thiazol-2-yl)urea |
| **246** | 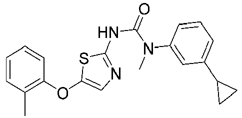 | 1-(3-cyclopropylphenyl)-1-methyl-3-(5-(o-tolyloxy)thiazol-2-yl)urea |
| **247** | 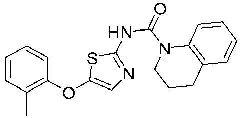 | N-(5-(o-tolyloxy)thiazol-2-yl)-3,4-dihydroquinoline-1 (2H)-carboxamide |
| **248** | 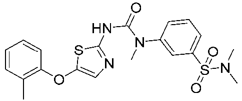 | N,N-dimethyl-3-(1-methyl-3-(5-(o-tolyloxy)thiazol-2-yl)ureido)benzenesulfonamide |
| **249** | 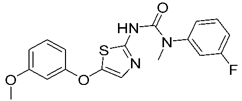 | 1-(3-fluorophenyl)-3-(5-(3-methoxyphenoxy)thiazol-2-yl)-1-methylurea |
| **250** | 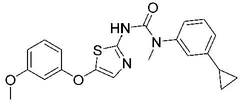 | 1-(3-cyclopropylphenyl)-3-(5-(3-methoxyphenoxy)thiazol-2-yl)-1-methylurea |
| **251** | 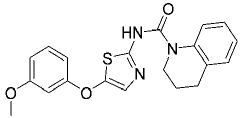 | N-(5-(3-methoxyphenoxy)thiazol-2-yl)-3,4-dihydroquinoline-1(2H)-carboxamide |
| **252** | 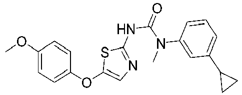 | 1-(3-cyclopropylphenyl)-3-(5-(4-methoxyphenoxy)thiazol-2-yl)-1-methylurea |
| **253** | 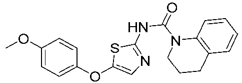 | N-(5-(4-methoxyphenoxy)thiazol-2-yl)-3,4-dihydroquinoline-1(2H)-carboxamide |
| **254** | 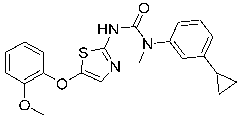 | 1-(3-cyclopropylphenyl)-3-(5-(2-methoxyphenoxy)thiazol-2-yl)-1-methylurea |
| **255** | 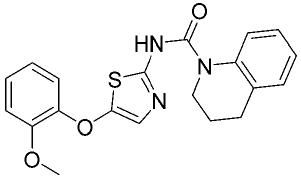 | N-(5-(2-methoxyphenoxy)thiazol-2-yl)-3,4-dihydroquinoline-1(2H)-carboxamide |
| **256** | 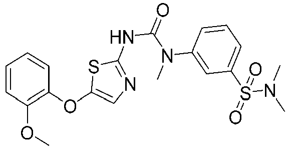 | 3-(3-(5-(2-methoxyphenoxy)thiazol-2-yl)-1-methylureido)-N,N-dimethylbenzenesulfonamide |
| **257** | 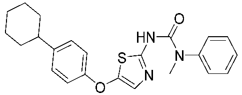 | 3-(5-(4-cyclohexylphenoxy)thiazol-2-yl)-1-methyl-1-phenylurea |
| **258** | 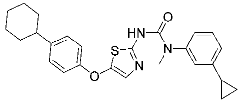 | 3-(5-(4-cyclohexylphenoxy)thiazol-2-yl)-1-(3-cyclopropylphenyl)-1-methylurea |
| **259** | 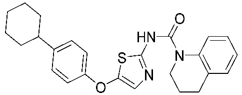 | N-(5-(4-cyclohexylphenoxy)thiazol-2-yl)-3,4-dihydroquinoline-1(2H)-carboxamide |
| **260** | 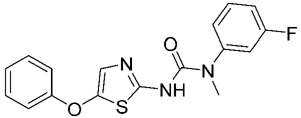 | 1-(3-fluorophenyl)-1-methyl-3-(5-phenoxythiazol-2-yl)urea |
| **261** | 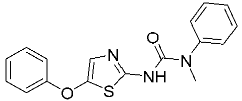 | 1-methyl-3-(5-phenoxythiazol-2-yl)-1-phenylurea |
| **262** | 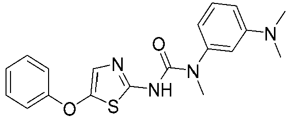 | 1-(3-(dimethylamino)phenyl)-1-methyl-3-(5-phenoxythiazol-2-yl)urea |
| **263** | 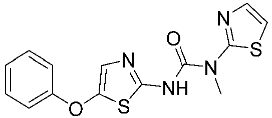 | 1-methyl-3-(5-phenoxythiazol-2-yl)-1-(thiazol-2-yl)urea |
| **264** | 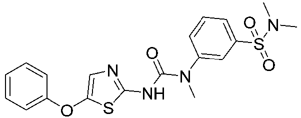 | N,N-dimethyl-3-(1-methyl-3-(5-phenoxythiazol-2-yl)ureido)benzenesulfonamide |
| **265** | 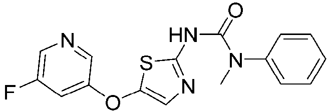 | 3-(5-((5-fluoropyridin-3-yl)oxy)thiazol-2-yl)-1-methyl-1-phenylurea |
| **266** | 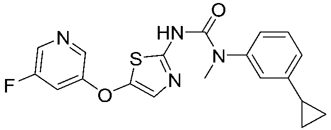 | 1-(3-cyclopropylphenyl)-3-(5-((5-fluoropyridin-3-yl)oxy)thiazol-2-yl)-1-methylurea |
| **267** | 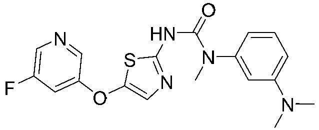 | 1-(3-(dimethylamino)phenyl)-3-(5-((5-fluoropyridin-3-yl)oxy)thiazol-2-yl)-1-methylurea |
| **268** | 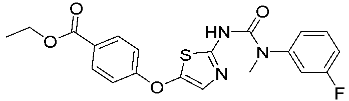 | Ethyl 4-((2-(3-(3-fluorophenyl)-3-methylureido)thiazol-5-yl)oxy)benzoate |
| **269** | 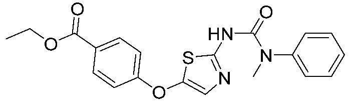 | Ethyl 4-((2-(3-methyl-3-phenylureido)thiazol-5-yl)oxy)benzoate |
| **270** | 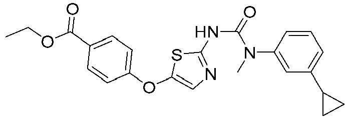 | Ethyl 4-((2-(3-(3-cyclopropylphenyl)-3-methylureido)thiazol-5-yl)oxy)benzoate |
| **271** | 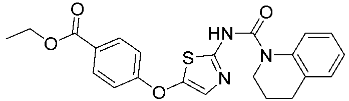 | Ethyl 4-((2-(1,2,3,4-tetrahydroquinoline-1-carboxamido)thiazol-5-yl)oxy)benzoate |
| **272** | 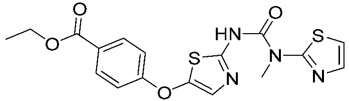 | Ethyl 4-((2-(3-methyl-3-(thiazol-2-yl)ureido)thiazol-5-yl)oxy)benzoate |
| **273** | 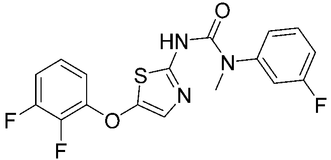 | 3-(5-(2,3-difluorophenoxy)thiazol-2-yl)-1-(3-fluorophenyl)-1-methylurea |
| **274** | 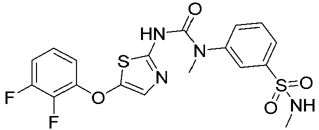 | 3-(3-(5-(2,3-difluorophenoxy)thiazol-2-yl)-1-methylureido)-N-methylbenzenesulfonamide |
| **275** | 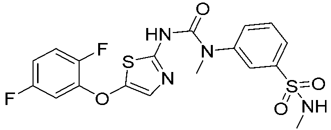 | 3-(3-(5-(2,5-difluorophenoxy)thiazol-2-yl)-1-methylureido)-N-methylbenzenesulfonamide |
| **276** | 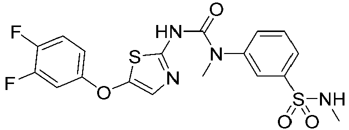 | 3-(3-(5-(3,4-difluorophenoxy)thiazol-2-yl)-1-methylureido)-N-methylbenzenesulfonamide |
| **277** | 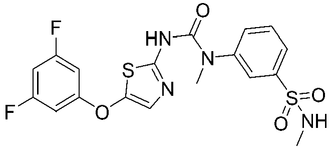 | 3-(3-(5-(3,5-difluorophenoxy)thiazol-2-yl)-1-methylureido)-N-methylbenzenesulfonamide |
| **278** | 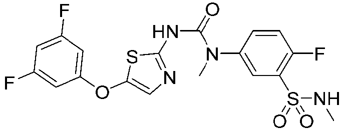 | 5-(3-(5-(3,5-difluorophenoxy)thiazol-2-yl)-1-methylureido)-2-fluoro-N-methylbenzenesulfonamide |
| **279** | 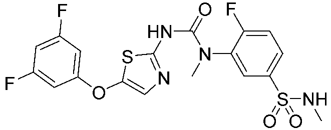 | 3-(3-(5-(3,5-difluorophenoxy)thiazol-2-yl)-1-methylureido)-4-fluoro-N-methylbenzenesulfonamide |
| **280** | 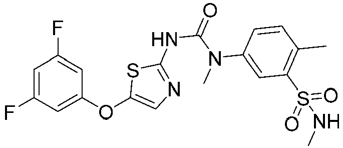 | 5-(3-(5-(3,5-difluorophenoxy)thiazol-2-yl)-1-methylureido)-N,2-dimethylbenzenesulfonamide |
| **281** | 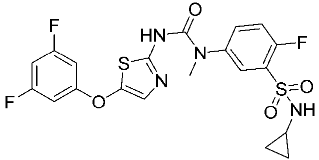 | N-cyclopropyl-5-(3-(5-(3,5-difluorophenoxy)thiazol-2-yl)-1-methylureido)-2-fluorobenzenesulfonamide |
| **282** | 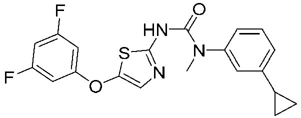 | 1-(3-cyclopropylphenyl)-3-(5-(3,5-difluorophenoxy)thiazol-2-yl)-1-methylurea |
| **283** | 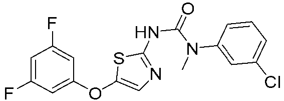 | 1-(3-chlorophenyl)-3-(5-(3,5-difluorophenoxy)thiazol-2-yl)-1-methylurea |
| **284** | 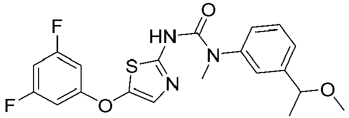 | 3-(5-(3,5-difluorophenoxy)thiazol-2-yl)-1-(3-(1-methoxyethyl)phenyl)-1-methylurea |
| **285** | 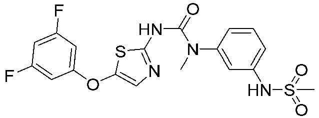 | N-(3-(3-(5-(3,5-difluorophenoxy)thiazol-2-yl)-1-methylureido)phenyl)methanesulfonamide |
| **286** | 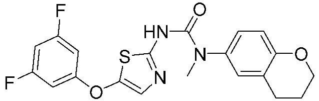 | 1-(chroman-6-yl)-3-(5-(3,5-difluorophenoxy)thiazol-2-yl)-1-methylurea |
| **287** | 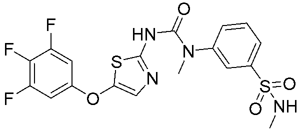 | N-methyl-3-(1-methyl-3-(5-(3,4,5-trifluorophenoxy)thiazol-2-yl)ureido)benzenesulfonamide |
| **288** | 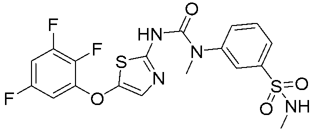 | N-methyl-3-(1-methyl-3-(5-(2,3,5-trifluorophenoxy)thiazol-2-yl)ureido)benzenesulfonamide |
| **289** | 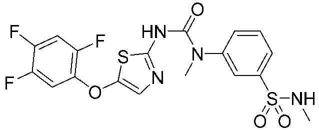 | N-methyl-3-(1-methyl-3-(5-(2,4,5-trifluorophenoxy)thiazol-2-yl)ureido)benzenesulfonamide |
| **290** | 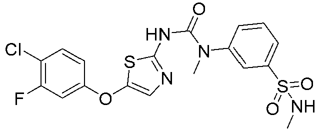 | 3-(3-(5-(4-chloro-3-fluorophenoxy)thiazol-2-yl)-1-methylureido)-N-methylbenzenesulfonamide |
| **291** | 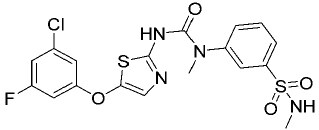 | 3-(3-(5-(3-chloro-5-fluorophenoxy)thiazol-2-yl)-1-methylureido)-N-methylbenzenesulfonamide |
| **292** | 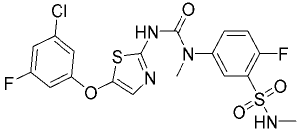 | 5-(3-(5-(3-chloro-5-fluorophenoxy)thiazol-2-yl)-1-methylureido)-2-fluoro-N-methylbenzenesulfonamide |
| **293** | 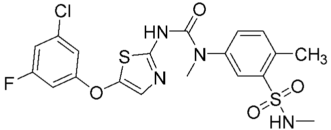 | 5-(3-(5-(3-chloro-5-fluorophenoxy)thiazol-2-yl)-1-methylureido)-N,2-dimethylbenzenesulfonamide |
| **294** | 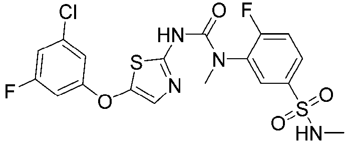 | 3-(3-(5-(3-chloro-5-fluorophenoxy)thiazol-2-yl)-1-methylureido)-4-fluoro-N-methylbenzenesulfonamide |
| **295** | 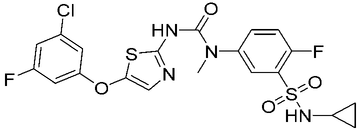 | 5-(3-(5-(3-chloro-5-fluorophenoxy)thiazol-2-yl)-1-methylureido)-N-cyclopropyl-2-fluorobenzenesulfonamide |
| **296** | 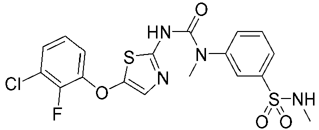 | 3-(3-(5-(3-chloro-2-fluorophenoxy)thiazol-2-yl)-1-methylureido)-N-methylbenzenesulfonamide |
| **297** | 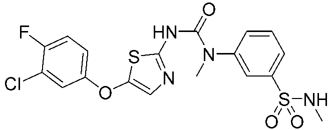 | 3-(3-(5-(3-chloro-4-fluorophenoxy)thiazol-2-yl)-1-methylureido)-N-methylbenzenesulfonamide |
| **298** | 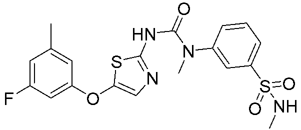 | 3-(3-(5-(3-fluoro-5-methylphenoxy)thiazol-2-yl)-1-methylureido)-N-methylbenzenesulfonamide |
| **299** | 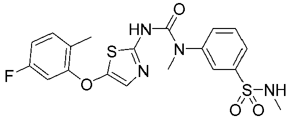 | 3-(3-(5-(5-fluoro-2-methylphenoxy)thiazol-2-yl)-1-methylureido)-N-methylbenzenesulfonamide |
| **300** | 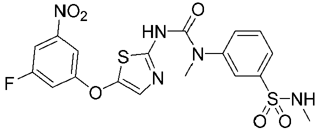 | 3-(3-(5-(3-fluoro-5-nitrophenoxy)thiazol-2-yl)-1-methylureido)-N-methylbenzenesulfonamide |
| **301** | 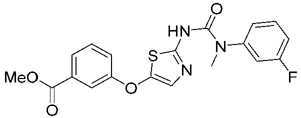 | Methyl 3-((2-(3-(3-fluorophenyl)-3-methylureido)thiazol-5-yl)oxy)benzoate |
| **302** | 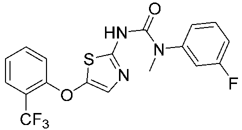 | 1-(3-fluorophenyl)-1-methyl-3-(5-(2-(trifluoromethyl)phenoxy)thiazol-2-yl)urea |
| **303** | 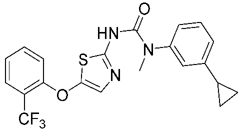 | 1-(3-cyclopropylphenyl)-1-methyl-3-(5-(2-(trifluoromethyl)phenoxy)thiazol-2-yl)urea |
| **304** | 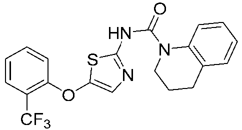 | N-(5-(2-(trifluoromethyl)phenoxy)thiazol-2-yl)-3,4-dihydroquinoline-1(2H)-carboxamide |
| **305** | 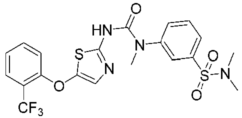 | N,N-dimethyl-3-(1-methyl-3-(5-(2-(trifluoromethyl)phenoxy)thiazol-2-yl)ureido)benzenesulfonamide |
| **306** | 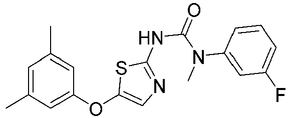 | 3-(5-(3,5-dimethylphenoxy)thiazol-2-yl)-1-(3-fluorophenyl)-1-methylurea |
| **307** | 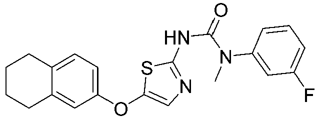 | 1-(3-fluorophenyl)-1-methyl-3-(5-((5,6,7,8-tetrahydronaphthalen-2-yl)oxy)thiazol-2-yl)urea |
| **308** | 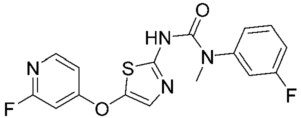 | 1-(3-fluorophenyl)-3-(5-((2-fluoropyridin-4-yl)oxy)thiazol-2-yl)-1-methylurea |
| **309** | 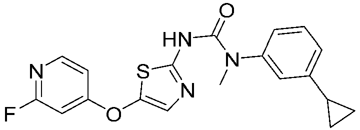 | 1-(3-cyclopropylphenyl)-3-(5-((2-fluoropyridin-4-yl)oxy)thiazol-2-yl)-1-methylurea |
| **310** | 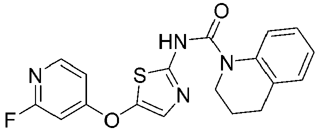 | N-(5-((2-fluoropyridin-4-yl)oxy)thiazol-2-yl)-3,4-dihydroquinoline-1(2H)-carboxamide |
| **311** | 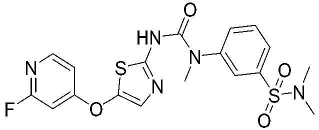 | 3-(3-(5-((2-fluoropyridin-4-yl)oxy)thiazol-2-yl)-1-methylureido)-N,N-dimethylbenzenesulfonamide |
| **312** | 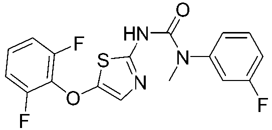 | 3-(5-(2,6-difluorophenoxy)thiazol-2-yl)-1-(3-fluorophenyl)-1-methylurea |
| **313** | 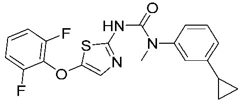 | 1-(3-cyclopropylphenyl)-3-(5-(2,6-difluorophenoxy)thiazol-2-yl)-1-methylurea |
| **314** | 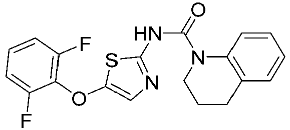 | N-(5-(2,6-difluorophenoxy)thiazol-2-yl)-3,4-dihydroquinoline-1(2H)-carboxamide |
| **315** | 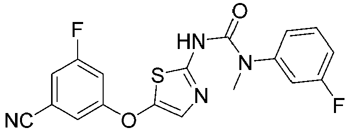 | 3-(5-(3-cyano-5-fluorophenoxy)thiazol-2-yl)-1-(3-fluorophenyl)-1-methylurea |
| **316** | 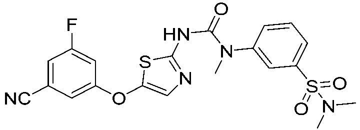 | 3-(3-(5-(3-cyano-5-fluorophenoxy)thiazol-2-yl)-1-methylureido)-N,N-dimethylbenzenesulfonamide |
| **317** | 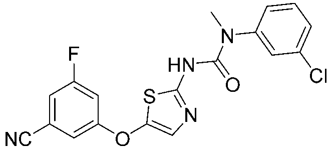 | 1-(3-chlorophenyl)-3-(5-(3-cyano-5-fluorophenoxy)thiazol-2-yl)-1-methylurea |
| **318** | 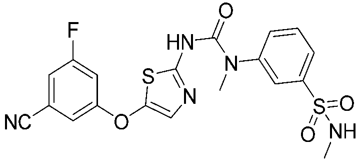 | 3-(3-(5-(3-cyano-5-fluorophenoxy)thiazol-2-yl)-1-methylureido)-N-methylbenzenesulfonamide |
| **319** | 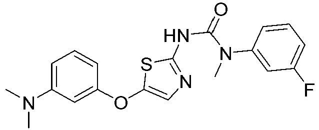 | 3-(5-(3-(dimethylamino)phenoxy)thiazol-2-yl)-1-(3-fluorophenyl)-1-methylurea |
| **320** | 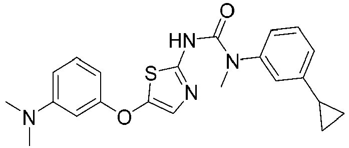 | 1-(3-cyclopropylphenyl)-3-(5-(3-(dimethylamino)phenoxy)thiazol-2-yl)-1-methylurea |
| **321** | 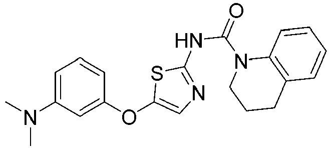 | N-(5-(3-(dimethylamino)phenoxy)thiazol-2-yl)-3,4-dihydroquinoline-1(2H)-carboxamide |
| **322** | 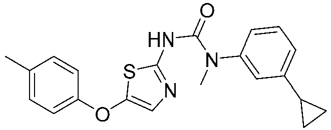 | 1-(3-cyclopropylphenyl)-1-methyl-3-(5-(p-tolyloxy)thiazol-2-yl)urea |
| **323** | 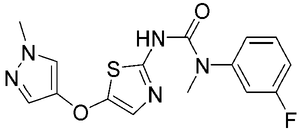 | 1-(3-fluorophenyl)-1-methyl-3-(5-((1-methyl-1H-pyrazol-4-yl)oxy)thiazol-2-yl)urea |
| **324** | 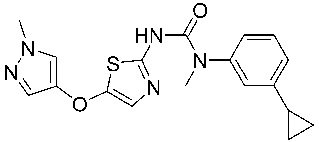 | 1-(3-cyclopropylphenyl)-1-methyl-3-(5-((1-methyl-1H-pyrazol-4-yl)oxy)thiazol-2-yl)urea |
| **325** | 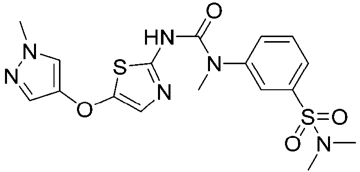 | N,N-dimethyl-3-(1-methyl-3-(5-((1-methyl-1H-pyrazol-4-yl)oxy)thiazol-2-yl)ureido)benzenesulfonamide |
| **326** | 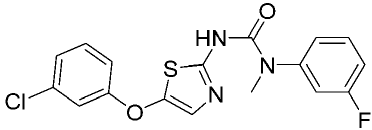 | 3-(5-(3-chlorophenoxy)thiazol-2-yl)-1-(3-fluorophenyl)-1-methylurea |
| **327** | 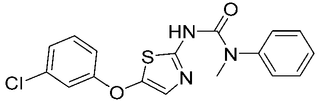 | 3-(5-(3-chlorophenoxy)thiazol-2-yl)-1-methyl-1-phenylurea |
| **328** | 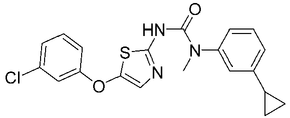 | 3-(5-(3-chlorophenoxy)thiazol-2-yl)-1-(3-cyclopropylphenyl)-1-methylurea |
| **329** | 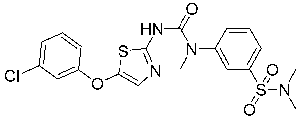 | 3-(3-(5-(3-chlorophenoxy)thiazol-2-yl)-1-methylureido)-N,N-dimethylbenzenesulfonamide |
| **330** | 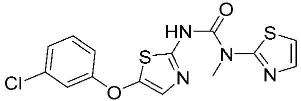 | 3-(5-(3-chlorophenoxy)thiazol-2-yl)-1-methyl-1-(thiazol-2-yl)urea |
| **331** | 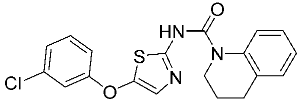 | N-(5-(3-chlorophenoxy)thiazol-2-yl)-3,4-dihydroquinoline-1(2H)-carboxamide |
| **332** | 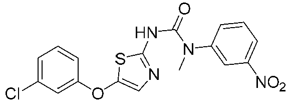 | 3-(5-(3-chlorophenoxy)thiazol-2-yl)-1-methyl-1-(3-nitrophenyl)urea |
| **333** | 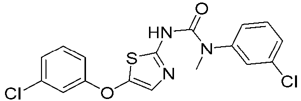 | 3-(5-(3-chlorophenoxy)thiazol-2-yl)-1-(3-chlorophenyl)-1-methylurea |
| **334** | 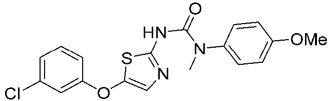 | 3-(5-(3-chlorophenoxy)thiazol-2-yl)-1-(4-methoxyphenyl)-1-methylurea |
| **335** | 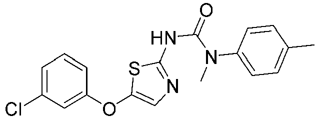 | 3-(5-(3-chlorophenoxy)thiazol-2-yl)-1-methyl-1-(p-tolyl)urea |
| **336** | 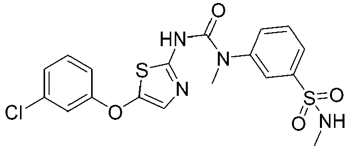 | 3-(3-(5-(3-chlorophenoxy)thiazol-2-yl)-1-methylureido)-N-methylbenzenesulfonamide |
| **337** | 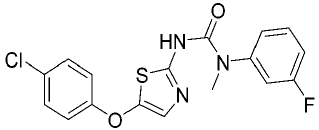 | 3-(5-(4-chlorophenoxy)thiazol-2-yl)-1-(3-fluorophenyl)-1-methylurea |
| **338** | 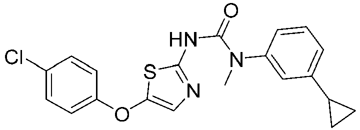 | 3-(5-(4-chlorophenoxy)thiazol-2-yl)-1-(3-cyclopropylphenyl)-1-methylurea |
| **339** | 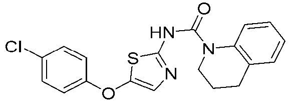 | N-(5-(4-chlorophenoxy)thiazol-2-yl)-3,4-dihydroquinoline-1(2H)-carboxamide |
| **340** | 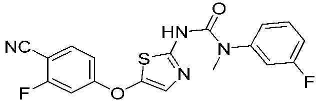 | 3-(5-(4-cyano-3-fluorophenoxy)thiazol-2-yl)-1-(3-fluorophenyl)-1-methylurea |
| **341** | 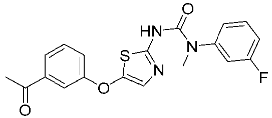 | 3-(5-(3-acetylphenoxy)thiazol-2-yl)-1-(3-fluorophenyl)-1-methylurea |
| **342** | 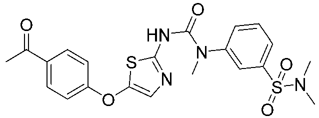 | 3-(3-(5-(4-acetylphenoxy)thiazol-2-yl)-1-methylureido)-N,N-dimethylbenzenesulfonamide |
| **343** | 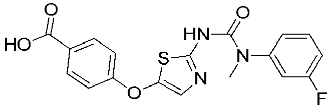 | 4-((2-(3-(3-fluorophenyl)-3-methylureido)thiazol-5-yl)oxy)benzoic acid |
| **344** | 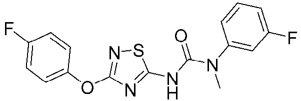 | 3-(3-(4-fluorophenoxy)-1,2,4-thiadiazol-5-yl)-1-(3-fluorophenyl)-1-methylurea |
| **345** | 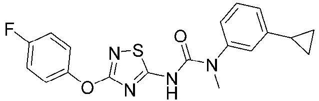 | 1-(3-cyclopropylphenyl)-3-(3-(4-fluorophenoxy)-1,2,4-thiadiazol-5-yl)-1-methylurea |
| **346** | 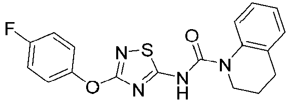 | N-(3-(4-fluorophenoxy)-1,2,4-thiadiazol-5-yl)-3,4-dihydroquinoline-1(2H)-carboxamide |
| **347** | 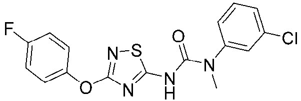 | 1-(3-chlorophenyl)-3-(3-(4-fluorophenoxy)-1,2,4-thiadiazol-5-yl)-1-methylurea |
| **348** | 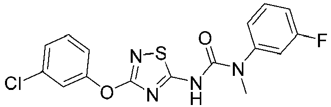 | 3-(3-(3-chlorophenoxy)-1,2,4-thiadiazol-5-yl)-1-(3-fluorophenyl)-1-methylurea |
| **349** | 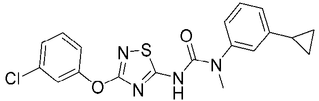 | 3-(3-(3-chlorophenoxy)-1,2,4-thiadiazol-5-yl)-1-(3-cyclopropylphenyl)-1-methylurea |
| **350** | 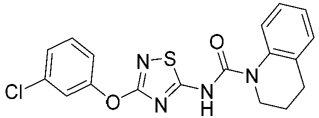 | N-(3-(3-chlorophenoxy)-1,2,4-thiadiazol-5-yl)-3,4-dihydroquinoline-1(2H)-carboxamide |
| **351** | 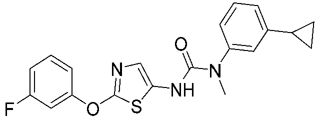 | 1-(3-cyclopropylphenyl)-3-(2-(3-fluorophenoxy)thiazol-5-yl)-1-methylurea |
| **352** | 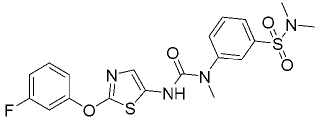 | 3-(3-(2-(3-fluorophenoxy)thiazol-5-yl)-1-methylureido)-N,N-dimethylbenzenesulfonamide |
| **353** | 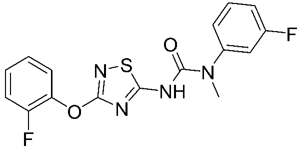 | 3-(3-(2-fluorophenoxy)-1,2,4-thiadiazol-5-yl)-1-(3-fluorophenyl)-1-methylurea |
| **354** | 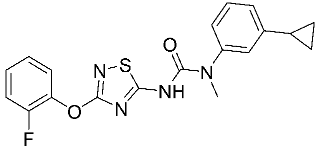 | 1-(3-cyclopropylphenyl)-3-(3-(2-fluorophenoxy)-1,2,4-thiadiazol-5-yl)-1-methylurea |
| **355** | 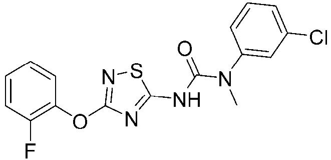 | 1-(3-chlorophenyl)-3-(3-(2-fluorophenoxy)-1,2,4-thiadiazol-5-yl)-1-methylurea |
| **356** | 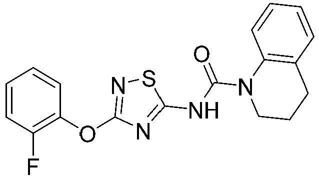 | N-(3-(2-fluorophenoxy)-1,2,4-thiadiazol-5-yl)-3,4-dihydroquinoline-1(2H)-carboxamide |
| **357** | 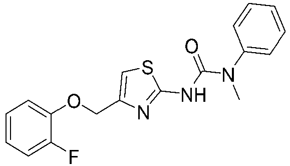 | 3-(4-((2-fluorophenoxy)methyl)thiazol-2-yl)-1-methyl-1-phenylurea |
| **358** | 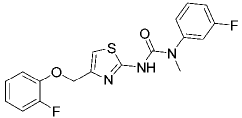 | 3-(4-((2-fluorophenoxy)methyl)thiazol-2-yl)-1-(3-fluorophenyl)-1-methylurea |
| **359** | 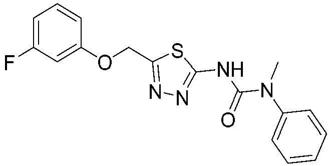 | 3-(5-((3-fluorophenoxy)methyl)-1,3,4-thiadiazol-2-yl)-1-methyl-1-phenylurea |
| **360** | 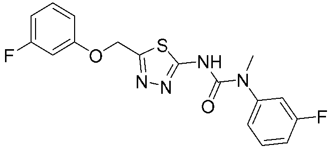 | 3-(5-((3-fluorophenoxy)methyl)-1,3,4-thiadiazol-2-yl)-1-(3-fluorophenyl)-1-methylurea |
| **361** | 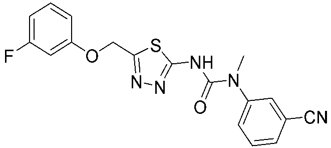 | 1-(3-cyanophenyl)-3-(5-((3-fluorophenoxy)methyl)-1,3,4-thiadiazol-2-yl)-1-methylurea |
| **362** | 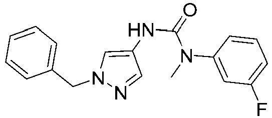 | 3-(1-benzyl-1H-pyrazol-4-yl)-1-(3-fluorophenyl)-1-methylurea |
| **363** | 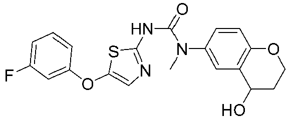 | 3-(5-(3-fluorophenoxy)thiazol-2-yl)-1-(4-hydroxychroman-6-yl)-1-methylurea |
| **364** | 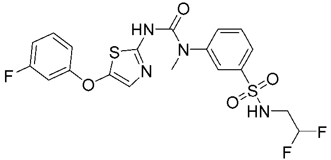 | N-(2,2-difluoroethyl)-3-(3-(5-(3-fluorophenoxy)thiazol-2-yl)-1-methylureido)benzenesulfonamide |

**(8)** In any one of above **(1)** to **(6),** the novel compound according to the present invention, the stereoisomer thereof, or the pharmaceutically acceptable salt thereof may be a compound represented by above Table 1, a stereoisomer thereof, or a pharmaceutically acceptable salt thereof.

In the present invention, "stereoisomer" comprises diastereomers and optical isomers, which comprise a single enantiomer, a mixture of enantiomers comprising a racemate, a single diastereomer, and a mixture of diastereomers all. Such isomers are separated by resolution according to the conventional art, for example, column chromatography, HPLC, or the like. Alternatively, stereospecific synthesis may be performed by using optically pure starting materials and/or reagents of known configuration. Specifically, the isomer may be an optical isomer.

In the present invention, "pharmaceutically acceptable" may mean that which is physiologically acceptable and does not typically cause an allergic reaction such as gastrointestinal disorder and dizziness, or a similar reaction thereto, when administered to a subject.

In the present invention, "pharmaceutically acceptable salts" mean salts conventionally used in the pharmaceutical industry, and the pharmaceutically acceptable salts of the present invention may be prepared by a conventional method known to those skilled in the art. As one example, the pharmaceutically acceptable salts comprise inorganic ion salts prepared from calcium, potassium, sodium, magnesium, or the like; inorganic acid salts prepared from hydrochloric acid, nitric acid, phosphoric acid, bromic acid, iodic acid, perchloric acid, sulfuric acid, or the like; organic acid salts prepared from acetic acid, trifluoroacetic acid, citric acid, maleic acid, succinic acid, oxalic acid, benzoic acid, tartaric acid, fumaric acid, mandelic acid, propionic acid, lactic acid, glycolic acid, gluconic acid, galacturonic acid, glutamic acid, glutaric acid, glucuronic acid, aspartic acid, ascorbic acid, carbonic acid, vanillic acid, or the like; sulfonic acid salts prepared from methanesulfonic acid, ethanesulfonic acid, benzenesulfonic acid, p-toluenesulfonic acid, naphthalenesulfonic acid, or the like; amino acid salts prepared from glycine, arginine, lysine, or the like; amine salts prepared from trimethylamine, triethylamine, ammonia, pyridine, picoline, or the like; and the like, but the types of salts meant in the present invention are not limited to those listed salts. For example, the pharmaceutically acceptable salt may be a hydrochloride.

The pharmaceutically acceptable salts of the present invention may be prepared by conventional methods known to those skilled in the art.

The compound having a novel structure (the compound represented by chemical formula I or chemical formula II and the compound shown in Table 1), the stereoisomer thereof, or the pharmaceutically acceptable salt thereof according to the present invention may effectively inhibit MRGPRX2 and effectively suppress the expression of MRGPRX2 at a low concentration. The compound having a novel structure, the stereoisomer thereof, or the pharmaceutically acceptable salt thereof according to the present invention may effectively inhibit calcium mobilization in cells and significantly inhibit calcium mobilization in cells even at a low concentration.

The compound having a novel structure, the stereoisomer thereof, or the pharmaceutically acceptable salt thereof according to the present invention may effectively inhibit degranulation of mast cells and may significantly inhibit the degranulation of mast cells even at a low concentration.

The compound having a novel structure, the stereoisomer thereof, or the pharmaceutically acceptable salt thereof according to the present invention may significantly and excellently prevent or treat allergic diseases, inflammatory bowel disease, arthritis, or migraine.

The compound having a novel structure, the stereoisomer thereof, or the pharmaceutically acceptable salt thereof according to the present invention may significantly and excellently prevent or treat allergic diseases comprising chronic urticaria, anaphylaxis, non-histaminergic itching, psoriasis, asthma, atopic dermatitis, or rosacea; inflammatory bowel disease; arthritis; or migraine.

For example, the compound having a novel structure, the stereoisomer thereof, or the pharmaceutically acceptable salt thereof according to the present invention may have little or no side effects, may exhibit excellent pharmacokinetic properties (excellent absorption rate and bioavailability), may exhibit desired pharmacological effects (effects of preventing or treating allergic diseases, inflammatory bowel disease, arthritis, migraine, or the like) in each tissue by being appropriately distributed to each tissue according to administration, and may be effectively metabolized in the body.

### Pharmaceutical composition, treatment method, and use

The present invention provides a pharmaceutical composition comprising a compound having a novel structure, a stereoisomer thereof, or a pharmaceutically acceptable salt thereof as an active ingredient.

Hereinafter, "the compound having a novel structure, the stereoisomer thereof, or the pharmaceutically acceptable salt thereof" according to the present invention are substantially the same as those described above, and thus redundant detailed description is omitted.

Hereinafter, "the compound having a novel structure, the stereoisomer thereof, or the pharmaceutically acceptable salt thereof" according to the present invention may be a compound, a stereoisomer thereof, or a pharmaceutically acceptable salt thereof according to any one selected from above (1) to (8).

The present invention provides a pharmaceutical composition for preventing or treating diseases comprising at least one selected from allergic diseases, inflammatory bowel disease, arthritis, and migraine, comprising a compound having a novel structure, a stereoisomer thereof, or a pharmaceutically acceptable salt thereof as an active ingredient.

In the present invention, the allergic diseases may comprise chronic urticaria, anaphylaxis, non-histaminergic itching, psoriasis, asthma, atopic dermatitis, or rosacea.

The pharmaceutical composition of the present invention may further comprise at least one pharmaceutically acceptable carrier in addition to the compound, the stereoisomer thereof, or the pharmaceutically acceptable salt according to the present invention for administration. The pharmaceutically acceptable carriers may be those which are conventionally used in the art, specifically comprising, but not limited thereto, lactose, dextrose, sucrose, sorbitol, mannitol, starch, acacia gum, calcium phosphate, alginate, gelatin, calcium silicate, microcrystalline cellulose, polyvinylpyrrolidine, cellulose, water, syrup, methyl cellulose, methyl hydroxybenzoate, propyl hydroxybenzoate, talc, magnesium stearate, or mineral oil. The pharmaceutical composition of the present invention may further comprise lubricants, humectants, sweetening agents, flavoring agents, emulsifiers, suspending agents, preservatives, dispersing agents, stabilizing agents, etc., in addition to the above components. In addition, the pharmaceutical composition of the present invention may be formulated into an oral dosage form, such as tablets, powders, granules, pills, capsules, suspensions, emulsions, liquids for internal use, emulsions, syrups, etc., as well as a form of external preparations, suppositories, or sterile solutions for injection by using a pharmaceutically acceptable carrier and an excipient, and thus may be prepared in a unit dosage form or contained within a multi-dose container. The preparations may be prepared according to a conventional method used for formulation in the art or a method disclosed in Remington's Pharmaceutical Sciences (19th ed., 1995), and may be formulated into various preparations depending on each disease or component.

The pharmaceutical composition of the present invention may be orally or parenterally administered (for example, applied intravenously, subcutaneously, intraperitoneally, or locally) according to an intended method, in which the dosage thereof varies depending on a patient's weight, age, gender, health condition, diet, administration time, administration method, excretion rate, severity of a disease, and the like. The compound, the stereoisomer thereof, or the pharmaceutically acceptable salt thereof of the present invention may be administered once a day or several times a day by dividing the daily dosage of the composition, but is not necessarily limited thereto. The daily dosage of the compound having a novel structure, the stereoisomer thereof, or the pharmaceutically acceptable salt thereof according to the present invention may be specifically about 0.1 to about 10,000 mg/kg, about 1 to about 8,000 mg/kg, about 5 to about 6,000 mg/kg, or about 10 to about 4,000 mg/kg, and more specifically about 50 to about 2,000 mg/kg, but is not limited thereto.

In addition to the compound according to the present invention, the stereoisomer thereof, or the pharmaceutically acceptable salt thereof, the pharmaceutical composition of the present invention may further comprise at least one ingredient which may exhibit the same or similar medicinal effects or may bring synergy to medicinal effects in combination. The pharmaceutical composition of the present invention may be administered in combination with other therapeutic agents, and may be administered sequentially or simultaneously with conventional therapeutic agents.

The compound having a novel structure, the stereoisomer thereof, or the pharmaceutically acceptable salt thereof according to the present invention may show an effect of preventing or treating diseases comprising at least one selected from allergic diseases, inflammatory bowel disease, arthritis, and migraine.

In the present invention, the "prevention" means all the acts, which inhibit diseases comprising at least one selected from allergic diseases, inflammatory bowel disease, arthritis, and migraine, or delay the occurrence thereof by administering a pharmaceutical composition comprising the compound having a novel structure, the compound of above Table 1, the stereoisomer thereof, or the pharmaceutically acceptable salt thereof according to the present invention (as an active ingredient).

In the present invention, the "treatment" means all the acts, by which symptoms of diseases comprising at least one selected from allergic diseases, inflammatory bowel disease, arthritis, and migraine are ameliorated or beneficially altered by administering a pharmaceutical composition comprising the compound having a novel structure, the compound of above Table 1, the stereoisomer thereof, or the pharmaceutically acceptable salt thereof according to the present invention (as an active ingredient).

The present invention provides a method for preventing or treating diseases comprising at least one selected from allergic diseases, inflammatory bowel disease, arthritis, and migraine, comprising administering the compound having a novel structure, the stereoisomer thereof, the pharmaceutically acceptable salt thereof, or the pharmaceutical composition comprising the same into a subject.

In the present invention, the "administration" refers to introducing a predetermined substance to a subject by an appropriate method.

In the present invention, the "subject" refers to all the animals such as rats, mice, livestock, etc., comprising humans, in which a disease has developed or has the potential to develop, and specifically may refer to mammals comprising humans, but is not limited thereto.

The method for preventing or treating allergic diseases of the present invention may refer to administering a therapeutically effective amount of the compound, the stereoisomer thereof, or the pharmaceutically acceptable salt thereof of the present invention.

In the present invention, the "therapeutically effective amount" may refer to an amount enough to treat a disease at a reasonable risk/benefit ratio applicable to medical treatment and not to cause side effects, and may be determined by those skilled in the art according to factors comprising a patient's gender, age, weight, health condition, type of disease, severity, activity of a drug, sensitivity to a drug, administration method, administration time, administration route, excretion rate, treatment period, drugs combined or concurrently used, as well as other factors well known in the medical field. It may be preferable to differently apply a specific therapeutically effective amount for a certain patient depending on various factors comprising the type and degree of reaction to be achieved therefrom, a specific composition comprising whether other preparations are used in some cases, a patient's age, weight, general health condition, gender, diet, administration time, administration route and secretion rate of the composition, treatment period, and drugs used together with the specific composition or simultaneously therewith, as well as other similar factors well known in the pharmaceutical field.

The present invention provides a use of the compound having a novel structure, the stereoisomer thereof, or the pharmaceutically acceptable salt thereof, or the pharmaceutical composition comprising the same for preventing or treating diseases comprising at least one selected from allergic diseases, inflammatory bowel disease, arthritis, and migraine.

The present invention provides a use of the compound having a novel structure, the stereoisomer thereof, or the pharmaceutically acceptable salt thereof, or the pharmaceutical composition comprising the same in the manufacture of a medicament for preventing or treating diseases comprising at least one selected from allergic diseases, inflammatory bowel disease, arthritis, and migraine.

(9) The present invention provides a method for preventing or treating diseases comprising at least one selected from allergic diseases, inflammatory bowel disease, arthritis, and migraine, comprising administering the compound, the stereoisomer thereof, or the pharmaceutically acceptable salt thereof according to any one selected from above **(1)** to **(8).**

**(10)** The present invention provides a use of the compound, the stereoisomer thereof, or the pharmaceutically acceptable salt thereof according to any one selected from above **(1)** to **(8)** for preventing or treating diseases comprising at least one selected from allergic diseases, inflammatory bowel disease, arthritis, and migraine.

**(11)** The present invention provides a use of the compound, the stereoisomer thereof, or the pharmaceutically acceptable salt thereof according to any one selected from above **(1)** to **(8)** in the manufacture of a medicament for preventing or treating diseases comprising at least one selected from allergic diseases, inflammatory bowel disease, arthritis, and migraine.

Matters mentioned in the compound, the pharmaceutical composition comprising the same, the method using the same, and the use thereof according to the present invention are equally applied, if not contradictory to each other.

### Advantageous Effects

The compound, the stereoisomer thereof, or the pharmaceutically acceptable salt thereof of the present invention can be advantageously used for preventing or treating allergic diseases such as chronic urticaria, anaphylaxis, non-histaminergic itching, psoriasis, asthma, atopic dermatitis, and rosacea, as well as inflammatory bowel disease, arthritis, or migraine as an excellent MRGPRX2 antagonist.

### Mode for Invention

Hereinafter, the present invention will be described in more detail through Examples. These Examples are provided only for the purpose of illustrating the present invention, and thus it will be apparent to those skilled in the art that the scope of the present invention is not limited thereto.

The compound of the present invention may be prepared through the methods described below. Starting materials may be commercially available or may be prepared by known methods, unless otherwise described. The compounds obtained in each Preparation Example were identified by 400 MHz ¹H-NMR (BRUKER, 400-MR), LC-Mass (Waters, SQD2 or Agilent, LC/MSD) analysis.

### <Preparation Example> Compound synthesis method and NMR analysis

### [Example 1]

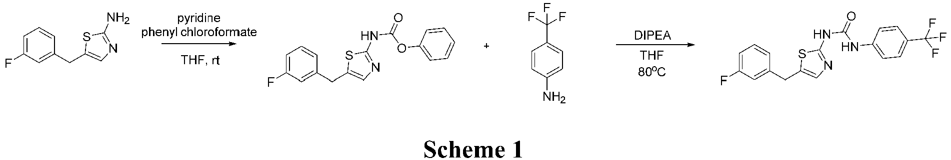

### Synthesis of phenyl (5-(3-fluorobenzyl)thiazol-2-yl)carbamate

After dissolving 1 eq (equivalent) of 5-(3-fluorobenzyl)thiazol-2-amine in tetrahydrofuran (10 mL/g), 1.5 eq of pyridine was added thereto, and the mixture was cooled to 0°C. After slowly adding 1.2 eq of phenyl chloroformate, the mixture was slowly warmed to room temperature and stirred for 16 hours. After completion of the reaction, the mixture was extracted with ethyl acetate and water. The ethyl acetate layer was dried over magnesium sulfate and then concentrated under reduced pressure. The precipitated solid was washed with methylene chloride and filtered. The product obtained by filtration was dried to obtain phenyl (5-(3-fluorobenzyl)thiazol-2-yl)carbamate.

### Synthesis of 1-(5-(3-fluorobenzyl)thiazol-2-yl)-3-(4-(trifluoromethyl)phenyl)urea (Example 1)

After dissolving 1 eq of phenyl (5-(3-fluorobenzyl)thiazol-2-yl)carbamate in tetrahydrofuran (10 mL/g), 2 eq of N,N-diisopropylethylamine and 2 eq of 4-(trifluoromethyl)aniline as **reactant A1** were added thereto and the mixture was warmed to 80°C, and then stirred for 16 hours. After completion of the reaction, the mixture was extracted with methylene chloride, dried over magnesium sulfate, and then concentrated under reduced pressure. The concentrate was purified and separated by column chromatography (hexane:ethyl acetate) to obtain the title **compound of Example 1.**

### [Examples 2-11]

The **compounds of Examples 2-11** were synthesized in substantially the same manner as described in the method for preparing the compound of Example 1 (**Scheme 1**) by using **reactant A1 of Table 2** instead of reactant A1 in the method for preparing the compound of Example 1.

The structures of the compounds of Examples 1-11, reactant A1, and analytical data are shown in Table 2.

**[Table 2]**

| **No** | **Structure** | **Reactant A1** | **Analysis data** |
|---|---|---|---|
| **1** | 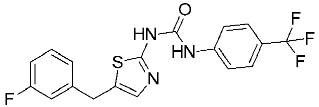 | 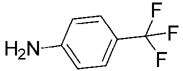 | ¹H NMR (400 MHz, CDCl3) δ 7.58 (q, J = 8.9 Hz, 4H), 7.28 (dd, J = 14.2, 8.1 Hz, 1H), 7.07 (s, 1H), 7.00 (t, J = 6.8 Hz, 1H), 6.99 - 6.87 (m, 2H), 4.11 - 3.92 (m, 2H). LCMS: m/z 396.3 [M+H]+ |
| **2** | 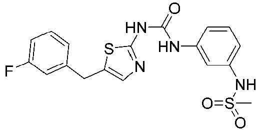 | 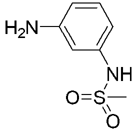 | ¹H NMR (400 MHz, DMSO-d6) δ 9.04 (s, 1H), 7.54 - 7.31 (m, 2H), 7.31 - 6.97 (m, 6H), 6.86 (d, J = 7.9 Hz, 1H), 4.09 (s, 2H), 2.96 (d, J = 22.1 Hz, 3H). LCMS: m/z 421 [M+H]+ |
| **3** | 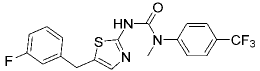 | 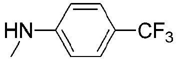 | ¹H NMR (400 MHz, CDCl3) δ 7.74 (d, J = 8.4 Hz, 2H), 7.43 (d, J = 8.3 Hz, 2H), 7.05 - 6.86 (m, 5H), 4.00 (d, J = 15.4 Hz, 2H), 3.39 (s, 3H). LCMS: m/z 410 [M+H]+ |
| **4** | 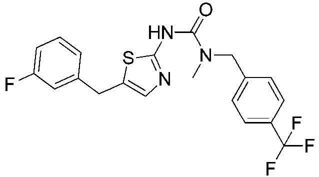 | 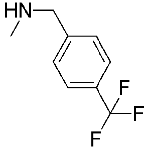 | ¹H NMR (400 MHz, CDCl3) δ 7.58 (d, J = 8.1 Hz, 2H), 7.35 (d, J = 8.0 Hz, 2H), 7.29 - 7.17 (m, 1H), 7.01 (d, J = 3.6 Hz, 2H), 6.97 - 6.79 (m, 2H), 4.64 (s, 2H), 4.01 (s, 2H), 2.98 (s, 3H). LCMS: m/z 424 [M+H]+ |
| **5** | 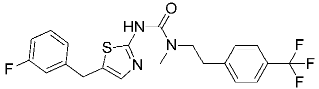 | 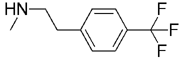 | ¹H NMR (400 MHz, CDCl3) δ 7.64 - 7.48 (m, 2H), 7.33 (d, J = 8.0 Hz, 2H), 7.29 - 7.18 (m, 1H), 7.01 (d, J = 10.7 Hz, 2H), 6.92 (t, J = 7.3 Hz, 2H), 4.01 (s, 2H), 3.71 - 3.52 (m, 2H), 3.02 - 2.81 (m, 5H). LCMS: m/z 438.1 [M+H]+ |
| **6** | 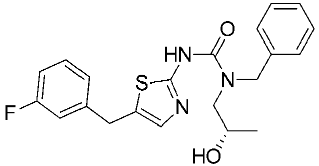 | 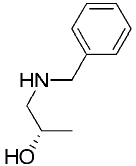 | ¹H NMR (400 MHz, CDCl3) δ 13.71 - 13.63 (m, 1H), 7.43 - 7.07 (m, 7H), 7.05 - 6.66 (m, 3H), 4.85 - 4.40 (m, 2H), 4.04 - 3.81 (m, 2H), 3.37 (dt, J = 26.9, 13.4 Hz, 1H), 3.15 (d, J = 15.5 Hz, 1H), 1.34 - 1.20 (m, 1H), 1.16 (t, J = 7.5 Hz, 3H). LCMS: m/z 400 [M+H]+ |
| **7** | 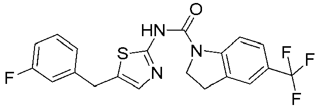 | 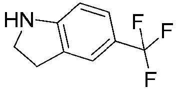 | ¹H NMR (400 MHz, CDCl3) δ 8.07 (d, J = 8.5 Hz, 1H), 7.43 (d, J = 8.6 Hz, 1H), 7.38 (s, 1H), 7.31 - 7.16 (m, 1H), 7.09 - 6.97 (m, 2H), 6.93 (t, J = 7.5 Hz, 2H), 4.17 - 4.06 (m, 2H), 4.02 (d, J = 8.8 Hz, 2H), 3.37 - 3.09 (m, 2H). LCMS: m/z 422.4 [M+H]+ |
| **8** | 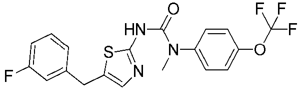 | 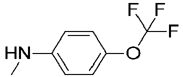 | ¹H NMR (400 MHz, CDCl3) δ 7.43 - 7.29 (m, 2H), 7.27 - 7.18 (m, 3H), 7.00 (s, 1H), 6.92 (t, J = 7.3 Hz, 1H), 6.82 (d, J = 8.5 Hz, 2H), 4.02 (s, 2H), 3.33 (s, 3H). LCMS: m/z 426 [M+H]+ |
| **9** | 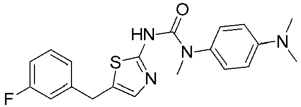 | 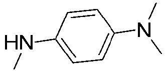 | ¹H NMR (400 MHz, CDCl3) δ 7.26 - 7.19 (m, 1H), 7.14 - 7.04 (m, 2H), 7.04 - 6.96 (m, 2H), 6.90 (t, J = 7.5 Hz, 2H), 6.71 (d, J = 8.9 Hz, 2H), 4.00 (s, 2H), 3.28 (d, J = 5.9 Hz, 3H), 2.98 (s, 6H). LCMS: m/z 385.1 [M+H]+ |
| **10** | 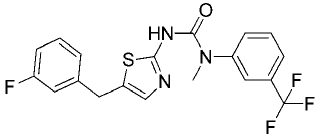 | 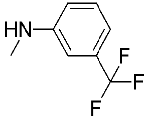 | ¹H NMR (400 MHz, CDCl3) δ 7.75 - 7.56 (m, 2H), 7.51 (dd, J = 18.1, 8.3 Hz, 2H), 7.25 (s, 1H), 7.07 - 6.81 (m, 4H), 4.02 (s, 2H), 3.43 (s, 3H). LCMS: m/z 410.1 [M+H]+ |
| **11** | 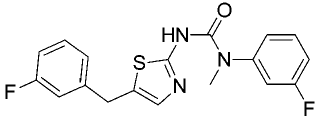 | 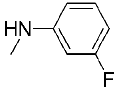 | ¹H NMR (400 MHz, CDCl3) δ 7.41 (td, J = 8.1, 6.3 Hz, 1H), 7.30 - 7.24 (m, 1H), 7.10 (d, J = 8.3 Hz, 1H), 7.06 (dd, J = 8.4, 2.4 Hz, 1H), 7.03 (d, J =7.2 Hz, 2H), 6.95 (s, 1H), 6.93 (dd, J = 5.0, 2.7 Hz, 2H), 4.03 (s, 2H), 3.36 (s, 3H). LCMS: m/z 360.23 [M+H]+ |

### [Example 12]

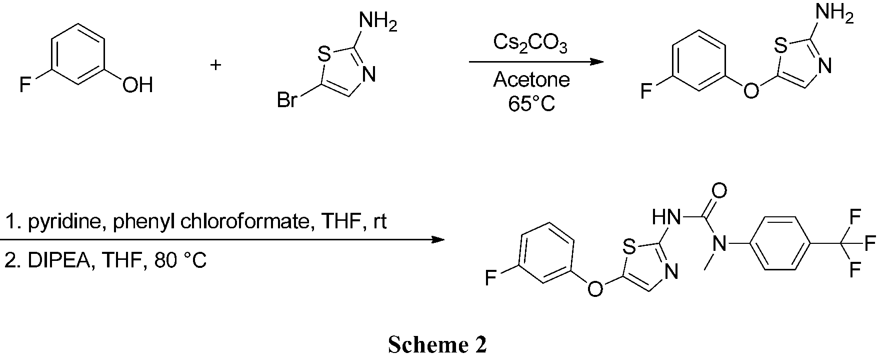

### Synthesis of 5-(3-fluorophenoxy)thiazol-2-amine

After dissolving 1.3 eq of 3-fluorophenol in acetone (30 mL/g), 2 eq of cesium carbonate was added thereto. After stirring for 10 minutes, 1 eq of 5-bromothiazol-2-amine was added thereto, and the mixture was heated to 65°C for 12 hours. After completion of the reaction, the mixture was filtered through Celite with acetone, concentrated, and extracted with ethyl acetate and water. The ethyl acetate layer was dried over magnesium sulfate, concentrated under reduced pressure, purified, and separated by column chromatography (hexane:ethyl acetate) to obtain 5-(3-fluorophenoxy)thiazol-2-amine.

### Synthesis of 3-(5-(3-fluorophenoxy)thiazol-2-yl)-1-methyl-1-(4-(trifluoromethyl)phenyl)urea (Example 12)

After dissolving 1 eq of 5-(3-fluorophenoxy)thiazol-2-amine in tetrahydrofuran (10 mL/g), 1.5 eq of pyridine was added thereto, and the mixture was cooled to 0°C. After slowly adding 1.2 eq of phenyl chloroformate, the mixture was slowly warmed to room temperature and stirred for 4 hours. 1.5 eq of N-methyl-4-(trifluoromethyl)aniline as **reactant B1** and 2 eq of N,N-diisopropylethylamine were added in situ, and the mixture was heated to 80°C. After completion of the reaction, the ethyl acetate layer was dried over magnesium sulfate, concentrated under reduced pressure, purified, and separated by column chromatography (hexane:ethyl acetate) to obtain the title **compound of Example 12.**

**[Table 3]**

| **No** | **Structure** | **Reactant B1** | **Analysis data** |
|---|---|---|---|
| **12** | 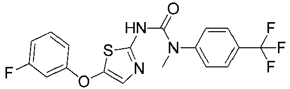 | 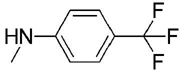 | ¹H NMR (400 MHz, CDCl3) δ 7.76 (d, J = 8.3 Hz, 2H), 7.47 (t, J = 13.0 Hz, 2H), 7.33 - 7.17 (m, 1H), 6.96 (d, J = 17.7 Hz, 1H), 6.90 - 6.84 (m, 1H), 6.79 (dd, J = 8.6, 7.7 Hz, 2H), 3.38 (s, 3H). LCMS: m/z 412.3 [M+H]+ |

### [Example 13]

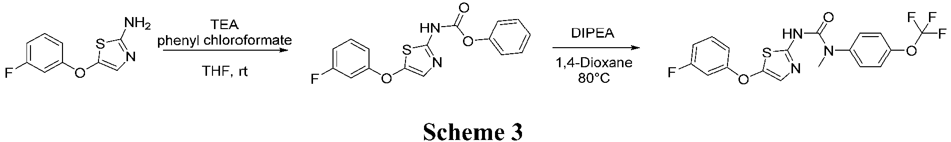

### Synthesis of phenyl (5-(3-fluorophenoxy)thiazol-2-yl)carbamate

After dissolving 5-(3-fluorophenoxy)thiazol-2-amine in tetrahydrofuran (10 mL/g), 1.5 eq of triethylamine was added thereto, and the mixture was cooled to 0°C. After slowly adding 1.2 eq of phenyl chloroformate, the mixture was slowly warmed to room temperature and stirred for 4 hours. After completion of the reaction, the mixture was extracted with ethyl acetate and water, dried over magnesium sulfate, and then concentrated under reduced pressure. The concentrate was purified and separated by column chromatography (hexane:ethyl acetate) to obtain phenyl (5-(3-fluorophenoxy)thiazol-2-yl)carbamate.

### Synthesis of 3-(5-(3-fluorophenoxy)thiazol-2-yl)-1-methyl-1-(4-(trifluoromethoxy)phenyl)urea (Example 13)

1 eq of phenyl (5-(3-fluorophenoxy)thiazol-2-yl)carbamate was dissolved in 1,4-dioxane (10 mL/g). 1.5 eq of N-methyl-4-(trifluoromethoxy)aniline as **reactant C1** and 2 eq of N,N-diisopropylethylamine were added thereto, and the mixture was heated to 80°C. After completion of the reaction, the mixture was extracted with ethyl acetate and water. The ethyl acetate layer was dried over magnesium sulfate, concentrated under reduced pressure, purified, and separated by column chromatography (hexane:ethyl acetate) to obtain the title **compound of Example 13.**

### [Examples 14-137]

The **compounds of Examples 14-137** were synthesized in substantially the same manner as described in the method for preparing the compound of Example 13 (**Scheme 3**) by using **reactant C1 of Table 4** instead of reactant C1 in the method for preparing the compound of Example 13.

The structures of the compounds of Examples 13-137, reactant C1, and analytical data are shown in Table 4.

**[Table 4]**

| **No** | **Structure** | **Reactant C1** | **Analysis data** |
|---|---|---|---|
| **13** | 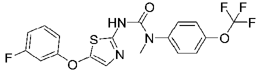 | 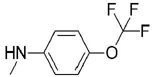 | ¹H NMR (400 MHz, MeOD) δ 7.48 (d, J = 8.9 Hz, 2H), 7.45 - 7.22 (m, 3H), 7.01 (d, J = 17.4 Hz, 1H), 6.97 - 6.73 (m, 3H), 3.35 (s, 3H). LCMS: m/z 428.3 [M+H]+ |
| **14** | 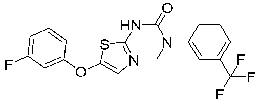 | 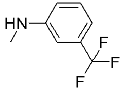 | ¹H NMR (400 MHz, CDCl3) δ 7.66 (dt, J = 15.5, 7.8 Hz, 2H), 7.58 (s, 1H), 7.55 - 7.41 (m, 1H), 7.28 (t, J = 6.7 Hz, 1H), 6.95 (t, J = 16.7 Hz, 1H), 6.90 - 6.69 (m, 3H), 3.39 (s, 3H). LCMS: m/z 412 [M+H]+ |
| **15** | 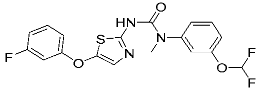 | 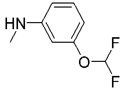 | ¹H NMR (400 MHz, DMSO-d6) δ 10.81 (s, 1H), 7.47 - 7.39 (m, 2H), 7.27 - 7.18 (m, 3H), 7.09 - 7.05 (m, 1H), 6.98 (ddt, J = 14.3, 7.5, 1.7 Hz, 3H), 3.32 (s, 3H), 3.29 (s, 1H). LCMS: m/z 410.1 [M+H]+ |
| **16** | 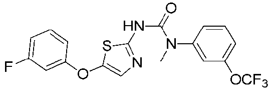 | 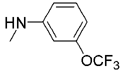 | ¹H NMR (400 MHz, CDCl3) δ 8.02 (s, 1H), 7.52 (t, J = 8.2 Hz, 1H), 7.29 - 7.24 (m, 3H), 7.19 (d, J = 2.4 Hz, 1H), 6.91 (s, 1H), 6.88 - 6.84 (m, 1H), 6.79 (td, J = 8.8, 7.6, 2.8 Hz, 2H), 3.36 (s, 3H). LCMS: m/z 428.1 [M+H]+ |
| **17** | 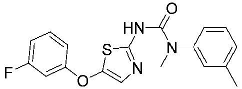 | 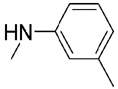 | ¹H NMR (400 MHz, CDCl3) δ 7.58 (s, 1H), 7.30 (td, J = 7.3, 1.6 Hz, 1H), 7.23 - 7.12 (m, 2H), 7.06 - 7.01 (m, 2H), 6.86 (s, 1H), 6.81 - 6.77 (m, 1H), 6.75 - 6.69 (m, 2H), 3.26 (s, 3H), 2.33 (s, 3H). LCMS: m/z 358.1 [M+H]+ |
| **18** | 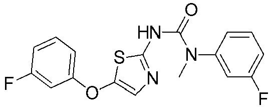 | 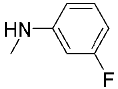 | ¹H NMR (400 MHz, CDCl3) δ 7.51 (td, J = 8.2, 6.3 Hz, 1H), 7.28 (s, 1H), 7.19 - 7.14 (m, 2H), 7.08 (dt, J = 9.1, 2.3 Hz, 1H), 6.98 (s, 1H), 6.92 - 6.87 (m, 1H), 6.86 - 6.80 (m, 2H), 3.38 (s, 3H). LCMS: m/z 362.1 [M+H]+ |
| **19** | 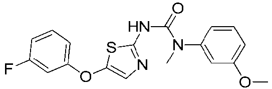 | 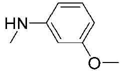 | ¹H NMR (400 MHz, CDCl3) δ 7.67 (s, 1H), 7.33 (t, J = 8.1 Hz, 1H), 7.24 -7.13 (m, 1H), 6.88 (ddd, J = 8.5, 2.6, 0.9 Hz, 1H), 6.86 (s, 1H), 6.84 - 6.78 (m, 2H), 6.76 - 6.68 (m, 3H), 3.77 (s, 3H), 3.27 (s, 3H). LCMS: m/z 374.1 [M+H]+ |
| **20** | 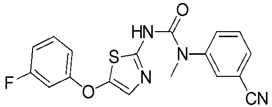 | 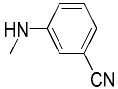 | ¹H NMR (400 MHz, CDCl3) δ 7.67 - 7.63 (m, 2H), 7.60 - 7.55 (m, 2H), 7.30 - 7.21 (m, 1H), 6.89 - 6.84 (m, 2H), 6.82 - 6.76 (m, 2H), 3.38 (s, 3H). LCMS: m/z 369.1 [M+H]+ |
| **21** | 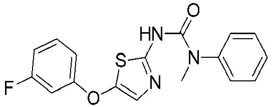 | 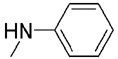 | ¹H NMR (400 MHz, CDCl3) δ 7.71 (s, 1H), 7.52 (dd, J = 8.4, 6.9 Hz, 2H), 7.48 - 7.41 (m, 1H), 7.36 - 7.32 (m, 2H), 7.31 - 7.23 (m, 1H), 6.94 (s, 1H), 6.91 - 6.87 (m, 1H), 6.82 (ddt, J = 9.3, 4.4, 2.1 Hz, 2H), 3.38 (s, 3H). LCMS: m/z 344.1 [M+H]+ |
| **22** | 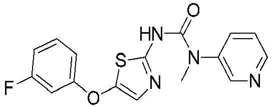 | 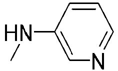 | ¹H NMR (400 MHz, CDCl3) δ 8.70 - 8.58 (m, 2H), 8.40 (s, 1H), 7.68 (ddd, J = 8.2, 2.6, 1.5 Hz, 1H), 7.42 (dd, J = 8.1, 4.7 Hz, 1H), 7.28 (td, J = 8.2, 7.7, 6.3 Hz, 1H), 6.88 (d, J = 8.6 Hz, 2H), 6.82 (tq, J = 10.1, 2.3, 1.6 Hz, 2H), 3.40 (s, 3H). LCMS: m/z 345.1 [M+H]+ |
| **23** | 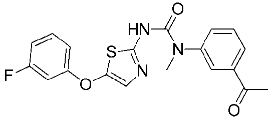 | 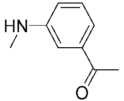 | ¹H NMR (400 MHz, CDCl3) δ 8.44 (s, 1H), 7.95 (d, J = 2.0 Hz, 1H), 7.89 (dp, J = 6.5, 1.6 Hz, 1H), 7.59 - 7.49 (m, 2H), 7.28 - 7.21 (m, 1H), 6.84 (dd, J = 8.4, 2.3 Hz, 1H), 6.82 - 6.71 (m, 3H), 3.36 (s, 3H), 2.64 (s, 3H). LCMS: m/z 386.1 [M+H]+ |
| **24** | 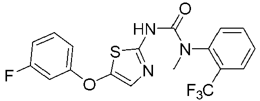 | 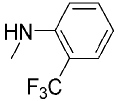 | ¹H NMR (400 MHz, CDCl3) δ 7.84 (dd, J = 8.0, 1.6 Hz, 1H), 7.72 (td, J = 7.7, 1.6 Hz, 1H), 7.65 - 7.58 (m, 1H), 7.43 (d, J = 7.8 Hz, 1H), 7.26 (td, J = 8.6, 6.5 Hz, 1H), 6.91 (s, 1H), 6.89 - 6.84 (m, 1H), 6.82 - 6.76 (m, 2H), 3.30 (s, 3H). LCMS: m/z 412.1 [M+H]+ |
| **25** | 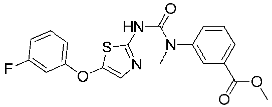 | 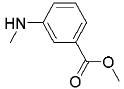 | ¹H NMR (400 MHz, DMSO-d6) δ 11.05 (s, 1H), 7.89 (t, J = 1.9 Hz, 1H), 7.84 (d, J = 7.7 Hz, 1H), 7.63 (ddd, J = 8.0, 2.2,1.2 Hz, 1H), 7.56 (t, J = 7.8 Hz, 1H), 7.43 (td, J = 8.3, 6.6 Hz, 1H), 7.21 (s, 1H), 7.04 - 6.95 (m, 3H), 3.87 (s, 3H), 3.35 (s, 3H). LCMS: m/z 402.1 [M+H]+ |
| **26** | 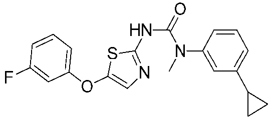 | 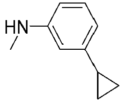 | ¹H NMR (400 MHz, CDCl3) δ 7.80 (s, 1H), 7.35 (t, J = 7.8 Hz, 1H), 7.30 - 7.17 (m, 1H), 7.06 (dd, J = 7.8, 2.0 Hz, 2H), 7.01 (d, J = 2.0 Hz, 1H), 6.92 (s, 1H), 6.88 - 6.84 (m, 1H), 6.82 - 6.75 (m, 2H), 3.32 (s, 3H), 1.91 (tt, J = 8.4, 5.1 Hz, 1H), 1.07 - 1.01 (m, 2H), 0.76 - 0.70 (m, 2H). LCMS: m/z 384.1 [M+H]+ |
| **27** | 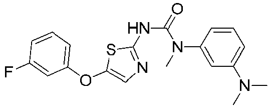 | 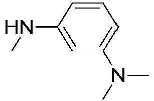 | ¹H NMR (400 MHz, CDCl3) δ 7.82 (s, 1H), 7.29 (dd, J = 8.5, 7.6 Hz, 1H), 7.26 -7.21 (m, 1H), 6.92 (s, 1H), 6.86 (ddd, J = 8.6, 1.8, 0.8 Hz, 1H), 6.81 - 6.75 (m, 2H), 6.70 (ddd, J = 8.5, 2.6, 0.9 Hz, 1H), 6.58 (ddd, J = 7.7, 2.0, 0.9 Hz, 1H), 6.53 (t, J = 2.3 Hz, 1H), 3.33 (s, 3H), 2.98 (s, 6H). LCMS: m/z 387.1 [M+H]+ |
| **28** | 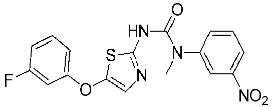 | 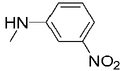 | ¹H NMR (400 MHz, CDCl3) δ 8.30 - 8.26 (m, 1H), 8.25 (q, J = 1.5 Hz, 1H), 7.74 - 7.69 (m, 2H), 7.30 (s, 1H), 6.93 (s, 1H), 6.91 - 6.87 (m, 1H), 6.84 - 6.79 (m, 2H), 3.45 (s, 3H). LCMS: m/z 389.1 [M+H]+ |
| **29** | 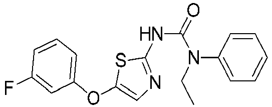 | 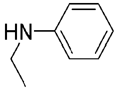 | ¹H NMR (400 MHz, CDCl3) δ 7.69 (s, 1H), 7.52 - 7.46 (m, 2H), 7.45 - 7.40 (m, 1H), 7.29 - 7.26 (m, 2H), 7.22 (d, J = 8.6 Hz, 1H), 6.89 (d, J = 1.5 Hz, 1H), 6.87 - 6.83 (m, 1H), 6.81 - 6.75 (m, 2H), 3.79 (q, J = 7.2 Hz, 2H), 1.16 (td, J = 7.1, 1.4 Hz, 3H). LCMS: m/z 358.1 [M+H]+ |
| **30** | 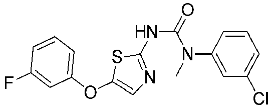 | 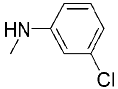 | ¹H NMR (400 MHz, CDCl3) δ 8.43 (s, 1H), 7.42 - 7.37 (m, 1H), 7.36 - 7.32 (m, 2H), 7.30 - 7.19 (m, 2H), 6.88 - 6.75 (m, 4H), 3.34 (s, 3H). LCMS: m/z 378.1 [M+H]+ |
| **31** | 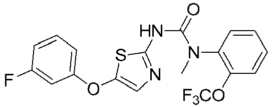 | 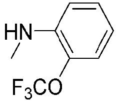 | ¹H NMR (400 MHz, CDCl3) δ 7.54 - 7.40 (m, 4H), 7.32 - 7.21 (m, 1H), 6.92 (s, 1H), 6.89 - 6.85 (m, 1H), 6.84 - 6.77 (m, 2H), 3.31 (s, 3H). LCMS: m/z 428.1 [M+H]+ |
| 32 | 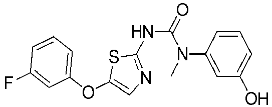 | 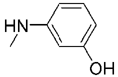 | ¹H NMR (400 MHz, CDCl3) δ 7.52 (s, 1H), 7.36 (s, 2H), 7.00 (s, 1H), 6.94 (d, J = 8.5 Hz, 2H), 6.88 - 6.79 (m, 3H), 3.76 (s, 3H). LCMS: m/z 360.1 [M+H]+ |
| **33** | 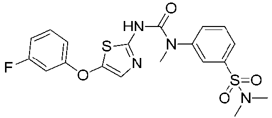 | 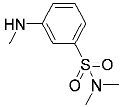 | ¹H NMR (400 MHz, CDCl3) δ 7.86 (d, J = 7.9 Hz, 1H), 7.75 - 7.70 (m, 2H), 7.57 - 7.51 (m, 1H), 7.31 (dd, J = 14.9, 8.3 Hz, 1H), 6.96 (s, 1H), 6.88 (dt, J = 8.2, 2.9 Hz, 2H), 6.83 - 6.79 (m, 1H), 3.42 (s, 3H), 2.81 (s, 6H). LCMS: m/z 451.10 [M+H]+ |
| **34** | 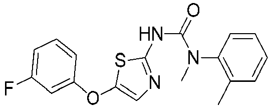 | 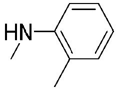 | ¹H NMR (400 MHz, DMSO-d6) δ 10.50 (s, 1H), 7.43 (dd, J = 15.5, 8.5 Hz, 1H), 7.35 - 7.20 (m, 4H), 7.16 (s, 1H), 7.03 - 6.93 (m, 3H), 3.18 (s, 3H), 2.16 (s, 3H). LCMS: m/z 358.24 [M+H]+ |
| **35** | 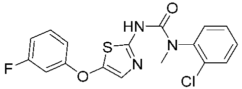 | 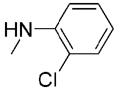 | ¹H NMR (400 MHz, DMSO-d6) δ 11.00 (s, 1H), 7.58 (dd, J = 7.3, 1.8 Hz, 1H), 7.50 - 7.36 (m, 4H), 7.18 (s, 1H), 7.04 - 6.93 (m, 3H), 3.19 (s, 3H). LCMS: m/z 378.13 [M+H]+ |
| **36** | 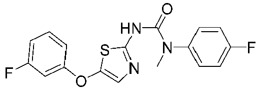 | 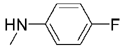 | ¹H NMR (400 MHz, DMSO-d6) δ 10.83 (s, 1H), 7.47 - 7.34 (m, 3H), 7.24 (t, J = 8.8 Hz, 2H), 7.19 (s, 1H), 7.04 - 6.93 (m, 3H), 3.28 (s, 3H). LCMS: m/z 362.16 [M+H]+ |
| **37** | 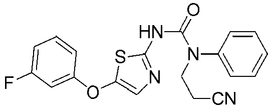 | 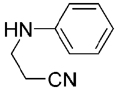 | ¹H NMR (400 MHz, DMSO-d6) δ 10.84 (m, 1H), 7.50 - 7.39 (m, 3H), 7.35 - 7.33 (m, 3H), 7.19 (s, 1H), 7.05 - 6.94 (m, 3H), 3.98 (t, J = 6.5 Hz, 2H), 2.75 (t, J = 6.6 Hz, 2H). LCMS: m/z 383.18 [M+H]+ |
| **38** | 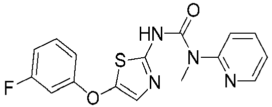 | 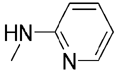 | ¹H NMR (400 MHz, DMSO-d6) δ 13.75 (s, 1H), 8.48 (d, J = 3.8 Hz, 1H), 7.96 (t, J = 7.4 Hz, 1H), 7.50 - 7.39 (m, 2H), 7.28 (s, 1H), 7.25 (dd, J = 7.0, 5.3 Hz, 1H), 7.08 - 6.95 (m, 3H), 3.43 (s, 3H). LCMS: m/z 345.17 [M+H]+ |
| **39** | 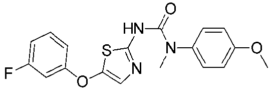 | 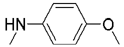 | ¹H NMR (400 MHz, DMSO-d6) δ 10.40 (s, 1H), 7.43 (dd, J = 15.8, 8.3 Hz, 1H), 7.28 - 7.22 (m, 2H), 7.17 (s, 1H), 7.05 - 6.91 (m, 5H), 3.78 (s, 3H), 3.24 (s, 3H). LCMS: m/z 374.18 [M+H]+ |
| **40** | 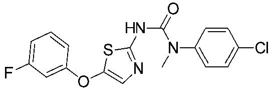 | 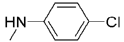 | ¹H NMR (400 MHz, DMSO-d6) δ 11.02 (s, 1H), 7.47 - 7.35 (m, 5H) 7.20 (s, 1H), 7.03 - 6.94 (m, 3H), 3.30 (s, 3H). LCMS: m/z 378.11 [M+H]+ |
| **41** | 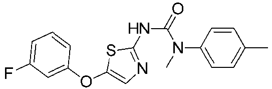 | 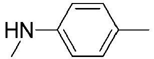 | ¹H NMR (400 MHz, DMSO-d6) δ 10.57 (s, 1H), 7.47 - 7.39 (m, 1H), 7.25 - 7.16 (m, 5H), 7.04 - 6.92 (m, 3H), 3.27 (s, 3H), 2.33 (s, 3H). LCMS: m/z 358.21 [M+H]+ |
| **42** | 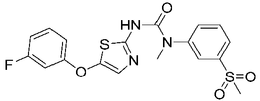 | 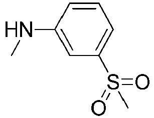 | ¹H NMR (400 MHz, CDCl3) δ 8.08 (s, 1H), 7.93 (d, J = 19.0 Hz, 2H), 7.63 (s, 1H), 7.54 (s, 1H), 7.02 (s, 2H), 6.87 (s, 2H), 3.62 (s, 3H), 3.14 (s, 3H). LCMS: m/z 422.12 [M+H]+ |
| **43** | 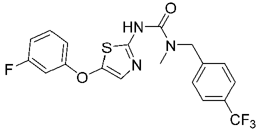 | 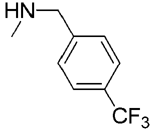 | ¹H NMR (400 MHz, CDCl3) δ 7.65 (d, J = 8.2 Hz, 2H), 7.41 (d, J = 7.6 Hz, 2H), 7.37 (d, J = 6.5 Hz, 1H), 7.07 (s, 1H), 6.95 - 6.91 (m, 2H), 6.88 - 6.84 (m, 1H), 4.72 (s, 2H), 3.18 (s, 3H). LCMS: m/z 426.17 [M+H]+ |
| **44** | 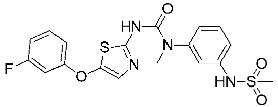 | 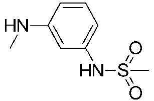 | ¹H NMR (400 MHz, CDCl3) δ 7.54 (s, 1H), 7.46 (s, 1H), 7.40 (d, J = 6.2 Hz, 1H), 7.35 (s, 1H), 7.11 (d, J = 7.7 Hz, 1H), 7.01 (s, 1H), 6.98 (s, 1H), 6.89 (d, J = 10.7 Hz, 2H), 3.42 (s, 3H), 3.29 (s, 3H). LCMS: m/z 437.14 [M+H]+ |
| **45** | 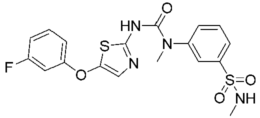 | 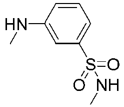 | ¹H NMR (400 MHz, DMSO-d6) δ 7.73 (s, 1H), 7.62 (s, 2H), 7.50 (q, J = 4.8 Hz, 1H), 7.44 (dd, J = 15.4, 8.4 Hz, 1H), 7.23 (s, 1H), 7.04 - 6.95 (m, 3H), 3.37 (s, 3H), 2.45 (d, J = 5.0 Hz, 3H). LCMS: m/z 437.17 [M+H]+ |
| **46** | 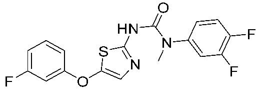 | 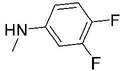 | ¹H NMR (400 MHz, DMSO-d6) δ 11.00 (s, 1H), 7.55 (ddd, J = 12.1, 7.4, 2.6 Hz, 1H), 7.51 - 7.39 (m, 2H), 7.24 - 7.21 (m, 1H), 7.20 (s, 1H), 7.04 - 6.94 (m, 3H), 3.29 (s, 3H). LCMS: m/z 380.19 [M+H]+ |
| **47** | 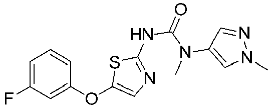 | 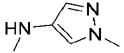 | 1H NMR (400 MHz, CDCl3) δ 7.57 (s, 1H), 7.54 - 7.49 (m, 1H), 7.34 (d d, J = 14.8, 8.3 Hz, 1H), 7.01 (s, 1H), 6.92 - 6.87 (m, 2H), 6.85 - 6.81 (m , 1H), 4.00 (s, 3H), 3.33 (s, 3H). LC MS: m/z 348.16 [M+H]+ |
| **48** | 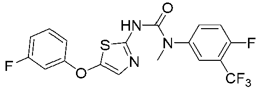 | 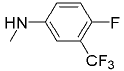 | ¹H NMR (400 MHz, DMSO-d6) δ 11.07 (s, 1H), 7.80 (dd, J = 6.4, 2.4 Hz, 1H), 7.77 - 7.69 (m, 1H), 7.55 (t, J = 9.7 Hz, 1H), 7.47 - 7.39 (m, 1H), 7.21 (s, 1H), 7.03 - 6.94 (m, 3H), 3.32 (s, 3H). LCMS: m/z 430.15 [M+H]+ |
| **49** | 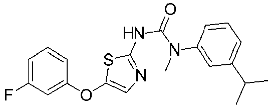 | 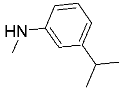 | ¹H NMR (400 MHz, DMSO-d6) δ 10.36 (s, 1H), 7.47 - 7.36 (m, 1H), 7.32 (t, J = 7.8 Hz, 1H), 7.16 (d, J = 5.7 Hz, 3H), 7.10 (d, J = 7.9 Hz, 1H), 7.01 - 6.92 (m, 3H), 3.27 (s, 3H), 2.93 - 2.84 (m, 1H), 1.20 (d, J = 6.9 Hz, 6H). LCMS: m/z 386.21 [M+H]+ |
| **50** | 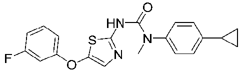 | 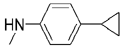 | ¹H NMR (400 MHz, DMSO) δ 10.50 (s, 1H), 7.43 (dd, J = 15.9, 7.9 Hz, 1 H), 7.19 (d, J = 8.6 Hz, 3H), 7.11 (d, J = 8.1 Hz, 2H), 6.98 (dd, J = 17.5, 7. 6 Hz, 3H), 3.25 (s, 3H), 1.99 - 1.90 ( m, 1H), 1.00 - 0.93 (m, 2H), 0.73 - 0 .65 (m, 2H). LCMS: m/z 384.13 [M+H]+ |
| **51** | 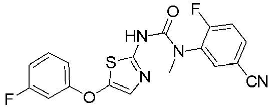 | 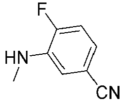 | ¹H NMR (400 MHz, DMSO-d6) δ 11.33 (s, 1H), 8.10 (d, J = 7.0 Hz, 1H), 7.89 (s, 1H), 7.56 (t, J = 9.4 Hz, 1H), 7.43 (dd, J = 15.3, 8.4 Hz, 1H), 7.19 (s, 1H), 6.99 (dd, J = 15.3, 8.4 Hz, 3H), 3.26 (s, 3H). LCMS: m/z 387.0 [M+H]+ |
| **52** | 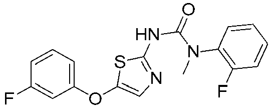 | 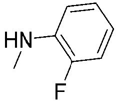 | ¹H NMR (400 MHz, DMSO-d6) δ 11.08 (s, 1H), 7.52 - 7.07 (m, 6H), 6.99 (dd, J = 14.6, 8.6 Hz, 3H), 3.24 (s, 3H). LCMS: m/z 362.1 [M+H]+ |
| **53** | 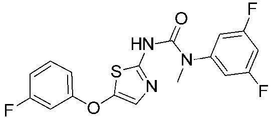 | 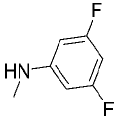 | ¹H NMR (400 MHz, DMSO-d6) δ 11.16 (s, 1H), 7.47 - 7.33 (m, 3H), 7.27 - 7.17 (m, 2H), 6.99 (dd, J = 15.1, 8.8 Hz, 3H), 3.24 (s, 3H). LCMS: m/z 380.0 [M+H]+ |
| **54** | 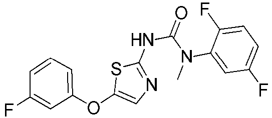 | 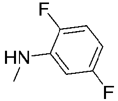 | ¹H NMR (400 MHz, DMSO-d6) δ 11.35 (s, 1H), 7.49 - 7.31 (m, 3H), 7.31 - 7.15 (m, 2H), 6.99 (dd, J = 15.2, 9.0 Hz, 3H), 3.25 (s, 3H). LCMS: m/z 380.0 [M+H]+ |
| **55** | 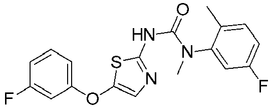 | 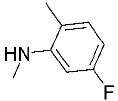 | ¹H NMR (400 MHz, DMSO-d6) δ 10.73 (s, 1H), 7.49 - 7.28 (m, 2H), 7.27 - 7.05 (m, 3H), 7.05 - 6.92 (m, 3H), 3.18 (s, 3H), 2.12 (s, 3H). LCMS: m/z 376.0 [M+H]+ |
| **56** | 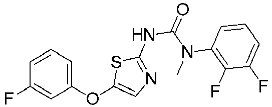 | 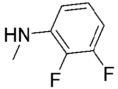 | ¹H NMR (400 MHz, DMSO-d6) δ 11.23 (s, 1H), 7.47 - 7.37 (m, 2H), 7.31 - 7.22 (m, 2H), 7.19 (s, 1H), 7.05 - 6.94 (m, 3H), 3.27 (s, 3H). LCMS: m/z 380.0 [M+H]+ |
| **57** | 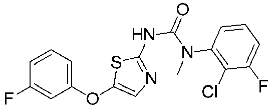 | 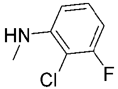 | ¹H NMR (400 MHz, DMSO-d6) δ 11.10 (s, 1H), 7.53 - 7.28 (m, 4H), 7.17 (s, 1H), 6.99 (dd, J = 15.1, 8.9 Hz, 3H), 3.21 (s, 3H). LCMS: m/z 396.0 [M+H]+ |
| **58** | 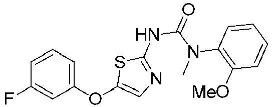 | 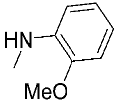 | ¹H NMR (400 MHz, DMSO-d6) δ 10.24 (s, 1H), 7.43 (dd, J = 16.0, 8.1 Hz, 1H), 7.35 (t, J = 7.8 Hz, 1H), 7.25 (d, J = 7.7 Hz, 1H), 7.13 (d, J = 5.5 Hz, 2H), 7.05 - 6.92 (m, 4H), 3.80 (s, 3H), 3.13 (s, 3H). LCMS: m/z 374.1 [M+H]+ |
| **59** | 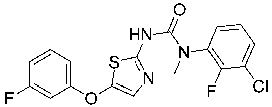 | 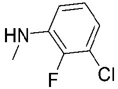 | ¹H NMR (400 MHz, DMSO-d6) δ 11.29 (s, 1H), 7.56 (t, J = 7.2 Hz, 1H), 7.43 (q, J = 7.7 Hz, 2H), 7.32 - 7.12 (m, 2H), 7.10 - 6.93 (m, 3H), 3.25 (s, 3H). LCMS: m/z 396.0 [M+H]+ |
| **60** | 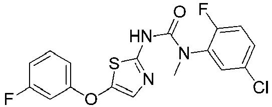 | 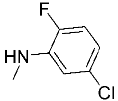 | ¹H NMR (400 MHz, DMSO-d6) δ 11.33 (s, 1H), 7.61 (d, J = 4.6 Hz, 1H), 7.52 - 7.31 (m, 3H), 7.19 (s, 1H), 6.99 (dd, J = 15.3, 8.5 Hz, 3H), 3.24 (s, 3H). LCMS: m/z 396.0 [M+H]+ |
| **61** | 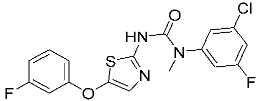 | 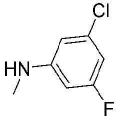 | ¹H NMR (400 MHz, DMSO-d6) δ 11.32 (s, 1H), 7.49 - 7.24 (m, 4H), 7.21 (s, 1H), 7.03 - 6.94 (m, 3H), 3.33 (s, 3H). LCMS: m/z 396.0 [M+H]+ |
| **62** | 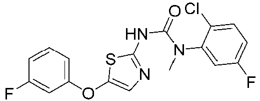 | 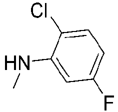 | ¹H NMR (400 MHz, DMSO-d6) δ 10.66 (s, 1H), 7.70 - 7.56 (m, 1H), 7.53 - 7.39 (m, 2H), 7.29 (s, 1H), 7.18 (s, 1H), 6.99 (dd, J = 16.3, 8.9 Hz, 3H), 3.19 (s, 3H). LCMS: m/z 396.0 [M+H]+ |
| **63** | 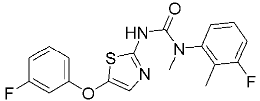 | 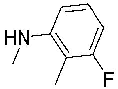 | ¹H NMR (400 MHz, DMSO-d6) δ 10.6 (s, 1H), 7.47 - 7.39 (m, 1H), 7.33 - 7.25 (m, 1H), 7.23 - 7.14 (m, 2H), 7.12 (d, J = 7.7 Hz, 1H), 7.04 - 6.93 (m, 3H), 3.19 (s, 3H), 2.06 (s, 3H). LCMS: m/z 376.0 [M+H]+ |
| **64** | 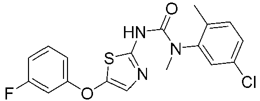 | 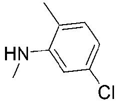 | ¹H NMR (400 MHz, DMSO-d6) δ 10.65 (s, 1H), 7.46 - 7.39 (m, 1H), 7.39 - 7.32 (m, 3H), 7.17 (s, 1H), 7.04 - 6.93 (m, 3H), 3.18 (s, 3H), 2.13 (s, 3H). LCMS: m/z 392.0 [M+H]+ |
| **65** | 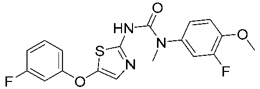 | 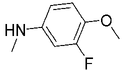 | ¹H NMR (400 MHz, DMSO-d6) δ 10.62 (s, 1H), 7.47 - 7.39 (m, 1H), 7.29 (d, J = 12.6 Hz, 1H), 7.23 - 7.15 (m, 2H), 7.15 - 7.09 (m, 1H), 7.04 - 6.92 (m, 3H), 3.86 (s, 3H), 3.24 (s, 3H). LCMS: m/z 392.0 [M+H]+ |
| **66** | 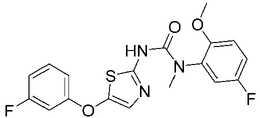 | 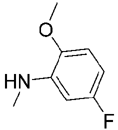 | ¹H NMR (400 MHz, DMSO-d6) δ 10.60 (s, 1H), 7.43 (q, J = 8.3 Hz, 1H), 7.25 - 7.10 (m, 4H), 7.04 - 6.90 (m, 3H), 3.78 (s, 3H), 3.13 (s, 3H). LCMS: m/z 392.0 [M+H]+ |
| **67** | 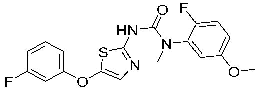 | 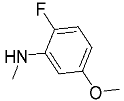 | ¹H NMR (400 MHz, DMSO-d6) δ 10.87 (s, 1H), 7.43 (dd, J = 15.6, 8.2 Hz, 1H), 7.29 - 7.11 (m, 2H), 7.08 - 6.82 (m, 5H), 3.76 (s, 3H), 3.24 (s, 3H). LCMS: m/z 392.1 [M+H]+ |
| **68** | 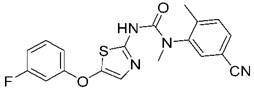 | 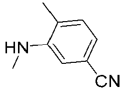 | ¹H NMR (400 MHz, DMSO-d6) δ 10.84 (s, 1H), 7.82 (s, 1H), 7.73 (d, J = 7.9 Hz, 1H), 7.53 (d, J = 8.0 Hz, 1H), 7.43 (dd, J = 15.8, 7.9 Hz, 1H), 7.17 (s, 1H), 6.98 (dd, J = 16.6, 8.9 Hz, 3H), 3.19 (s, 3H), 2.23 (s, 3H). LCMS: m/z 383.0 [M+H]+ |
| **69** | 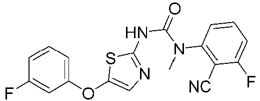 | 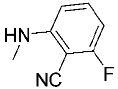 | ¹H NMR (400 MHz, DMSO-d6) δ 8.97 (d, J = 16.5 Hz, 1H), 7.75 - 7.46 (m, 3H), 7.32 - 6.95 (m, 5H), 3.45 (s, 3H). LCMS: m/z 387.0 [M+H]+ |
| **70** | 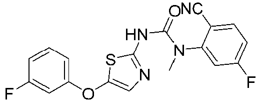 | 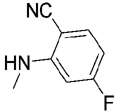 | ¹H NMR (400 MHz, DMSO-d6) δ 9.35 (s, 1H), 8.29 (s, 1H), 7.57 - 7.45 (m, 1H), 7.30 (d, J = 10.9 Hz, 2H), 7.21 - 7.02 (m, 4H), 3.43 (s, 3H). LCMS: m/z 387.1 [M+H]+ |
| **71** | 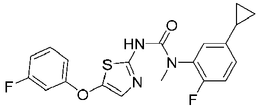 | 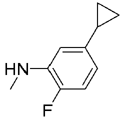 | ¹H NMR (400 MHz, DMSO-d6) δ 11.13 (s, 1H), 7.47 - 7.39 (m, 1 H), 7.19 - 7.10 (m, 3H), 7.07 - 6.92 (m, 4H), 3.21 (s, 3H), 1.98 - 1.89 (m , 1H), 0.98 - 0.91 (m, 2H), 0.72 - 0.6 3 (m, 2H). LCMS: m/z 402.22 [M+H]+ |
| **72** | 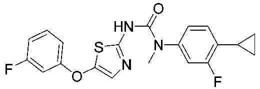 | 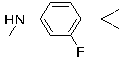 | ¹H NMR (400 MHz, DMSO-d6) δ 10.48 (s, 1H), 7.43 (dd, J = 15.5, 7.7 Hz, 1H), 7.32 - 7.23 (m, 1H), 7.16 (s, 1H), 7.08 - 6.92 (m, 4H), 6.81 (d, J = 10.0 Hz, 1H), 3.21 (s, 3H), 1.86 - 1.73 (m, 1H), 1.03 - 0.59 (m, 4H). LCMS: m/z 402.12 [M+H]+ |
| **73** | 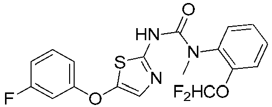 | 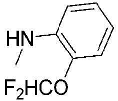 | ¹H NMR (400 MHz, DMSO-d6) δ 10.87 (s, 1H), 7.49 - 7.36 (m, 3H), 7.35 - 6.87 (m, 7H), 3.19 (s, 3H). LCMS: m/z 410.1 [M+H]+ |
| **74** | 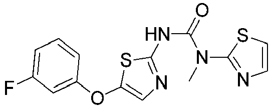 | 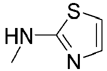 | ¹H NMR (400 MHz, DMSO-d6) δ 12.71 (s, 1H), 7.49 - 7.43 (m, 2H), 7.40 (s, 1H), 7.19 (s, 1H), 7.11 (d, J = 10.3 Hz, 1H), 7.07 - 7.00 (m, 2H), 3.68 (s, 3H). LCMS: m/z 351.0 [M+H]+ |
| **75** | 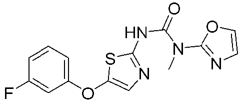 | 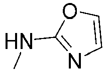 | ¹H NMR (400 MHz, DMSO-d6) δ 12.75 (s, 1H), 8.04 (s, 1H), 7.44 (q, *J* = 7.7 Hz, 1H), 7.31 (d, *J* = 4.6 Hz, 2H), 7.06 - 6.97 (m, 3H), 3.39 (s, 3H). LCMS: m/z 335.09 [M+H]+ |
| **76** | 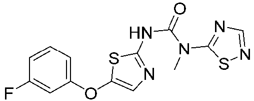 | 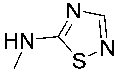 | ¹H NMR (400 MHz, DMSO-d6) δ 13.04 (s, 1H), 8.34 (s, 1H), 7.45 (d, *J* = 7.8 Hz, 2H), 7.12 (dd, *J* = 10.3, 2.9 Hz, 1H), 7.07 - 7.00 (m, 2H), 3.69 (s, 3H). LCMS: m/z 352.04 [M+H]+ |
| **77** | 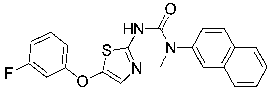 | 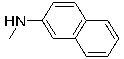 | ¹H NMR (400 MHz, DMSO-d6) δ 10.72 (s, 1H), 7.93 (t, J = 9.5 Hz, 3H), 7.89 - 7.84 (m, 1H), 7.57 - 7.51 (m, 2H), 7.50 - 7.45 (m, 1H), 7.44 - 7.38 (m, 1H), 7.18 (s, 1H), 7.03 - 6.93 (m, 3H), 3.39 (s, 3H). LCMS: m/z 394.1 [M+H]+ |
| **78** | 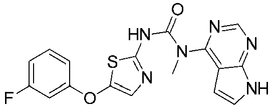 | 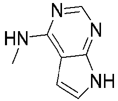 | ¹H NMR (400 MHz, DMSO-d6) δ 13.05 (s, 1H), 8.48 (s, 1H), 8.20 (d, J = 5.4 Hz, 1H), 7.68 (d, J = 3.9 Hz, 1H), 7.50 - 7.43 (m, 1H), 7.41 (s, 1H), 7.11 - 7.06 (m, 1H), 7.03 (dt, J = 8.3, 2.6 Hz, 2H), 6.88 (d, J = 3.9 Hz, 1H), 3.02 (d, J = 4.5 Hz, 3H). LCMS: m/z 385.1 [M+H]+ |
| **79** | 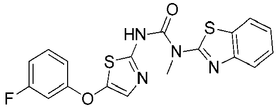 | 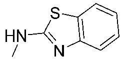 | ¹H NMR (400 MHz, DMSO-d6) δ 12.93 (s, 1H), 7.89 (d, J = 7.8 Hz, 1H), 7.74 (d, J = 8.0 Hz, 1H), 7.50 - 7.43 (m, 2H), 7.42 - 7.37 (m, 1H), 7.26 (t, J = 7.5 Hz, 1H), 7.13 (dt, J = 10.4, 2.5 Hz, 1H), 7.09 - 7.01 (m, 2H), 3.77 (s, 3H). LCMS: m/z 401.1 [M+H]+ |
| **80** | 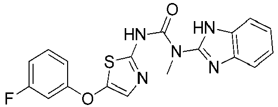 | 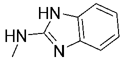 | ¹H NMR (400 MHz, DMSO-d6) δ 14.03 (s, 1H), 12.51 (s, 1H), 7.51 (s, 2H), 7.46 (td, J = 8.3, 6.8 Hz, 1H), 7.36 (s, 1H), 7.18 (s, 2H), 7.10 - 7.00 (m, 3H), 3.53 (s, 3H). LCMS: m/z 384.1 [M+H]+ |
| **81** | 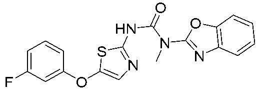 | 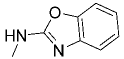 | ¹H NMR (400 MHz, Acetone-d6) δ 13.03 (s, 1H), 7.69 (dd, J = 7.9, 1.2 Hz, 1H), 7.67 - 7.63 (m, 1H), 7.49 - 7.39 (m, 2H), 7.35 (td, J = 7.8, 1.3 Hz, 1H), 7.25 (s, 1H), 7.00 (dd, J = 8.8, 2.1 Hz, 1H), 6.98 - 6.92 (m, 2H), 3.64 (s, 3H). LCMS: m/z 385.1 [M+H]+ |
| **82** | 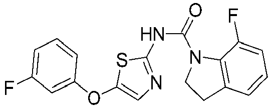 | 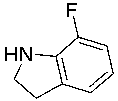 | ¹H NMR (400 MHz, CDCl3) δ 7.29 - 7.23 (m, 1H), 7.06 - 6.94 (m, 3H), 6.86 (dd, J = 8.3, 2.4 Hz, 1H), 6.83 (s, 1H), 6.82 - 6.75 (m, 2H), 4.27 (t, J = 8.1 Hz, 2H), 3.15 (t, J = 8.0 Hz, 2H). LCMS: m/z 374.1 [M+H]+ |
| **83** | 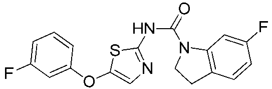 | 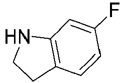 | ¹H NMR (400 MHz, CDCl3) δ 7.78 (dd, J = 10.5, 2.4 Hz, 1H), 7.35 - 7.27 (m, 1H), 7.14 - 7.09 (m, 1H), 7.07 (s, 1H), 6.94 - 6.90 (m, 1H), 6.88 - 6.81 (m, 2H), 6.71 (td, J = 8.5, 2.5 Hz, 1H), 4.14 (t, J = 8.5 Hz, 2H), 3.24 (t, J = 8.5 Hz, 2H). LCMS: m/z 374.1 [M+H]+ |
| **84** | 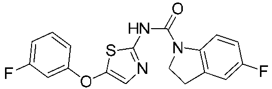 | 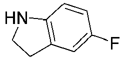 | ¹H NMR (400 MHz, CDCl3) δ 7.96 - 7.89 (m, 1H), 7.33 - 7.22 (m, 1H), 7.04 (s, 1H), 6.92 - 6.86 (m, 3H), 6.85 - 6.79 (m, 2H), 4.12 - 4.08 (m, 2H), 3.25 (t, J = 8.5 Hz, 2H). LCMS: m/z 374.1 [M+H]+ |
| **85** | 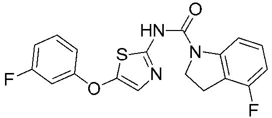 | 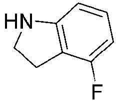 | ¹H NMR (400 MHz, CDCl3) δ 7.68 (d, J = 8.1 Hz, 1H), 7.26 - 7.16 (m, 1H), 7.10 (td, J = 8.2, 5.7 Hz, 1H), 6.96 (s, 1H), 6.85 - 6.79 (m, 1H), 6.75 (tt, J = 7.5, 2.0 Hz, 2H), 6.65 (t, J = 8.5 Hz, 1H), 4.06 (t, J = 8.6 Hz, 2H), 3.19 (t, J = 8.5 Hz, 2H). LCMS: m/z 374.1 [M+H]+ |
| **86** | 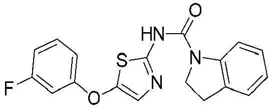 | 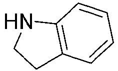 | ¹H NMR (400 MHz, DMSO-d6) δ 11.03 (s, 1H), 7.87 (s, 1H), 7.45 (td, J = 8.2, 6.7 Hz, 1H), 7.28 (s, 1H), 7.22 (d, J = 7.1 Hz, 1H), 7.15 (t, J = 7.7 Hz, 1H), 7.08 - 6.91 (m, 4H), 4.20 - 4.05 (m, 2H), 3.23 - 3.10 (m, 2H). LCMS: m/z 356.17 [M+H]+ |
| **87** | 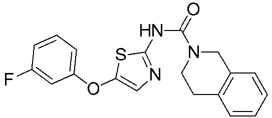 | 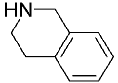 | ¹H NMR (400 MHz, CDCl3) δ 10.39 - 9.81 (m, 1H), 7.18 (dt, J = 8.7, 7.6 Hz, 1H), 7.15 - 7.05 (m, 3H), 7.03 - 6.97 (m, 1H), 6.93 (s, 1H), 6.83 - 6.78 (m, 1H), 6.77 - 6.69 (m, 2H), 4.59 (s, 2H), 3.67 (t, J = 5.9 Hz, 2H), 2.84 (t, J = 5.9 Hz, 2H). LCMS: m/z 370.1 [M+H]+ |
| **88** | 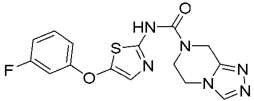 | 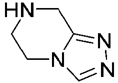 | ¹H NMR (400 MHz, DMSO-d6) δ 8.49 (s, 1H), 7.43 (dd, J = 15.3, 8.5 Hz, 1H), 7.25 (s, 1H), 7.06 - 6.92 (m, 3H), 4.89 (s, 2H), 4.11 (t, J = 5.2 Hz, 2H), 3.95 (s, 2H). LCMS: m/z 361.24 [M+H]+ |
| **89** | 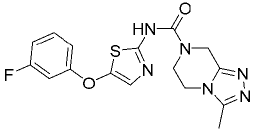 | 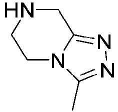 | ¹H NMR (400 MHz, DMSO-d6) δ 7.43 (dd, J = 15.4, 8.8 Hz, 1H), 7.25 (s, 1H), 6.99 (dd, J = 16.2, 8.7 Hz, 3H), 4.84 (s, 2H), 3.95 (s, 4H), 2.30 (s, 3H). LCMS: m/z 375.22 [M+H]+ |
| **90** | 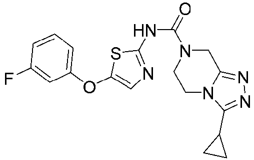 | 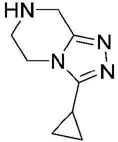 | ¹H NMR (400 MHz, DMSO-d6) δ 7.43 (dd, J = 15.3, 8.3 Hz, 1H), 7.25 (s, 1H), 6.99 (dd, J = 16.0, 8.5 Hz, 3H), 4.83 (s, 2H), 4.12 - 3.92 (m, 4H), 2.00 - 1.84 (m, 1H), 1.03 - 0.90 (m, 2H), 0.90 - 0.83 (m, 2H). LCMS: m/z 401.26 [M+H]+ |
| **91** | 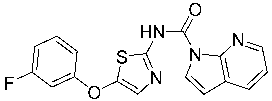 | 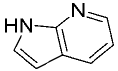 | ¹H NMR (400 MHz, DMSO-d6) δ 13.10 (s, 1H), 8.56 (d, J = 4.6 Hz, 1H), 8.27 (d, J = 7.6 Hz, 1H), 8.06 (d, J = 4.0 Hz, 1H), 7.53 - 7.33 (m, 3H), 7.12 - 7.00 (m, 3H), 6.99 - 6.84 (m, 1H). LCMS: m/z 355.1 [M+H]+ |
| **92** | 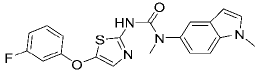 | 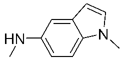 | ¹H NMR (400 MHz, DMSO-d6) δ 9.86 (s, 1H), 7.51 - 7.36 (m, 3H), 7.21 (t, J = 7.8 Hz, 1H), 7.11 (s, 1H), 7.05 - 6.91 (m, 4H), 6.30 (d, J = 3.0 Hz, 1H), 3.84 (s, 3H), 3.33 (s, 3H). LCMS: m/z 397.20 [M+H]+ |
| **93** | 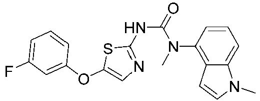 | 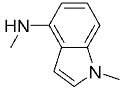 | ¹H NMR (400 MHz, DMSO-d6) δ 9.85 (s, 1H), 7.50 (d, J = 8.5 Hz, 2H), 7.47 - 7.39 (m, 2H), 7.15 - 7.07 (m, 2H), 7.03 - 6.93 (m, 3H), 6.46 (d, J = 2.9 Hz, 1H), 3.83 (s, 3H), 3.29 (s, 3H). LCMS: m/z 397.19 [M+H]+ |
| **94** | 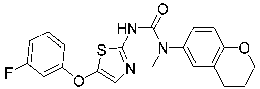 | 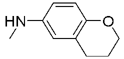 | ¹H NMR (400 MHz, DMSO-d6) δ 10.24 (s, 1H), 7.43 (dd, J = 15.5, 8.1 Hz, 1H), 7.15 (s, 1H), 7.03 - 6.93 (m, 5H), 6.76 (d, J = 8.5 Hz, 1H), 4.20 - 4.10 (m, 2H), 3.21 (s, 3H), 2.75 (t, J = 6.3 Hz, 2H), 1.93 (dd, J = 11.1, 5.9 Hz, 2H). LCMS: m/z 400.15 [M+H]+ |
| **95** | 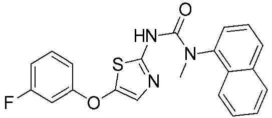 | 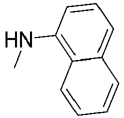 | ¹H NMR (400 MHz, DMSO-d6) δ 10.59 (s, 1H), 8.05 - 8.01 (m, 1H), 7.96 (d, J = 8.1 Hz, 1H), 7.77 - 7.70 (m, 1H), 7.63 - 7.55 (m, 3H), 7.52 (d, J = 7.2 Hz, 1H), 7.47 - 7.39 (m, 1H), 7.11 (s, 1H), 7.02 - 6.92 (m, 3H), 3.33 (s, 3H). LCMS: m/z 394.17 [M+H]+ |
| **96** | 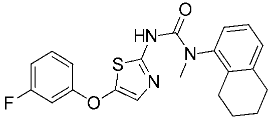 | 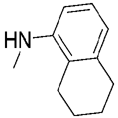 | ¹H NMR (400 MHz, DMSO-d6) δ 10.02 (s, 1H), 7.47 - 7.35 (m, 1H), 7.20 - 7.12 (m, 2H), 7.08 (d, J = 7.3 Hz, 1H), 7.05 - 6.91 (m, 4H), 3.13 (s, 2H), 2.76 (s, 2H), 2.51 (s, 3H), 1.77 - 1.67 (m, 4H). LCMS: m/z 398.17 [M+H]+ |
| **97** | 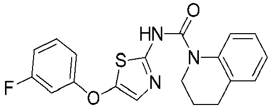 | 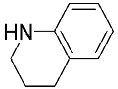 | ¹H NMR (400 MHz, CDCl3) δ 9.05 (s, 1H), 7.37 (dd, J = 8.0, 1.2 Hz, 1H), 7.33 - 7.19 (m, 3H), 7.15 (td, J = 7.4, 1.2 Hz, 1H), 6.90 - 6.87 (m, 1H), 6.86 (s, 1H), 6.84 - 6.77 (m, 2H), 3.85 (t, J = 6.4 Hz, 2H), 2.79 (t, J = 6.7 Hz, 2H), 2.01 (p, J = 6.6 Hz, 2H). LCMS: m/z 370.1 [M+H]+ |
| **98** | 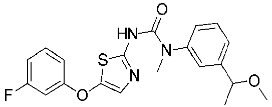 | 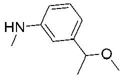 | ¹H NMR (400 MHz, DMSO-d6) δ 10.81 (s, 1H), 7.48 - 7.36 (m, 2H), 7.27 - 7.18 (m, 4H), 7.03 - 6.94 (m, 3H), 4.33 (q, J = 6.3 Hz, 1H), 3.32 (s, 3H), 3.16 (s, 3H), 1.35 (d, J = 6.4 Hz, 3H). LCMS: m/z 402.22 [M+H]+ |
| **99** | 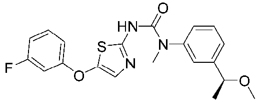 | 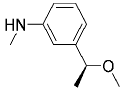 | ¹H NMR (400 MHz, DMSO-d6) δ 10.85 (s, 1H), 7.48 - 7.33 (m, 2H), 7.24 (d, J = 8.1 Hz, 2H), 7.20 - 7.11 (m, 2H), 7.03 - 6.91 (m, 3H), 4.38 - 4.26 (m, 1H), 3.32 (s, 3H), 3.16 (s, 3H), 1.35 (d, J = 6.3 Hz, 3H). LCMS: m/z 402.17 [M+H]+ |
| **100** | 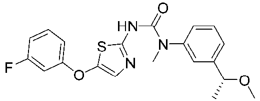 | 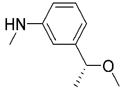 | ¹H NMR (400 MHz, DMSO-d6) δ 10.65 (s, 1H), 7.48 - 7.36 (m, 2 H), 7.27 - 7.16 (m, 4H), 7.04 - 6.93 (m, 3H), 4.33 (q, J = 6.0 Hz, 1H), 3.3 2 (s, 3H), 3.16 (s, 3H), 1.35 (d, J = 6. 2 Hz, 3H). LCMS: m/z 402.33 [M+H]+ |
| **101** | 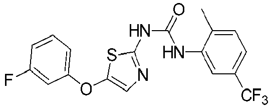 | 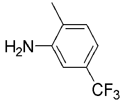 | ¹H NMR (400 MHz, DMSO-d6) δ 11.02 (s, 1H), 8.52 (s, 1H), 8.29 (s, 1H), 7.48 - 7.41 (m, 2H), 7.39 - 7.32 (m, 1H), 7.27 (s, 1H), 7.05 - 6.95 (m, 3H), 2.34 (s, 3H). LCMS: m/z 412.1 [M+H]+ |
| **102** | 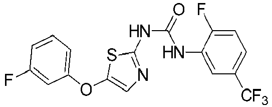 | 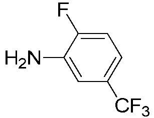 | ¹H NMR (400 MHz, DMSO-d6) δ 10.88 (s, 1H), 9.21 (s, 1H), 8.51 (dd, J = 7.2, 2.3 Hz, 1H), 7.59 - 7.52 (m, 1H), 7.51 -7.42 (m, 2H), 7.28 (s, 1H), 7.06 - 6.98 (m, 3H). LCMS: m/z 416.1 [M+H]+ |
| **103** | 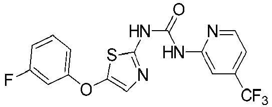 | 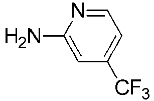 | ¹H NMR (400 MHz, Acetone-d6) δ 11.74 (s, 1H), 9.68 (s, 1H), 8.65 (d, J = 5.3 Hz, 1H), 7.88 (s, 1H), 7.49 - 7.39 (m, 2H), 7.21 (s, 1H), 7.02 - 6.87 (m, 3H). LCMS: m/z 399.1 [M+H]+ |
| **104** | 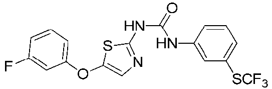 | 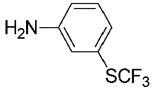 | ¹H NMR (400 MHz, CDCl3) δ 7.81 (s, 1H), 7.61 - 7.54 (m, 1H), 7.41 - 7.34 (m, 2H), 7.33 - 7.27 (m, 1H), 7.04 (s, 1H), 6.91 - 6.86 (m, 1H), 6.85f - 6.78 (m, 2H). LCMS: m/z 430.0 [M+H]+ |
| **105** | 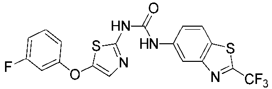 | 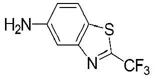 | ¹H NMR (400 MHz, DMSO-d6) δ 10.75 (s, 1H), 9.38 (s, 1H), 8.45 (s, 1H), 8.27 (d, J = 8.9 Hz, 1H), 7.72 (d, J = 7.7 Hz, 1H), 7.45 (dd, J = 15.2, 8.0 Hz, 1H), 7.27 (s, 1H), 7.09 - 6.96 (m, 3H). LCMS: m/z 455.08 [M+H]+ |
| **106** | 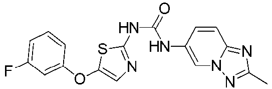 | 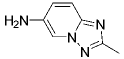 | ¹H NMR (400 MHz, DMSO-d6) δ 10.91 (s, 1H), 9.21 (s, 1H), 9.10 (s, 1H), 7.68 (d, J = 9.5 Hz, 1H), 7.56 (dd, J = 9.5, 1.7 Hz, 1H), 7.45 (dd, J = 15.1, 8.1 Hz, 1H), 7.26 (s, 1H), 7.07 - 6.96 (m, 3H), 2.45 (s, 3H). LCMS: m/z 385.16 [M+H]+ |
| **107** | 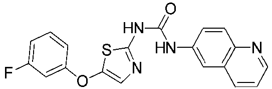 | 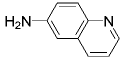 | ¹H NMR (400 MHz, DMSO-d6) δ 11.53 (s, 1H), 10.15 (s, 1H), 8.83 (d, J = 2.9 Hz, 1H), 8.27 (s, 2H), 7.91 (d, J = 8.8 Hz, 1H), 7.67 (d, J = 8.9 Hz, 1H), 7.50 - 7.35 (m, 2H), 7.24 (s, 1H), 7.08 - 6.96 (m, 3H). LCMS: m/z 381.12 [M+H]+ |
| **108** | 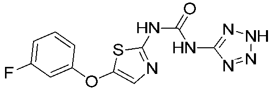 | 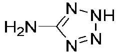 | ¹H NMR (400 MHz, DMSO-d6) δ 16.18 - 15.25 (m, 1H), 10.95 - 10.61 (m, 1H), 7.45 (dd, J = 15.1, 8.2 Hz, 1H), 7.33 (s, 1H), 7.13 - 6.96 (m, 3H), 6.88 (s, 1H). LCMS: m/z 322.0 [M+H]+ |
| **109** | 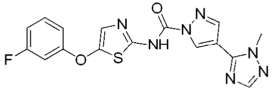 | 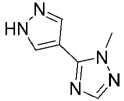 | ¹H NMR (400 MHz, CDCl3) δ 8.14 (s, 1H), 7.35 - 7.26 (m, 3H), 7.08 (s, 1H), 6.93 - 6.89 (m, 1H), 6.85 (t, J = 7.9 Hz, 2H), 3.89 (s, 3H). LCMS: m/z 386.1 [M+H]+ |
| **110** | 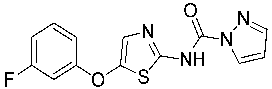 | 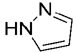 | ¹H NMR (400 MHz, CDCl3) δ 8.31 (d, J = 2.8 Hz, 1H), 7.76 (s, 1H), 7.36 - 7.29 (m, 1H), 7.20 (s, 1H), 6.93 (t, J = 7.7 Hz, 1H), 6.90 - 6.83 (m, 2H), 6.54 (dd, J = 2.6, 1.6 Hz, 1H). LCMS: m/z 303.00 [M-H]- |
| **111** | 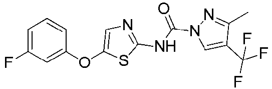 | 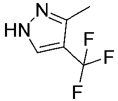 | ¹H NMR (400 MHz, CDCl3) δ 7.27 - 7.20 (m, 1H), 7.10 (s, 1H), 6.87 - 6.81 (m, 1H), 6.80 - 6.72 (m, 2H), 6.26 (s, 1H), 2.31 (s, 3H). LCMS: m/z 387.0 [M+H]+ |
| **112** | 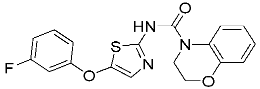 | 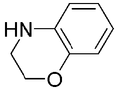 | ¹H NMR (400 MHz, DMSO-d6) δ 11.86 (s, 1H), 7.84 (s, 1H), 7.45 (dd, J = 15.2, 8.1 Hz, 1H), 7.29 (s, 1H), 7.08 - 6.94 (m, 4H), 6.89 - 6.85 (m, 2H), 4.30 - 4.19 (m, 2H), 4.03 - 3.95 (m, 2H). LCMS: m/z 372.18 [M+H]+ |
| **113** | 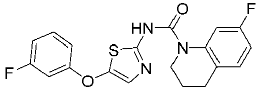 | 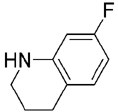 | ¹H NMR (400 MHz, DMSO-d6) δ 11.65 (s, 1H), 7.52 (s, 1H), 7.44 (dd, J = 15.2, 8.1 Hz, 1H), 7.29 (s, 1H), 7.17 - 7.12 (m, 1H), 7.07 - 6.98 (m, 3H), 6.83 (td, J = 8.4, 2.6 Hz, 1H), 3.83 (t, J = 5.7 Hz, 2H), 2.72 (t, J = 6.4 Hz, 2H), 1.91 - 1.83 (m, 2H). LCMS: m/z 388.19 [M+H]+ |
| **114** | 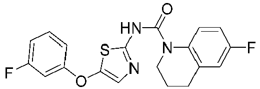 | 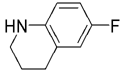 | ¹H NMR (400 MHz, DMSO-d6) δ 11.44 (s, 1H), 7.56 (s, 1H), 7.44 (dd, J = 15.1, 8.0 Hz, 1H), 7.26 (s, 1H), 7.05 - 6.94 (m, 5H), 3.80 (t, J = 5.9 Hz, 2H), 2.74 (t, J = 6.5 Hz, 2H), 1.91 - 1.82 (m, 2H). LCMS: m/z 388.18 [M+H]+ |
| **115** | 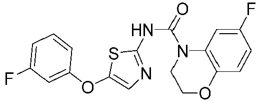 | 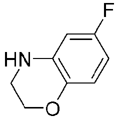 | ¹H NMR (400 MHz, DMSO-d6) δ 12.15 (s, 1H), 7.90 (s, 1H), 7.45 (dd, J = 15.2, 8.2 Hz, 1H), 7.33 (s, 1H), 7.09 - 6.98 (m, 3H), 6.88 (dd, J = 9.0, 5.8 Hz, 1H), 6.84 - 6.75 (m, 1H), 4.25 - 4.18 (m, 2H), 4.02 (s, 2H). LCMS: m/z 390.16 [M+H]+ |
| **116** | 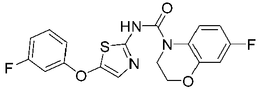 | 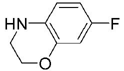 | ¹H NMR (400 MHz, DMSO-d6) δ 11.92 (s, 1H), 7.86 (s, 1H), 7.44 (dd, J = 15.1, 8.2 Hz, 1H), 7.29 (s, 1H), 7.08 - 6.96 (m, 3H), 6.73 (ddd, J = 11.7, 9.4, 2.9 Hz, 2H), 4.31 - 4.23 (m, 2H), 4.00 (s, 2H). LCMS: m/z 390.19 [M+H]+ |
| **117** | 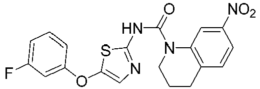 | 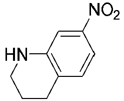 | ¹H NMR (400 MHz, DMSO-d6) δ 11.90 (s, 1H), 8.66 (s, 1H), 7.83 (dd, J = 8.4, 2.1 Hz, 1H), 7.49 - 7.37 (m, 2H), 7.32 (s, 1H), 7.10 - 6.98 (m, 3H), 3.97 - 3.87 (m, 2H), 2.88 (t, J = 6.4 Hz, 2H), 1.95 - 1.86 (m, 2H). LCMS: m/z 415.20 [M+H]+ |
| **118** | 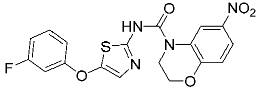 | 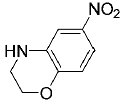 | ¹H NMR (400 MHz, DMSO-d6) δ 12.29 (s, 1H), 9.03 (s, 1H), 7.87 (dd, J = 9.0, 2.8 Hz, 1H), 7.45 (dd, J = 15.1, 8.3 Hz, 1H), 7.35 (s, 1H), 7.12 - 6.98 (m, 4H), 4.43 - 4.35 (m, 2H), 4.12 (s, 2H). LCMS: m/z 415.24 [M-H]- |
| **119** | 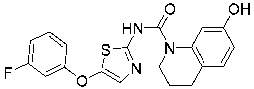 | 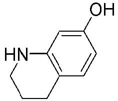 | ¹H NMR (400 MHz, DMSO-d6) δ 11.32 (s, 1H), 9.17 (s, 1H), 7.44 (dd, J = 15.2, 8.1 Hz, 1H), 7.26 (s, 1H), 7.07 - 6.95 (m, 4H), 6.91 (d, J = 8.3 Hz, 1H), 6.44 (dd, J = 8.2, 2.3 Hz, 1H), 3.74 (d, J = 5.3 Hz, 2H), 2.62 (t, J = 6.5 Hz, 2H), 1.88 - 1.79 (m, 2H). LCMS: m/z 386.25 [M+H]+ |
| **120** | 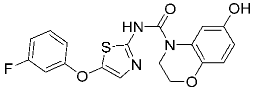 | 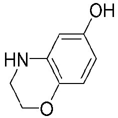 | ¹H NMR (400 MHz, DMSO-d6) δ 11.82 (s, 1H), 8.90 (s, 1H), 7.45 (dd, J = 15.2, 8.2 Hz, 1H), 7.29 (s, 1H), 7.07 - 6.97 (m, 3H), 6.66 (d, J = 8.7 Hz, 1H), 6.38 (dd, J = 8.7, 2.7 Hz, 1H), 4.18 - 4.12 (m, 2H), 3.94 (s, 2H). LCMS: m/z 388.17[M+H]+ |
| **121** | 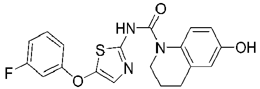 | 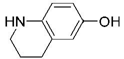 | ¹H NMR (400 MHz, DMSO-d6) δ 11.07 (s, 1H), 9.19 (s, 1H), 7.43 (dd, J = 15.4, 8.3 Hz, 1H), 7.28 (d, J = 8.2 Hz, 1H), 7.23 (s, 1H), 7.04 - 6.95 (m, 3H), 6.55 (dd, J = 11.7, 3.0 Hz, 2H), 3.73 (t, J = 5.9 Hz, 2H), 2.64 (t, J = 6.6 Hz, 2H), 1.87 - 1.78 (m, 2H). LCMS: m/z 386.22 [M+H]+ |
| **122** | 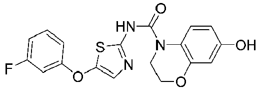 | 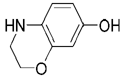 | ¹H NMR (400 MHz, DMSO-d6) δ 11.61 (s, 1H), 9.26 (s, 1H), 7.54 (s, 1H), 7.44 (dd, J = 15.1, 8.2 Hz, 1H), 7.26 (s, 1H), 7.07 - 6.96 (m, 3H), 6.31 (dd, J = 8.9, 2.6 Hz, 1H), 6.25 (d, J = 2.6 Hz, 1H), 4.23 - 4.16 (m, 2H), 3.92 (s, 2H). LCMS: m/z 388.19 [M+H]+ |
| **123** | 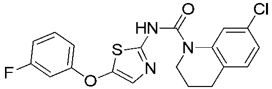 | 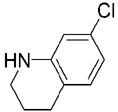 | ¹H NMR (400 MHz, CDCl3) δ 10.46 (s, 1H), 7.60 (d, J = 1.8 Hz, 1H), 7.30 (q, J = 8.1 Hz, 1H), 7.10 (d, J = 8.2 Hz, 1H), 7.04 (dd, J = 8.2, 1.9 Hz, 1H), 6.91-6.75 (m, 3H), 6.55 (s, 1H), 3.84 (t, J = 6.4 Hz, 2H), 2.76 (t, J = 6.6 Hz, 2H), 2.18 - 1.86 (m, 2H). LCMS: m/z 402.20 [M-H]⁻ |
| **124** | 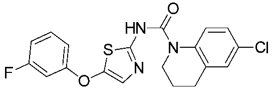 | 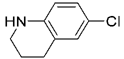 | ¹H NMR (400 MHz, CDCl3) δ 8.93 (s, 1H), 7.39 - 7.14 (m, 4H), 6.94 - 6.76 (m, 4H), 3.85 (q, J = 6.6 Hz, 2H), 2.78 (t, J = 6.6 Hz, 2H), 2.10 - 1.94 (m, 2H). LCMS: m/z 404.1 [M+H]+ |
| **125** | 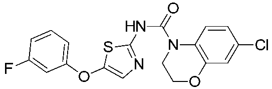 | 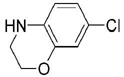 | ¹H NMR (400 MHz, CDCl3) δ 9.37 (s, 1H), 7.31 (ddd, J = 10.7, 8.2, 6.5 Hz, 2H), 7.00 (d, J = 2.3 Hz, 1H), 6.97 - 6.71 (m, 5H), 4.40 - 4.27 (m, 2H), 4.05 - 3.91 (m, 2H). LCMS: m/z 406.0 [M+H]+ |
| **126** | 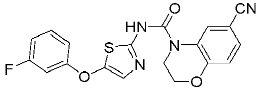 | 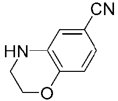 | ¹H NMR (400 MHz, DMSO-d6) δ 12.18 (s, 1H), 8.41 (s, 1H), 7.49 - 7.39 (m, 2H), 7.34 (s, 1H), 7.09 - 6.99 (m, 4H), 4.34 (t, J = 4.3 Hz, 2H), 4.06 (s, 2H). LCMS: m/z 397.15 [M+H]+ |
| **127** | 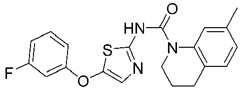 | 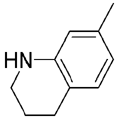 | ¹H NMR (400 MHz, DMSO-d6) δ 11.35 (s, 1H), 7.44 (dd, J = 15.1, 8.1 Hz, 1H), 7.35 (s, 1H), 7.25 (s, 1H), 7.05 - 6.97 (m, 4H), 6.83 (d, J = 7.5 Hz, 1H), 3.77 (t, J = 5.9 Hz, 2H), 2.68 (t, J = 6.5 Hz, 2H), 2.25 (s, 3H), 1.90 - 1.82 (m, 2H). LCMS: m/z 384.18 [M+H]+ |
| **128** | 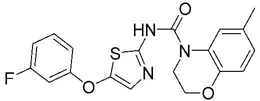 | 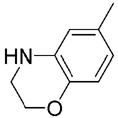 | ¹H NMR (400 MHz, DMSO-d6) δ 11.82 (s, 1H), 7.66 (s, 1H), 7.45 (dd, J = 15.2, 8.2 Hz, 1H), 7.30 (s, 1H), 7.09 - 6.98 (m, 3H), 6.81 - 6.72 (m, 2H), 4.24 - 4.17 (m, 2H), 3.96 (s, 2H), 2.22 (s, 3H). LCMS: m/z 386.22 [M+H]+ |
| **129** | 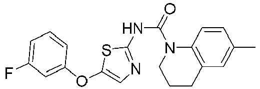 | 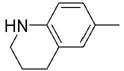 | ¹H NMR (400 MHz, DMSO-d6) δ 11.27 (s, 1H), 7.47 - 7.39 (m, 2H), 7.24 (s, 1H), 7.05 - 6.91 (m, 5H), 3.81 - 3.73 (m, 2H), 2.69 (t, J = 6.5 Hz, 2H), 2.24 (s, 3H), 1.90 - 1.82 (m, 2H). LCMS: m/z 384.22 [M+H]+ |
| **130** | 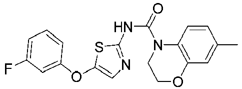 | 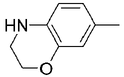 | ¹H NMR (400 MHz, DMSO-d6) δ 11.82 (s, 1H), 7.71 (s, 1H), 7.49 - 7.40 (m, 1H), 7.27 (s, 1H), 7.06 - 6.97 (m, 3H), 6.70 - 6.65 (m, 2H), 4.26 - 4.18 (m, 2H), 3.96 (s, 2H), 2.22 (s, 3H). LCMS: m/z 386.21 [M+H]+ |
| **131** | 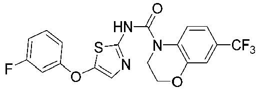 | 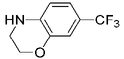 | ¹H NMR (400 MHz, DMSO-d6) δ 12.20 (s, 1H), 8.20 (s, 1H), 7.45 (dd, J = 15.2, 8.2 Hz, 1H), 7.34 (s, 1H), 7.23 - 7.18 (m, 2H), 7.09 - 6.99 (m, 3H), 4.33 - 4.28 (m, 2H), 4.07 (s, 2H). LCMS: m/z 440.13 [M+H]+ |
| **132** | 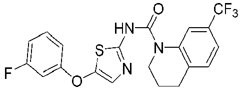 | 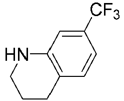 | ¹H NMR (400 MHz, DMSO-d6) δ 11.72 (s, 1H), 8.04 (s, 1H), 7.44 (dd, J = 15.2, 8.2 Hz, 1H), 7.38 - 7.34 (m, 1H), 7.32 - 7.29 (m, 2H), 7.08 - 6.98 (m, 3H), 3.89 (s, 2H), 2.82 (t, J = 6.4 Hz, 2H), 1.97 - 1.84 (m, 2H). LCMS: m/z 438.15 [M+H]+ |
| **133** | 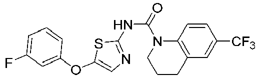 | 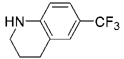 | ¹H NMR (400 MHz, DMSO-d6) δ 11.77 (s, 1H), 7.84 (d, J = 8.3 Hz, 1H), 7.50 (s, 1H), 7.49 - 7.40 (m, 2H), 7.29 (s, 1H), 7.08 - 6.96 (m, 3H), 3.91 - 3.81 (m, 2H), 2.82 (t, J = 6.4 Hz, 2H), 1.95 - 1.85 (m, 2H). LCMS: m/z 438.15 [M+H]+ |
| **134** | 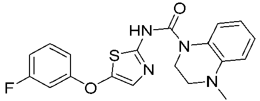 | 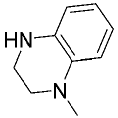 | ¹H NMR (400 MHz, CDCl3) δ 7.34 (dd, J = 14.6, 8.1 Hz, 2H), 7.19 (t, J = 7.8 Hz, 1H), 7.05 (s, 1H), 6.92 (s, 1H), 6.88 - 6.82 (m, 2H), 6.76 (dd, J = 19.8, 11.4 Hz, 2H), 3.99 (s, 2H), 3.45 (d, J = 5.4 Hz, 2H), 3.00 (s, 3H). LCMS: m/z 385.23 [M+H]⁺ |
| **135** | 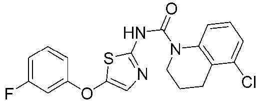 | 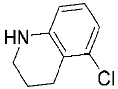 | ¹H NMR (400 MHz, CDCl3) δ 7.38 (d, J = 7.7 Hz, 1H), 7.33 (dd, J = 8.4, 6.6 Hz, 1H), 7.26 (s, 1H), 7.06 (s, 1H), 6.96 (d, J = 7.4 Hz, 1H), 6.91 (dd, J = 8.0, 2.7 Hz, 1H), 6.85 (d, J = 5.3 Hz, 2H), 3.95 - 3.89 (m, 2H), 2.90 (t, J = 6.9 Hz, 2H), 2.11 - 2.06 (m, 2H). LCMS: m/z 404.18 [M+H]+ |
| **136** | 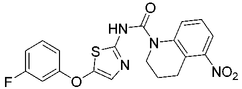 | 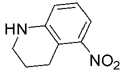 | ¹H NMR (400 MHz, CDCl3) δ 7.79 (dd, J = 13.8, 8.3 Hz, 2H), 7.44 (t, J = 8.1 Hz, 1H), 7.39 - 7.33 (m, 1H), 7.08 (s, 1H), 6.96 - 6.90 (m, 2H), 6.88 - 6.83 (m, 2H), 3.99 (t, J = 6.3 Hz, 2H), 3.11 (dt, J = 13.1, 6.5 Hz, 2H), 2.10 (t, J = 6.4 Hz, 2H). LCMS: m/z 415.20 [M+H]+ |
| **137** | 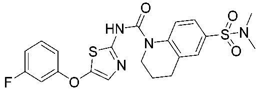 | 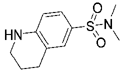 | ¹H NMR (400 MHz, CDCl3) δ 7.70 (d, J = 8.5 Hz, 1H), 7.64 (dd, J = 11.4, 2.8 Hz, 2H), 7.31 (d, J = 8.4 Hz, 1H), 7.03 (s, 1H), 6.92 - 6.88 (m, 1H), 6.83 (dd, J = 14.0, 5.0 Hz, 2H), 3.89 (t, J = 6.4 Hz, 2H), 2.86 (t, J = 6.5 Hz, 2H), 2.76 (s, 6H), 1.45 - 1.39 (m, 2H). LCMS: m/z 477.1 [M+H]⁺ |

### [Example 138]

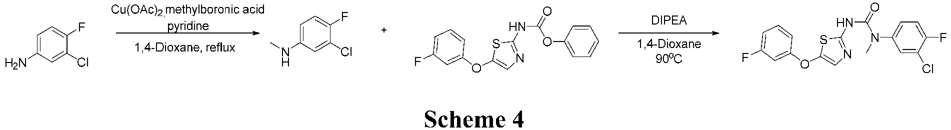

### Synthesis of 3-chloro-4-fluoro-N-methylaniline (reactant D2)

1 eq of 3-chloro-4-fluoroaniline as **reactant D1** was dissolved in 1,4-dioxane (10 mL/g). 2.5 eq of methylboronic acid, 2.5 eq of copper acetate, and 3.5 eq of pyridine were added thereto, and the mixture was stirred under reflux until the reaction was completed. After completion of the reaction, the mixture was filtered through Celite with ethyl acetate and then concentrated. The organic layer was obtained by extraction using ethyl acetate and brine. The ethyl acetate layer was dried over magnesium sulfate, concentrated under reduced pressure, purified, and separated by column chromatography (hexane:ethyl acetate) to obtain 3-chloro-4-fluoro-N-methylaniline.

### Synthesis of 1-(3-chloro-4-fluorophenyl)-3-(5-(3-fluorophenoxy)thiazol-2-yl)-1-methylurea (Example 138)

1 eq of phenyl (5-(3-fluorophenoxy)thiazol-2-yl)carbamate was dissolved in 1,4-dioxane (10 mL/g). 1.5 eq of 3-chloro-4-fluoro-N-methylaniline as **reactant D2** and 2 eq of N,N-diisopropylethylamine were added thereto, and the mixture was stirred at 90°C for about 1 hour. After confirming the completion of the reaction, water was added thereto, and then the organic layer was obtained by extraction using ethyl acetate and brine. The ethyl acetate layer was dried over magnesium sulfate, concentrated under reduced pressure, purified, and separated by column chromatography (hexane:ethyl acetate) to obtain the title **compound of**

### Example 138.

### [Examples 139-141]

The **compounds of Examples 139-141** were synthesized in substantially the same manner as described in the method for preparing the compound of Example 138 (**Scheme 4**) by using **reactants D1 and D2 of Table 5** instead of reactants D1 and D2 in the method for preparing the compound of Example 138.

The structures of the compounds of Examples 138-141, reactants D1, D2, and analytical data are shown in Table 5.

**[Table 5]**

| **No** | **Structure** | **Reactant D1** | **Reactant D2** | **Analysis data** |
|---|---|---|---|---|
| **138** | 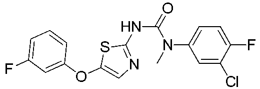 | 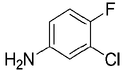 | 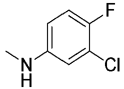 | ¹H NMR (400 MHz, DMSO-d6) δ 11.00 (s, 1H), 7.65 (dd, J = 6.7, 2.5 Hz, 1H), 7.48 - 7. 34 (m, 2H), 7.39 - 7.34 (m, 1H), 7.19 (s, 1H), 7.04 - 6.93 (m, 3H), 3.28 (s, 3H). LCMS: m/z 396.12 [M+H]+ |
| **139** | 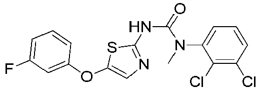 | 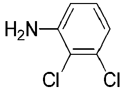 | 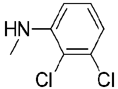 | ¹H NMR (400 MHz, DMSO-d6) δ 10.86 (s, 1H), 7.65 (d, J = 7.2 Hz, 1H), 7.50 - 7.39 (m, 3H), 7.16 (s, 1H), 7.02 - 6.94 (m, 3H), 3.19 (s, 3H). LCMS: m/z 413.95 [M+H]+ |
| **140** | 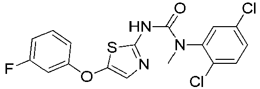 | 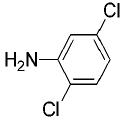 | 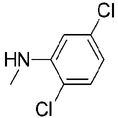 | ¹H NMR (400 MHz, DMSO-d6) δ 11.34 (s, 1H), 7.66 (s, 1H), 7.61 (d, J = 8.6 Hz, 1H), 7.50 - 7.39 (m, 2H), 7.17 (s, 1H), 6.99 (dd, J = 14.8, 8.8 Hz, 3H), 3.19 (s, 3H). LCMS: m/z 413.97 [M+H]+ |
| **141** | 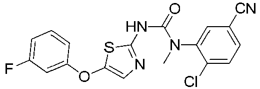 | 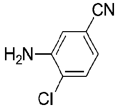 | 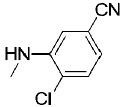 | ¹H NMR (400 MHz, DMSO-d6) δ 10.99 (s, 1H), 8.12 (s, 1H), 7.92 - 7.79 (m, 2H), 7.48 - 7.38 (m, 1H), 7.19 (s, 1H), 7.06 - 6.94 (m, 3H), 3.20 (s, 3H). LCMS: m/z 403.04 [M+H]+ |

### [Example 142]

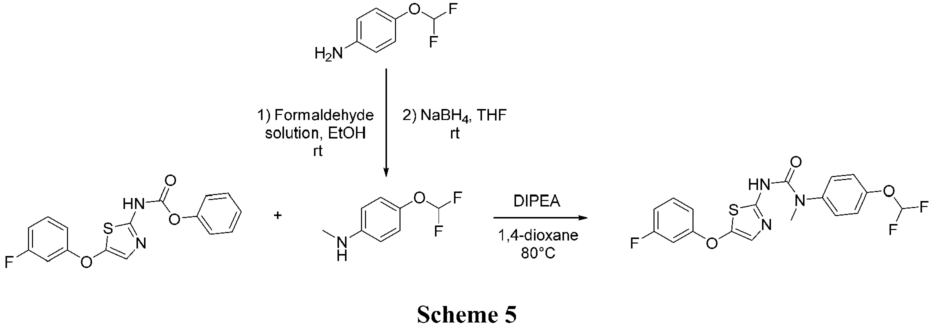

### Synthesis of 4-(difluoromethoxy)-N-methylaniline (Reactant E2)

After dissolving 1 eq of 4-(difluoromethoxy)aniline as **reactant E1** in ethanol (10 mL/g), 1.5 eq of 37% formaldehyde aqueous solution was added thereto and the mixture was stirred at room temperature for 2 hours, and concentrated under reduced pressure. After dissolving the residue in tetrahydrofuran (10 mL/g), 2 eq of sodium borohydride were added thereto and the mixture was stirred at room temperature for 18 hours. After confirming the completion of the reaction, water was added thereto, and then the organic layer was obtained by extraction using ethyl acetate and brine. The ethyl acetate layer was dried over magnesium sulfate, concentrated under reduced pressure, purified, and separated by column chromatography (hexane:ethyl acetate) to obtain 4-(difluoromethoxy)-N-methylaniline.

### Synthesis of 1-(4-(difluoromethoxy)phenyl)-3-(5-(3-fluorophenoxy)thiazol-2-yl)-1-methylurea (Example 142)

After dissolving 1 eq of phenyl (5-(3-fluorophenoxy)thiazol-2-yl)carbamate and 2 eq of N,N-diisopropylethylamine in 1,4-dioxane (10 mL/g), 1.5 eq of 4-(difluoromethoxy)-N-methylaniline as **reactant E2** was added thereto, and the mixture was stirred at 80°C for 18 hours. After confirming the completion of the reaction, water was added thereto, and then the organic layer was obtained by extraction using ethyl acetate and brine. The organic layer was dried over magnesium sulfate, concentrated under reduced pressure, purified, and separated by column chromatography (hexane:ethyl acetate) to obtain the title **compound of Example 142.**

### [Examples 143-147]

The **compounds of Examples 143-147** were synthesized in substantially the same manner as described in the method for preparing the compound of Example 142 (**Scheme 5**) by using **reactants E1 and E2 of Table 6** instead of reactants E1 and E2 in the method for preparing the compound of Example 142.

The structures of the compounds of Examples 142-147, reactants E1, E2, and analytical data are shown in Table 6.

**[Table 6]**

| **No** | **Structure** | **Reactant E1** | **Reactant E2** | **Analysis data** |
|---|---|---|---|---|
| **142** | 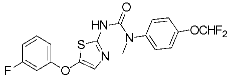 | 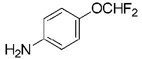 | 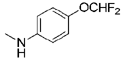 | ¹H NMR (400 MHz, DMSO-d6) δ 10.93 (s, 1H), 7.48 - 7.35 (m, 3H), 7.29 - 6.92 (m, 7H), 3.29 (s, 3H). LCMS: m/z 410.1 [M+H]+ |
| **143** | 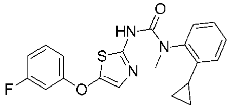 | 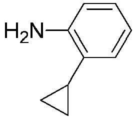 | 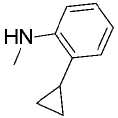 | ¹H NMR (400 MHz, DMSO-d6) δ 10.06 (s, 1H), 7.43 (dd, J = 16.2, 8.1 Hz, 1H), 7.34 - 7.20 (m, 3H), 7.16 (s, 1H), 6.99 (dd, J = 17.9, 7.9 Hz, 4H), 3.24 (s, 3H), 1.82 (dd, J = 11.9, 6.8 Hz, 1H), 0.99 - 0.80 (m, 2H), 0.80 - 0.55 (m, 2H). LCMS: m/z 384.1 [M+H]+ |
| **144** | 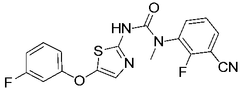 | 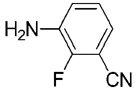 | 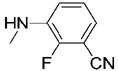 | ¹H NMR (400 MHz, DMSO-d6) δ 11.34 (s, 1H), 8.01 - 7.78 (m, 2H), 7.49 - 7.39 (m, 2H), 7.20 (s, 1H), 7.00 (dd, J = 15.7, 8.0 Hz, 3H), 3.27 (s, 3H). LCMS: [M+H]+: 387.0 |
| **145** | 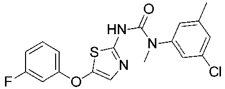 | 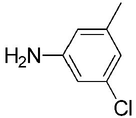 | 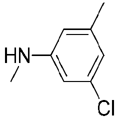 | ¹H NMR (400 MHz, DMSO-d6) δ 10.99 (s, 1H), 7.43 (dd, J = 15.4, 7.6 Hz, 1H), 7.30 - 7.12 (m, 4H), 6.99 (dd, J = 15.5, 8.7 Hz, 3H), 3.29 (s, 3H), 2.31 (s, 3H). LCMS: m/z 392.0 [M+H]+ |
| **146** | 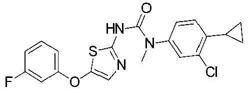 | 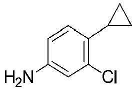 | 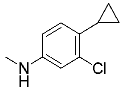 | ¹H NMR (400 MHz, DMSO-d6) δ 10.69 (s, 1H), 7.50 -7.35 (m, 2H), 7.20 (d, J = 7.8 Hz, 2H), 7.08 - 6.91 (m, 4H), 3.27 (s, 3H), 2.17 - 2.10 (m, 1H), 1.06 - 0.98 (m, 2H), 0.75 - 0.69 (m, 2H). LCMS: m/z 418.1 [M+H]+ |
| **147** | 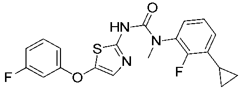 | 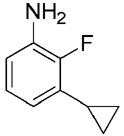 | 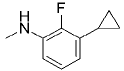 | ¹H NMR (400 MHz, DMSO-d6) δ 10.97 (s, 1H), 7.43 (q, J = 7.7 Hz, 1H), 7.28 - 7.06 (m, 3H), 7.06 - 6.83 (m, 4H), 3.23 (s, 3H), 2.12 - 2.02 (m, 1H), 1.03 - 0.97 (m, 2H), 0.79 - 0.71 (m, 2H). LCMS: m/z 402.1 [M+H]+ |

### [Example 148]

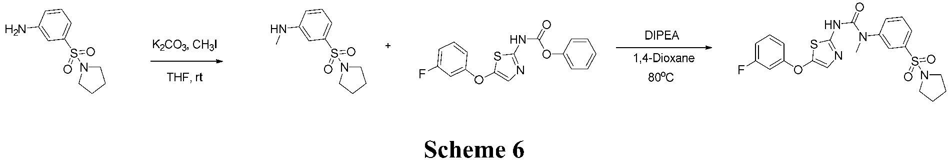

### Synthesis of N-methyl-3-(pyrrolidin-1-ylsulfonyl)aniline (Reactant F2)

After dissolving 1 eq of 3-(pyrrolidin-1-ylsulfonyl)aniline as **reactant F1** and 2 eq of potassium carbonate in tetrahydrofuran (10 mL/g), 2 eq of methyl iodide was added thereto and the mixture was stirred at room temperature for 16 hours. After confirming the completion of the reaction, water was added thereto, and then the organic layer was obtained by extraction using ethyl acetate and brine. The ethyl acetate layer was dried over magnesium sulfate, concentrated under reduced pressure, purified, and separated by column chromatography (hexane:ethyl acetate) to obtain N-methyl-3-(pyrrolidin-1-ylsulfonyl)aniline.

### Synthesis of 3-(5-(3-fluorophenoxy)thiazol-2-yl)-1-methyl-1-(3-(pyrrolidin-1-ylsulfonyl)phenyl)urea (Example 148)

After dissolving 1 eq of phenyl (5-(3-fluorophenoxy)thiazol-2-yl)carbamate in 1,4-dioxane (10 mL/g), 1.5 eq of N-methyl-3-(pyrrolidin-1-ylsulfonyl)aniline as **reactant F2** and 2 eq of N,N-diisopropylethylamine were added thereto, and the mixture was stirred at 80°C for 2 hours. After confirming the completion of the reaction, water was added thereto, and then the organic layer was obtained by extraction using ethyl acetate and brine. The ethyl acetate layer was dried over magnesium sulfate, concentrated under reduced pressure, purified, and separated by column chromatography (hexane:ethyl acetate) to obtain the title **compound of Example 148.**

**[Table 7]**

| **No** | **Structure** | **Reactant F1** | **Reactant F2** | **Analysis data** |
|---|---|---|---|---|
| **148** | 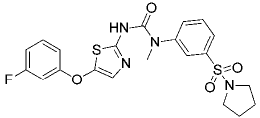 | 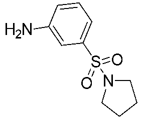 | 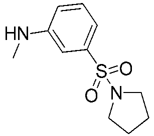 | ¹H NMR (400 MHz, DMSO-d6) δ 7.75 (s, 1H), 7.71 - 7.61 (m, 3H), 7.44 (dd, J = 15.3, 8.3 Hz, 1H), 7.23 (s, 1H), 7.05 - 6.96 (m, 3H), 3.38 (s, 3H), 3.17 (dd, J = 9.1, 4.2 Hz, 4H), 1.72 - 1.65 (m, 4H). LCMS: m/z 477.23 [M+H]+ |

### [Examples 149-151]

The **compounds of Examples 149-151** were synthesized in substantially the same manner as described in the method for preparing Example 148 (**Scheme 6**) by using dimethylformamide (10 mL/g) instead of tetrahydrofuran (10 mL/g) and 1 eq of methyl iodide instead of 2 eq thereof and using **reactants F3 and F4 of Table 8** instead of reactants F1 and F2 in the method for preparing the compound of Example 148.

The structures of the compounds of Examples 149-151, reactants F3, F4, and analytical data are shown in Table 8.

**[Table 8]**

| **No** | **Structure** | **Reactant F3** | **Reactant F4** | **Analysis data** |
|---|---|---|---|---|
| **149** | 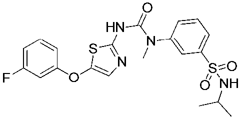 | 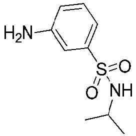 | 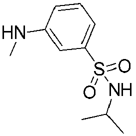 | ¹H NMR (400 MHz, CDCl3) δ 7.89 (d, J = 8.0 Hz, 1H), 7.86 (s, 1H), 7.67 (t, J = 7.9 Hz, 1H), 7.55 (d, J = 6.9 Hz, 1H), 7.32 (d, J = 8.3 Hz, 1H), 6.96 (s, 1H), 6.90 (d, J = 7.4 Hz, 1H), 6.86 - 6.80 (m, 2H), 4.91 (d, J = 7.9 Hz, 1H), 3.56 (dd, J = 13.6, 6.9 Hz, 1H), 3.40 (s, 3H), 1.17 (d, J = 6.5 Hz, 6H). LCMS: m/z 465.11 [M+H]+ |
| **150** | 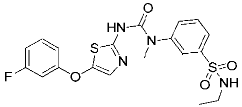 | 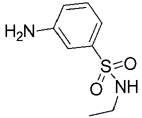 | 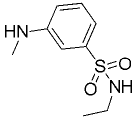 | ¹H NMR (400 MHz, CDCl3) δ 7.86 (d, J = 6.6 Hz, 2H), 7.66 (d, J = 8.1 Hz, 1H), 7.58 - 7.51 (m, 1H), 7.35 - 7.30 (m, 1H), 6.94 (s, 1H), 6.92 - 6.82 (m, 3H), 5.31 (d, J = 12.2 Hz, 1H), 3.42 (s, 3H), 3.16 - 3.07 (m, 2H), 1.20 (t, J = 7.2 Hz, 3H). LCMS: m/z 451.16 [M+H] + |
| **151** | 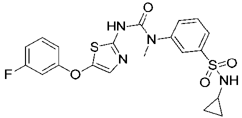 | 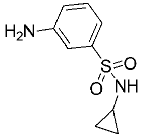 | 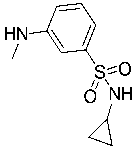 | ¹H NMR (400 MHz, CDCl3) δ 7.93 - 7.88 (m, 2H), 7.70 (d, J = 7.8 Hz, 1H), 7.59 (d, J = 8.0 Hz, 1H), 7.32 (d, J = 8.8 Hz, 1H), 6.96 (s, 1H), 6.90 (d, J = 7.6 Hz, 1H), 6.83 (t, J = 7.9 Hz, 2H), 5.42 (s, 1H), 3.42 (s, 3H), 2.37 - 2.31 (m, 1H), 0.69 (d, J = 7.0 Hz, 4H). LCMS: m/z 463.04 [M+H]+ |

### [Example 152]

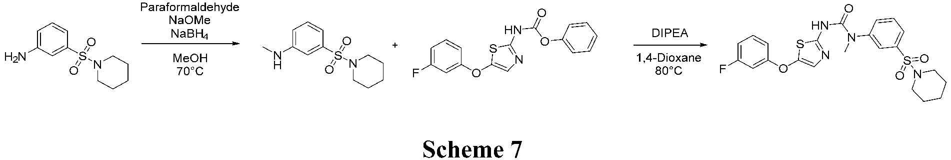

### Synthesis of N-methyl-3-(piperidin-1-ylsulfonyl)aniline (Reactant G2)

After dissolving 1 eq of 3-(piperidin-1-ylsulfonyl)aniline as **reactant G1** in methanol (30 mL/g), 5 eq of sodium methoxide and 1.4 eq of paraformaldehyde were added thereto, and the mixture was stirred at room temperature for 2 hours and stirred at 70°C for 30 minutes. After that, 1 eq of sodium borohydride was added at 0°C, the temperature was raised to 70°C, and the mixture was stirred for 2 hours. After confirming the completion of the reaction, water was added thereto, and then the organic layer was obtained by extraction using ethyl acetate and brine. The ethyl acetate layer was dried over magnesium sulfate, concentrated under reduced pressure, purified, and separated by column chromatography (hexane:ethyl acetate) to obtain N-methyl-3-(piperidin-1-ylsulfonyl)aniline.

### Synthesis of 3-(5-(3-fluorophenoxy)thiazol-2-yl)-1-methyl-1-(3-(pineridin-1-ylsulfonyl)phenyl)urea (Example 152)

1 eq of phenyl (5-(3-fluorophenoxy)thiazol-2-yl)carbamate was dissolved in 1,4-dioxane (10 mL/g). 1.5 eq of N-methyl-3-(piperidin-1-ylsulfonyl)aniline as **reactant G2** and 2 eq of N,N-diisopropylethylamine were added thereto, and the mixture was heated to 80°C. After completion of the reaction, the mixture was extracted with ethyl acetate and water. The ethyl acetate layer was dried over magnesium sulfate, concentrated under reduced pressure, purified, and separated by column chromatography (hexane:ethyl acetate) to obtain the title **compound of Example 152.**

### [Examples 153-167]

The **compounds of Examples 153-167** were synthesized in substantially the same manner as described in the method for preparing the compound of Example 152 (**Scheme 7)** by using **reactants G1 and G2 of Table 9** instead of reactants G1 and G2 in the method for preparing the compound of Example 152.

The structures of the compounds of Examples 152-167, reactants G1, G2, and analytical data are shown in Table 9.

**[Table 9]**

| **No** | **Structure** | **Reactant G1** | **Reactant G2** | **Analysis data** |
|---|---|---|---|---|
| **152** | 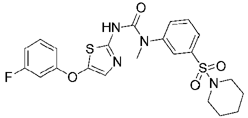 | 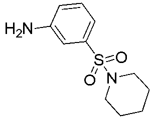 | 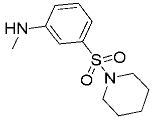 | ¹H NMR (400 MHz, CDCl3) δ 7.81 (d, J = 7.9 Hz, 1H), 7.75 (s, 1H), 7.69 (t, J = 7.8 Hz, 1H), 7.59 (d, J = 9.0 Hz, 2H), 6.98 (s, 1H), 6.90 (d, J = 7.9 Hz, 1H), 6.83 (t, J = 7.9 Hz, 2H), 3.42 (s, 3H), 3.14 - 3.05 (m, 4H), 1.75 - 1.66 (m, 4H), 1.55 - 1.48 (m, 2H). LCMS: m/z 491.10 [M+H]+ |
| **153** | 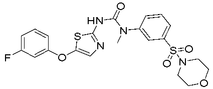 | 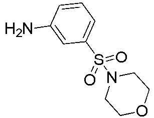 | 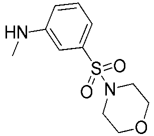 | ¹H NMR (400 MHz, CDCl3) δ 7.81 (d, J = 7.9 Hz, 1H), 7.77 - 7.71 (m, 2H), 7.63 (d, J = 7.9 Hz, 1H), 7.31 (d, J = 8.6 Hz, 1H), 6.98 (s, 1H), 6.89 (d, J = 7.8 Hz, 1H), 6.83 (t, J = 8.0 Hz, 2H), 3.84 - 3.77 (m, 4H), 3.43 (s, 3H), 3.14 - 3.07 (m, 4H). LCMS: m/z 493.08 [M+H]+ |
| **154** | 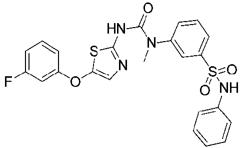 | 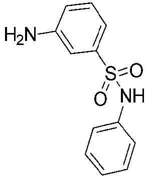 | 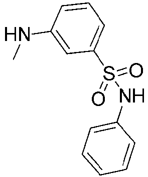 | ¹H NMR (400 MHz, CDCl3) δ 7.88 (s, 1H), 7.59 - 7.48 (m, 3H), 7.35 (t, J = 5.2 Hz, 3H), 7.20 (dd, J = 13.7, 6.7 Hz, 2H), 7.03 (s, 1H), 6.97 - 6.90 (m, 2H), 6.87 (d, J = 9.6 Hz, 1H), 3.39 (s, 3H). LCMS: m/z 499.06 [M+H]+ |
| **155** | 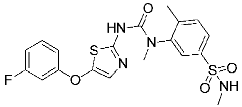 | 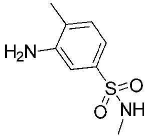 | 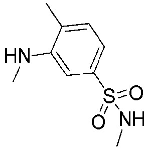 | ¹H NMR (400 MHz, CDCl3) δ 7.83 (d, J = 8.0 Hz, 1H), 7.79 (s, 1H), 7.60 - 7.53 (m, 1H), 7.36 - 7.30 (m, 1H), 6.96 (s, 1H), 6.93 - 6.81 (m, 3H), 4.92 (s, 1H), 3.34 (d, J = 9.0 Hz, 3H), 2.78 (d, J = 5.1 Hz, 3H), 2.39 (s, 3H). LCMS: m/z 451.18 [M+H]+ |
| **156** | 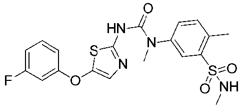 | 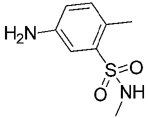 | 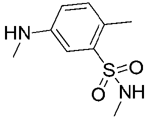 | ¹H NMR (400 MHz, CDCl3) δ 7.95 (d, J = 1.9 Hz, 1H), 7.74 (s, 1H), 7.50 - 7.41 (m, 2H), 6.96 (s, 1H), 6.90 (d, J = 7.2 Hz, 1H), 6.86 - 6.79 (m, 2H), 3.38 (s, 3H), 2.74 (d, J = 5.3 Hz, 3H), 2.69 (s, 3H). LCMS: m/z 451.26 [M+H]+ |
| **157** | 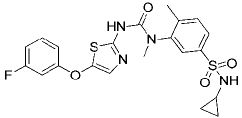 | 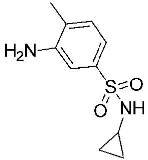 | 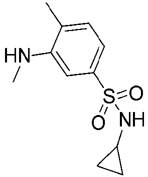 | ¹H NMR (400 MHz, CDCl3) δ 7.86 (d, J = 8.0 Hz, 1H), 7.83 (s, 1H), 7.61 - 7.53 (m, 2H), 7.31 (d, J = 8.7 Hz, 1H), 6.95 (s, 1H), 6.90 (d, J = 7.2 Hz, 1H), 6.86 - 6.79 (m, 2H), 4.73 (s, 1H), 3.32 (s, 3H), 2.41 (s, 3H), 2.33 (d, J = 5.0 Hz, 1H), 0.71 - 0.64 (m, 4H). LCMS: m/z 477.26 [M+H]+ |
| **158** | 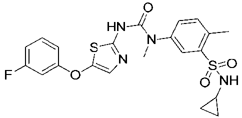 | 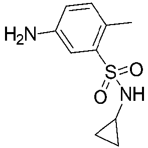 | 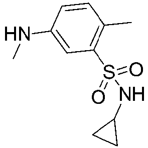 | ¹H NMR (400 MHz, CDCl3) δ 8.00 (d, J = 1.6 Hz, 1H), 7.45 (d, J = 3.8 Hz, 1H), 7.31 (d, J = 8.7 Hz, 1H), 6.94 (s, 1H), 6.90 (d, J = 7.3 Hz, 1H), 6.87 - 6.81 (m, 2H), 6.17 (s, 1H), 4.73 (s, 1H), 3.38 (s, 3H), 2.62 (s, 3H), 2.38 (dd, J = 6.7, 3.2 Hz, 1H), 0.64 - 0.57 (m, 2H), 0.54 - 0.47 (m, 2H). LCMS: m/z 477.12 [M+H]+ |
| **159** | 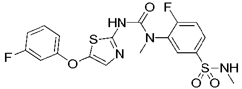 | 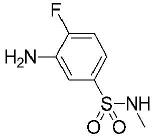 | 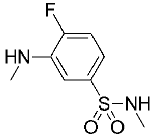 | ¹H NMR (400 MHz, DMSO-d6) δ 11.23 (s, 1H), 7.86 - 7.73 (m, 2H), 7.57 (t, J = 9.2 Hz, 2H), 7.47 - 7.38 (m, 1H), 7.21 (s, 1H), 7.05 - 6.95 (m, 3H), 3.29 (s, 3H), 2.47 (s, 3H). LCMS: m/z 455.37 [M+H]+ |
| **160** | 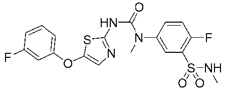 | 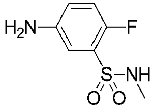 | 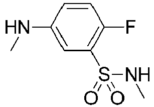 | ¹H NMR (400 MHz, DMSO-d6) δ 10.98 (s, 1H), 7.82 - 7.76 (m, 1H), 7.71 - 7.63 (m, 2H), 7.53 - 7.39 (m, 2H), 7.21 (s, 1H), 7.04 - 6.94 (m, 3H), 3.32 (s, 3H), 2.55 (d, J = 4.5 Hz, 3H). LCMS: m/z 455.26 [M+H]+ |
| **161** | 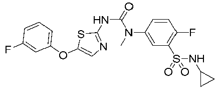 | 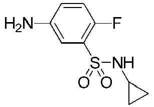 | 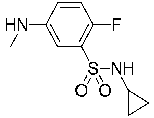 | ¹H NMR (400 MHz, DMSO-d6) δ 10.95 (s, 1H), 8.31 (s, 1H), 7.74 - 7.65 (m, 2H), 7.53 - 7.39 (m, 2H), 7.22 (s, 1H), 7.05 - 6.95 (m, 3H), 3.33 (s, 3H), 2.29 (s, 1H), 0.56 - 0.41 (m, 4H). LCMS: m/z 481.20 [M+H]+ |
| **162** | 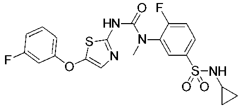 | 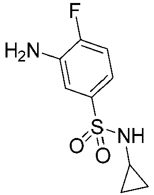 | 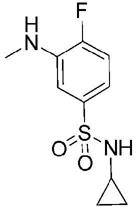 | ¹H NMR (400 MHz, C DCl3) δ 8.24 (s, 1H), 7 .94 (d, J = 6.9 Hz, 2H), 7.41 (t, J = 8.8 Hz, 1 H), 6.95 (s, 1H), 6.90 ( d, J = 7.2 Hz, 1H), 6.8 3 (d, J = 8.3 Hz, 3H), 5 .99 (s, 1H), 3.37 (s, 3H ), 2.35 - 2.28 (m, 1H), 0.94 - 0.87 (m, 2H), 0. 68 - 0.66 (m, 2H). LCMS: m/z 481.96 [M+H]+ |
| **163** | 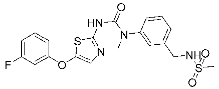 | 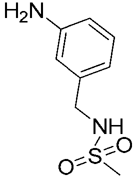 | 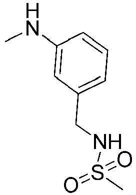 | ¹H NMR (400 MHz, CDCl3) δ 7.59 (s, 1H), 7.54 (t, J = 7.8 Hz, 1H), 7.46 (d, J = 7.9 Hz, 1H), 7.36 (s, 1H), 7.31 (s, 1H), 6.95 (s, 1H), 6.89 (d, J = 7.3 Hz, 1H), 6.86 - 6.79 (m, 2H), 4.79 (s, 1H), 4.42 (d, J = 6.3 Hz, 2H), 3.38 (s, 3H), 3.03 (s, 3H). LCMS: m/z 451.17 [M+H]+ |
| **164** | 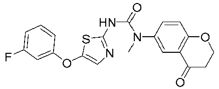 | 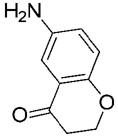 | 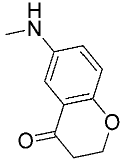 | ¹H NMR (400 MHz, DMSO-d6) δ 10.64 (s, 1H), 7.66 - 7.61 (m, 1H), 7.55 - 7.50 (m, 1H), 7.47 - 7.39 (m, 1H), 7.19 (s, 1H), 7.09 (d, J = 8.9 Hz, 1H), 7.04 - 6.94 (m, 3H), 4.57 (t, J = 6.4 Hz, 2H), 3.27 (s, 3H), 2.82 (t, J= 6.3 Hz, 2H). LCMS: m/z 414.07 [M+H]+ |
| **165** | 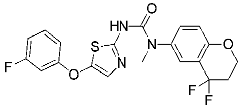 | 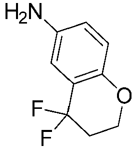 | 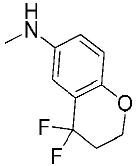 | ¹H NMR (400 MHz, DMSO-d6) δ 10.66 (s, 1H), 7.50 (s, 1H), 7.43 (dd, J = 16.0, 8.1 Hz, 1H), 7.37 (d, J = 8.9 Hz, 1H), 7.19 (s, 1H), 7.03 - 6.94 (m, 4H), 4.40 - 4.32 (m, 2H), 3.27 (s, 3H), 2.61 - 2.49 (m, 2H). LCMS: m/z 436.12 [M+H]+ |
| **166** | 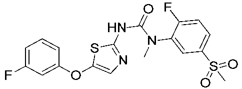 | 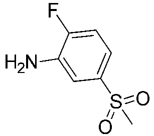 | 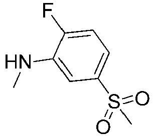 | ¹H NMR (400 MHz, DMSO-d6) δ 11.30 (s, 1H), 8.03 (d, *J* = 7.0 Hz, 1H), 7.92 (s, 1H), 7.60 (t, *J* = 9.3 Hz, 1H), 7.42 (q, *J* = 7.7 Hz, 1H), 7.19 (s, 1H), 7.04 - 6.94 (m, 3H), 3.29 (s, 3H), 3.27 (s, 3H). LCMS: m/z 440.04 [M+H]+ |
| **167** | 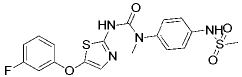 | 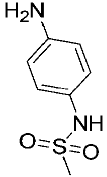 | 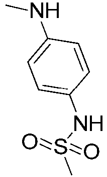 | ¹H NMR (400 MHz, DMSO-d6) δ 10.46 (s, 1H), 9.85 (s, 1H), 7.46 -7.39 (m, 1H), 7.29 (d, *J* = 8.4 Hz, 2H), 7.21 (d, *J* = 8.4 Hz, 2H), 7.17 (d, *J* = 1.3 Hz, 1H), 7.02 - 6.93 (m, 3H), 3.26 (s, 3H), 3.02 (s, 3H). LCMS: m/z 437.29 [M+H]+ |

### [Example 168]

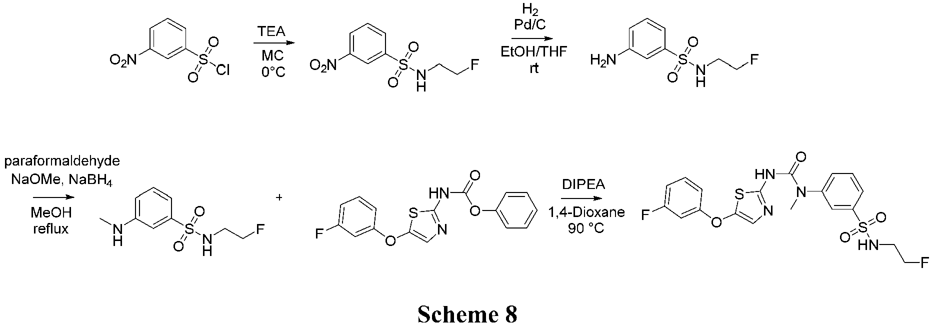

### Synthesis of N-(2-fluoroethyl)-3-nitrobenzenesulfonamide

1 eq of 3-nitrobenzenesulfonyl chloride as **reactant H1** was dissolved in methylene chloride (10 mL/g). 1.2 eq of 2-fluoroethylamine hydrochloride as **reactant H2** and 3 eq of triethylamine were added thereto at 0°C and the mixture was stirred. After confirming the completion of the reaction, water was added thereto, and then the organic layer was obtained by extraction using methylene chloride and brine. Moisture remaining in the organic layer was completely removed with magnesium sulfate and then filtered. The filtrate was concentrated under reduced pressure to obtain a crude product, which was then purified by column chromatography and concentrated under vacuum to obtain N-(2-fluoroethyl)-3-nitrobenzenesulfonamide.

### Synthesis of 3-amino-N-(2-fluoroethyl)benzenesulfonamide

1 eq of N-(2-fluoroethyl)-3-nitrobenzenesulfonamide was dissolved in ethanol (10 mL/g) and tetrahydrofuran (10 mL/g). 10 wt% of palladium/carbon was added thereto, and the mixture was hydrogenated under a hydrogen atmosphere to perform the reaction. The reaction mixture was stirred at room temperature for 16 hours. After confirming the completion of the reaction, the mixture was filtered through Celite. The filtrate was concentrated under reduced pressure to obtain a crude product, which was then purified by column chromatography and concentrated under vacuum to obtain 3-amino-N-(2-fluoroethyl)benzenesulfonamide.

### Synthesis of N-(2-fluoroethyl)-3-(methylamino)benzenesulfonamide

1 eq of 3-amino-N-(2-fluoroethyl)benzenesulfonamide was dissolved in methanol (10 mL/g). 1.5 eq of paraformaldehyde and 5 eq of sodium methoxide were added thereto, and the mixture was stirred under reflux for about 1 hour. 1 eq of sodium borohydride was added thereto at 0°C and stirred for about 30 minutes. Water was added thereto, and then the organic layer was obtained by extraction using ethyl acetate and brine. Moisture remaining in the organic layer was completely removed with magnesium sulfate and then filtered. The filtrate was concentrated under reduced pressure to obtain a crude product, which was then purified by column chromatography and concentrated under vacuum to obtain N-(2-fluoroethyl)-3-(methylamino)benzenesulfonamide.

### Synthesis of N-(2-fluoroethyl)-3-(3-(5-(3-fluorophenoxy)thiazol-2-yl)-1-methylureido)benzenesulfonamide (Example 168)

1 eq of phenyl (5-(3-fluorophenoxy)thiazol-2-yl)carbamate was dissolved in 1,4-dioxane (10 mL/g). 1.5 eq of N-(2-fluoroethyl)-3-(methylamino)benzenesulfonamide and 2 eq of N,N-diisopropylethylamine were added thereto, and the mixture was stirred at 90°C for about 1 hour. After confirming the completion of the reaction, water was added thereto, and then the organic layer was obtained by extraction using ethyl acetate and brine. Moisture remaining in the organic layer was completely removed with magnesium sulfate and then filtered. The filtrate was concentrated under reduced pressure to obtain a crude product, which was then purified by column chromatography and concentrated under vacuum to obtain the title **compound of Example 168.**

### [Examples 169-177]

The **compounds of Examples 169-177** were synthesized in substantially the same manner as described in the method for preparing the compound of Example 168 (**Scheme 8**) by using **reactants H1 and H2 of Table 10** instead of reactants H1 and H2 in the method for preparing the compound of Example 168.

The structures of the compounds of Examples 168-177, reactants H1, H2, and analytical data are shown in Table 10.

**[Table 10]**

| **No** | **Structure** | **Reactant H1** | **Reactant H2** | Analysis data |
|---|---|---|---|---|
| **168** | 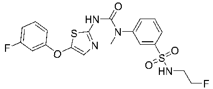 | 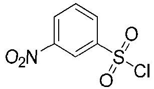 | 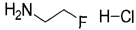 | ¹H NMR (400 MHz, DMSO-d6) δ 11.17 (s, 1H), 8.01 (s, 1H), 7.76 (s, 1H), 7.69 - 7.59 (m, 3H), 7.44 (q, J = 7.9 Hz, 1H), 7.22 (s, 1H), 7.05 - 6.95 (m, 3H), 4.47 (t, J = 4.6 Hz, 1H), 4.35 (t, J = 4.7 Hz, 1H), 3.37 (s, 3H), 3.14 (s, 1H), 3.07 (s, 1H). LCMS: m/z 469.23 [M+H]+ |
| **169** | 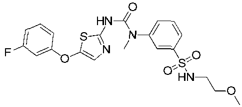 | 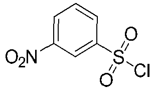 | 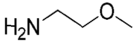 | ¹H NMR (400 MHz, DMSO-d6) δ 11.19 (s, 1H), 7.81 - 7.73 (m, 2H), 7.67 - 7.58 (m, 3H), 7.44 (dd, J = 15.4, 8.3 Hz, 1H), 7.22 (s, 1H), 6.99 (dd, J = 15.2, 9.0 Hz, 3H), 3.37 (s, 3H), 3.32 (t, J = 5.7 Hz, 2H), 3.17 (s, 3H), 2.94 (q, J = 5.7 Hz, 2H). LCMS: m/z 481.17 [M+H]+ |
| **170** | 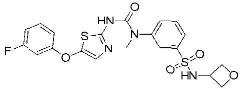 | 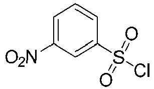 | 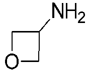 | ¹H NMR (400 MHz, DMSO-d6) δ 11.17 (s, 1H), 8.59 (d, J = 8.0 Hz, 1H), 7.74 (s, 1H), 7.61 (s, 3H), 7.44 (dd, J = 15.3, 8.2 Hz, 1H), 7.23 (s, 1H), 7.05 - 6.93 (m, 3H), 4.53 (t, J = 6.7 Hz, 2H), 4.46 - 4.36 (m, 1H), 4.28 (t, J = 6.2 Hz, 2H), 3.37 (s, 3H). LCMS: m/z 479.02 [M+H]+ |
| **171** | 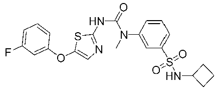 | 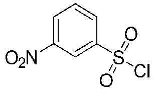 | 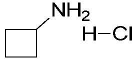 | ¹H NMR (400 MHz, DMSO-d6) δ 11.24 (s, 1H), 8.00 (d, J = 7.3 Hz, 1H), 7.73 (s, 1H), 7.66 - 7.56 (m, 3H), 7.48 - 7.39 (m, 1H), 7.22 (s, 1H), 7.04 - 6.96 (m, 3H), 3.70 - 3.60 (m, 1H), 3.37 (s, 3H), 1.98 - 1.88 (m, 2H), 1.82 - 1.69 (m, 2H), 1.55 - 1.40 (m, 2H). LCMS: m/z 477.02 [M+H]+ |
| **172** | 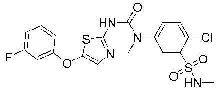 | 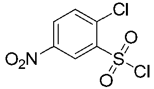 | 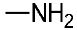 | ¹H NMR (400 MHz, MeOD) δ 8.02 (d, J = 2.3 Hz, 1H), 7.68 (d, J = 8.4 Hz, 1H), 7.62 (dd, J = 8.5, 2.3 Hz, 1H), 7.38 (dd, J = 15.1, 7.9 Hz, 1H), 7.03 (s, 1H), 6.97 - 6.85 (m, 3H), 3.43 (s, 3H), 2.63 (s, 3H). LCMS: m/z 471.07 [M+H]+ |
| **173** | 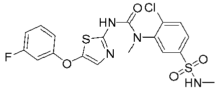 | 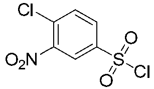 | 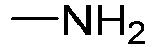 | ¹H NMR (400 MHz, DMSO-d6) δ 11.10 (s, 1H), 7.88 - 7.78 (m, 2H), 7.78 - 7.72 (m, 1H), 7.61 (s, 1H), 7.43 (dd, J = 15.1, 7.7 Hz, 1H), 7.19 (s, 1H), 6.99 (dd, J = 16.9, 8.6 Hz, 3H), 3.23 (s, 3H), 2.48 (s, 3H). LCMS: m/z 470.94 [M+H]+ |
| **174** | 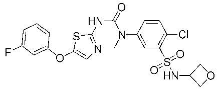 | 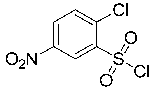 | 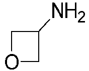 | ¹H NMR (400 MHz, DMSO-d6) δ 11.33 (s, 1H), 8.93 (s, 1H), 7.86 (s, 1H), 7.70 - 7.60 (m, 2H), 7.49 - 7.37 (m, 1H), 7.23 (s, 1H), 7.05 - 6.93 (m, 3H), 4.59 - 4.42 (m, 5H), 3.18 (d, J = 4.4 Hz, 3H). LCMS: m/z 512.97 [M+H]+ |
| **175** | 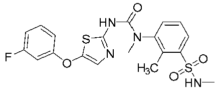 | 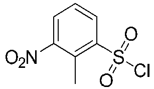 | -NH₂ | ¹H NMR (400 MHz, DMSO-d6) δ 10.74 (s, 1H), 7.81 (d, J = 7.7 Hz, 1H), 7.65 - 7.57 (m, 1H), 7.57 - 7.51 (m, 1H), 7.50 - 7.39 (m, 2H), 7.18 (s, 1H), 7.04 - 6.94 (m, 3H), 3.25 - 3.13 (m, 3H), 2.49 (s, 3H), 2.39 (s, 3H). LCMS: m/z 451.30 [M+H]+ |
| **176** | 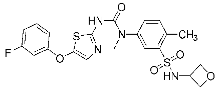 | 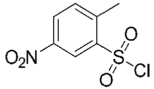 | 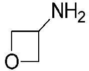 | ¹H NMR (400 MHz, DMSO-d6) δ 10.97 (s, 1H), 8.66 (d, J = 5.5 Hz, 1H), 7.67 (s, 1H), 7.52 - 7.39 (m, 3H), 7.22 (s, 1H), 7.05 - 6.95 (m, 3H), 4.56 - 4.46 (m, 2H), 4.41 - 4.30 (m, 3H), 3.32 (s, 3H), 2.58 (s, 3H). LCMS: m/z 493.03 [M+H]+ |
| **177** | 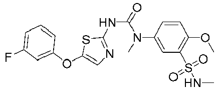 | 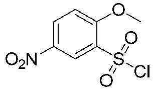 | -NH₂ | ¹H NMR (400 MHz, DMSO-d6) δ 10.84 (s, 1H), 7.62 - 7.53 (m, 2H), 7.47 - 7.39 (m, 1H), 7.26 (d, J = 8.7 Hz, 1H), 7.19 (s, 1H), 7.15 - 7.08 (m, 1H), 7.03 - 6.93 (m, 3H), 3.92 (s, 3H), 3.28 (s, 3H), 2.45 (d, J = 4.6 Hz, 3H). LCMS: m/z 467.27 [M+H]+ |

### [Example 178]

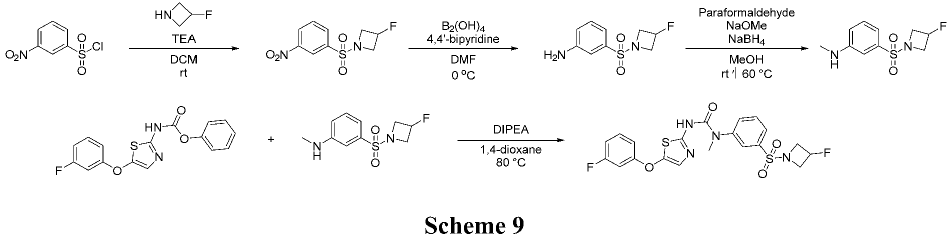

### Synthesis of 3-fluoro-1-((3-nitrophenyl)sulfonyl)azetidine

1 eq of 3-nitrobenzenesulfonyl chloride and 1.5 eq of 3-fluoroazetidine hydrochloride as **reactant I1** were dissolved in methylene chloride (5 mL/g) and then 2 eq of triethylamine was added thereto, and the mixture was stirred at room temperature for 30 minutes. After completion of the reaction, the mixture was extracted with methylene chloride and water. The methylene chloride layer was dried over magnesium sulfate, and then subjected to the following reaction without further purification.

### Synthesis of 3-((3-fluoroazetidin-1-yl)sulfonyl)aniline

After dissolving 1 eq of 3-fluoro-1-((3-nitrophenyl)sulfonyl)azetidine in dimethylformamide (25 mL/g), 3 eq of tetrahydroxydiboron was added thereto. 5 mol% of 4,4-dipyridine was dissolved in dimethylformamide (10 mL/g) and then slowly added dropwise to the solution at 0°C, and the mixture was stirred for 1 minute. After completion of the reaction, the mixture was extracted with ethyl acetate and water. The ethyl acetate layer was dried over magnesium sulfate, concentrated under reduced pressure, and then subjected to the following reaction without further purification.

### Synthesis of 3-((3-fluoroazetidin-1-yl)sulfonyl)-N-methylaniline

After dissolving 1 eq of 3-((3-fluoroazetidin-1-yl)sulfonyl)aniline in methanol (40 mL/g), 1.4 eq of paraformaldehyde and 5 eq of sodium methoxide were added thereto and the mixture was stirred at room temperature for 16 hours. After that, 1 eq of sodium borohydride was added at 0°C, and the mixture was stirred at 60°C for 1 hour. After completion of the reaction, the mixture was extracted with ethyl acetate and water. The ethyl acetate layer was dried over magnesium sulfate and then concentrated under reduced pressure. The concentrate was purified and separated by column chromatography (hexane:ethyl acetate) to obtain 3-((3-fluoroazetidin-1-yl)sulfonyl)-N-methylaniline.

### Synthesis of 1-(3-((3-fluoroazetidin-1-yl)sulfonyl)phenyl)-3-(5-(3-fluorophenoxy)thiazol-2-yl)-1-methylurea (Example 178)

After dissolving 1 eq of phenyl (5-(3-fluorophenoxy)thiazol-2-yl)carbamate in 1,4-dioxane (10 mL/g), 1.2 eq of 3-((3-fluoroazetidin-1-yl)sulfonyl)-N-methylaniline as **reactant 12** and 3 eq of N,N-diisopropylethylamine were added thereto, and the mixture was stirred at 80°C for 3 hours. After completion of the reaction, the mixture was extracted with methylene chloride and water. The methylene chloride layer was dried over magnesium sulfate and then concentrated under reduced pressure. The concentrate was purified and separated by column chromatography (hexane:ethyl acetate) to obtain the title **compound of Example 178.**

### [Examples 179-180]

The **compounds of Examples 179-180** were synthesized in substantially the same manner as described in the method for preparing the compound of Example 178 (**Scheme 9**) by using **reactants I1 and I2 of Table 11** instead of reactants I1 and I2 in the compound of Example 178.

The structures of the compounds of Examples 178-180, reactants I1, I2, and analytical data are shown in Table 11.

**[Table 11]**

| **No** | **Structure** | **Reactant I1** | **Reactant I2** | **Analysis data** |
|---|---|---|---|---|
| 178 | 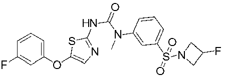 | 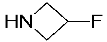 | 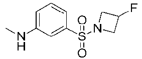 | ¹H NMR (400 MHz, DMSO-d6) δ 11.18 (s, 1H), 7.81 (s, 1H), 7.76 (dd, *J* = 7.6, 1.7 Hz, 1H), 7.73 - 7.65 (m, 2H), 7.43 (q, *J* = 7.8 Hz, 1H), 7.22 (s, 1H), 7.03 - 6.94 (m, 3H), 5.31 - 5.09 (m, 1H), 4.14 - 4.00 (m, 2H), 3.86 - 3.74 (m, 2H), 3.40 (s, 3H). LCMS: m/z 480.98 [M+H]+ |
| 179 | 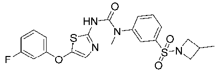 | 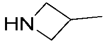 | 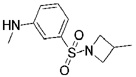 | ¹H NMR (400 MHz, DMSO-d6) δ 11.14 (s, 1H), 7.77 (s, 1H), 7.76 - 7.66 (m, 2H), 7.63 (d, *J* = 7.5 Hz, 1H), 7.43 (q, *J* = 7.7 Hz, 1H), 7.22 (d, *J* = 1.3 Hz, 1H), 7.02 - 6.94 (m, 3H), 3.83 (t, *J* = 8.1 Hz, 2H), 3.40 (s, 3H), 3.28 (dd, *J* = 8.0, 5.9 Hz, 2H), 2.47 - 2.40 (m, 1H), 0.91 (d, *J* = 6.8 Hz, 3H). LCMS: m/z 477.02 [M+H]+ |
| 180 | 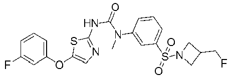 | 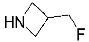 | 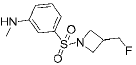 | ¹H NMR (400 MHz, DMSO-d6) δ 10.99 (s, 1H), 7.78 (s, 1H), 7.77 - 7.62 (m, 3H), 7.43 (q, J = 7.8 Hz, 1H), 7.22 (s, 1H), 7.05 - 6.94 (m, 3H), 4.37 (d, J = 5.2 Hz, 1H), 4.26 (d, J = 5.2 Hz, 1H), 3.82 (t, J = 8.5 Hz, 2H), 3.60 - 3.53 (m, 2H), 3.39 (s, 3H), 2.84 - 2.70 (m, 1H). LCMS: m/z 494.98 [M+H]+ |

### [Example 181]

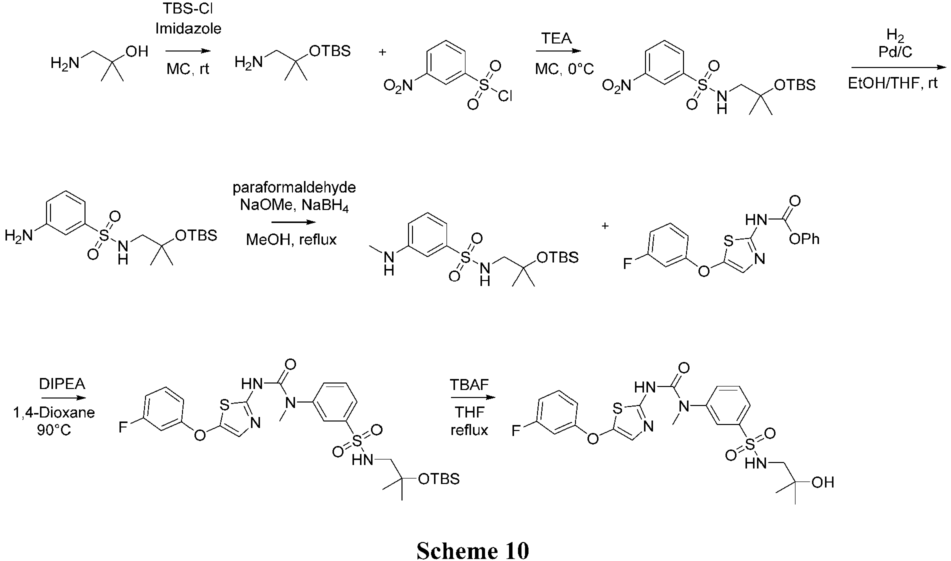

### Synthesis of 2-((tert-butyldimethylsilyl)oxy)-2-methylpropan-1-amine

1 eq of 1-amino-2-methylpropan-2-ol was dissolved in methylene chloride (10 mL/g). 1.5 eq of tert-butyldimethylsilyl chloride and 2 eq of imidazole were added thereto at 0°C and the mixture was stirred at room temperature for about 2 hours. After confirming the completion of the reaction, water was added thereto, and then the organic layer was obtained by extraction using methylene chloride and brine. Moisture remaining in the organic layer was completely removed with magnesium sulfate and then filtered. The filtrate was concentrated under reduced pressure to obtain a crude product, which was then purified by column chromatography and concentrated under vacuum to obtain 2-((tert-butyldimethylsilyl)oxy)-2-methylpropan-1-amine.

### Synthesis of N-(2-((tert-butyldimethylsilyl)oxy)-2-methylpropyl)-3-nitrobenzenesulfonamide

1 eq of 3-nitrobenzenesulfonyl chloride was dissolved in methylene chloride (10 mL/g). 1.2 eq of 2-((tert-butyldimethylsilyl)oxy)-2-methylpropan-1-amine and 1.5 eq of triethylamine were added at 0°C and the mixture was stirred. After confirming the completion of the reaction, water was added thereto, and then the organic layer was obtained by extraction using methylene chloride and brine. Moisture remaining in the organic layer was completely removed with magnesium sulfate and then filtered. The filtrate was concentrated under reduced pressure to obtain a crude product, which was then purified by column chromatography and concentrated under vacuum to obtain N-(2-((tert-butyldimethylsilyl)oxy)-2-methylpropyl)-3-nitrobenzenesulfonamide.

### Synthesis of 3-amino-N-(2-((tert-butyldimethylsilyl)oxy)-2-methylpropyl)benzenesulfonamide

1 eq of N-(2-((tert-butyldimethylsilyl)oxy)-2-methylpropyl)-3-nitrobenzenesulfonamide was dissolved in ethanol (10 mL/g) and tetrahydrofuran (10 mL/g). 10 wt% of palladium/carbon was added thereto, and the mixture was hydrogenated under a hydrogen atmosphere to perform the reaction. The mixture was stirred at room temperature for 16 hours. After confirming the completion of the reaction, the mixture was filtered through Celite. The filtrate was concentrated under reduced pressure to obtain a crude product, which was then purified by column chromatography and concentrated under vacuum to obtain 3-amino-N-(2-((tert-butyldimethylsilyl)oxy)-2-methylpropyl)benzenesulfonamide.

### Synthesis of N-(2-((tert-butyldimethylsilyl)oxy)-2-methylpropyl)-3-(methylamino)benzenesulfonamide

1 eq of 3-amino-N-(2-((tert-butyldimethylsilyl)oxy)-2-methylpropyl)benzenesulfonamide was dissolved in methanol (10 mL/g). 1.5 eq of paraformaldehyde and 5 eq of sodium methoxide were added thereto, and the mixture was stirred under reflux for about 1 hour. 1 eq of sodium borohydride was added thereto at 0°C and the mixture was stirred for about 30 minutes. Water was added thereto, and then the organic layer was obtained by extraction using ethyl acetate and brine. Moisture remaining in the organic layer was completely removed with magnesium sulfate and then filtered. The filtrate was concentrated under reduced pressure to obtain a crude product, which was then purified by column chromatography and concentrated under vacuum to obtain N-(2-((tert-butyldimethylsilyl)oxy)-2-methylpropyl)-3-(methylamino)benzenesulfonamide.

### Synthesis of N-(2-((tert-butyldimethylsilyl)oxy)-2-methylpropyl)-3-(3-(5-(3-fluorophenoxy)thiazol-2-yl)-1-methylureido)benzenesulfonamide

1 eq of phenyl (5-(3-fluorophenoxy)thiazol-2-yl)carbamate was dissolved in 1,4-dioxane (10 mL/g). 1.5 eq of N-(2-((tert-butyldimethylsilyl)oxy)-2-methylpropyl)-3-(methylamino)benzenesulfonamide and 2 eq of N,N-diisopropylethylamine were added thereto, and the mixture was stirred at 90°C for about 1 hour. After confirming the completion of the reaction, water was added thereto, and then the organic layer was obtained by extraction using ethyl acetate and brine. Moisture remaining in the organic layer was completely removed with magnesium sulfate and then filtered. The filtrate was concentrated under reduced pressure to obtain a crude product, which was then purified by column chromatography and concentrated under vacuum to obtain N-(2-((tert-butyldimethylsilyl)oxy)-2-methylpropyl)-3-(3-(5-(3-fluorophenoxy)thiazol-2-yl)-1-methylureido)benzenesulfonamide.

### Synthesis of 3-(3-(5-(3-fluorophenoxy)thiazol-2-yl)-1-methylureido)-N-(2-hydroxy-2-methylpropyl)benzenesulfonamide (Example 181)

1 eq of N-(2-((tert-butyldimethylsilyl)oxy)-2-methylpropyl)-3-(3-(5-(3-fluorophenoxy)thiazol-2-yl)-1-methylureido)benzenesulfonamide was dissolved in tetrahydrofuran (10 mL/g). 1.2 eq of tetrabutylammonium fluoride was added thereto, and the mixture was stirred under reflux for about 1.5 hours. After confirming the completion of the reaction, water was added thereto, and then the organic layer was obtained by extraction using ethyl acetate and brine. Moisture remaining in the organic layer was completely removed with magnesium sulfate and then filtered. The filtrate was concentrated under reduced pressure to obtain a crude product, which was then purified by column chromatography and concentrated under vacuum to obtain the title **compound of Example 181.**

**[Table 12]**

| **No** | **Structure** | **Analysis data** |
|---|---|---|
| **181** | 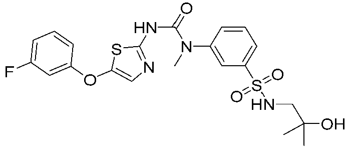 | ¹H NMR (400 MHz, DMSO-d6) δ 11.17 (s, 1H), 7.76 (s, 1H), 7.67 - 7.52 (m, 4H), 7.44 (q, J = 7.8 Hz, 1H), 7.22 (s, 1H), 6.99 (dd, J = 15.7, 8.7 Hz, 3H), 4.45 (s, 1H), 3.37 (s, 3H), 2.65 (d, J = 6.5 Hz, 2H), 1.06 (s, 6H). LC/MS m/z: 495.00 [M+H]+ |

### [Example 182]

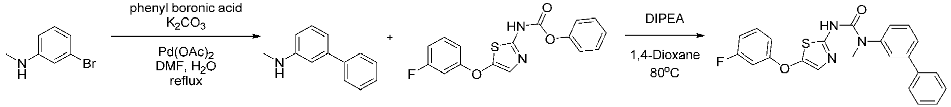

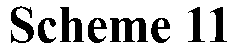

### Synthesis of N-methyl[1,1'-biphenyl]-3-amine (Reactant J2)

1 eq of 3-bromo-N-methylaniline, 2 eq of phenyl boronic acid as **reactant J1,** 4 eq of potassium carbonate, and 0.1 eq of palladium acetate were added to dimethylformamide (50 mL/g) and water (50 mL/g). The mixture was stirred under reflux for 16 hours. After confirming the completion of the reaction, the mixture was filtered through Celite with ethyl acetate, concentrated, and extracted with ethyl acetate and water. The ethyl acetate layer was dried over magnesium sulfate, concentrated under reduced pressure, purified, and separated by column chromatography (hexane:ethyl acetate) to obtain N-methyl[1,1'-biphenyl]-3-amine.

### Synthesis of 1-([1,1'-biphenyl]-3-yl)-3-(5-(3-fluorophenoxy)thiazol-2-yl)-1-methylurea (Example 182)

1 eq of phenyl (5-(3-fluorophenoxy)thiazol-2-yl)carbamate was dissolved in 1,4-dioxane (10 mL/g). 1.5 eq of N-methyl[1,1'-biphenyl]-3-amine as **reactant J2** and 2 eq of N,N-diisopropylethylamine were added thereto, and the mixture was heated to 80°C. After completion of the reaction, the mixture was extracted with ethyl acetate and water. The ethyl acetate layer was dried over magnesium sulfate, concentrated under reduced pressure, purified, and separated by column chromatography (hexane:ethyl acetate) to obtain the title **compound of Example 182.**

### [Example 183]

The **compound of Example 183** was synthesized in substantially the same manner as described in the method for preparing the compound of Example 182 (Scheme 11) by using **reactants J1 and J2 of Table 13** instead of reactants J1 and J2 in the method for preparing the compound of Example 182.

The structures of the compounds of Examples 182-183, reactants J1, J2, and analytical data are shown in Table 13.

**[Table 13]**

| **No** | **Structure** | **Reactant J1** | **Reactant J2** | **Analysis data** |
|---|---|---|---|---|
| **182** | 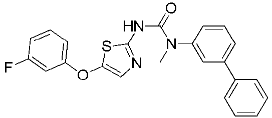 | 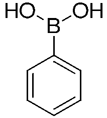 | 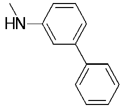 | ¹H NMR (400 MHz, DMSO-d6) δ 7.71 (s, 1H), 7.69 (s, 1H), 7.63 (s, 1H), 7.59 (d, J = 8.1 Hz, 1H), 7.49 (t, J = 6.8 Hz, 3H), 7.41 (dt, J = 14.7, 7.1 Hz, 2H), 7.33 (d, J = 7.6 Hz, 1H), 7.20 (s, 1H), 7.19 - 7.14 (m, 1H), 7.02 - 6.97 (m, 2H), 6.76 (t, J = 8.2 Hz, 1H), 3.33 (s, 3H). LCMS: m/z 420.13 [M+H]+ |
| **183** | 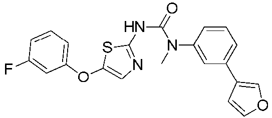 | 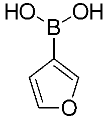 | 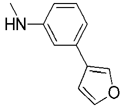 | ¹H NMR (400 MHz, CDCl3) δ 7.80 (s, 1H), 7.58 - 7.50 (m, 3H), 7.43 (s, 1H), 7.24 (t, J = 6.8 Hz, 2H), 6.96 (s, 1H), 6.91 - 6.87 (m, 1H), 6.84 - 6.79 (m, 2H), 6.73 (s, 1H), 3.40 (s, 3H). LCMS: m/z 410.10 [M+H] |

### [Example 184]

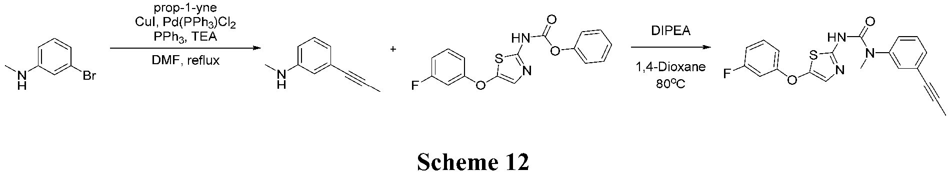

### Synthesis of N-methyl-3-(prop-1-yn-1-yl)aniline

1 eq of 3-bromo-N-methylaniline, 0.1 eq of copper(I) iodide, 0.1 eq of bis(triphenylphosphine)palladium chloride, and 0.1 eq of triphenylphosphine were dissolved in dimethylformamide (1.45 mL/g). After nitrogen substitution, 1.5 eq of triethylamine and 3 eq of propyne were added thereto. The mixture was stirred under reflux for 16 hours. After confirming the completion of the reaction, the mixture was filtered through Celite with ethyl acetate, concentrated, and extracted with ethyl acetate and water. The ethyl acetate layer was dried over magnesium sulfate, concentrated under reduced pressure, purified, and separated by column chromatography (hexane:ethyl acetate) to obtain N-methyl-3-(prop-1-yn-1-yl)aniline.

### Synthesis of 3-(5-(3-fluorophenoxy)thiazol-2-yl)-1-methyl-1-(3-(prop-1-yn-1-yl)phenyl)urea (Example 184)

1 eq of phenyl (5-(3-fluorophenoxy)thiazol-2-yl)carbamate was dissolved in 1,4-dioxane (10 mL/g). 1.5 eq of N-methyl-3-(prop-1-yn-1-yl)aniline and 2 eq of N,N-diisopropylethylamine were added thereto, and the mixture was heated to 80°C. After completion of the reaction, the mixture was extracted with ethyl acetate and water. The ethyl acetate layer was dried over magnesium sulfate, concentrated under reduced pressure, purified, and separated by column chromatography (hexane:ethyl acetate) to obtain the title **compound of Example 184.**

**[Table 14]**

| **No** | **Structure** | **Analysis data** |
|---|---|---|
| **184** | 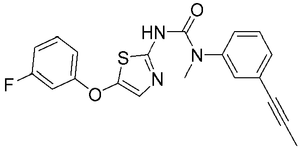 | ¹H NMR (400 MHz, CDCl3) δ 7.42 (d, J = 6.2 Hz, 2H), 7.31 (d, J = 9.1 Hz, 1H), 7.21 (d, J = 6.4 Hz, 1H), 6.95 (s, 1H), 6.86 (dd, J = 12.7, 4.6 Hz, 2H), 6.82 - 6.76 (m, 2H), 3.34 (s, 3H), 2.06 (s, 3H). LCMS: m/z 382.13 [M+H]+ |

### [Reactant K1, K2]

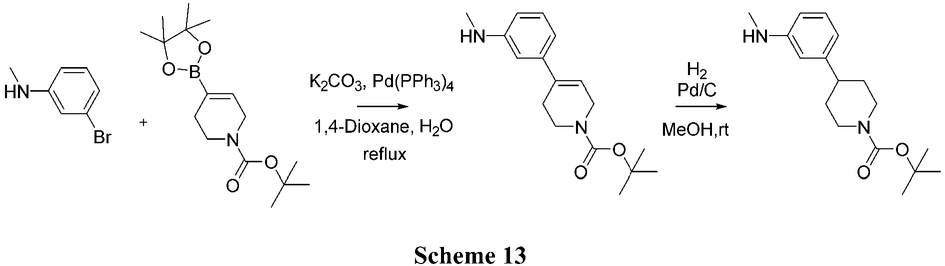

### Synthesis of tert-butyl 4-(3-(methylamino)phenyl)-3,6-dihydro(pyridine)-1(2H)-carboxylate (Reactant K1)

1 eq of 3-bromo-N-methylaniline, 1.2 eq of tert-butyl 4-(4,4,5,5-tetramethyl-1,3,2-dioxaborolan-2-yl)-3,6-dihydro(pyridine)-1(2H)-carboxylate, 4 eq of potassium carbonate, and 0.1 eq of tetrakis(triphenylphosphine)palladium(0) were added to 1,4-dioxane (50 mL/g) and water (10 mL/g). The mixture was stirred under reflux for 16 hours. After confirming the completion of the reaction, the mixture was filtered through Celite with ethyl acetate, concentrated, and extracted with ethyl acetate and water. The ethyl acetate layer was dried over magnesium sulfate, concentrated under reduced pressure, purified, and separated by column chromatography (hexane:ethyl acetate) to obtain tert-butyl 4-(3-(methylamino)phenyl)-3,6-dihydro(pyridine)-1(2H)-carboxylate.

### Synthesis of tert-butyl 4-(3-(methylamino)phenyl)piperidin-1-carboxylate (Reactant K2)

1 eq of tert-butyl 4-(3-(methylamino)phenyl)-3,6-dihydro(pyridine)-1(2H)-carboxylate as **reactant K1** was added to methanol (10 mL/g), and 10 wt% of palladium/carbon was added thereto, and the mixture was hydrogenated under a hydrogen atmosphere to perform the reaction. The mixture was stirred at room temperature for 16 hours. After confirming the completion of the reaction, the mixture was filtered through Celite with ethyl acetate, concentrated, and extracted with ethyl acetate and water. The ethyl acetate layer was dried over magnesium sulfate, concentrated under reduced pressure, purified, and separated by column chromatography (hexane:ethyl acetate) to obtain tert-butyl 4-(3-(methylamino)phenyl)piperidin-1-carboxylate.

### [Example 185]

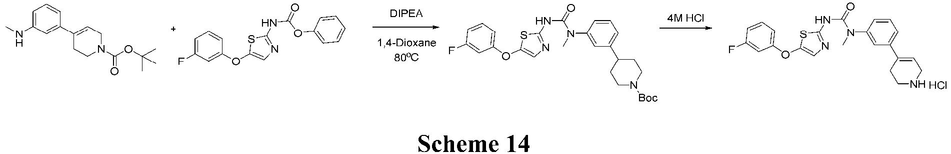

### Synthesis of tert-butyl 4-(3-(3-(5-(3-fluorophenoxy)thiazol-2-yl)-1-methylureido)phenyl)-3,6-dihydro(pyridine)-1(2H)-carboxylate

1 eq of phenyl (5-(3-fluorophenoxy)thiazol-2-yl)carbamate was dissolved in 1,4-dioxane (10 mL/g). 1.5 eq of tert-butyl 4-(3-(methylamino)phenyl)-3,6-dihydro(pyridine)-1(2H)-carboxylate as **reactant K1** and 2 eq of N,N-diisopropylethylamine were added, and the mixture was heated to 80°C. After completion of the reaction, the mixture was extracted with ethyl acetate and water. The ethyl acetate layer was dried over magnesium sulfate, concentrated under reduced pressure, purified, and separated by column chromatography (hexane:ethyl acetate) to obtain tert-butyl 4-(3-(3-(5-(3-fluorophenoxy)thiazol-2-yl)-1-methylureido)phenyl)-3,6-dihydro(pyridine)-1(2H)-carboxylate.

### Synthesis of 3-(5-(3-fluorophenoxy)thiazol-2-yl)-1-methyl-1-(3-(1,2,3,6-tetrahydropyridin-4-yl)phenyl)urea hydrochloride (1:1) (Example 185)

1 eq of tert-butyl 4-(3-(3-(5-(3-fluorophenoxy)thiazol-2-yl)-1-methylureido)phenyl)-3,6-dihydro(pyridine)-1(2H)-carboxylate as **reactant K3** was added to 5 eq of hydrochloric acid having a molar concentration of 4, and the mixture was stirred at room temperature for 16 hours. After confirming the completion of the reaction, the reaction mixture was concentrated under reduced pressure to remove the solvent therefrom. The concentrate was subjected to recrystallization under the conditions of hexane and tetrahydrofuran to obtain the title **compound of Example 185.**

**[Table 15]**

| **No** | **Structure** | **Reactant K1** | **Analysis data** |
|---|---|---|---|
| **185** | 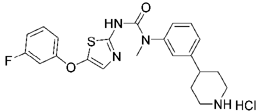 | 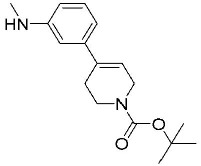 | ¹H NMR (400 MHz, DMSO-d6) δ 10.64 (s, 1H), 8.96 (s, 2H), 7.44 (t, J = 11.6 Hz, 3H), 7.28 (d, J = 7.4 Hz, 1H), 7.21 (s, 1H), 7.05 - 6.95 (m, 2H), 6.24 (s, 1H), 3.8 1 - 3.65 (m, 2H), 3.33 (s, 3H), 2.7 5 - 2.64 (m, 2H), 2.39 - 2.27 (m, 2H).LCMS: m/z 425.47 [M+H]+ |

### [Example 186]

The **compound of Example 186** was synthesized in substantially the same manner as described in the method for preparing the compound of Example 185 **(Scheme 14)** by using **reactant K2 of Table 16** instead of reactant K2 in the method for preparing the compound of Example 185.

**[Table 16]**

| **No** | **Structure** | **Reactant K2** | **Analysis data** |
|---|---|---|---|
| **186** | 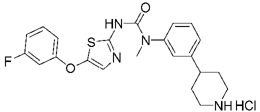 | 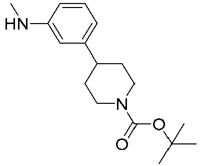 | ¹H NMR (400 MHz, DMSO-d6) δ 10.49 (d, J = 4.6 Hz, 1H), 8. 62 - 8.45 (m, 1H), 8.25 (s, 1H), 7. 37 - 7.22 (m, 2H), 7.09 (d, J = 8.6 Hz, 2H), 7.04 (s, 1H), 6.91 - 6.8 4 (m, 2H), 3.50 (s, 1H), 3.20 (s, 3 H), 2.89 (d, J = 11.8 Hz, 2H), 2.8 2 - 2.68 (m, 2H), 1.85 (d, J = 14.4 Hz, 2H), 1.76 - 1.62 (m, 2H). LCMS: m/z 427.26 [M+H]+ |

### [Example 187]

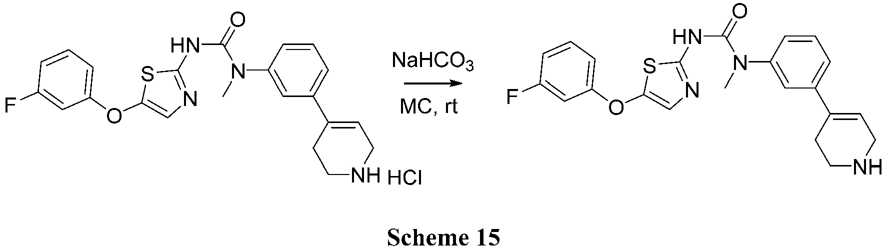

### Synthesis of 3-(5-(3-fluorophenoxy)thiazol-2-yl)-1-methyl-1-(3-(1,2,3,6-tetrahydropyridin-4-yl)phenyl)urea (Example 187)

1 eq of 3-(5-(3-fluorophenoxy)thiazol-2-yl)-1-methyl-1-(3-(1,2,3,6-tetrahydropyridin-4-yl)phenyl)urea hydrochloride (1:1), which is the **compound of Example 185,** was dissolved as a reactant in methylene chloride (50 mL/g), and a saturated sodium hydrogen carbonate solution (50 mL/g) was added thereto, and the mixture was stirred at room temperature for 16 hours. After confirming the completion of the reaction, water was added thereto, and then the organic layer was obtained by extraction using methylene chloride. Moisture remaining in the organic layer was completely removed with magnesium sulfate and then filtered. The filtrate was concentrated under reduced pressure to obtain a crude product, which was then purified and separated by column chromatography (hexane:ethyl acetate) to obtain the title **compound of Example 187.**

**[Table 17]**

| **No** | **Structure** | **Reactant (Example 185)** | **Analysis data** |
|---|---|---|---|
| **187** | 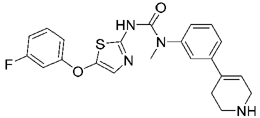 | 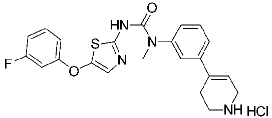 | ¹H NMR (400 MHz, CDC13) δ 7.54 - 7.39 (m, 2H), 7.31 (s, 1H), 6.99 (s, 1H), 6.94 (s, 1H), 6.90 - 6.84 (m, 2H), 6.79 (t, J = 8.1 Hz, 3H), 6.14 (s, 1H), 3 .82 - 3.72 (m, 2H), 3.36 (s, 3H ), 2.70 - 2.55 (m, 2H), 2.04 - 1.96 (m, 2H). LCMS: m/z 425.07 [M+H]+ |

### [Example 188]

The **compound of Example 188** was synthesized in substantially the same manner as described in the method for preparing the compound of Example 187 **(Scheme 15)** by using the **compound of Example 186** of Table 18 instead of the compound of Example 185, which is a reactant in the method for preparing the compound of Example 187.

**[Table 18]**

| **No** | **Structure** | **Reactant (Example 186)** | **Analysis data** |
|---|---|---|---|
| **188** | 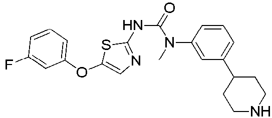 | 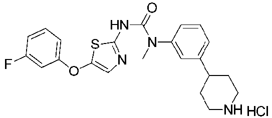 | ¹H NMR (400 MHz, DMSO-d6) δ 7.46 - 7.36 (m, 2H), 7.3 6 - 7.27 (m, 1H), 7.20 - 7.13 ( m, 3H), 6.99 - 6.95 (m, 3H), 6 .93 (s, 1H), 4.37 - 4.27 (m, 1H ), 3.27 (s, 3H), 2.92 - 2.85 (m, 2H), 2.84 - 2.66 (m, 2H), 2.0 0 - 1.91 (m, 2H), 1.83 - 1.75 ( m, 2H). LCMS: m/z 427.26 [M+H]+ |

### [Example 189]

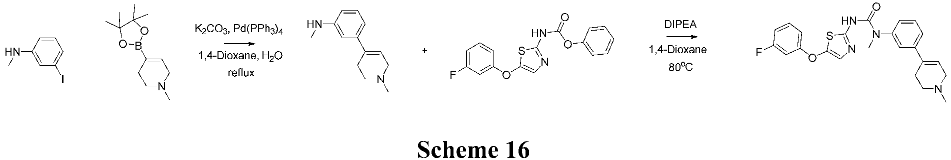

### Synthesis of N-methyl-3-(1-methyl-1,2,3,6-tetrahydropyridin-4-yl)aniline

1 eq of 3-iodo-N-methylaniline, 1.05 eq of 1-methyl-4-(4,4,5,5-tetramethyl-1,3,2-dioxaborolan-2-yl)-1,2,3,6-tetrahydropyridine, 3 eq of potassium carbonate, and 0.1 eq of tetrakis(triphenylphosphine)palladium(0) were added to 1,4-dioxane (2 mL/g) and water (1 mL/g). The mixture was stirred under reflux for 16 hours. After confirming the completion of the reaction, the mixture was filtered through Celite with ethyl acetate, concentrated, and extracted with ethyl acetate and water. The ethyl acetate layer was dried over magnesium sulfate, concentrated under reduced pressure, purified, and separated by column chromatography (hexane:ethyl acetate) to obtain N-methyl-3-(1-methyl-1,2,3,6-tetrahydropyridin-4-yl)aniline.

### Synthesis of 3-(5-(3-fluorophenoxy)thiazol-2-yl)-1-methyl-1-(3-(1-methyl-1,2,3,6-tetrahydropyridin-4-yl)phenyl)urea (Example 189)

After dissolving 1 eq of phenyl (5-(3-fluorophenoxy)thiazol-2-yl)carbamate in 1,4-dioxane (10 mL/g), 1.1 eq of N-methyl-3-(1-methyl-1,2,3,6-tetrahydropyridin-4-yl)aniline and 2 eq of N,N-diisopropylethylamine were added thereto, and the mixture was stirred at 80°C for 2 hours. After confirming the completion of the reaction, the mixture was extracted with ethyl acetate and water. The ethyl acetate layer was dried over magnesium sulfate, concentrated under reduced pressure, purified, and separated by column chromatography (hexane:ethyl acetate) to obtain the title **compound of Example 189.**

**[Table 19]**

| **No** | **Structure** | **Analysis data** |
|---|---|---|
| **189** | 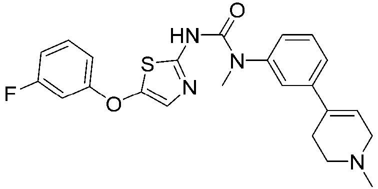 | ¹H NMR (400 MHz, CDC13) δ 7.58 - 7.52 (m, 1H), 7.48 (d, J = 7.9 Hz, 1H), 7.43 - 7.38 (m, 1H), 7.35 (s, 1H), 7.31 (s, 1H), 6.96 (s, 1H), 6.89 (d, J = 8.2 Hz, 1H), 6.84 (d, J = 7.6 Hz, 1H), 6.81 (d, J = 2.5 Hz, 1H), 6.11 (s, 1H), 4.18 (d, J = 17.7 Hz, 1H), 3.72 - 3.61 (m, 1H), 3.60 - 3.50 (m, 2H), 3.39 (s, 3H), 3.27 - 3.14 (m, 1H), 2.96 (s, 3H), 2.81 - 2.72 (m, 1H). LCMS: m/z 439.34 [M+H]+ |

### [Example 190]

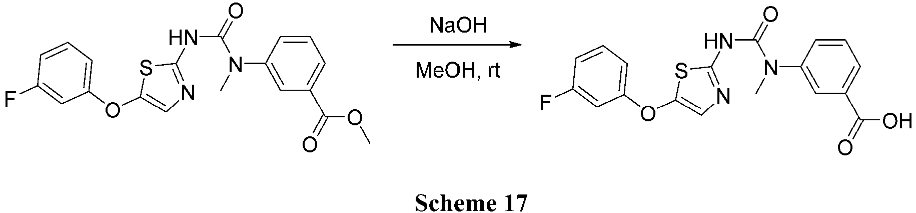

### Synthesis of 3-(3-(5-(3-fluorophenoxy)thiazol-2-yl)-1-methylureido)benzoic acid (Example 190)

After dissolving 1 eq of methyl 3-(3-(5-(3-fluorophenoxy)thiazol-2-yl)-1-methylureido)benzoate of **Example 25** in methanol (50 mL/g), 10 eq of sodium hydroxide was added thereto and the mixture was stirred at room temperature for 16 hours. After confirming the completion of the reaction, the mixture was extracted with ethyl acetate and water. Hydrochloric acid (1 N) was added to the water layer to obtain a solid, which was then filtered with water to obtain the title **compound of Example 190.**

**[Table 20]**

| **No** | **Structure** | **Analysis data** |
|---|---|---|
| **190** | 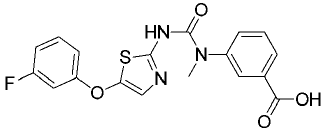 | ¹H NMR (400 MHz, DMSO-d6) δ 7.83 - 7.76 (m, 2H), 7.54 - 7.35 (m, 3H), 7.16 (s, 1H), 6.96 (ddd, J = 14.4, 8.6, 2.2 Hz, 3H), 3.30 (s, 3H). LCMS: m/z 388.1 [M+H]+ |

### [Example 191]

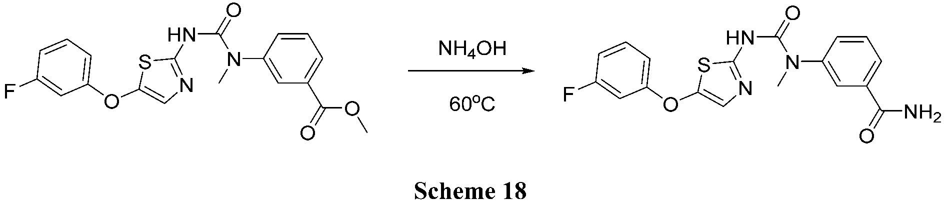

### Synthesis of 3-(3-(5-(3-fluorophenoxy)thiazol-2-yl)-1-methylureido)benzamide (Example 191)

1 eq of methyl 3-(3-(5-(3-fluorophenoxy)thiazol-2-yl)-1-methylureido)benzoate, which is the **compound of Example 25,** was added to a saturated ammonium hydroxide solution (50 mL/g), and the mixture was stirred at 60°C for 12 hours. After confirming the completion of the reaction, the mixture was extracted with ethyl acetate and water. The ethyl acetate layer was dried over magnesium sulfate, concentrated under reduced pressure, purified, and separated by column chromatography (methylene chloride:methanol) to obtain the title **compound of Example 191.**

**[Table 21]**

| **No** | **Structure** | **Analysis data** |
|---|---|---|
| **191** | 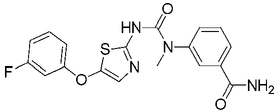 | ¹H NMR (400 MHz, DMSO-d6) δ 10.92 (s, 1H), 8.00 (s, 1H), 7.81 (d, *J* = 2.1 Hz, 1H), 7.78 - 7.75 (m, 1H), 7.50 - 7.45 (m, 2H), 7.44 - 7.39 (m, 2H), 7.18 (s, 1H), 7.03 - 6.92 (m, 3H), 3.30 (s, 3H). LCMS: m/z 387.1 [M+H]+ |

### [Example 192]

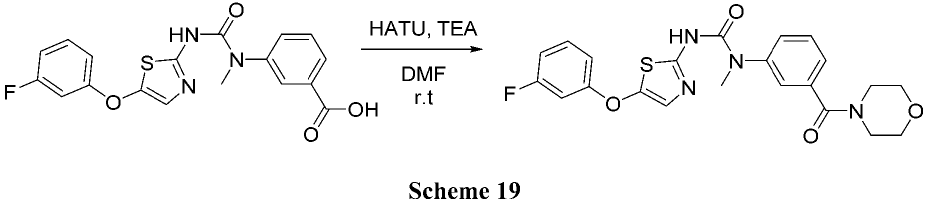

### Synthesis of 3-(5-(3-fluorophenoxy)thiazol-2-yl)-1-methyl-1-(3-(morpholine-4-carbonyl)phenyl)urea (Example 192)

After dissolving 1 eq of 3-(3-(5-(3-fluorophenoxy)thiazol-2-yl)-1-methylureido)benzoic acid, which is the **compound of Example 190,** in dimethylformamide (10 mL/g), 2 eq of hexafluorophosphate azabenzotriazole tetramethyl uronium and 3 eq of triethylamine were added thereto and the mixture was stirred at room temperature for 5 minutes. 1.5 eq of morpholine was added as **reactant L1** to the reaction mixture and the mixture was stirred for 3 hours. After confirming the completion of the reaction, the mixture was extracted with ethyl acetate and water. The ethyl acetate layer was dried over magnesium sulfate, concentrated under reduced pressure, purified, and separated by column chromatography (methylene chloride:methanol) to obtain the title **compound of Example 192.**

### [Examples 193-194]

The **compounds of Examples 193-194** were synthesized in substantially the same manner as described in the method for preparing the compound of Example 192 **(Scheme 19)** by using **reactant L1 of Table 22** instead of reactant L1 in the method for preparing the compound of Example 192.

The structures of the compounds of Examples 192-194, reactant L1, and analytical data are shown in Table 22.

**[Table 22]**

| **No** | **Structure** | **Reactant L1** | **Analysis data** |
|---|---|---|---|
| **192** | 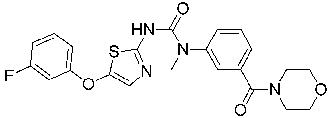 | 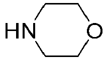 | ¹H NMR (400 MHz, DMSO-d6) δ 7.54 - 7.46 (m, 2H), 7.43 (s, 2H), 7.33 (s, 1H), 7.28 (d, J = 8.5 Hz, 2H), 7.17 (dd, J = 8.3, 2.4 Hz, 1H), 7.10 (td, J = 8.4, 2.4 Hz, 1H), 3.65 (s, 4H), 3.33 (s, 3H), 2.86 (s, 4H). LCMS: m/z 457.1 [M+H]+ |
| **193** | 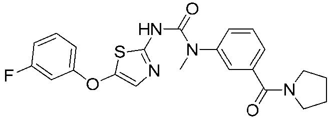 | 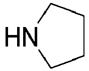 | ¹H NMR (400 MHz, CDCl3) δ 7.60 - 7.51 (m, 3H), 7.39 - 7.35 (m, 1H), 7.30 - 7.23 (m, 1H), 6.94 (s, 1H), 6.86 (dd, J = 8.0, 2.3 Hz, 1H), 6.84 - 6.76 (m 2H), 3.66 (t, J = 6.8 Hz, 2H), 3.52 (t, J = 6.4 Hz, 2H), 3.36 (s, 3H), 2.02 - 1.88 (m, 4H). LCMS: m/z 441.1 [M+H]+ |
| **194** | 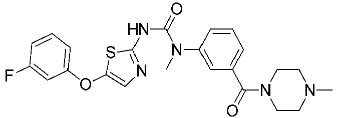 | 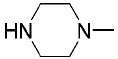 | ¹H NMR (400 MHz, CDCl3) δ 7.55 (t, J = 7.7 Hz, 1H), 7.48 - 7.35 (m, 3H), 7.25 (s, 1H), 6.94 (s, 1H), 6.86 (dd, J = 8.1, 2.0 Hz, 1H), 6.83 - 6.76 (m, 2H), 3.87 (s, 2H), 3.60 (s, 2H), 3.37 (s, 3H), 2.72 - 2.48 (m, 4H), 2.42 (s, 3H). LCMS: m/z 470.2 [M+H]+ |

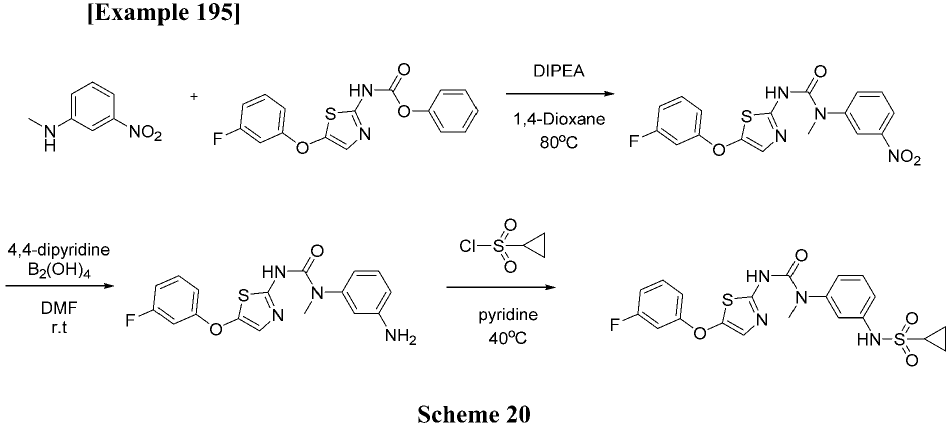

### Synthesis of 3-(5-(3-fluorophenoxy)thiazol-2-yl)-1-methyl-1-(3-nitrophenyl)urea

After dissolving 1 eq of phenyl (5-(3-fluorophenoxy)thiazol-2-yl)carbamate in 1,4-dioxane (10 mL/g), 2 eq of N-methyl-3-nitroaniline and 2 eq of N,N-diisopropylethylamine were added thereto, and the mixture was stirred at 80°C for 2 hours. After confirming the completion of the reaction, the mixture was extracted with ethyl acetate and water. The ethyl acetate layer was dried over magnesium sulfate, concentrated under reduced pressure, purified, and separated by column chromatography (hexane:ethyl acetate) to obtain 3-(5-(3-fluorophenoxy)thiazol-2-yl)-1-methyl-1-(3-nitrophenyl)urea.

### Synthesis of 1-(3-aminophenyl)-3-(5-(3-fluorophenoxy)thiazol-2-yl)-1-methylurea (Example 195)

After dissolving 1 eq of 3-(5-(3-fluorophenoxy)thiazol-2-yl)-1-methyl-1-(3-nitrophenyl)urea in dimethylformamide (25 mL/g), 3 eq of tetrahydroxydiboron was added thereto. 0.05 eq of 4,4-dipyridine was dissolved in dimethylformamide (10 mL/g), and then slowly added dropwise to the solution at 0°C and the mixture was stirred for 1 minute. After completion of the reaction, the mixture was extracted with ethyl acetate, dried over magnesium sulfate, and then concentrated under reduced pressure. The concentrate was purified and separated by column chromatography (hexane:ethyl acetate) to obtain the title **compound of Example 195.**

**[Table 23]**

| **No** | **Structure** | **Analysis data** |
|---|---|---|
| **195** | 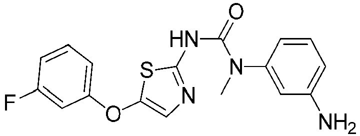 | ¹H NMR (400 MHz, CDCl3) δ 7.77 (s, 1H), 7.27 (s, 2H), 6.97 (d, J = 1.7 Hz, 1H), 6.89 (d, J = 8.5 Hz, 1H), 6.85 - 6.79 (m, 2H), 6.72 (d, J = 8.2 Hz, 1H), 6.68 (d, J = 7.9 Hz, 1H), 6.59 (q, J = 2.1 Hz, 1H), 3.90 (s, 2H), 3.34 (s, 3H). LCMS: m/z 359.1 [M+H]+ |

### [Example 196]

### Synthesis of N-(3-(3-(5-(3-fluorophenoxy)thiazol-2-yl)-1-methylureido)phenyl)cyclopropanesulfonamide (Example 196)

After dissolving 1 eq of 1-(3-aminophenyl)-3-(5-(3-fluorophenoxy)thiazol-2-yl)-1-methylurea as the **compound of Example 195** in 12 eq of pyridine, 1.2 eq of cyclopropanesulfonyl chloride, which is **reactant M1,** was added thereto, and the mixture was stirred at 40°C for 16 hours. After completion of the reaction, the mixture was extracted with ethyl acetate, dried over magnesium sulfate, and then concentrated under reduced pressure. The concentrate was purified and separated by column chromatography (hexane:ethyl acetate) to obtain the title **compound of Example 196.**

### [Example 197]

The **compound of Example 197** was synthesized in substantially the same manner as described in the method for preparing the compound of Example 196 **(Scheme 20)** by using **reactant M1 of Table 24** instead of reactant M1 in the method for preparing the compound of Example 196.

The structures of the compounds of Examples 196-197, reactant M1, and analytical data are shown in Table 24.

**[Table 24]**

| **No** | **Structure** | **Reactant M1** | **Analysis data** |
|---|---|---|---|
| **196** | 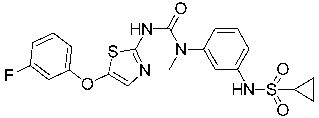 | 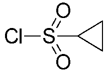 | ¹H NMR (400 MHz, CDCl3) δ 7.73 (s, 1H), 7.54 (s, 1H), 7.41 (d, J = 6.3 Hz, 1H), 7.35 (s, 1H), 7.23 (s, 1H), 7.11 (s, 1H), 7.02 (s, 1H), 6.95 (d, J = 8.6 Hz, 1H), 6.90 (d, J = 9.1 Hz, 1H), 3.37 (s, 3H), 2.41 (s, 1H), 1.11 (s, 2H), 0.97 (s, 2H). LCMS: m/z 463.18 [M+H]+ |
| **197** | 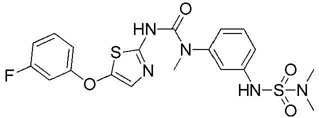 | 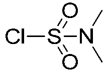 | ¹H NMR (400 MHz, CDC13) δ 7.71 (s, 1H), 7.39 - 7.32 (m, 3H), 7.19 (d, J = 7.8 Hz, 1H), 7.09 (s, 1H), 6.93 (d, J = 7.9 Hz, 2H), 6.88 - 6.85 (m, 1H), 3.28 (s, 3H), 2.82 (d, J = 5.4 Hz, 6H). LCMS: m/z 466.19 [M+H]+ |

### [Example 198]

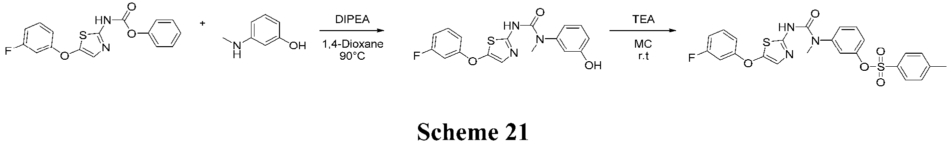

### Synthesis of 3-(5-(3-fluorophenoxy)thiazol-2-yl)-1-(3-hydroxyphenyl)-1-methylurea

1 eq of phenyl (5-(3-fluorophenoxy)thiazol-2-yl)carbamate was dissolved in 1,4-dioxane (10 mL/g). 1.5 eq of 3-(methylamino)phenol and 2 eq of N,N-diisopropylethylamine were added thereto, and the mixture was stirred at 90°C for about 1 hour. After confirming the completion of the reaction, water was added thereto, and then the organic layer was obtained by extraction using ethyl acetate and brine. Moisture remaining in the organic layer was dried over magnesium sulfate, concentrated under reduced pressure, purified, and separated by column chromatography (hexane:ethyl acetate) to obtain 3-(5-(3-fluorophenoxy)thiazol-2-yl)-1-(3-hydroxyphenyl)-1-methylurea.

### Synthesis of 3-(3-(5-(3-fluorophenoxy)thiazol-2-yl)-1-methylureido)phenyl 4-methylbenzenesulfonate (Example 198)

1 eq of 3-(5-(3-fluorophenoxy)thiazol-2-yl)-1-(3-hydroxyphenyl)-1-methylurea of **Example 32** was dissolved in methylene chloride (10 mL/g). 1.2 eq of 4-toluenesulfonyl chloride as **reactant N1** and 2 eq of triethylamine were added thereto and the mixture was stirred at room temperature for about 1 hour. After confirming the completion of the reaction, the mixture was extracted with ethyl acetate and water. The ethyl acetate layer was dried over magnesium sulfate, concentrated under reduced pressure, purified, and separated by column chromatography (hexane:ethyl acetate) to obtain the title **compound of Example 198.**

### [Example 199]

The **compound of Example 199** was synthesized in substantially the same manner as described in the method for preparing the compound of Example 198 **(Scheme 21)** by using **reactant N1 of Table 25** instead of reactant N1 in the method for preparing the compound of Example 198.

The structures of the compounds of Examples 198-199, reactant N1, and analytical data are shown in Table 25.

**[Table 25]**

| **No** | **Structure** | **Reactant N1** | **Analysis data** |
|---|---|---|---|
| **198** | 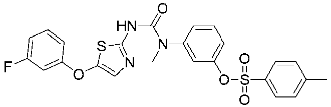 | 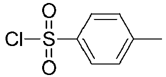 | ¹H NMR (400 MHz, DMSO-d6) δ 11.16 (s, 1H), 7.77 (d, J = 8.1 Hz, 2H), 7.49 - 7.36 (m, 4H), 7.31 (d, J = 8.0 Hz, 1H), 7.23 (s, 1H), 7.04 - 6.89 (m, 5H), 3.22 (s, 3H), 2.39 (s, 3H). LCMS: m/z 514.06 [M+H]+ |
| **199** | 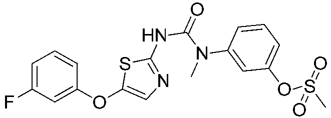 | 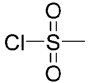 | ¹HNMR (400 MHz, DMSO-d6) δ 11.06 (s, 1H), 7.50 (t, J = 8.1 Hz, 1H), 7.46 - 7.39 (m, 2H), 7.38 - 7.35 (m, 1H), 7.23 (d, J = 8.1 Hz, 1H), 7.20 (s, 1H), 7.02 - 6.94 (m, 3H), 3.40 (s, 3H), 3.33 (s, 3H). LCMS: m/z 438.14 [M+H]+ |

### [Example 200]

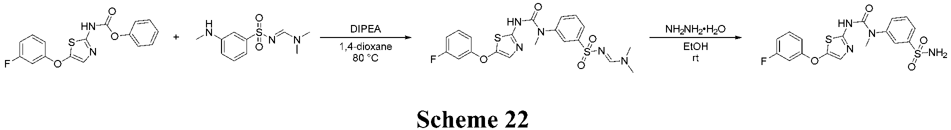

### Synthesis of N'-((3-(3-(5-(3-fluorophenoxy)thiazol-2-yl)-1-methylureido)phenyl)sulfonyl)-N,N-dimethylformimidamide

After dissolving 1 eq of phenyl (5-(3-fluorophenoxy)thiazol-2-yl)carbamate in 1,4-dioxane (10 mL/g), 1.2 eq of N,N-dimethyl-N'-((3-(methylamino)phenyl)sulfonyl)formimidamide and 3 eq of N,N-diisopropylethylamine were added thereto, and the mixture was stirred at 80°C for 2 hours. After completion of the reaction, the mixture was extracted with methylene chloride and water. The methylene chloride layer was dried over magnesium sulfate and then concentrated under reduced pressure. The concentrate was purified and separated by column chromatography (hexane:ethyl acetate) to obtain N'-((3-(3-(5-(3-fluorophenoxy)thiazol-2-yl)-1-methylureido)phenyl)sulfonyl)-N,N-dimethylformimidamide.

### Synthesis of 3-(3-(5-(3-fluorophenoxy)thiazol-2-yl)-1-methylureido)benzenesulfonamide (Example 200)

After dissolving 1 eq of N'-((3-(3-(5-(3-fluorophenoxy)thiazol-2-yl)-1-methylureido)phenyl)sulfonyl)-N,N-dimethylformimidamide in ethanol (15 mL/g), 10 eq of hydrazine hydrate was added thereto and the mixture was stirred at room temperature for 1 hour. After completion of the reaction, the mixture was extracted with ethyl acetate and water. The ethyl acetate layer was dried over magnesium sulfate and then concentrated under reduced pressure. The concentrate was subjected to recrystallization with diethyl ether and hexane to obtain the title **compound of Example 200.**

**[Table 26]**

| **No** | **Structure** | **Analysis data** |
|---|---|---|
| **200** | 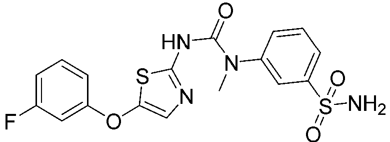 | ¹H NMR (400 MHz, DMSO-d6) δ 11.07 (s, 1H), 7.77 (s, 1H), 7.66 (s, 1H), 7.60 - 7.53 (m, 2H), 7.47 - 7.38 (m, 3H), 7.21 (s, 1H), 7.04 - 6.94 (m, 3H), 3.36 (s, 3H). LCMS: m/z 423.14 [M+H]+ |

### [Example 201]

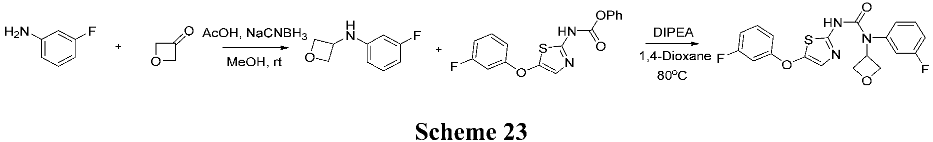

### Synthesis of N-(3-fluorophenyl)oxetan-3-amine (Reactant O2)

1 eq of 3-fluoroaniline was dissolved in methanol (10 mL/g). 2.5 eq of oxetan-3-one and 2 eq of acetic acid were added to the reaction mixture at 0°C and the mixture was stirred at room temperature for about 3 hours. After confirming the disappearance of the starting material, 2 eq of sodium cyanoborohydride was slowly added thereto at 0°C and the mixture was stirred at room temperature for 1 hour. The reaction was terminated using a saturated sodium hydrogen carbonate solution. The reaction mixture was extracted with ethyl acetate and water. The ethyl acetate layer was dried over magnesium sulfate, concentrated under reduced pressure, purified, and separated by column chromatography (hexane:ethyl acetate) to obtain N-(3-fluorophenyl)oxetan-3-amine.

### Synthesis of 3-(5-(3-fluorophenoxy)thiazol-2-yl)-1-(3-fluorophenyl)-1-(oxetan-3-yl)urea (Example 201)

1 eq of phenyl (5-(3-fluorophenoxy)thiazol-2-yl)carbamate was dissolved in 1,4-dioxane (10 mL/g). 1.5 eq of N-(3-fluorophenyl)oxetan-3-amine and 2 eq of N,N-diisopropylethylamine were added to the reaction mixture, and the mixture was stirred at 80°C for 2 hours. After confirming the completion of the reaction, the mixture was extracted with ethyl acetate and water. The ethyl acetate layer was dried over magnesium sulfate, concentrated under reduced pressure, purified, and separated by column chromatography (hexane:ethyl acetate) to obtain the title **compound of Example 201.**

**[Table 27]**

| **No** | **Structure** | **Analysis data** |
|---|---|---|
| **201** | 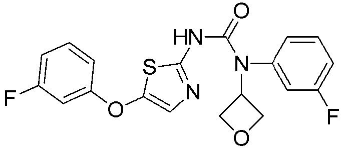 | ¹H NMR (400 MHz, DMSO-d6) δ 7.56 (d, J = 11.7 Hz, 1H), 7.49 - 7.39 (m, 3H), 7.33 (s, 1H), 7.06 - 6.97 (m, 4H), 5.13 (t, J = 5.2 Hz, 1H), 4.73 (m, 1H), 4.06 (dd, J = 10.1, 3.9 Hz, 1H), 3.68 - 3.57 (m, 1H), 3.55 - 3.47 (m, 1H). LCMS: m/z 404.11 [M+H]+ |

### [Example 202]

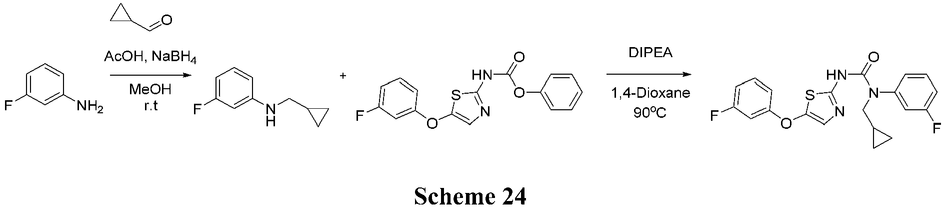

### Synthesis of N-(cyclopropylmethyl)-3-fluoroaniline

1 eq of 3-fluoroaniline was dissolved in methanol (10 mL/g). 1.2 eq of cyclopropanecarbaldehyde and 1.5 eq of acetic acid were added thereto and the mixture was stirred at 0°C for about 1 hour. 1.5 eq of sodium borohydride was added to the reaction mixture and the mixture was stirred at 0°C for about 1 hour. After confirming the completion of the reaction, the mixture was extracted with ethyl acetate and brine. The ethyl acetate layer was dried over magnesium sulfate, concentrated under reduced pressure, purified, and separated by column chromatography (hexane:ethyl acetate) to obtain N-(cyclopropylmethyl)-3-fluoroaniline.

### Synthesis of 1-(cyclopropylmethyl)-3-(5-(3-fluorophenoxy)thiazol-2-yl)-1-(3-fluorophenyl)urea (Example 202)

1 eq of phenyl (5-(3-fluorophenoxy)thiazol-2-yl)carbamate was dissolved in 1,4-dioxane (10 mL/g). 1.5 eq of N-(cyclopropylmethyl)-3-fluoroaniline and 2 eq of N,N-diisopropylethylamine were added to the reaction mixture, and the mixture was stirred at 90°C for 1 hour. After confirming the completion of the reaction, the mixture was extracted with ethyl acetate and brine. The ethyl acetate layer was dried over magnesium sulfate, concentrated under reduced pressure, purified, and separated by column chromatography (hexane:ethyl acetate) to obtain the title **compound of Example 202.**

**[Table 28]**

| **No** | **Structure** | **Analysis data** |
|---|---|---|
| **202** | 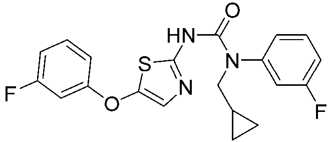 | ¹H NMR (400 MHz, DMSO-d6) δ 10.84 (s, 1H), 7.40 - 7.31 (m, 2H), 7.14 (d, J = 10.3 Hz, 1H), 7.11 - 7.04 (m, 3H), 6.94 - 6.83 (m, 3H), 3.52 (d, J = 6.9 Hz, 2H), 0.91 - 0.80 (m, 1H), 0.34 - 0.23 (m, 2H), 0.03 - -0.06 (m, 2H). LCMS: m/z 402.16 [M+H]+ |

### [Example 203]

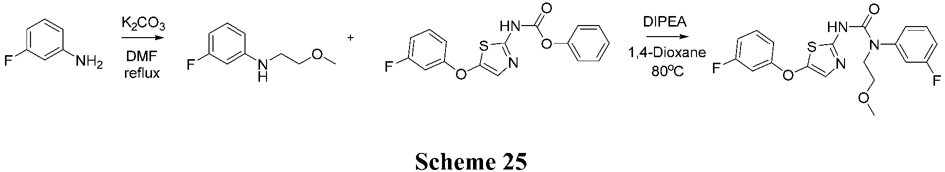

### Synthesis of 3-fluoro-N-(2-methoxyethyl)aniline (Reactant O2)

1 eq of 3-fluoroaniline was dissolved in dimethylformamide (10 mL/g). 1.2 eq of 1-bromo-2-methoxy ethane as **reactant O1** and 2.5 eq of potassium carbonate were added thereto, and the mixture was stirred under reflux for about 24 hours. After confirming the completion of the reaction, the mixture was extracted with ethyl acetate and brine. The ethyl acetate layer was dried over magnesium sulfate, concentrated under reduced pressure, purified, and separated by column chromatography (hexane:ethyl acetate) to obtain 3-fluoro-N-(2-methoxyethyl)aniline.

### Synthesis of 3-(5-(3-fluorophenoxy)thiazol-2-yl)-1-(3-fluorophenyl)-1-(2-methoxyethyl)urea (Example 203)

1 eq of phenyl (5-(3-fluorophenoxy)thiazol-2-yl)carbamate was dissolved in 1,4-dioxane (10 mL/g). 1.5 eq of 3-fluoro-N-(2-methoxyethyl)aniline as **reactant O2** and 2 eq of N,N-diisopropylethylamine were added thereto, and the mixture was stirred at 80°C for about 1 hour. After confirming the completion of the reaction, the mixture was extracted with ethyl acetate and water. The ethyl acetate layer was dried over magnesium sulfate, concentrated under reduced pressure, purified, and separated by column chromatography (hexane:ethyl acetate) to obtain the title **compound of Example 203.**

### [Example 204]

The **compound of Examples 204** was synthesized in substantially the same manner as described in the method for preparing the compound of Example 203 **(Scheme 25)** by using **reactants O1 and O2 of Table 29** instead of reactants O1 and O2 in the method for preparing the compound of Example 203.

The structures of the compounds of Examples 203-204, reactants O1, O2, and analytical data are shown in Table 29.

**[Table 29]**

| **No** | **Structure** | **Reactant O1** | **Reactant O2** | **Analysis data** |
|---|---|---|---|---|
| **203** | 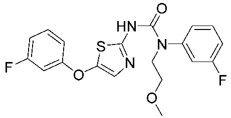 | 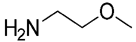 | 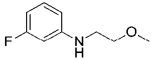 | ¹H NMR (400 MHz, DMSO-d6) δ 10.60 (s, 1H), 7.49 - 7.38 (m, 2H), 7.23 (d, J = 10.6 Hz, 1H), 7.18 (d, J = 9.5 Hz, 3H), 7.04 - 6.94 (m, 3H), 3.88 (t, J = 5.5 Hz, 2H), 3.45 (t, J = 5.7 Hz, 2H), 3.23 (s, 3H). LCMS: m/z 406.23 [M+H]+ |
| **204** | 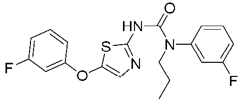 | 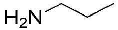 | 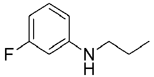 | ¹H NMR (400 MHz, DMSO-d6) δ 10.59 (s, 1H), 7.44 (t, J = 7.6 Hz, 2H), 7.23 (d, J = 10.4 Hz, 1H), 7.18 (s, 1H), 7.15 (d, J = 7.9 Hz, 2H), 7.04 - 6.94 (m, 3H), 3.72 - 3.64 (m, 2H), 1.46 (dd, J = 14.8, 7.3 Hz, 2H), 0.83 (t, J = 7.4 Hz, 3H). LCMS: m/z 390.20 [M+H]+ |

### [Example 205]

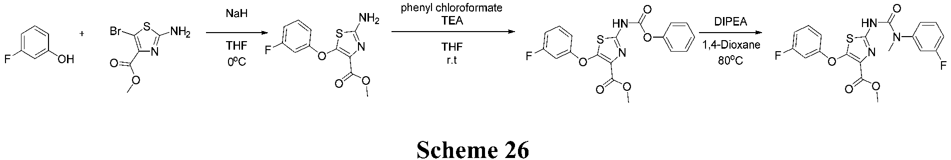

### Synthesis of methyl 2-amino-5-(3-fluorophenoxy)thiazol-4-carboxylate

1 eq of 3-fluorophenol was dissolved in tetrahydrofuran (10 mL/g). 1.5 eq of sodium hydride was slowly added to the reaction mixture at 0°C and the mixture was stirred for about 20 minutes. 1 eq of methyl 2-amino-5-bromothiazol-4-carboxylate was added as **reactant P1** thereto and the mixture was stirred at 0°C for about 1 hour. After confirming the completion of the reaction, water was slowly added at 0°C. The mixture was extracted with ethyl acetate and water. The ethyl acetate layer was dried over magnesium sulfate, concentrated under reduced pressure, purified, and separated by column chromatography (hexane:ethyl acetate) to obtain methyl 2-amino-5-(3-fluorophenoxy)thiazol-4-carboxylate.

### Synthesis of methyl 5-(3-fluorophenoxy)-2-((phenoxycarbonyl)amino)thiazol-4-carboxylate

1 eq of methyl 2-amino-5-(3-fluorophenoxy)thiazol-4-carboxylate was dissolved in tetrahydrofuran (10 mL/g). 1.2 eq of phenyl chloroformate and 1.5 eq of triethylamine were added to the reaction mixture at 0°C. The mixture was stirred at room temperature for about 1 hour. After confirming the completion of the reaction, the mixture was extracted with ethyl acetate and water. The ethyl acetate layer was dried over magnesium sulfate, concentrated under reduced pressure, purified, and separated by column chromatography (hexane:ethyl acetate) to obtain methyl 5-(3-fluorophenoxy)-2-((phenoxycarbonyl)amino)thiazol-4-carboxylate.

### Synthesis of methyl 5-(3-fluorophenoxy)-2-(3-(3-fluorophenyl)-3-methylureido)thiazol-4-carboxylate (Example 205)

1 eq of methyl 5-(3-fluorophenoxy)-2-((phenoxycarbonyl)amino)thiazol-4-carboxylate was dissolved in 1,4-dioxane (10 mL/g). 1.5 eq of 3-fluoro-N-methylaniline as **reactant P2** and 2 eq of N,N-diisopropylethylamine were added thereto, and the mixture was stirred at 90°C for about 1 hour. After confirming the completion of the reaction, the mixture was extracted with ethyl acetate and brine. The ethyl acetate layer was dried over magnesium sulfate, concentrated under reduced pressure, purified, and separated by column chromatography (hexane:ethyl acetate) to obtain the title **compound of Example 205.**

### [Examples 206-210]

The **compounds of Examples 206-210** were synthesized in substantially the same manner as described in the method for preparing the compound of Example 205 **(Scheme 26)** by using **reactants P1 and P2 of Table 30** instead of reactants P1 and P2 in the method for preparing the compound of Example 205.

The structures of the compounds of Examples 205-210, reactants P1, P2, and analytical data are shown in Table 30.

**[Table 30]**

| **No** | **Structure** | **Reactant P1** | **Reactant P2** | **Analysis data** |
|---|---|---|---|---|
| **205** | 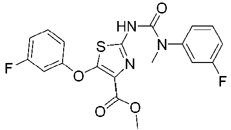 | 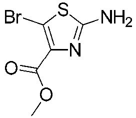 | 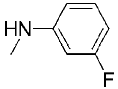 | ¹H NMR (400 MHz, DMSO-d6) δ 11.28 (s, 1H), 7.45 (dt, J = 15.1, 4.3 Hz, 2H), 7.27 (dt, J = 10.6, 2.2 Hz, 1H), 7.21 - 6.97 (m, 5H), 3.68 (s, 3H), 3.31 (s, 3H). LCMS: m/z 420.17 [M+H]+ |
| **206** | 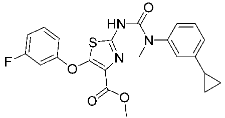 | 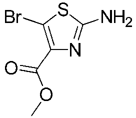 | 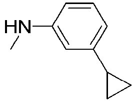 | ¹H NMR (400 MHz, DMSO-d6) δ 11.02 (s, 1H), 7.45 (dd, J = 15.1, 8.2 Hz, 1H), 7.28 (t, J = 7.7 Hz, 1H), 7.11 - 6.96 (m, 6H), 3.67 (s, 3H), 3.27 (s, 3H), 1.98 - 1.90 (m, 1H), 1.00 - 0.92 (m, 2H), 0.74 - 0.67 (m, 2H). LCMS: m/z 442.22 [M+H]+ |
| **207** | 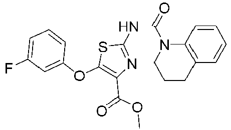 | 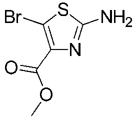 | 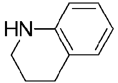 | ¹H NMR (400 MHz, DMSO-d6) δ 11.59 (s, 1H), 7.48 - 7.41 (m, 2H), 7.17 - 6.99 (m, 6H), 3.77 (t, J = 6.1 Hz, 2H), 3.70 (s, 3H), 2.72 (t, J = 6.5 Hz, 2H), 1.93 - 1.85 (m, 2H). LCMS: m/z 428.21 [M+H]+ |
| **208** | 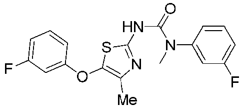 | 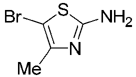 | 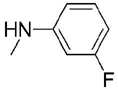 | ¹H NMR (400 MHz, DMSO-d6) δ 11.59 (s, 1s, 1H), 7.42 (dd, J = 15.1, 8.1 Hz, 2H), 7.26 (dt, J = 10.8, 2.2 Hz, 1H), 7.19 (d, J = 8.1 Hz, 1H), 7.08 (t, J = 7.4 Hz, 1H), 6.94 (ddd, J = 22.7, 10.5, 2.2 Hz, 3H), 3.32 (s, 3H), 2.02 (s, 3H). LCMS: m/z 376.24 [M+H]+ |
| **209** | 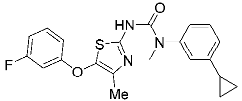 | 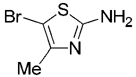 | 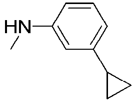 | ¹H NMR (400 MHz, DMSO-d6) δ 10.44 (s, 1H), 7.42 (dd, J = 15.2, 8.2 Hz, 1H), 7.27 (t, J = 7.7 Hz, 1H), 7.06 (d, J = 8.5 Hz, 2H), 7.00 - 6.86 (m, 4H), 3.27 (s, 3H), 2.01 (s, 3H), 1.96 - 1.89 (m, 1H), 1.00 - 0.93 (m, 2H), 0.73 - 0.67 (m, 2H). LCMS: m/z 398.23 [M+H]+ |
| **210** | 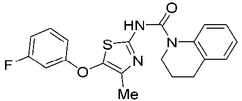 | 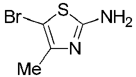 | 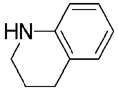 | ¹H NMR (400 MHz, DMSO-d6) δ 11.37 (s, 1H), 7.56 (s, 1H), 7.42 (dd, J = 15.2, 7.8 Hz, 1H), 7.12 (t, J = 7.3 Hz, 2H), 7.02 - 6.90 (m, 4H), 3.80 (t, J = 5.4 Hz, 2H), 2.73 (t, J = 6.5 Hz, 2H), 2.05 (s, 3H), 1.92 - 1.83 (m, 2H). LCMS: m/z 384.25 [M+H]+ |

### [Example 211]

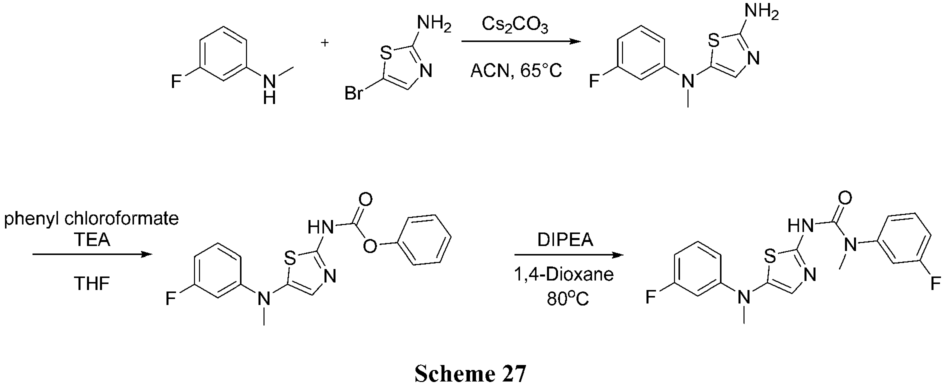

### Synthesis of N⁵-(3-fluorophenyl)-N⁵-methylthiazol-2,5-diamine

After dissolving 1.3 eq of 3-fluoro-N-methylaniline as **reactant Q1** in acetonitrile (30 mL/g), 2 eq of cesium carbonate was added thereto at room temperature. After stirring for 10 minutes, 1 eq of 5-bromothiazol-2-amine was added thereto, and the mixture was stirred at 65°C for 12 hours. After completion of the reaction, the mixture was filtered through Celite with acetone, concentrated, and extracted with ethyl acetate and water. The ethyl acetate layer was dried over magnesium sulfate, concentrated under reduced pressure, purified, and separated by column chromatography (hexane:ethyl acetate) to obtain N⁵-(3-fluorophenyl)-N⁵-methylthiazol-2,5-diamine.

### Synthesis of phenyl (5-((3-fluorophenyl)(methyl)amino)thiazol-2-yl)carbamate

After dissolving 1 eq of N⁵-(3-fluorophenyl)-N⁵-methylthiazol-2,5-diamine in tetrahydrofuran (10 mL/g), 1.5 eq of triethylamine was added thereto and the mixture was stirred at 0°C for 10 minutes. After slowly adding 1.2 eq of phenyl chloroformate, the mixture was stirred at room temperature for 4 hours. After completion of the reaction, the mixture was extracted with methylene chloride, dried over magnesium sulfate, and then concentrated under reduced pressure. The concentrate was purified and separated by column chromatography (hexane:ethyl acetate) to obtain phenyl (5-((3-fluorophenyl)(methyl)amino)thiazol-2-yl)carbamate.

### Synthesis of 1-(3-fluorophenyl)-3-(5-((3-fluorophenyl)(methyl)amino)thiazol-2-vl)-1-methylurea (Example 211)

After dissolving 1 eq of phenyl (5-((3-fluorophenyl)(methyl)amino)thiazol-2-yl)carbamate in 1,4-dioxane (10 mL/g), 1.5 eq of 3-fluoro-N-methylaniline as **reactant Q2** and 2 eq of N,N-diisopropylethylamine were added thereto, and the mixture was stirred at 80°C for 2 hours. After confirming the completion of the reaction, the mixture was extracted with ethyl acetate and water. The ethyl acetate layer was dried over magnesium sulfate, concentrated under reduced pressure, purified, and separated by column chromatography (hexane:ethyl acetate) to obtain the title **compound of Example 211.**

### [Example 212]

The **compound of Examples 212** was synthesized in substantially the same manner as described in the method for preparing the compound of Example 211 **(Scheme 27)** by using **reactants Q1 and Q2 of Table 31** instead of reactants Q1 and Q2 in the method for preparing the compound of Example 211.

The structures of the compounds of Examples 211-212, reactants Q1, Q2, and analytical data are shown in Table 31.

**[Table 31]**

| **No** | **Structure** | **Reactant Q1** | **Reactant Q2** | **Analysis data** |
|---|---|---|---|---|
| **211** | 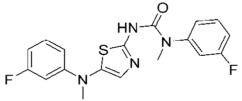 | 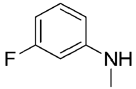 | 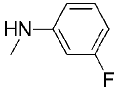 | ¹H NMR (400 MHz, CDCl3) δ 7.52 (dd, J = 14.5, 8.0 Hz, 1H), 7.19 (dt, J = 15.3, 8.6 Hz, 3H), 7.10 - 7.04 (m, 1H), 7.03 (s, 1H), 6.69 - 6.63 (m, 1H), 6.63 - 6.55 (m, 2H), 3.41 (s, 3H), 3.30 (s, 3H). LCMS: m/z 375.22 [M+H] + |
| **212** | 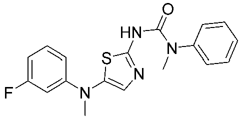 | 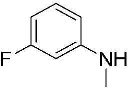 | 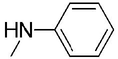 | ¹H NMR (400 MHz, CDCl3) δ 7.56 - 7.53 (m, 2H), 7.51 - 7.47 (m, 1H), 7.34 (d, J = 7.3 Hz, 2H), 7.25 - 7.18 (m, 1H), 6.99 (s, 1H), 6.67 (d, J = 8.4 Hz, 1H), 6.64 - 6.57 (m, 2H), 3.41 (s, 3H), 3.30 (s, 3H). LCMS: m/z 357.23 [M+H]+ |

### [Example 213]

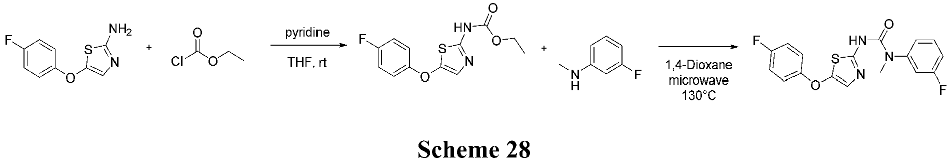

### Synthesis of ethyl (5-(4-fluorophenoxy)thiazol-2-yl)carbamate

After dissolving 1 eq of 5-(4-fluorophenoxy)thiazol-2-amine in tetrahydrofuran (10 mL/g), 1.5 eq of pyridine was added thereto at room temperature and the mixture was stirred for 10 minutes. After slowly adding 1.2 eq of ethyl chloroformate, the mixture was stirred at room temperature for 1 hour. After completion of the reaction, the mixture was concentrated under reduced pressure. The concentrated material was purified and separated by column chromatography (hexane:ethyl acetate) to obtain ethyl (5-(4-fluorophenoxy)thiazol-2-yl)carbamate.

### Synthesis of 3-(5-(4-fluorophenoxy)thiazol-2-yl)-1-(3-fluorophenyl)-1-methylurea (Example 213)

After dissolving 1 eq of ethyl (5-(4-fluorophenoxy)thiazol-2-yl)carbamate in 1,4-dioxane (10 mL/g), 2 eq of 3-fluoro-N-methylaniline as **reactant R1** was added thereto, and the mixture was stirred at 130°C in a microwave for 30 minutes. After completion of the reaction, the mixture was concentrated under reduced pressure. The concentrated material was purified and separated by column chromatography (hexane:ethyl acetate) to obtain the title **compound of Example 213.**

### [Example 214]

The **compound of Example 214** was synthesized in substantially the same manner as described in the method for preparing the compound of Example 213 **(Scheme 28)** by using **reactant R1 of Table 32** instead of reactant R1 in the method for preparing the compound of Example 213.

The structures of the compounds of Examples 213-214, reactant R1, and analytical data are shown in Table 32.

**[Table 32]**

| **No** | **Structure** | **Reactant R1** | **Analysis data** |
|---|---|---|---|
| **213** | 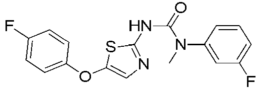 | 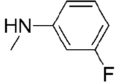 | ¹H NMR (400 MHz, DMSO-d6) δ 10.99 (s, 1H), 7.47 - 7.33 (m, 1H), 7.31 - 7.02 (m, 8H), 3.31 (s, 3H). LCMS: m/z 362.0 [M+H]+ |
| **214** | 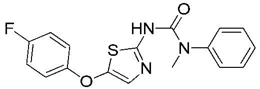 | 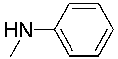 | ¹H NMR (400 MHz, DMSO-d6) δ 10.56 (s, 1H), 7.41 (t, J = 7.7 Hz, 2H), 7.37 - 6.91 (m, 8H), 3.30 (s, 3H). LCMS: m/z 344.1 [M+H]+ |

### [Example 215]

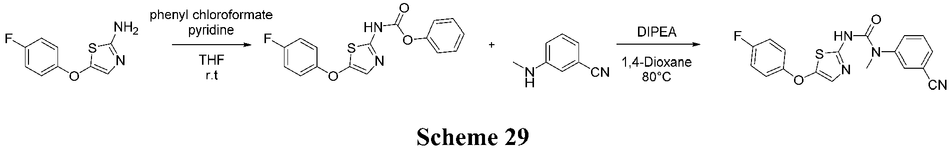

### Synthesis of phenyl (5-(4-fluorophenoxy)thiazol-2-yl)carbamate

After dissolving 1 eq of 5-(4-fluorophenoxy)thiazol-2-amine in tetrahydrofuran (10 mL/g), 1.5 eq of pyridine was added thereto at room temperature and the mixture was stirred for 10 minutes. After slowly adding 1.2 eq of phenyl chloroformate, the mixture was stirred at room temperature for 4 hours. After completion of the reaction, water was added thereto, the organic layer was obtained by extraction using ethyl acetate and tetrahydrofuran, and moisture remaining in the organic layer was removed over magnesium sulfate and then filtered. The filtrate was concentrated under reduced pressure to obtain a crude product, which was then purified and separated by column chromatography (hexane:ethyl acetate) to obtain phenyl (5-(4-fluorophenoxy)thiazol-2-yl)carbamate.

### Synthesis of 1-(3-cyanophenyl)-3-(5-(4-fluorophenoxy)thiazol-2-yl)-1-methylurea (Example 215)

After dissolving 1 eq of phenyl (5-(4-fluorophenoxy)thiazol-2-yl)carbamate and 2 eq of N,N-diisopropylethylamine in 1,4-dioxane (10 mL/g), 1.5 eq of 3-(methylamino)benzonitrile as **reactant S1** was added thereto, and the mixture was stirred at 80°C for 18 hours. After completion of the reaction, the mixture was concentrated under reduced pressure. The concentrated material was purified and separated by column chromatography (hexane:ethyl acetate) to obtain the title **compound of Example 215.**

### [Examples 216-219]

The **compounds of Examples 216-219** were synthesized in substantially the same manner as described in the method for preparing the compound of Example 215 **(Scheme 29)** by using **reactant S1** of **Table 33** instead of reactant S1 in the method for preparing the compound of Example 215.

The structures of the compounds of Examples 215-219, reactant S1, and analytical data are shown in Table 33.

**[Table 33]**

| **No** | **Structure** | **Reactant S1** | **Analysis data** |
|---|---|---|---|
| **215** | 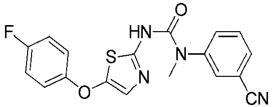 | 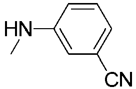 | ¹H NMR (400 MHz, DMSO-d6) δ 11.12 (s, 1H), 7.87 (s, 1H), 7.69 (d, J = 7.7 Hz, 2H), 7.58 (t, J = 8.0 Hz, 1H), 7.28 - 7.12 (m, 5H), 3.33 (s, 3H). LCMS: m/z 369.0 [M+H]+ |
| **216** | 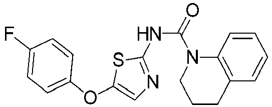 | 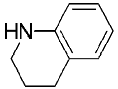 | ¹H NMR (400 MHz, DMSO-d6) δ 11.32 (s, 1H), 7.52 (s, 1H), 7.30 - 7.02 (m, 7H), 7.00 (t, J = 7.4 Hz, 1H), 3.79 (t, J = 5.7 Hz, 2H), 2.73 (t, J = 6.5 Hz, 2H), 1.94 - 1.82 (m, 2H). LCMS: m/z 370.17 [M+H]+ |
| **217** | 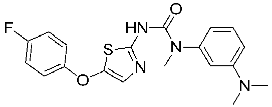 | 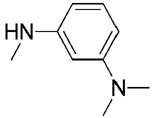 | ¹H NMR (400 MHz, DMSO-d6) δ 9.95 (s, 1H), 7.28 - 7.08 (m, 6H), 6.66 (d, J = 12.6 Hz, 2H), 6.58 (d, J = 7.8 Hz, 1H), 3.25 (s, 3H), 2.87 (s, 6H). LCMS: m/z 387.24 [M+H]+ |
| **218** | 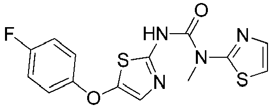 | 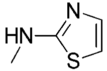 | ¹H NMR (400 MHz, DMSO-d6) δ 12.53 (s, 1H), 7.97 - 8.85 (m, 1H), 7.62 - 7.41 (m, 1H), 7.36 - 7.09 (m, 5H), 3.66 (s, 3H). LCMS: m/z 351.13 [M+H]+ |
| **219** | 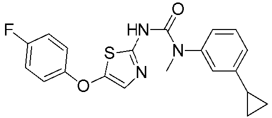 | 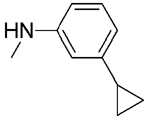 | ¹H NMR (400 MHz, DMSO-d6) δ 10.29 (s, 1H), 7.36 - 7.09 (m, 6H), 7.08 - 6.93 (m, 3H), 3.26 (s, 3H), 1.98 - 1.87 (m, 1H), 1.04 - 0.89 (m, 2H), 0.77 - 0.64 (m, 2H). LCMS: m/z 384.16 [M+H]+ |

### [Example 220]

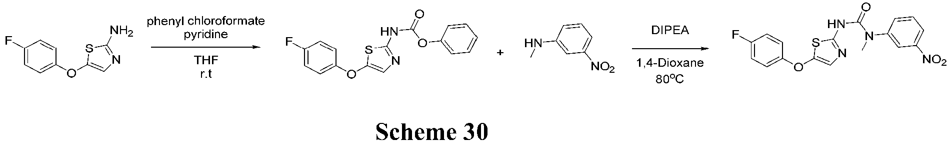

### Synthesis of phenyl (5-(4-fluorophenoxy)thiazol-2-yl)carbamate

After dissolving 5-(4-fluorophenoxy)thiazol-2-amine in tetrahydrofuran (10 mL/g), 1.5 eq of triethylamine was added thereto, and the mixture was cooled to 0°C. After slowly adding 1.2 eq of phenyl chloroformate, the mixture was slowly warmed to room temperature and stirred for 4 hours. After completion of the reaction, the mixture was extracted with ethyl acetate and water, dried over magnesium sulfate, and then concentrated under reduced pressure. The concentrate was purified and separated by column chromatography (hexane:ethyl acetate) to obtain phenyl (5-(4-fluorophenoxy)thiazol-2-yl)carbamate.

### Synthesis of 3-(5-(4-fluorophenoxy)thiazol-2-yl)-1-methyl-1-(3-nitrophenyl)urea (Example 220)

1 eq of phenyl (5-(4-fluorophenoxy)thiazol-2-yl)carbamate was dissolved in 1,4-dioxane (10 mL/g). 1.5 eq of N-methyl-3-nitroaniline as reactant T1 and 2 eq of N,N-diisopropylethylamine were added thereto, and the mixture was heated to 80°C. After completion of the reaction, the mixture was extracted with ethyl acetate and water. The ethyl acetate layer was dried over magnesium sulfate, concentrated under reduced pressure, purified, and separated by column chromatography (hexane:ethyl acetate) to obtain the title **compound of Example 220.**

### [Examples 221-224]

The **compounds of Examples 221-224** were synthesized in substantially the same manner as described in the method for preparing the compound of Example 220 **(Scheme 30)** by using **reactant T1 of Table 34** instead of reactant T1 in the method for preparing the compound of Example 220.

The structures of the compounds of Examples 220-224, reactant T1, and analytical data are shown in Table 34.

**[Table 34]**

| **No** | **Structure** | **Reactant T1** | **Analysis data** |
|---|---|---|---|
| **220** | 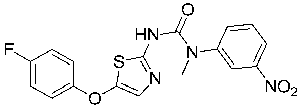 | 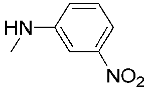 | ¹H NMR (400 MHz, DMSO-d6) δ 8.24 (t, J = 2.2 Hz, 1H), 8.09 (d, J = 8.3 Hz, 1H), 7.83 (ddd, J = 8.1, 2.1, 1.0 Hz, 1H), 7.68 (t, J = 8.1 Hz, 1H), 7.27 - 7.20 (m, 2H), 7.22 - 7.14 (m, 3H), 3.40 (s, 3H). LCMS: m/z 389.25 [M+H]+ |
| **221** | 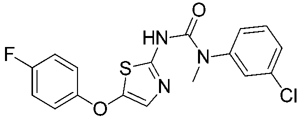 | 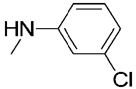 | ¹H NMR (400 MHz, DMSO-d6) δ 10.83 (s, 1H), 7.46 (t, J = 2.0 Hz, 1H), 7.42 (t, J = 8.0 Hz, 1H), 7.34 - 7.29 (m, 2H), 7.27 - 7.20 (m, 2H), 7.20 - 7.15 (m, 2H), 7.15 (s, 1H), 3.31 (s, 3H). LCMS: m/z 378.22 [M+H]+ |
| **222** | 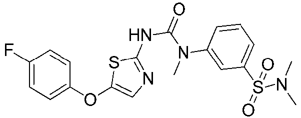 | 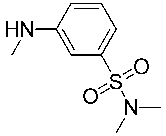 | ¹H NMR (400 MHz, DMSO-d6) δ 10.91 (s, 1H), 7.68 (d, J = 1.9 Hz, 2H), 7.65 (d, J = 8.4 Hz, 1H), 7.60 (s, 1H), 7.28 - 7.19 (m, 2H), 7.20 - 7.14 (m, 3H), 3.37 (s, 3H), 2.64 (s, 6H). LCMS: m/z 451.24 [M+H]+ |
| **223** | 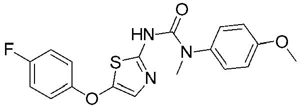 | 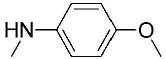 | ¹H NMR (400 MHz, DMSO-d6) δ 10.17 (s, 1H), 7.27 - 7.20 (m, 4H), 7.18 - 7.13 (m, 2H), 7.11 (s, 1H), 7.00 - 6.95 (m, 2H), 3.78 (s, 3H), 3.23 (s, 3H). LCMS: m/z 374.29 [M+H]+ |
| **224** | 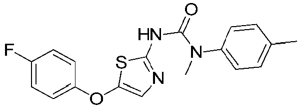 | 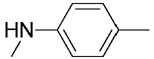 | ¹H NMR (400 MHz, DMSO-d6) δ 10.30 (s, 1H), 7.26 - 7.22 (m, 2H), 7.22 - 7.19 (m, 4H), 7.19 - 7.14 (m, 2H), 7.11 (s, 1H), 3.26 (s, 3H), 2.33 (s, 3H). LCMS: m/z 358.30 [M+H]+ |

### [Example 225]

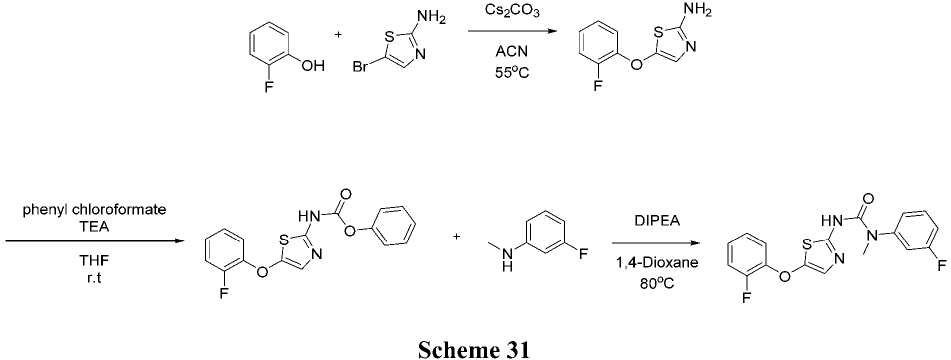

### Synthesis of 5-(2-fluorophenoxy)thiazol-2-amine

After dissolving 1.3 eq of 2-fluorophenol as **reactant U1** in acetonitrile (10 mL/g), 1.5 eq of cesium carbonate was added thereto. After stirring for 10 minutes, 1 eq of 5-bromothiazol-2-amine was added thereto, and the mixture was stirred at 55°C for 15 hours. After completion of the reaction, the mixture was filtered through Celite with acetonitrile and extracted with ethyl acetate and water. The ethyl acetate layer was dried over magnesium sulfate, concentrated under reduced pressure, purified, and separated by column chromatography (hexane:ethyl acetate) to obtain 5-(2-fluorophenoxy)thiazol-2-amine.

### Synthesis of phenyl (5-(2-fluorophenoxy)thiazol-2-yl)carbamate

After dissolving 5-(2-fluorophenoxy)thiazol-2-amine in tetrahydrofuran (10 mL/g), 1.5 eq of triethylamine was added thereto, and the mixture was cooled to 0°C. After slowly adding 1.2 eq of phenyl chloroformate, the mixture was slowly warmed to room temperature and stirred for 4 hours. After completion of the reaction, the mixture was extracted with ethyl acetate and water, dried over magnesium sulfate, and then concentrated under reduced pressure. The concentrate was purified and separated by column chromatography (hexane:ethyl acetate) to obtain phenyl (5-(2-fluorophenoxy)thiazol-2-yl)carbamate.

### Synthesis of 3-(5-(2-fluorophenoxy)thiazol-2-yl)-1-(3-fluorophenyl)-1-methylurea (Example 225)

1 eq of phenyl (5-(2-fluorophenoxy)thiazol-2-yl)carbamate was dissolved in 1,4-dioxane (10 mL/g). 1.5 eq of 3-fluoro-N-methylaniline as **reactant U2** and 2 eq of N,N-diisopropylethylamine were added thereto, and the mixture was the mixture was heated to 80°C. After completion of the reaction, the mixture was extracted with ethyl acetate and water. The ethyl acetate layer was dried over magnesium sulfate, concentrated under reduced pressure, purified, and separated by column chromatography (hexane:ethyl acetate) to obtain the title **compound of Example 225.**

### [Examples 226-325]

The **compounds of Examples 226-325** were synthesized in substantially the same manner as described in the method for preparing the compound of Example 225 **(Scheme 31)** by using **reactants U1 and U2 of Table 35** instead of reactants U1 and U2 in the method for preparing the compound of Example 225.

The structures of the compounds of Examples 225-325, reactants U1, U2, and analytical data are shown in Table 35.

**[Table 35]**

| **No** | **Structure** | **Reactant U1** | **Reactant U2** | **Analysis data** |
|---|---|---|---|---|
| **225** | 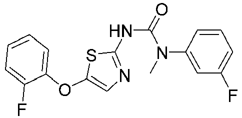 | 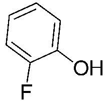 | 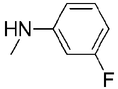 | ¹H NMR (400 MHz, CDCl3) δ 7.44 (td, J = 8.1, 6.5 Hz, 1H), 7.16 (dd, J = 7.8, 2.2 Hz, 1H), 7.14 - 7.07 (m, 3H), 7.07 - 7.03 (m, 2H), 7.02 (t, J = 2.2 Hz, 1H), 6.87 (s, 1H), 3.34 (s, 3H). LCMS: m/z 362.23 [M+H]+ |
| **226** | 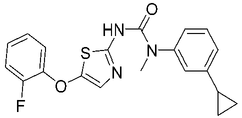 | 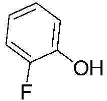 | 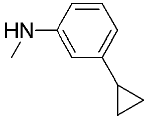 | ¹H NMR (400 MHz, CDCl3) δ 7.33 (t, J = 7.8 Hz, 1H), 7.17 - 7.07 (m, 2H), 7.07 - 7.01 (m, 4H), 6.99 (t, J = 1.9 Hz, 1H), 6.86 (s, 1H), 3.31 (s, 3H), 1.95 - 1.85 (m, 1H), 1.07 - 0.98 (m, 2H), 0.72 (dt, J = 6.7, 5.0 Hz, 2H). LCMS: m/z 384.21 [M+H]+ |
| **227** | 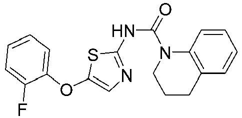 | 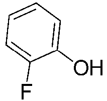 | 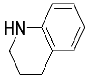 | ¹H NMR (400 MHz, CDCl3) δ 7.34 (d, J = 7.9 Hz, 1H), 7.22 (t, J = 7.7 Hz, 1H), 7.21 - 7.08 (m, 3H), 7.11 - 7.02 (m, 3H), 6.78 (s, 1H), 3.82 (t, J = 6.4 Hz, 2H), 2.76 (t, J = 6.6 Hz, 2H), 1.99 (p, J = 6.6 Hz, 2H). LCMS: m/z 370.23 [M+H]+ |
| **228** | 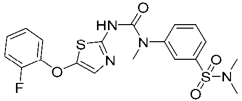 | 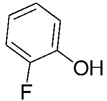 | 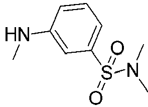 | ¹H NMR (400 MHz, DMSO-d6) δ 10.92 (s, 1H), 7.70 - 7.51 (m, 4H), 7.39 (ddd, J = 11.3, 6.0, 3.3 Hz, 1H), 7.21 (dt, J = 6.7, 3.9 Hz, 3H), 7.18 (s, 1H), 3.33 (s, 3H), 2.64 (s, 6H). LCMS: m/z 451.18 [M+H]+ |
| **229** | 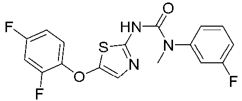 | 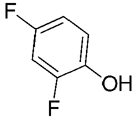 | 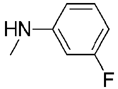 | ¹H NMR (400 MHz, CDCl3) δ 7.36 (t, J = 7.9 Hz, 2H), 7.35 - 7.24 (m, 3H), 6.94 - 6.88 (m, 2H), 6.88 - 6.81 (m, 1H), 1.36 (s, 3H). LCMS: m/z 380.1 [M+H]+ |
| **230** | 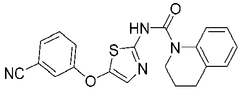 | 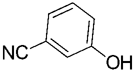 | 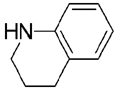 | ¹H NMR (400 MHz, CDCl3) δ 7.39 - 7.33 (m, 1H), 7.33 - 7.28 (m, 2H), 7.24 (d, J = 7.0 Hz, 2H), 7.21 - 7.16 (m, 1H), 7.16 - 7.11 (m, 2H), 7.06 (t, J = 7.3 Hz, 1H), 6.73 (s, 1H), 3.76 (t, J = 6.4 Hz, 2H), 2.71 (t, J = 6.6 Hz, 2H), 1.93 (p, J = 6.6 Hz, 2H). LCMS: m/z 377.28 [M+H]+ |
| **231** | 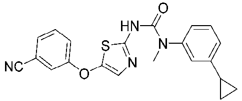 | 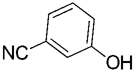 | 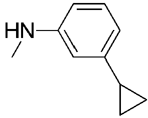 | ¹H NMR (400 MHz, CDCl3) δ 7.84 (s, 1H), 7.45 - 7.28 (m, 5H), 7.06 (dd, J = 7.8, 2.0 Hz, 2H), 7.00 (t, J = 2.0 Hz, 1H), 6.91 (s, 1H), 3.32 (s, 3H), 1.91 (tt, J = 8.3, 5.0 Hz, 1H), 1.04 (dt, J = 6.9, 4.8 Hz, 2H), 0.73 (dt, J = 6.9, 4.8 Hz, 2H). LCMS: m/z 391.29 [M+H]+ |
| **232** | 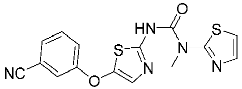 | 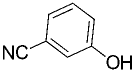 | 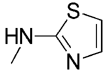 | ¹H NMR (400 MHz, CDCl3) δ 13.48 (s, 1H), 7.51 (d, *J =* 3.7 Hz, 1H), 7.43 (td, *J =* 7.6, 1.1 Hz, 1H), 7.38 (dt, *J* = 7.6, 1.5 Hz, 1H), 7.36 - 7.31 (m, 2H), 7.14 (s, 1H), 7.00 (d, *J =* 3.7 Hz, 1H), 3.54 (s, 3H). LCMS: m/z 358.21 [M+H]+ |
| **233** | 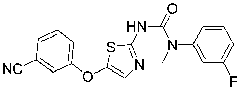 | 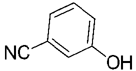 | 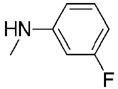 | ¹H NMR (400 MHz, DMSO-d6) δ 10.84 (s, 1H), 7.67 - 7.56 (m, 3H), 7.49 (dt, *J* = 7.5, 2.3 Hz, 1H), 7.43 (q, *J* = 7.8 Hz, 1H), 7.27 (dt, *J* = 10.7, 2.3 Hz, 1H), 7.24 (s, 1H), 7.21 - 7.17 (m, 1H), 7.10 (t, *J* = 7.5 Hz, 1H), 3.33 (s, 3H). LCMS: m/z 369.26 [M+H]+ |
| **234** | 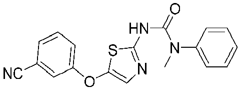 | 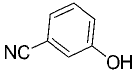 | 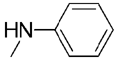 | ¹H NMR (400 MHz, CDCl3) δ 7.56 - 7.50 (m, 2H), 7.48 - 7.39 (m, 3H), 7.37 - 7.32 (m, 4H), 6.97 (s, 1H), 3.38 (s, 3H). LCMS: m/z 351.28 [M+H]+ |
| **235** | 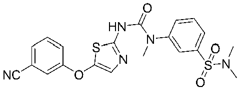 | 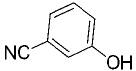 | 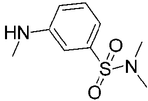 | ¹H NMR (400 MHz, DMSO-d6) δ 10.99 (s, 1H), 7.71 - 7.66 (m, 3H), 7.66 - 7.58 (m, 4H), 7.49 (dt, *J* = 7.4, 2.3 Hz, 1H), 7.25 (s, 1H), 3.38 (s, 3H), 2.64 (s, 6H). LCMS: m/z 458.19 [M+H]+ |
| **236** | 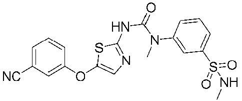 | 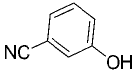 | 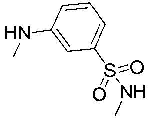 | ¹H NMR (400 MHz, CDCl3) δ 7.93 - 7.88 (m, 2H), 7.71 (t, J = 7.8 Hz, 1H), 7.59 - 7.54 (m, 1H), 7.48 (t, J = 8.6 Hz, 2H), 7.36 (d, J = 8.0 Hz, 2H), 7.02 (s, 1H), 5.06 (s, 1H), 3.44 (s, 3H), 2.81 (d, J = 4.5 Hz, 3H). LCMS: m/z 444.13 [M+H]+ |
| **237** | 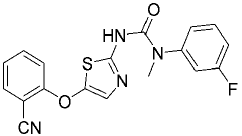 | 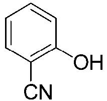 | 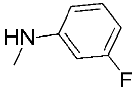 | ¹H NMR (400 MHz, DMSO-d6) δ 10.90 (s, 1H), 7.91 (dd, *J* = 7.7, 1.7 Hz, 1H), 7.72 (ddd, *J =* 8.9, 7.5, 1.7 Hz, 1H), 7.44 (q, *J* = 7.7 Hz, 1H), 7.35 - 7.30 (m, 2H), 7.27 (dt, *J* = 10.8, 2.3 Hz, 1H), 7.23 (d, *J* = 8.5 Hz, 1H), 7.21 - 7.18 (m, 1H), 7.11 (t, *J* = 8.4 Hz, 1H), 3.33 (s, 3H). LCMS: m/z 369.26 [M+H]+ |
| **238** | 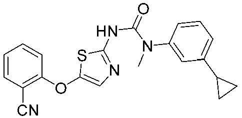 | 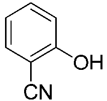 | 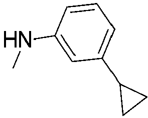 | ¹H NMR (400 MHz, DMSO-d6) δ 10.52 (s, 1H), 7.90 (dd, *J* = 7.7, 1.7 Hz, 1H), 7.72 (ddd, *J =* 8.9, 7.5, 1.7 Hz, 1H), 7.36 - 7.28 (m, 1H), 7.32 - 7.24 (m, 2H), 7.22 (d, *J* = 8.5 Hz, 1H), 7.10 - 7.03 (m, 2H), 6.98 (d, *J* = 7.5 Hz, 1H), 3.28 (s, 3H), 1.94 (tt, *J* = 8.4, 5.1 Hz, 1H), 1.01 - 0.92 (m, 2H), 0.71 (dt, *J* = 6.5, 3.2 Hz, 2H). LCMS: m/z 391.29 [M+H]+ |
| **239** | 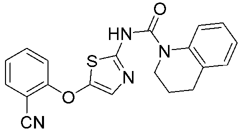 | 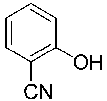 | 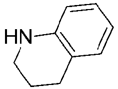 | ¹H NMR (400 MHz, DMSO-d6) δ 11.23 (s, 1H), 7.91 (dd, *J* = 7.7, 1.5 Hz, 1H), 7.73 (ddd, *J =* 8.9, 7.5, 1.6 Hz, 1H), 7.52 (s, 1H), 7.37 (s, 1H), 7.33 (td, *J* = 7.6, 1.0 Hz, 1H), 7.26 (d, *J* = 8.5 Hz, 1H), 7.14 (t, *J =* 7.4 Hz, 2H), 7.01 (t, *J* = 7.3 Hz, 1H), 3.80 (t, *J* = 6.3 Hz, 2H), 2.74 (t, *J* = 6.5 Hz, 2H), 1.89 (p, *J* = 6.4 Hz, 2H). LCMS: m/z 377.26 [M+H]+ |
| **240** | 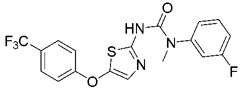 | 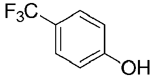 | 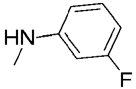 | ¹H NMR (400 MHz, CDCl3) δ 7.57 (d, J = 8.7 Hz, 2H), 7.47 (td, J = 8.2, 6.4 Hz, 1H), 7.18 - 7.09 (m, 4H), 7.04 (dt, J = 9.1, 2.2 Hz, 1H), 6.96 (s, 1H), 3.35 (s, 3H). LCMS: m/z 412.1 [M+H]⁺ |
| **241** | 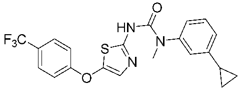 | 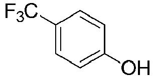 | 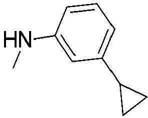 | ¹H NMR (400 MHz, CDCl3) δ 7.57 (t, J = 8.6 Hz, 2H), 7.36 (dd, J = 13.6, 5.8 Hz, 1H), 7.14 (d, J = 8.6 Hz, 2H), 7.10 - 7.03 (m, 2H), 7.00 (t, J = 1.8 Hz, 1H), 6.95 (s, 1H), 3.33 (d, J = 9.7 Hz, 3H), 1.97 - 1.87 (m, 1H), 1.09 - 0.98 (m, 2H), 0.78 - 0.68 (m, 2H). LCMS: m/z 434.1 [M+H]⁺ |
| **242** | 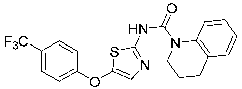 | 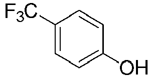 | 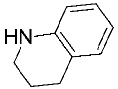 | ¹H NMR (400 MHz, CDCl3) δ 8.62 (s, 1H), 7.59 (t, J = 8.8 Hz, 2H), 7.37 (d, J = 7.5 Hz, 1H), 7.33 - 7.13 (m, 5H), 7.03 - 6.96 (m, 1H), 3.87 (t, J = 6.4 Hz, 2H), 2.81 (t, J = 6.7 Hz, 2H), 2.03 (dd, J = 13.1, 6.6 Hz, 2H). LCMS: m/z 420.1 [M+H]⁺ |
| **243** | 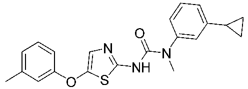 | 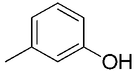 | 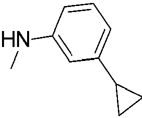 | ¹H NMR (400 MHz, CDCl3) δ 7.38 (t, J = 7.8 Hz, 1H), 7.24 - 7.18 (m, 1H), 7.09 (t, J = 7.8 Hz, 2H), 7.03 (s, 1H), 6.96 - 6.88 (m, 4H), 3.35 (s, 3H), 2.35 (s, 3H), 1.94 (ddd, J = 13.5, 8.5, 5.1 Hz, 1H), 1.06 (dt, J = 6.4, 4.7 Hz, 2H), 0.79 - 0.74 (m, 2H). LCMS: m/z 380.1 [M+H]+ |
| **244** | 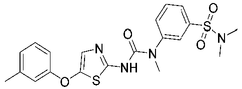 | 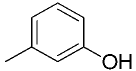 | 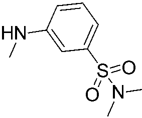 | ¹H NMR (400 MHz, CDCl3) δ 7.83 (d, J = 7.9 Hz, 1H), 7.77 (s, 1H), 7.71 (t, J = 7.9 Hz, 1H), 7.60 (d, J = 7.7 Hz, 1H), 7.22 (t, J = 8.2 Hz, 1H), 6.92 (q, J = 6.5 Hz, 4H), 3.45 - 3.39 (m, 3H), 2.87 - 2.79 (m, 6H), 2.35 (s, 3H). LCMS: m/z 447.2 [M+H]+ |
| **245** | 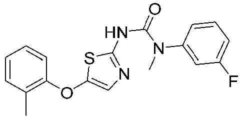 | 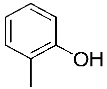 | 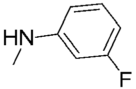 | ¹H NMR (400 MHz, CDCl3) δ 7.39 (td, *J* = 8.2, 6.3 Hz, 1H), 7.13 (dd,*J* = 7.4, 1.7 Hz, 1H), 7.08 - 7.03 (m, 3H), 6.99 - 6.96 (m, 1H), 6.95 - 6.89 (m, 2H), 6.74 (s, 1H), 3.27 (s, 3H), 2.26 (s, 3H). LCMS: m/z 358.26 [M+H]+ |
| **246** | 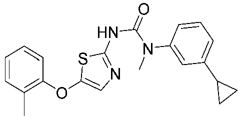 | 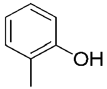 | 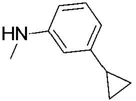 | ¹H NMR (400 MHz, CDCl3) δ 7.33 (t, *J* = 7.8 Hz, 1H), 7.18 (d, *J* = 7.4 Hz, 1H), 7.10 (td, *J* = 7.8, 1.8 Hz, 1H), 7.04 (dd, *J* = 7.8, 1.9 Hz, 2H), 7.02 - 6.95 (m, 3H), 6.78 (s, 1H), 3.30 (s, 3H), 2.32 (s, 3H), 1.90 (tt, *J* = 8.3, 5.0 Hz, 1H), 1.02 (dt, *J =* 6.6, 4.8 Hz, 2H), 0.72 (dt, *J* = 6.6, 4.8 Hz, 2H). LCMS: m/z 380.30 [M+H]+ |
| **247** | 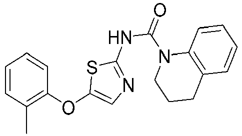 | 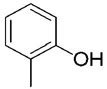 | 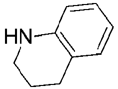 | ¹H NMR (400 MHz, CDCl3) δ 7.35 (d, *J* = 7.9 Hz, 1H), 7.26 - 7.18 (m, 3H), 7.17 - 7.08 (m, 2H), 7.07 - 6.95 (m, 2H), 6.74 (s, 1H), 3.82 (t, *J* = 6.4 Hz, 2H), 2.76 (t, *J* = 6.7 Hz, 2H), 2.34 (s, 3H), 1.99 (p, *J* = 6.5 Hz, 2H). LCMS: m/z 366.25 [M+H]+ |
| **248** | 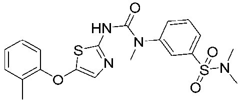 | 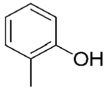 | 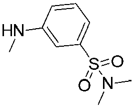 | ¹H NMR (400 MHz, CDCl3) δ 7.77 (dt, *J* = 7.8, 1.2 Hz, 1H), 7.74 (t, *J =* 1.8 Hz, 1H), 7.65 (t, *J =* 7.8 Hz, 1H), 7.56 (ddd, *J* = 7.8, 2.2, 1.1 Hz, 1H), 7.20 (dd, *J* = 7.4, 1.8 Hz, 1H), 7.13 (td, *J* = 7.7, 1.7 Hz, 1H), 7.03 (dd, *J* = 7.4, 1.2 Hz, 1H), 6.99 (t, *J* = 7.2 Hz, 1H), 6.78 (s, 1H), 3.39 (s, 3H), 2.78 (s, 6H), 2.33 (s, 3H). LCMS: m/z 447.19 [M+H]+ |
| **249** | 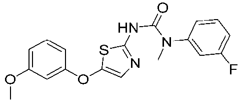 | 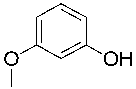 | 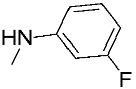 | ¹H NMR (400 MHz, CDCl3) δ 7.70 (s, 1H), 7.46 (td, J = 8.1, 6.4 Hz, 1H), 7.22 - 7.16 (m, 1H), 7.12 (td, J = 8.0, 2.1 Hz, 2H), 7.04 (dt, J = 9.1, 2.2 Hz, 1H), 6.90 (s, 1H), 6.69 - 6.57 (m, 3H), 3.77 (s, 3H), 3.33 (s, 3H). LCMS: m/z 374.25 [M+H]⁺ |
| **250** | 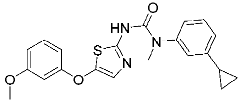 | 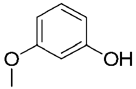 | 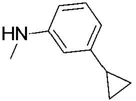 | ¹H NMR (400 MHz, CDCl3) δ 7.70 - 7.52 (m, 1H), 7.34 (t, J = 7.8 Hz, 1H), 7.22 - 7.15 (m, 1H), 7.09 - 7.02 (m, 2H), 7.00 (d, J = 1.8 Hz, 1H), 6.90 (d, J = 6.7 Hz, 1H), 6.68 - 6.57 (m, 3H), 3.76 (s, 3H), 3.31 (s, 3H), 1.98 - 1.84 (m, 1H), 1.09 - 0.95 (m, 2H), 0.78 - 0.65 (m, 2H). LCMS: m/z 396.25 [M+H]⁺ |
| **251** | 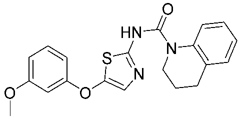 | 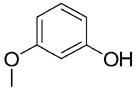 | 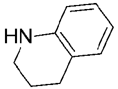 | ¹H NMR (400 MHz, CDCl3) δ 8.79 - 8.26 (m, 1H), 7.33 (d, J = 7.8 Hz, 1H), 7.29 - 7.11 (m, 4H), 6.93 (d, J = 8.4 Hz, 1H), 6.72 - 6.57 (m, 3H), 3.82 (t, J = 6.4 Hz, 2H), 3.77 (s, 3H), 2.77 (t, J = 6.7 Hz, 2H), 2.05 - 1.96 (m, 2H). LCMS: m/z 382.25 [M+H]⁺ |
| **252** | 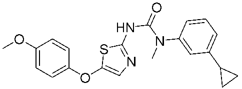 | 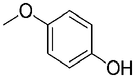 | 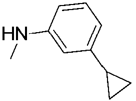 | ¹H NMR (400 MHz, CDCl3) δ 7.60 (s, 1H), 7.34 (dd, J = 14.2, 6.4 Hz, 1H), 7.08 - 6.95 (m, 5H), 6.86 - 6.78 (m, 3H), 3.77 (s, 3H), 3.29 (d, J = 10.0 Hz, 3H), 1.94 - 1.81 (m, 1H), 1.07 - 0.97 (m, 2H), 0.78 - 0.64 (m, 2H). LCMS: m/z 396.1 [M+H]⁺ |
| **253** | 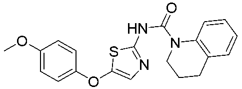 | 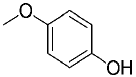 | 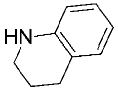 | ¹H NMR (400 MHz, CDCl3) δ 8.48 - 8.17 (m, 1H), 7.32 (d, J = 7.3 Hz, 1H), 7.25 - 7.19 (m, 2H), 7.18 - 7.10 (m, 1H), 7.07 - 7.00 (m, 2H), 6.88 (s, 1H), 6.86 - 6.80 (m, 2H), 3.82 (t, J = 6.4 Hz, 2H), 3.78 (s, 3H), 2.76 (t, J = 6.7 Hz, 2H), 1.98 (p, J = 6.6 Hz, 2H). LCMS: m/z 382.1 [M+H]⁺ |
| **254** | 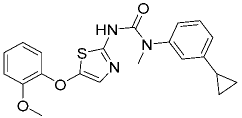 | 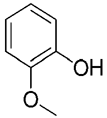 | 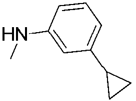 | ¹H NMR (400 MHz, CDCl3) δ 7.34 (t, *J* = 7.8 Hz, 1H), 7.10 - 7.03 (m, 4H), 6.99 (t, *J* = 1.9 Hz, 1H), 6.96 (dd, *J* = 8.1, 1.1 Hz, 1H), 6.90 - 6.84 (m, 2H), 3.90 (s, 3H), 3.31 (s, 3H), 1.95 - 1.86 (m, 1H), 1.06 - 1.00 (m, 2H), 0.76 - 0.70 (m, 2H). LCMS: m/z 396.27 [M+H]+ |
| **255** | 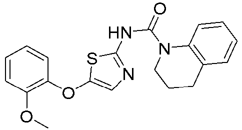 | 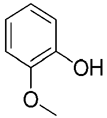 | 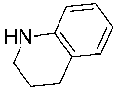 | ¹H NMR (400 MHz, CDCl3) δ 7.34 (dd, *J* = 8.0, 1.3 Hz, 1H), 7.29 - 7.16 (m, 2H), 7.14 (dd, *J* = 7.4, 1.2 Hz, 1H), 7.12 - 7.08 (m, 1H), 7.08 - 7.04 (m, 1H), 6.96 (dd, *J* = 8.1, 1.5 Hz, 1H), 6.90 - 6.87 (m, 1H), 6.86 (s, 1H), 3.91 (s, 3H), 3.82 (t, *J* = 6.4 Hz, 2H), 2.76 (t, *J* = 6.6 Hz, 2H), 1.99 (p, *J =* 6.6 Hz, 2H). LCMS: m/z 382.25 [M+H]+ |
| **256** | 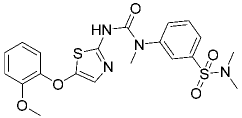 | 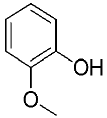 | 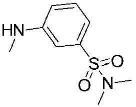 | ¹H NMR (400 MHz, CDCl3) δ 7.79 (dt, *J* = 7.9, 1.3 Hz, 1H), 7.73 (t, *J =* 1.6 Hz, 1H), 7.67 (t, *J =* 7.9 Hz, 1H), 7.56 (ddd, *J* = 7.9, 2.0, 1.0 Hz, 1H), 7.14 - 7.10 (m, 1H), 7.10 - 7.07 (m, 1H), 6.97 (dd, *J* = 8.2, 1.4 Hz, 1H), 6.93 - 6.84 (m, 1H), 6.87 (s, 1H), 3.90 (s, 3H), 3.39 (s, 3H), 2.79 (s, 6H). LCMS: m/z 463.24 [M+H]+ |
| **257** | 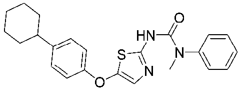 | 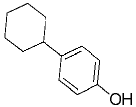 | 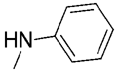 | ¹H NMR (400 MHz, DMSO-d6) δ 10.53 (s, 1H), 7.40 (t, *J* = 7.7 Hz, 2H), 7.33 - 7.29 (m, 2H), 7.29 - 7.19 (m, 3H), 7.08 (s, 1H), 7.03 - 6.99 (m, 2H), 3.29 (s, 3H), 1.80 - 1.72 (m, 5H), 1.69 (d, *J=* 13.2 Hz, 1H), 1.35 (t, *J =* 10.5 Hz, 4H), 1.27 - 1.16 (m, 1H). LCMS: m/z 408.26 [M+H]+ |
| **258** | 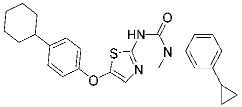 | 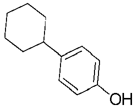 | 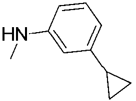 | ¹H NMR (400 MHz, CDCl3) δ 7.34 (t, *J* = 7.8 Hz, 1H), 7.16 - 7.11 (m, 2H), 7.09 - 7.02 (m, 2H), 7.01 - 6.97 (m, 3H), 6.86 (s, 1H), 3.31 (s, 3H), 2.52 - 2.40 (m, 1H), 1.95 - 1.87 (m, 1H), 1.86 - 1.78 (m, 4H), 1.77 - 1.66 (m, 1H), 1.41 - 1.32 (m, 4H), 1.06 - 1.00 (m, 2H), 0.88 (t, *J=* 6.7 Hz, 1H), 0.76 - 0.69 (m, 2H). LCMS: m/z 448.28 [M+H]+ |
| **259** | 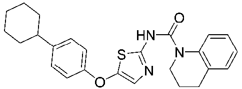 | 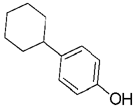 | 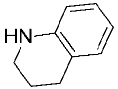 | ¹H NMR (400 MHz, DMSO-d6) δ 11.10 (s, 1H), 7.51 (s, 1H), 7.26 - 7.21 (m, 2H), 7.16 (s, 1H), 7.12 (t, *J* = 7.8 Hz, 2H), 7.06 - 7.02 (m, 2H), 7.00 (t, *J* = 7.6 Hz, 1H), 3.79 (s, 2H), 2.73 (t, *J* = 6.6 Hz, 2H), 1.88 (p, *J* = 6.3 Hz, 2H), 1.78 (d, *J* = 9.6 Hz, 4H), 1.70 (d, *J* = 12.9 Hz, 1H), 1.45 - 1.30 (m, 4H), 1.28 - 1.19 (m, 2H). LCMS: m/z 434.24 [M+H]+ |
| **260** | 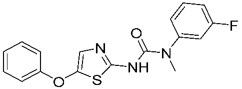 | 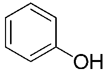 | 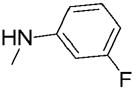 | ¹H NMR (400 MHz, CDCl3) δ 7.49 (dd, J = 14.5, 8.1 Hz, 1H), 7.34 (t, J = 8.0 Hz, 2H), 7.19 - 7.04 (m, 6H), 6.92 (s, 1H), 3.37 (s, 3H). LCMS: m/z 344.1 [M+H]+ |
| **261** | 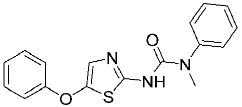 | 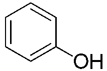 | 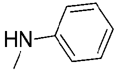 | ¹H NMR (400 MHz, CDCl3) δ 7.53 (t, J = 7.5 Hz, 2H), 7.45 (t, J = 7.4 Hz, 1H), 7.37 - 7.31 (m, 4H), 7.11 (t, J = 8.7 Hz, 3H), 6.93 (s, 1H), 3.38 (s, 3H). LCMS: m/z 326.0 [M+H]+ |
| **262** | 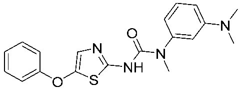 | 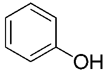 | 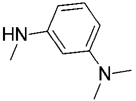 | ¹H NMR (400 MHz, CDCl3) δ 7.33 (td, J = 8.1, 2.8 Hz, 3H), 7.11 (dd, J = 7.9, 5.1 Hz, 3H), 6.93 (s, 1H), 6.74 (dd, J = 8.5, 2.4 Hz, 1H), 6.61 (d, J = 7.6 Hz, 1H), 6.55 (d, J = 2.0 Hz, 1H), 3.35 (s, 3H), 3.01 (s, 6H). LCMS: m/z 369.1 [M+H]+ |
| **263** | 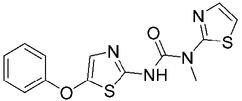 | 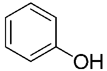 | 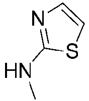 | ¹H NMR (400 MHz, CDCl3) δ 7.55 (d, J = 3.7 Hz, 1H), 7.38 - 7.32 (m, 2H), 7.13 (dd, J = 6.9, 5.2 Hz, 4H), 7.02 (d, J = 3.7 Hz, 1H), 3.56 (s, 3H). ). LCMS: m/z 333.0 [M+H]+ |
| **264** | 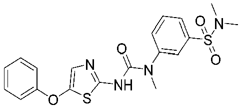 | 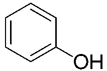 | 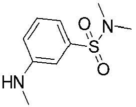 | ¹H NMR (400 MHz, CDCl3) δ 7.84 (d, J = 7.9 Hz, 1H), 7.77 (s, 1H), 7.71 (t, J = 7.9 Hz, 1H), 7.59 (d, J = 7.9 Hz, 1H), 7.36 (t, J = 8.0 Hz, 3H), 7.13 (dd, J = 18.8, 7.6 Hz, 3H), 6.93 (s, 1H), 3.43 (s, 3H), 2.83 (s, 6H). LCMS: m/z 433.1 [M+H]+ |
| **265** | 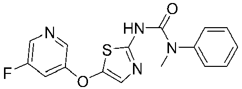 | 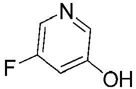 | 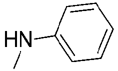 | ¹H NMR (400 MHz, CDCl3) δ 8.31 (d, J = 2.2 Hz, 1H), 8.24 (d, J = 2.3 Hz, 1H), 7.51 (t, J = 7.5 Hz, 2H), 7.43 (t, J = 7.4 Hz, 1H), 7.31 (d, J = 7.3 Hz, 2H), 7.11 (dt, J = 9.5, 2.3 Hz, 1H), 6.97 (s, 1H), 3.36 (s, 3H). LCMS: m/z 345.17 [M+H]⁺ |
| **266** | 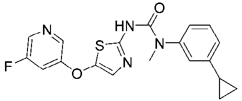 | 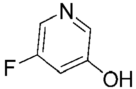 | 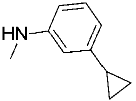 | ¹H NMR (400 MHz, CDCl3) δ 8.30 (d, J = 2.1 Hz, 1H), 8.22 (d, J = 2.3 Hz, 1H), 7.33 (t, J = 7.8 Hz, 1H), 7.10 (dt, J = 9.5, 2.3 Hz, 1H), 7.04 (d, J = 7.9 Hz, 2H), 6.99 (s, 1H), 6.94 (s, 1H), 3.32 (s, 3H), 1.89 (td, J = 8.4, 4.3 Hz, 1H), 1.08 - 0.95 (m, 2H), 0.78 - 0.56 (m, 2H). LCMS: m/z 385.1 [M+H]⁺ |
| **267** | 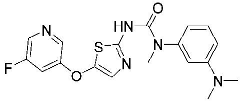 | 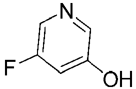 | 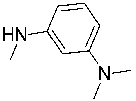 | ¹H NMR (400 MHz, CDCl3) δ 8.30 (d, J = 2.2 Hz, 1H), 8.22 (d, J = 2.3 Hz, 1H), 7.28 (t, J = 8.1 Hz, 1H), 7.12 (dt, J = 9.4, 2.3 Hz, 1H), 7.01 - 6.89 (m, 1H), 6.70 (dd, J = 8.5, 2.4 Hz, 1H), 6.57 (d, J = 7.6 Hz, 1H), 6.51 (d, J = 2.1 Hz, 1H), 3.38 - 3.24 (m, 3H), 3.00 - 2.88 (m, 6H). LCMS: m/z 388.23 [M+H]⁺ |
| **268** | 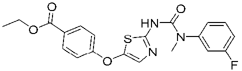 | 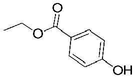 | 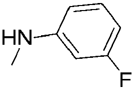 | ¹H NMR (400 MHz, DMSO-d6) δ 10.84 (s, 1H), 8.02 - 7.95 (m, 2H), 7.48 - 7.38 (m, 1H), 7.30 - 7.22 (m, 2H), 7.23 - 7.16 (m, 3H), 7.10 (s, 1H), 4.30 (q, *J* = 7.1 Hz, 2H), 3.32 (s, 3H), 1.31 (t, *J* = 7.1 Hz, 3H). LCMS: m/z 416.20 [M+H]+ |
| **269** | 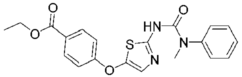 | 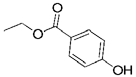 | 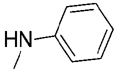 | ¹H NMR (400 MHz, CDCl3) δ 7.96 - 7.90 (m, 2H), 7.42 (dd, *J* = 8.5, 6.9 Hz, 2H), 7.38 - 7.31 (m, 1H), 7.28 - 7.23 (m, 2H), 7.04 - 6.99 (m, 2H), 6.85 (s, 1H), 4.28 (q, *J* = 7.1 Hz, 2H), 3.28 (s, 3H), 1.31 (t, *J* = 7.1 Hz, 3H). LCMS: m/z 398.28 [M+H]+ |
| **270** | 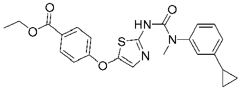 | 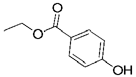 | 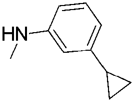 | ¹H NMR (400 MHz, CDCl3) δ 8.06 - 7.95 (m, 2H), 7.35 (t, *J* = 7.8 Hz, 1H), 7.10 - 7.04 (m, 4H), 7.01 (t, *J* = 2.0 Hz, 1H), 6.92 (s, 1H), 4.35 (q, *J =* 7.1 Hz, 2H), 3.32 (s, 3H), 1.95 - 1.86 (m, 1H), 1.37 (t, *J* = 7.1 Hz, 3H), 1.07 - 1.00 (m, 2H), 0.76 - 0.70 (m, 2H). LCMS: m/z 438.26 [M+H]+ |
| **271** | 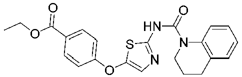 | 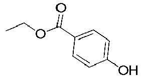 | 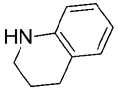 | ¹H NMR (400 MHz, CDCl3) δ 8.05 - 7.97 (m, 2H), 7.35 (dd, *J* = 8.0, 1.2 Hz, 1H), 7.30 - 7.18 (m, 2H), 7.15 (td, *J* = 7.4, 1.2 Hz, 1H), 7.12 - 7.06 (m, 2H), 6.92 (s, 1H), 4.36 (q, *J* = 7.1 Hz, 2H), 3.84 (t, *J* = 6.4 Hz, 2H), 2.78 (t, *J* = 6.7 Hz, 2H), 2.00 (p, *J =* 6.5 Hz, 2H), 1.38 (t, *J =* 7.1 Hz, 3H). LCMS: m/z 424.17 [M+H]+ |
| **272** | 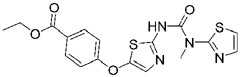 | 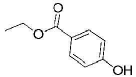 | 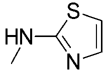 | ¹H NMR (400 MHz, CDCl3) δ 13.58 (s, 1H), 8.07 - 8.03 (m, 2H), 7.56 (d, *J =* 3.7 Hz, 1H), 7.16 (d, *J =* 4.5 Hz, 2H), 7.13 (d, *J =* 1.5 Hz, 1H), 7.03 (d, *J =* 3.7 Hz, 1H), 4.38 (q, *J* = 7.1 Hz, 2H), 3.57 (s, 3H), 1.41 (t, *J* = 7.1 Hz, 3H). LCMS: m/z 405.15 [M+H]+ |
| **273** | 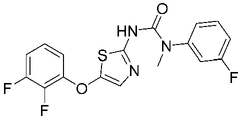 | 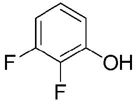 | 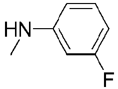 | ¹H NMR (400 MHz, DMSO-d6) δ 11.15 (s, 1H), 7.42 (dd, J = 15.3, 7.6 Hz, 1H), 7.32 - 7.15 (m, 5H), 7.13 - 6.99 (m, 2H), 3.34 (s, 3H). LCMS: m/z 380.13 [M+H]+ |
| **274** | 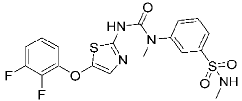 | 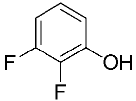 | 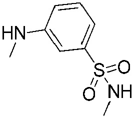 | ¹H NMR (400 MHz, DMSO-d6) δ 11.18 (br s, 1H), 7.72 (s, 1H), 7.61 (s, 3H), 7.49 (d, J = 5.0 Hz, 1H), 7.29 - 7.16 (m, 3H), 7.06 (t, J = 7.3 Hz, 1H), 3.36 (s, 3H), 2.44 (d, J = 4.4 Hz, 3H). LCMS: m/z 455.20 [M+H]+ |
| **275** | 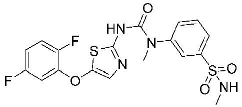 | 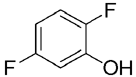 | 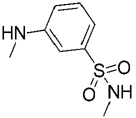 | ¹H NMR (400 MHz, DMSO-d6) δ 11.22 (s, 1H), 7.73 (s, 1H), 7.62 (s, 3H), 7.53 - 7.42 (m, 2H), 7.23 (s, 1H), 7.20 - 7.11 (m, 1H), 7.10 - 7.01 (m, 1H), 3.37 (s, 3H), 2.45 (s, 3H). LCMS: m/z 455.12 [M+H]+ |
| **276** | 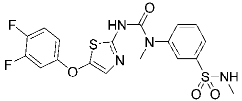 | 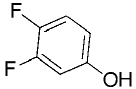 | 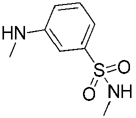 | ¹H NMR (400 MHz, DMSO-d6) δ 11.21 (s, 1H), 7.73 (s, 1H), 7.63 - 7.59 (m, 3H), 7.52 - 7.44 (m, 2H), 7.35 - 7.28 (m, 1H), 7.19 (s, 1H), 7.01 - 6.93 (m, 1H), 3.37 (s, 3H), 2.45 (d, J = 4.6 Hz, 3H). LCMS: m/z 455.15 [M+H]+ |
| **277** | 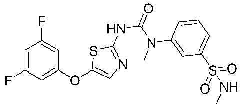 | 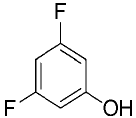 | 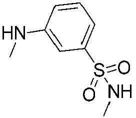 | ¹H NMR (400 MHz, DMSO-d6) δ 11.19 (s, 1H), 7.73 (s, 1H), 7.63 (s, 3H), 7.53 - 7.47 (m, 1H), 7.27 (s, 1H), 7.05 (t, J = 9.2 Hz, 1H), 6.92 (d, J = 7.3 Hz, 2H), 3.38 (s, 3H), 2.45 (d, J = 5.0 Hz, 3H). LCMS: m/z 455.10 [M+H]+ |
| **278** | 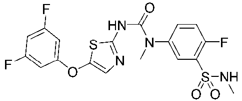 | 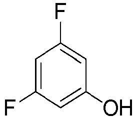 | 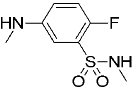 | ¹H NMR (400 MHz, CDCl3) δ 8.24 (s, 1H), 7.88 (dd, J = 6.1, 2.7 Hz, 1H), 7.59 - 7.52 (m, 1H), 7.33 (t, J = 9.0 Hz, 1H), 6.96 (s, 1H), 6.69 - 6.54 (m, 3H), 6.02 (s, 1H), 3.38 (s, 3H), 2.79 (d, J = 5.0 Hz, 3H). LCMS: m/z 473. 11 [M+H]+ |
| **279** | 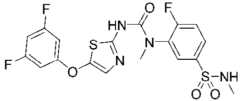 | 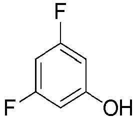 | 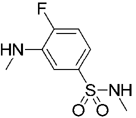 | ¹H NMR (400 MHz, CDCl3) δ 8.41 (s, 1H), 7.97 - 7.90 (m, 1H), 7.90 - 7.84 (m, 1H), 7.41 (t, J = 8.9 Hz, 1H), 6.96 (s, 1H), 6.69 - 6.54 (m, 3H), 5.30 (s, 1H), 3.37 (s, 3H), 2.75 (d, J = 5.2 Hz, 3H). LCMS: m/z 472.98 [M+H]+ |
| **280** | 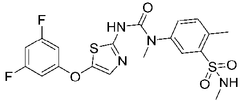 | 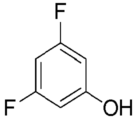 | 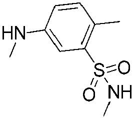 | ¹H NMR (400 MHz, CDCl3) δ 8.41 (s, 1H), 7.94 (d, J = 7.0 Hz, 1H), 7.86 (s, 1H), 7.41 (t, J = 8.9 Hz, 1H), 6.96 (s, 1H), 6.64 (d, J = 6.2 Hz, 2H), 6.59 (t, J = 8.9 Hz, 1H), 3.39 (s, 3H), 2.74 (d, J = 5.3 Hz, 3H), 2.69 (s, 3H). LCMS: m/z 396.06 [M+H]+ |
| **281** | 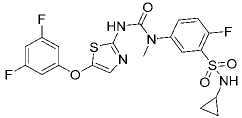 | 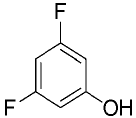 | 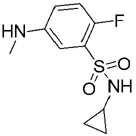 | ¹H NMR (400 MHz, CDCl3) δ 8.41 (s, 1H), 7.94 (d, J = 7.0 Hz, 1H), 7.60 - 7.51 (m, 1H), 7.34 (t, J = 9.0 Hz, 1H), 6.99 (s, 1H), 6.64 (d, J = 6.8 Hz, 2H), 6.59 (t, J = 9.5 Hz, 1H), 3.39 (s, 3H), 2.35 (s, 1H), 0.91 (t, J = 6.5 Hz, 2H), 0.67 (s, 2H). LCMS: m/z 498.96 [M+H]+ |
| **282** | 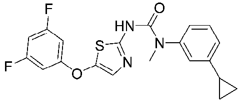 | 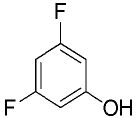 | 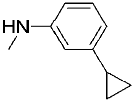 | ¹H NMR (400 MHz, CDCl3) δ 7.60 (s, 1H), 7.39 (t, J = 7.8 Hz, 1H), 7.10 (t, J = 7.6 Hz, 2H), 7.04 (s, 1H), 6.99 (s, 1H), 6.67 - 6.53 (m, 3H), 3.36 (s, 3H), 1.99 - 1.90 (m, 1H), 1.11 - 1.03 (m, 2H), 0.79 - 0.74 (m, 2H). LCMS: m/z 402.18 [M+H]+ |
| **283** | 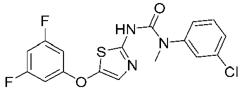 | 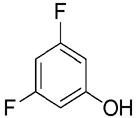 | 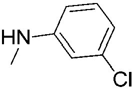 | ¹H NMR (400 MHz, DMSO-d6) δ 11.13 (br s, 1H), 7.46 (s, 1H), 7.41 (t, J = 8.0 Hz, 1H), 7.31 (d, J = 7.8 Hz, 2H), 7.24 (s, 1H), 7.04 (t, J = 9.3 Hz, 1H), 6.90 (d, J = 8.0 Hz, 2H), 3.33 (s, 3H). LCMS: m/z 396.06 [M+H]+ |
| **284** | 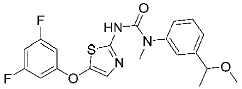 | 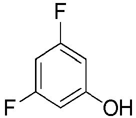 | 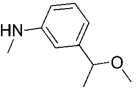 | ¹H NMR (400 MHz, DMSO-d6) δ 10.84 (s, 1H), 7.39 (t, J = 7.5 Hz, 1H), 7.22 (t, J = 10.0 Hz, 4H), 7.04 (t, J = 9.3 Hz, 1H), 6.93 - 6.85 (m, 2H), 4.33 (q, J = 6.4 Hz, 1H), 3.32 (s, 3H), 3.17 (s, 3H), 1.35 (d, J = 6.4 Hz, 3H). LCMS: m/z 420.14 [M+H]+ |
| **285** | 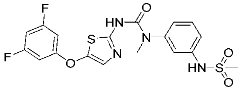 | 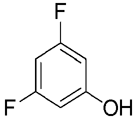 | 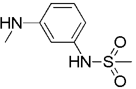 | ¹H NMR (400 MHz, DMSO-d6) δ 11.10 (br s, 1H), 7.72 (s, 1H), 7.62 (s, 3H), 7.53 - 7.43 (m, 1H), 7.26 (s, 1H), 7.04 (t, J = 9.3 Hz, 1H), 6.91 (d, J = 7.3 Hz, 2H), 3.37 (s, 3H), 2.44 (d, J = 5.0 Hz, 3H). LCMS: m/z 455.07 [M+H]+ |
| **286** | 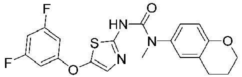 | 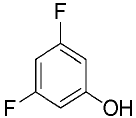 | 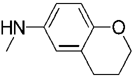 | ¹H NMR (400 MHz, DMSO-d6) δ 10.25 (s, 1H), 7.21 (s, 1H), 7.12 - 6.72 (m, 3H), 6.89 (d, J = 8.3 Hz, 2H), 6.76 (d, J = 8.5 Hz, 1H), 4.18 - 4.13 (m, 2H), 3.21 (s, 3H), 2.75 (t, J = 6.3 Hz, 2H), 1.97 - 1.88 (m, 2H). LCMS: m/z 418.26 [M+H]+ |
| **287** | 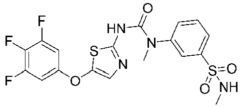 | 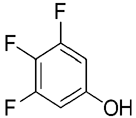 | 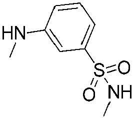 | ¹H NMR (400 MHz, DMSO-d6) δ 11.14 (s, 1H), 7.73 (s, 1H), 7.65 - 7.58 (m, 3H), 7.53 - 7.47 (m, 1H), 7.26 - 7.16 (m, 3H), 3.37 (s, 3H), 2.45 (d, J = 4.8 Hz, 3H). LCMS: m/z 473.06 [M+H]+ |
| **288** | 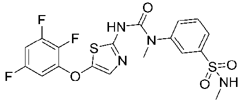 | 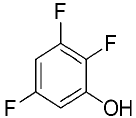 | 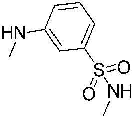 | ¹H NMR (400 MHz, DMSO-d6) δ 11.13 (s, 1H), 7.73 (s, 1H), 7.67 - 7.58 (m, 3H), 7.54 - 7.47 (m, 1H), 7.43 - 7.33 (m, 1H), 7.30 (s, 1H), 7.09 - 6.99 (m, 1H), 3.38 (s, 3H), 2.45 (d, J = 4.6 Hz, 3H). LCMS: m/z 473.11 [M+H]+ |
| **289** | 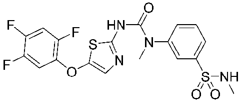 | 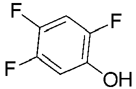 | 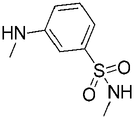 | ¹H NMR (400 MHz, DMSO-d6) δ 11.20 (s, 1H), 7.82 (dd, J = 19.0, 10.7 Hz, 1H), 7.73 (s, 1H), 7.61 (s, 3H), 7.56 - 7.47 (m, 2H), 7.18 (s, 1H), 3.37 (s, 3H), 2.45 (d, J = 4.1 Hz, 3H). LCMS: m/z 473.07 [M+H]+ |
| **290** | 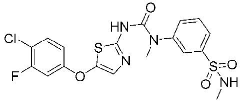 | 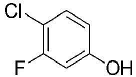 | 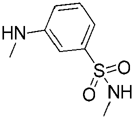 | ¹H NMR (400 MHz, DMSO-d6) δ 11.18 (br s, 1H), 7.72 (s, 1H), 7.65 - 7.54 (m, 4H), 7.49 (dd, J = 9.9, 5.2 Hz, 1H), 7.28 (d, J = 9.8 Hz, 1H), 7.23 (s, 1H), 7.01 (d, J = 7.8 Hz, 1H), 3.36 (s, 3H), 2.44 (d, J = 4.9 Hz, 3H). LCMS: m/z 471.08 [M+H]+ |
| **291** | 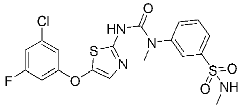 | 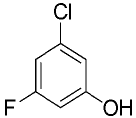 | 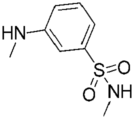 | ¹H NMR (400 MHz, DMSO-d6) δ 11.27 (s, 1H), 7.73 (s, 1H), 7.63 (s, 3H), 7.50 (s, 1H), 7.28 - 7.22 (m, 2H), 7.10 - 7.04 (m, 2H), 3.38 (s, 3H), 2.46 (s, 3H).LCMS: m/z 470.98 [M+H]+ |
| **292** | 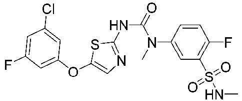 | 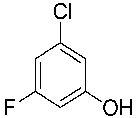 | 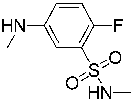 | ¹H NMR (400 MHz, DMSO-d6) δ 11.30 (s, 1H), 7.83 (d, J = 6.4 Hz, 1H), 7.76 (s, 1H), 7.62 - 7.51 (m, 2H), 7.28 - 7.22 (m, 2H), 7.12 - 7.03 (m, 2H), 3.29 (s, 3H), 2.47 (d, J = 4.5 Hz, 3H). LCMS: m/z 488.95 [M+H]+ |
| **293** | 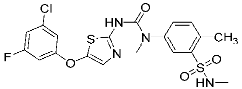 | 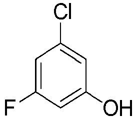 | 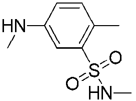 | ¹H NMR (400 MHz, DMSO-d6) δ 11.05 (s, 1H), 7.69 (s, 1H), 7.54 (q, J = 4.5 Hz, 1H), 7.50 (d, J = 8.2 Hz, 1H), 7.43 (d, J = 8.1 Hz, 1H), 7.27 - 7.22 (m, 2H), 7.10 - 7.02 (m, 2H), 3.33 (s, 3H), 2.57 (s, 3H), 2.47 (d, J = 4.7 Hz, 3H). LCMS: m/z 484.92 [M+H]+ |
| **294** | 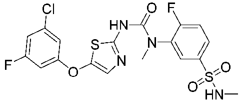 | 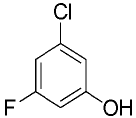 | 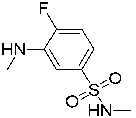 | ¹H NMR (400 MHz, DMSO-d6) δ 11.33 (s, 1H), 7.83 (d, J = 6.3 Hz, 1H), 7.80 - 7.74 (m, 1H), 7.61 - 7.51 (m, 2H), 7.28 - 7.22 (m, J = 4.9 Hz, 2H), 7.11 - 7.04 (m, J = 8.1 Hz, 2H), 3.29 (s, 3H), 2.47 (d, J = 3.0 Hz, 3H). LCMS: m/z 488.91 [M+H]+ |
| **295** | 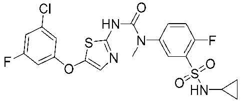 | 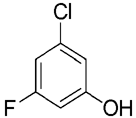 | 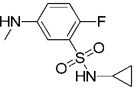 | ¹H NMR (400 MHz, DMSO-d6) δ 11.08 (s, 1H), 8.31 (s, 1H), 7.75 - 7.66 (m, 2H), 7.49 (t, J = 9.3 Hz, 1H), 7.29 - 7.21 (m, 2H), 7.10 - 7.02 (m, 2H), 3.33 (s, 3H), 2.34 - 2.27 (m, 1H), 0.56 - 0.47 (m, 2H), 0.47 - 0.41 (m, 2H). LCMS: m/z 514.93 [M+H]+ |
| **296** | 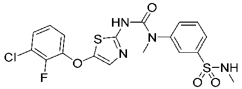 | 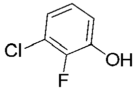 | 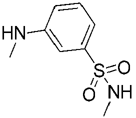 | ¹H NMR (400 MHz, DMSO-d6) δ 11.18 (s, 1H), 7.72 (s, 1H), 7.64 - 7.60 (m, 3H), 7.54 - 7.48 (m, 1H), 7.41 - 7.35 (m, 1H), 7.26 - 7.18 (m, 3H), 3.37 (s, 3H), 2.45 (s, 3H). LCMS: m/z 471.03 [M+H]+ |
| **297** | 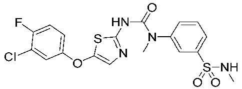 | 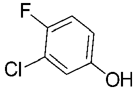 | 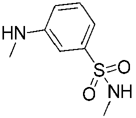 | ¹H NMR (400 MHz, DMSO-d6) δ 11.16 (s, 1H), 7.72 (s, 1H), 7.65 - 7.59 (m, 3H), 7.54 - 7.38 (m, 3H), 7.22 (s, 1H), 7.20 - 7.14 (m, 1H), 3.37 (s, 3H), 2.45 (d, J = 3.9 Hz, 3H). LCMS: m/z 470.99[M+H]+ |
| **298** | 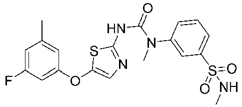 | 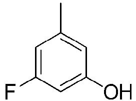 | 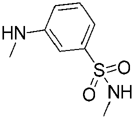 | ¹H NMR (400 MHz, DMSO-d6) δ 11.22 (s, 1H), 7.73 (s, 1H), 7.62 (s, 3H), 7.51 (s, 1H), 7.19 (s, 1H), 6.90 - 6.74 (m, 3H), 3.37 (s, 3H), 2.45 (s, 3H), 2.31 (s, 3H). LCMS: m/z 471.03 [M+H]+ |
| **299** | 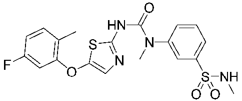 | 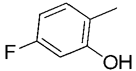 | 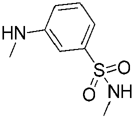 | ¹H NMR (400 MHz, DMSO-d6) δ 11.24 (s, 1H), 7.73 (s, 1H), 7.62 (s, 3H), 7.50 (dd, J = 9.2, 4.4 Hz, 1H), 7.33 (t, J = 7.4 Hz, 1H), 7.16 (s, 1H), 6.93 (t, J = 8.4 Hz, 1H), 6.85 (d, J = 10.1 Hz, 1H), 3.37 (s, 3H), 2.45 (d, J = 4.6 Hz, 3H), 2.25 (s, 3H). LCMS: m/z 451.21 [M+H]+ |
| **300** | 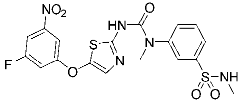 | 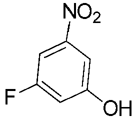 | 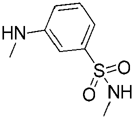 | ¹H NMR (400 MHz, DMSO-d6) δ 11.26 (s, 1H), 7.93 (d, J = 8.4 Hz, 1H), 7.73 (d, J = 7.4 Hz, 2H), 7.64 (d, J = 7.1 Hz, 4H), 7.50 (dd, J = 8.6, 4.0 Hz, 1H), 7.34 (s, 1H), 3.38 (s, 3H), 2.45 (d, J = 4.5 Hz, 3H). LCMS: m/z 481.93 [M+H]+ |
| **301** | 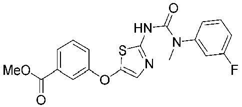 | 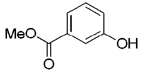 | 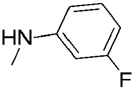 | ¹H NMR (400 MHz, DMSO-d6) δ 10.98 (br s, 1H), 7.74 (d, J = 7.9 Hz, 1H), 7.59 - 7.52 (m, 2H), 7.47 - 7.37 (m, 2H), 7.26 (dd, J = 10.8, 1.9 Hz, 1H), 7.23 (s, 1H), 7.19 (d, J = 8.0 Hz, 1H), 7.09 (t, J = 8.1 Hz, 1H), 3.84 (s, 3H), 3.32 (s, 3H). LCMS: m/z 402.21 [M+H]+ |
| **302** | 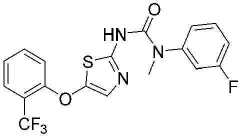 | 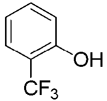 | 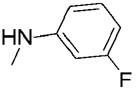 | ¹H NMR (400 MHz, DMSO-d6) δ 10.86 (s, 1H), 7.78 (dd, J = 7.9, 1.6 Hz, 1H), 7.75 - 7.66 (m, 1H), 7.43 (q, J = 7.7 Hz, 1H), 7.34 (t, J = 7.6 Hz, 1H), 7.30 (d, J = 8.5 Hz, 1H), 7.28 (s, 1H), 7.25 (t, J =2.3 Hz, 1H), 7.18 (ddd, J = 8.1, 2.1, 0.9 Hz, 1H), 7.10 (t, J = 7.7 Hz, 1H), 3.32 (s, 3H). LCMS: m/z 412.27 [M+H]+ |
| **303** | 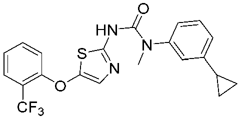 | 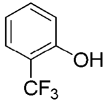 | 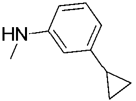 | ¹H NMR (400 MHz, DMSO-d6) δ 10.46 (s, 1H), 7.78 (dd, J = 8.0, 1.7 Hz, 1H), 7.73 - 7.67 (m, 1H), 7.34 (t, J = 7.6 Hz, 1H), 7.32 - 7.23 (m, 2H), 7.24 (s, 1H), 7.09 - 7.03 (m, 2H), 6.98 (d, J = 7.1 Hz, 1H), 3.27 (s, 3H), 2.00 - 1.88 (m, 1H), 0.99 - 0.94 (m, 2H), 0.75 - 0.66 (m, 2H). LCMS: m/z 434.25 [M+H]+ |
| **304** | 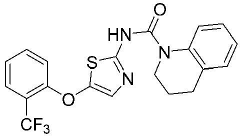 | 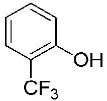 | 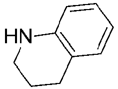 | ¹H NMR (400 MHz, DMSO-d6) δ 11.17 (s, 1H), 7.81 - 7.77 (m, 1H), 7.71 (dd, J = 8.9, 7.4 Hz, 1H), 7.50 (s, 1H), 7.36 (d, J = 7.6 Hz, 1H), 7.34 (s, 1H), 7.34 - 7.32 (m, 1H), 7.13 (t, J = 7.7 Hz, 2H), 7.01 (t, J = 7.4 Hz, 1H), 3.79 (s, 2H), 2.73 (t, J = 6.5 Hz, 2H), 1.88 (p, J = 6.4 Hz, 2H). LCMS: m/z 420.24 [M+H]+ |
| **305** | 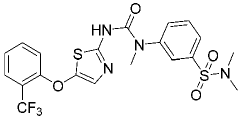 | 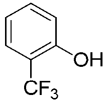 | 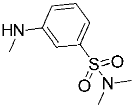 | ¹H NMR (400 MHz, DMSO-d6) δ 10.99 (s, 1H), 7.78 (dd, J = 7.8, 1.0 Hz, 2H), 7.74 - 7.64 (m, 3H), 7.61 (s, 1H), 7.36 (d, J = 7.7 Hz, 1H), 7.34 - 7.27 (m, 2H), 3.33 (s, 3H), 2.64 (s, 6H). LCMS: m/z 501.19 [M+H]+ |
| **306** | 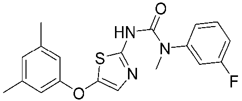 | 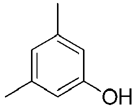 | 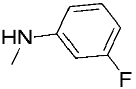 | ¹H NMR (400 MHz, DMSO-d6) δ 10.94 (s, 1H), 7.43 (dd, J = 15.1, 8.0 Hz, 1H), 7.26 (dd, J = 10.8, 2.2 Hz, 1H), 7.19 (d, J = 7.8 Hz, 1H), 7.13 - 7.06 (m, 2H), 6.78 (s, 1H), 6.72 (s, 2H), 3.32 (s, 3H), 2.26 (s, 6H). LCMS: m/z 372.0 [M+H]+ |
| **307** | 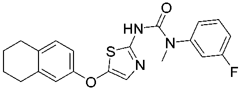 | 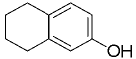 | 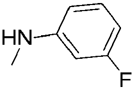 | ¹H NMR (400 MHz, DMSO-d6) δ 10.85 (s, 1H), 7.42 (dd, J = 15.4, 8.0 Hz, 1H), 7.22 (dd, J = 31.8, 10.3 Hz, 2H), 7.12 - 7.01 (m, 3H), 6.87 - 6.73 (m, 2H), 2.68 (s, 4H), 1.71 (s, 4H). LCMS: m/z 398.1 [M+H]+ |
| **308** | 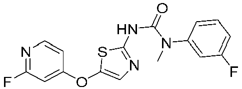 | 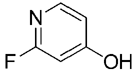 | 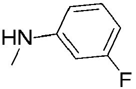 | ¹H NMR (400 MHz, DMSO-d6) δ 11.16 (s, 1H), 8.19 (d, J = 5.8 Hz, 1H), 7.43 (q, J = 8.2 Hz, 1H), 7.31 (s, 1H), 7.26 (dt, J = 10.7, 2.3 Hz, 1H), 7.18 (dd, J = 7.9, 1.5 Hz, 1H), 7.15 - 7.02 (m, 2H), 6.91 (d, J = 1.8 Hz, 1H), 3.32 (s, 3H). LCMS: m/z 363.24 [M+H]+ |
| **309** | 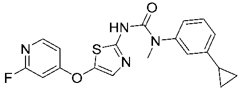 | 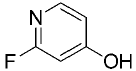 | 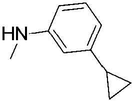 | ¹H NMR (400 MHz, DMSO-d6) δ 10.53 (s, 1H), 8.19 (d, J = 5.8 Hz, 1H), 7.29 (s, 1H), 7.25 (t, J = 7.7 Hz, 1H), 7.11 - 7.09 (m, 1H), 7.08 - 7.02 (m, 2H), 6.97 (d, J = 7.1 Hz, 1H), 6.90 (d, J = 1.2 Hz, 1H), 3.27 (s, 3H), 1.93 (tt, J = 8.5, 5.0 Hz, 1H), 1.03 -0.90 (m, 2H), 0.79 - 0.61 (m, 2H). LCMS: m/z 385.28 [M+H]+ |
| **310** | 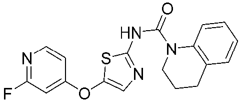 | 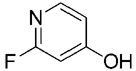 | 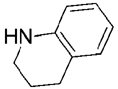 | ¹H NMR (400 MHz, DMSO-d6) δ 11.26 (s, 1H), 8.19 (d, J = 5.8 Hz, 1H), 7.51 (s, 1H), 7.36 (s, 1H), 7.13 (t, J = 7.2 Hz, 3H), 7.00 (t, J = 7.3 Hz, 1H), 6.93 (s, 1H), 3.79 (s, 2H), 2.73 (t, J = 6.5 Hz, 2H), 1.88 (p, J = 6.5 Hz, 2H). LCMS: m/z 371.24 [M+H]+ |
| **311** | 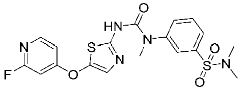 | 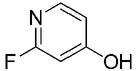 | 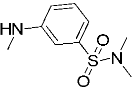 | ¹H NMR (400 MHz, DMSO-d6) δ 11.11 (s, 1H), 8.19 (d, J = 5.8 Hz, 1H), 7.73 - 7.57 (m, 4H), 7.33 (s, 1H), 7.11 (d, J = 4.7 Hz, 1H), 6.92 (s, 1H), 3.38 (s, 3H), 2.63 (s, 6H). LCMS: m/z 452.22 [M+H]+ |
| **312** | 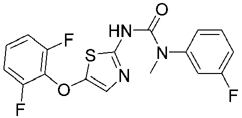 | 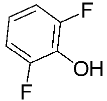 | 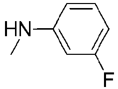 | ¹H NMR (400 MHz, CDCl3) δ 7.50 (dd, J = 14.8, 7.4 Hz, 1H), 7.43 (t, J = 7.9 Hz, 2H), 7.22 (d, J = 8.8 Hz, 2H), 7.12 (s, 1H), 7.04 (d, J = 9.2 Hz, 3H), 3.45 (s, 3H). LCMS: m/z 380.22 [M+H]+ |
| **313** | 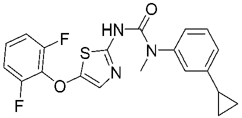 | 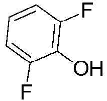 | 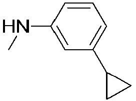 | ¹H NMR (400 MHz, CDCl3) δ 7.46 - 7.35 (m, 2H), 7.23 - 7.14 (m, 2H), 7.13 - 6.98 (m, 4H), 6.88 (s, 1H), 3.36 (s, 3H), 1.98 - 1.86 (m, 1H), 1.10 - 1.01 (m, 2H), 0.79 - 0.71 (m, 2H). LCMS: m/z 402.20 [M+H]+ |
| **314** | 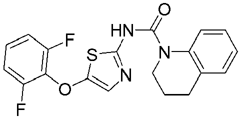 | 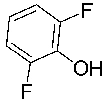 | 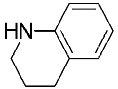 | ¹H NMR (400 MHz, CDCl3) δ 7.48 - 7.42 (m, 1H), 7.27 (s, 1H), 7.19 (dt, J = 14.7, 6.8 Hz, 3H), 7.03 (ddd, J = 10.9, 7.7, 3.1 Hz, 2H), 6.95 (d, J = 10.3 Hz, 1H), 3.91 (t, J = 6.4 Hz, 2H), 2.79 (t, J = 6.5 Hz, 2H), 2.08 - 1.98 (m, 2H). LCMS: m/z 388.26 [M+H]+ |
| **315** | 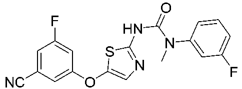 | 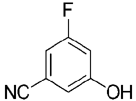 | 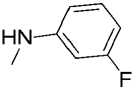 | ¹H NMR (400 MHz, DMSO-d6) δ 7.67 (d, J = 8.2 Hz, 1H), 7.55 (s, 1H), 7.49 - 7.40 (m, 2H), 7.27 (q, J = 2.6 Hz, 2H), 7.19 (d, J = 8.0 Hz, 1H), 7.11 (d, J = 7.6 Hz, 1H), 3.33 (s, 3H). LCMS: m/z 387.28 [M+H]+ |
| **316** | 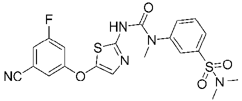 | 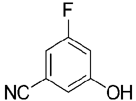 | 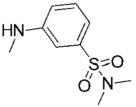 | ¹H NMR (400 MHz, DMSO-d6) δ 7.76 - 7.64 (m, 4H), 7.60 (d, J = 7.2 Hz, 1H), 7.55 (s, 1H), 7.47 (d, J = 10.0 Hz, 1H), 7.28 (s, 1H), 3.39 (s, 3H), 2.70 - 2.56 (m, 6H). LCMS: m/z 476.22 [M+H]+ |
| **317** | 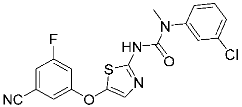 | 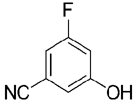 | 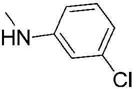 | ¹H NMR (400 MHz, CDCl3) δ 7.48 (s, 1H), 7.36 (s, 1H), 7.26 (d, J = 7.2 Hz, 2H), 7.16 (d, J = 9.9 Hz, 2H), 7.06 (s, 1H), 6.95 (t, J = 7.4 Hz, 1H), 6.86 (d, J = 7.8 Hz, 1H), 3.40 (s, 3H). LCMS: m/z 403.20 [M+H]+ |
| **318** | 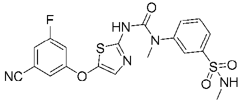 | 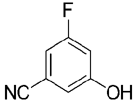 | 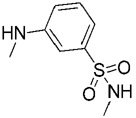 | ¹H NMR (400 MHz, CDCl3) δ 7.96 - 7.90 (m, 2H), 7.75 (t, J = 7.9 Hz, 1H), 7.54 (d, J = 3.0 Hz, 2H), 7.22 (s, 2H), 7.12 (d, J = 10.1 Hz, 2H), 3.47 (s, 3H), 2.84 (s, 3H). LCMS: m/z 462.10 [M+H]+ |
| **319** | 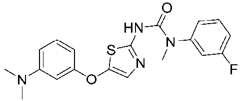 | 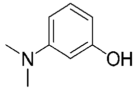 | 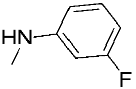 | ¹H NMR (400 MHz, CDCl3) δ 7.56 - 7.49 (m, 1H), 7.25 - 7.19 (m, 2H), 7.18 - 7.12 (m, 2H), 7.07 (dd, J = 9.0, 2.3 Hz, 1H), 6.94 (s, 1H), 6.65 (s, 1H), 6.52 (s, 1H), 3.40 (d, J = 5.8 Hz, 3H), 2.99 (s, 6H). LCMS: m/z 387.24 [M+H]+ |
| **320** | 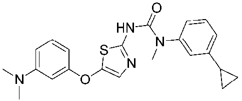 | 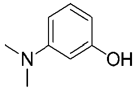 | 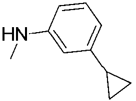 | ¹H NMR (400 MHz, CDCl3) δ 7.38 (t, J = 7.8 Hz, 1H), 7.27 - 7.21 (m, 1H), 7.18 (t, J = 8.2 Hz, 1H), 7.08 (dd, J = 14.6, 7.2 Hz, 2H), 7.03 (s, 1H), 6.91 (d, J = 9.3 Hz, 1H), 6.50 (dd, J = 19.7, 11.0 Hz, 2H), 3.37 (d, J = 16.1 Hz, 3H), 2.97 (s, 6H), 1.95 (td, J = 8.4, 4.2 Hz, 1H), 1.10 -1.02 (m, 2H), 0.80 - 0.72 (m, 2H). LCMS: m/z 409.23 [M+H]+ |
| **321** | 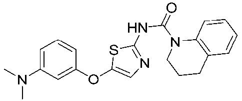 | 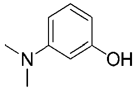 | 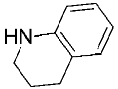 | ¹H NMR (400 MHz, CDCl3) δ 7.43 (d, J = 7.9 Hz, 1H), 7.21 (dt, J = 15.1, 7.7 Hz, 4H), 6.99 - 6.95 (m, 1H), 6.58 (s, 1H), 6.55 - 6.44 (m, 2H), 3.91 (dd, J = 17.7, 11.4 Hz, 2H), 2.98 (s, 6H), 2.81 (dd, J = 12.5, 5.9 Hz, 2H), 2.05 (dd, J = 12.7, 6.2 Hz, 2H). LCMS: m/z 395.20 [M+H]+ |
| **322** | 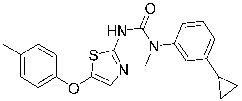 | 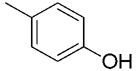 | 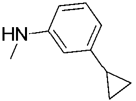 | ¹H NMR (400 MHz, DMSO-d6) δ 7.26 (s, 1H), 7.18 (d, J = 8.5 Hz, 2H), 7.06 (d, J = 9.1 Hz, 3H), 7.00 (d, J = 8.5 Hz, 2H), 6.98 - 6.93 (m, 1H), 3.26 (s, 3H), 2.28 (s, 3H), 1.91 (s, 1H), 0.96 (dd, J = 8.3, 2.2 Hz, 2H), 0.70 (d, J = 7.0 Hz, 2H). LCMS: m/z 380.1 [M+H]+ |
| **323** | 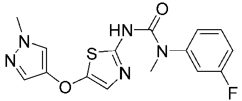 | 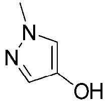 | 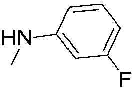 | ¹H NMR (400 MHz, CDCl3) δ 7.50 (td, J = 8.2, 6.4 Hz, 1H), 7.40 (d, J = 8.1 Hz, 1H), 7.32 (s, 1H), 7.19 - 7.11 (m, 2H), 7.06 (dt, J = 9.0, 2.2 Hz, 1H), 6.87 (s, 1H), 3.88 (d, J = 2.9 Hz, 3H), 3.39 (d, J = 5.1 Hz, 3H). LCMS: m/z 348.27 [M+H]+ |
| **324** | 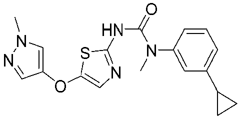 | 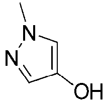 | 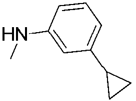 | ¹H NMR (400 MHz, DMSO-d6) δ 7.78 (s, 1H), 7.40 (s, 1H), 7.27 (t, J = 7.7 Hz, 1H), 7.04 (d, J = 11.7 Hz, 2H), 6.97 (d, J = 10.1 Hz, 2H), 3.78 (s, 3H), 3.26 (s, 3H), 1.98 - 1.89 (m, 1H), 0.99 - 0.92 (m, 2H), 0.73 - 0.66 (m, 2H). LCMS: m/z 370.1 [M+H]+ |
| **325** | 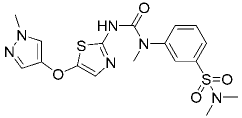 | 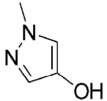 | 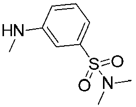 | ¹H NMR (400 MHz, DMSO-d6) δ 7.80 (s, 1H), 7.67 (d, J = 1.9 Hz, 3H), 7.59 (d, J = 6.3 Hz, 1H), 7.41 (s, 1H), 7.02 (d, J = 3.2 Hz, 1H), 3.78 (d, J = 2.4 Hz, 3H), 3.37 (s, 3H), 2.68 - 2.59 (m, 6H). LCMS: m/z 437.1 [M+H]+ |

### [Example 326]

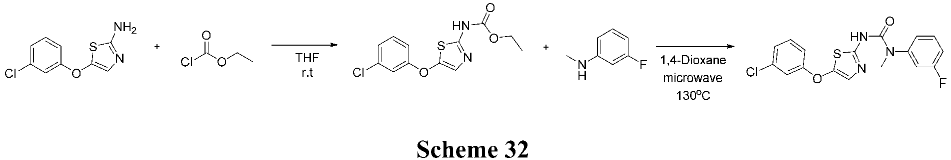

### Synthesis of ethyl (5-(3-chlorophenoxy)thiazol-2-yl)carbamate

After dissolving 1 eq of 5-(3-chlorophenoxy)thiazol-2-amine in tetrahydrofuran (10 mL/g), 1.5 eq of ethyl chloroformate was slowly added thereto, and the mixture was stirred at room temperature for 18 hours. After completion of the reaction, the mixture was concentrated under reduced pressure. The concentrated material was purified and separated by column chromatography (hexane:ethyl acetate) to obtain ethyl (5-(3-chlorophenoxy)thiazol-2-yl)carbamate.

### Synthesis of 3-(5-(3-chlorophenoxy)thiazol-2-yl)-1-(3-fluorophenyl)-1-methylurea (Example 326)

After dissolving 1 eq of ethyl (5-(3-chlorophenoxy)thiazol-2-yl)carbamate in 1,4-dioxane (10 mL/g), 2 eq of 3-fluoro-N-methylaniline was added as **reactant V1** thereto, and the mixture was stirred at 130°C in a microwave for 1 hour. After completion of the reaction, the mixture was concentrated under reduced pressure. The concentrated material was purified and separated by column chromatography (hexane:ethyl acetate) to obtain the title **compound of Example 326.**

### [Examples 327-328]

The **compounds of Examples 327-328** were synthesized in substantially the same manner as described in the method for preparing the compound of Example 326 **(Scheme 32)** by using **reactant V1 of Table 36** instead of reactant V1 in the method for preparing the compound of Example 326.

The structures of the compounds of Examples 326-328, reactant V1, and analytical data are shown in Table 36.

**[Table 36]**

| **No** | **Structure** | **Reactant V1** | **Analysis data** |
|---|---|---|---|
| **326** | 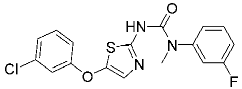 | 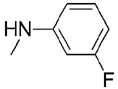 | ¹H NMR (400 MHz, DMSO-d6) δ 11.05 (s, 1H), 7.49 - 7.35 (m, 2H), 7.30 -7.15 (m, 5H), 7.13 - 7.06 (m, 2H), 3.32 (s, 3H). LCMS: m/z 378.0 [M+H]+ |
| **327** | 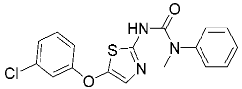 | 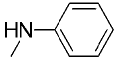 | ¹H NMR (400 MHz, DMSO-d6) δ 10.70 (s, 1H), 7.47 - 7.36 (m, 3H), 7.36 -7.15 (m, 6H), 7.14 - 7.04 (m, 1H), 3.31 (s, 3H). LCMS: m/z 360.0 [M+H]+ |
| **328** | 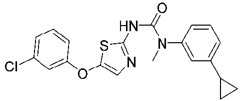 | 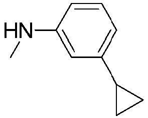 | ¹H NMR (400 MHz, DMSO-d6) *δ* 10.47 (s, 1H), 7.42 (t, J = 8.2 Hz, 1H), 7.32 - 7.14 (m, 4H), 7.12 - 7.01 (m, 3H), 6.97 (d, J = 7.7 Hz, 1H), 3.26 (s, 3H), 1.97 - 1.88 (m, 1H), 1.00 - 0.91 (m, 2H), 0.73 - 0.65 (m, 2H). LCMS: m/z 400.1 [M+H]+ |

### [Example 329]

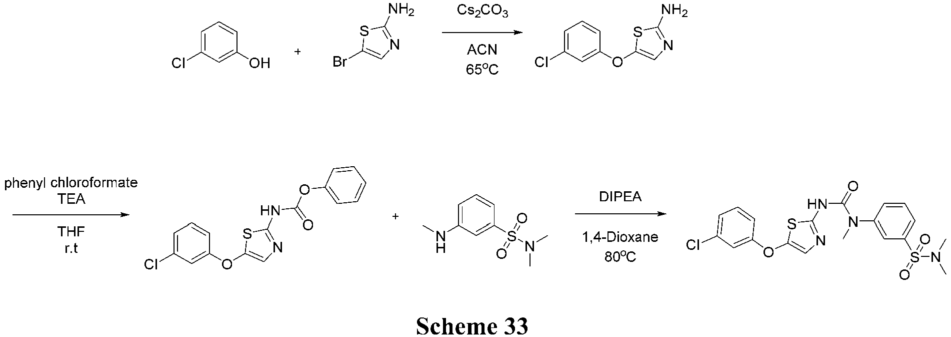

### Synthesis of 5-(3-chlorophenoxy)thiazol-2-amine

After dissolving 1.3 eq of 3-chlorophenol as **reactant W1** in acetonitrile (30 mL/g), 2 eq of cesium carbonate was added thereto. After stirring for 10 minutes, 1 eq of 5-bromothiazol-2-amine was added thereto, and the mixture was heated to 65°C for 12 hours. After completion of the reaction, the mixture was filtered through Celite with acetonitrile, concentrated, and extracted with ethyl acetate and water. The ethyl acetate layer was dried over magnesium sulfate, concentrated under reduced pressure, purified, and separated by column chromatography (hexane:ethyl acetate) to obtain 5-(3-chlorophenoxy)thiazol-2-amine.

### Synthesis of phenyl (5-(3-chlorophenoxy)thiazol-2-yl)carbamate

After dissolving 1 eq of 5-(3-chlorophenoxy)thiazol-2-amine in tetrahydrofuran (10 mL/g), 1.5 eq of triethylamine was added thereto, and the mixture was cooled to 0°C. After slowly adding 1.2 eq of phenyl chloroformate, the mixture was slowly warmed to room temperature and stirred for 4 hours. After completion of the reaction, the mixture was extracted with methylene chloride, dried over magnesium sulfate, and then concentrated under reduced pressure. The concentrate was purified and separated by column chromatography (hexane:ethyl acetate) to obtain phenyl (5-(3-chlorophenoxy)thiazol-2-yl)carbamate.

### Synthesis of 3-(3-(5-(3-chlorophenoxy)thiazol-2-yl)-1-methylureido)-N,N-dimethylbenzenesulfonamide (Example 329)

After dissolving 1 eq of phenyl (5-(3-chlorophenoxy)thiazol-2-yl)carbamate and 2 eq of N,N-diisopropylethylamine in 1,4-dioxane (10 mL/g), 1.5 eq of N,N-dimethyl-3-(methylamino)benzenesulfonamide was added thereto as **reactant W2,** and the mixture was stirred at 80°C for 18 hours. After completion of the reaction, the mixture was concentrated under reduced pressure. The concentrated material was purified and separated by column chromatography (hexane:ethyl acetate) to obtain the title **compound of Example 329.**

### [Examples 330-342]

The **compounds of Examples 330-342** were synthesized in substantially the same manner as described in the method for preparing the compound of Example 329 **(Scheme 33)** by using **reactants W1 and W2 of Table 37** instead of reactants W1 and W2 in the method for preparing the compound of Example 329.

The structures of the compounds of Examples 329-342, reactants W1, W2, and analytical data are shown in Table 37.

**[Table 37]**

| **No** | **Structure** | **Reactant W1** | **Reactant W2** | **Analysis data** |
|---|---|---|---|---|
| **329** | 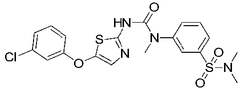 | 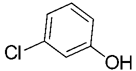 | 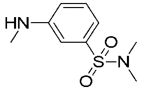 | ¹H NMR (400 MHz, DMSO-d6) *δ* 11.15 (s, 1H), 7.75 - 7.51 (m, 4H), 7.42 (t, J = 8.2 Hz, 1H), 7.21 (t, J = 9.2 Hz, 3H), 7.14 - 7.03 (m, 1H), 3.37 (s, 3H), 2.63 (s, 6H). LCMS: m/z 467.1 [M+H]+ |
| **330** | 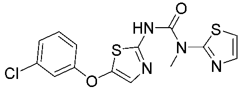 | 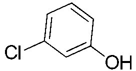 | 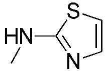 | ¹H NMR (400 MHz, DMSO-d6) *δ* 12.58 (s, 1H), 7.51 - 7.33 (m, 3H), 7.30 - 7.15 (m, 4H), 3.66 (s, 3H). LCMS: m/z 367.0 [M+H]+ |
| **331** | 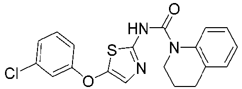 | 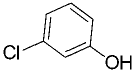 | 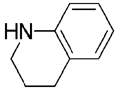 | ¹H NMR (400 MHz, DMSO-d6) *δ* 11.54 (s, 1H), 7.61 - 7.37 (m, 2H), 7.28 - 7.07 (m, 6H), 7.00 (t, J = 7.3 Hz, 1H), 3.79 (t, *J* = 5.9 Hz, 2H), 2.73 (t, *J* = 6.5 Hz, 2H), 1.94 - 1.81 (m, 2H). LCMS: m/z 386.0 [M+H]+ |
| **332** | 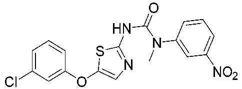 | 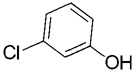 | 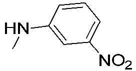 | ¹H NMR (400 MHz, CDCl3) δ 8.33 (d, J = 7.9 Hz, 1H), 8.23 (t, J = 2.0 Hz, 1H), 7.75 (t, J = 8.0 Hz, 1H), 7.70 (dd, J = 6.5, 5.3 Hz, 1H), 7.33 (t, J = 8.2 Hz, 1H), 7.20 (d, J = 8.0 Hz, 1H), 7.13 -7.11 (m, 1H), 7.02 (q, J = 2.8 Hz, 2H), 3.53 (s, 3H). LCMS: m/z 405.0 [M+H]+ |
| **333** | 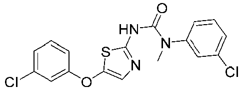 | 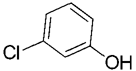 | 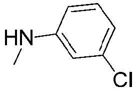 | ¹H NMR (400 MHz, CDCl3) δ 7.47 (t, J = 8.3 Hz, 2H), 7.35 (d, J = 1.9 Hz, 1H), 7.27 - 7.23 (m, 2H), 7.16 (d, J = 6.2 Hz, 1H), 7.10 (t, J = 2.1 Hz, 1H), 7.03 - 6.99 (m, 2H), 3.42 (s, 3H). LCMS: m/z 394.0 [M+H]+ |
| **334** | 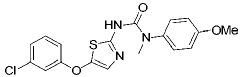 | 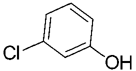 | 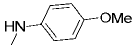 | ¹H NMR (400 MHz, CDCl3) δ 7.24 (dt, J = 5.8, 3.4 Hz, 3H), 7.13 (d, J = 8.1 Hz, 1H), 7.09 (t, J = 2.1 Hz, 1H), 7.03 (d, J = 8.8 Hz, 2H), 7.00 (dd, J = 8.3, 2.5 Hz, 1H), 6.97 (s, 1H), 3.89 (s, 3H), 3.34 (d, J = 12.3 Hz, 3H). LCMS: m/z 390.0 [M+H]+ |
| **335** | 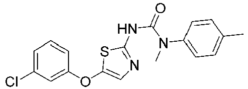 | 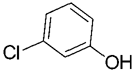 | 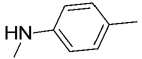 | ¹H NMR (400 MHz, CDCl3) δ 7.35 (dd, J = 9.3, 5.2 Hz, 3H), 7.24 (d, J = 9.6 Hz, 1H), 7.21 - 7.16 (m, 2H), 7.15 - 7.12 (m, 1H), 7.06 - 6.98 (m, 2H), 3.78 (s, 3H), 2.44 (s, 3H). LCMS: m/z 374.1 [M+H]+ |
| **336** | 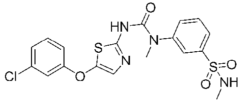 | 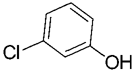 | 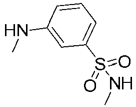 | ¹H NMR (400 MHz, CDCl3) δ 7.90 (d, J = 7.9 Hz, 1H), 7.86 (s, 1H), 7.70 (t, J = 7.9 Hz, 1H), 7.58 (d, J = 8.2 Hz, 1H), 7.26 (d, J = 8.0 Hz, 1H), 7.11 (d, J = 7.5 Hz, 2H), 7.02 - 6.98 (m, 1H), 6.96 (s, 1H), 4.63 (d, J = 5.4 Hz, 1H), 3.42 (d, J = 4.2 Hz, 3H), 2.80 (d, J = 5.3 Hz, 3H). LCMS: m/z 453.13 [M+H]+ |
| **337** | 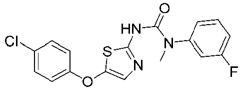 | 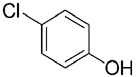 | 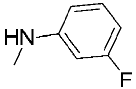 | ¹H NMR (400 MHz, CDCl3) δ 7.43 (dd, J = 14.3, 8.0 Hz, 1H), 7.25 - 7.21 (m, 2H), 7.10 (dd, J = 7.4, 3.1 Hz, 1H), 7.05 (d, J = 7.9 Hz, 1H), 6.99 - 6.92 (m, 3H), 6.86 (s, 1H), 3.30 (d, J = 4.6 Hz, 3H). LCMS: m/z 378.13 [M+H]+ |
| **338** | 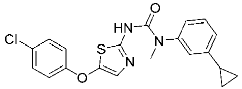 | 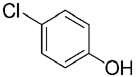 | 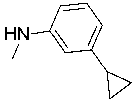 | ¹H NMR (400 MHz, CDCl3) δ 7.39 (dd, J = 8.7, 4.9 Hz, 3H), 7.15 (d, J = 8.1 Hz, 1H), 7.06 (d, J = 9.9 Hz, 3H), 7.02 (d, J = 4.2 Hz, 1H), 6.97 (s, 1H), 3.77 (s, 3H), 1.95 (d, J = 5.0 Hz, 1H), 1.13 - 1.05 (m, 2H), 0.78 - 0.73 (m, 2H). LCMS: m/z 400.18 [M+H]+ |
| **339** | 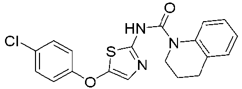 | 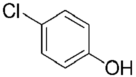 | 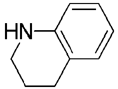 | ¹H NMR (400 MHz, CDCl3) δ 7.39 (d, J = 9.0 Hz, 2H), 7.31 (s, 2H), 7.20 (s, 2H), 7.09 (d, J = 9.0 Hz, 2H), 7.03 (d, J = 5.4 Hz, 1H), 4.31 (t, J = 6.8 Hz, 1H), 4.00 (t, J = 6.5 Hz, 1H), 2.88 - 2.77 (m, 2H), 2.17 - 2.07 (m, 2H). LCMS: m/z 386.16 [M+H]+ |
| **340** | 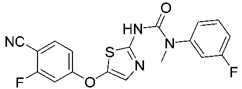 | 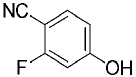 | 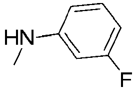 | ¹H NMR (400 MHz, DMSO-d6) *δ* 11.22 (s, 1H), 8.04 - 7.87 (m, 1H), 7.48 - 7.22 (m, 4H), 7.22 - 6.99 (m, 3H), 3.33 (s, 3H). LCMS: m/z 387.1 [M+H]+ |
| **341** | 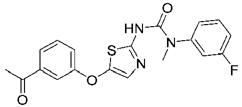 | 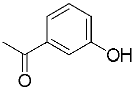 | 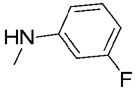 | ¹H NMR (400 MHz, DMSO-d6) *δ* 11.03 (s, 1H), 7.77 (d, *J* = 7.7 Hz, 1H), 7.61 - 7.52 (m, 2H), 7.47 - 7.38 (m, 2H), 7.32 - 7.18 (m, 3H), 7.09 (t, *J* = 8.3 Hz, 1H), 3.32 (s, 3H), 2.59 (s, 3H). LCMS: m/z 386.1 [M+H]+ |
| **342** | 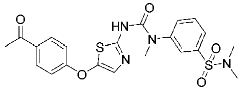 | 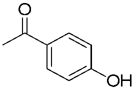 | 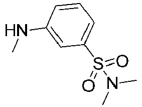 | ¹H NMR (400 MHz, DMSO-d6) *δ* 11.21 (s, 1H), 8.00 (d, *J* = 8.8 Hz, 2H), 7.74 - 7.63 (m, 4H), 7.26 - 7.15 (m, 3H), 3.37 (s, 3H), 2.63 (s, 6H) 2.55 (s, 3H). LCMS: m/z 475.2 [M+H]+ |

### [Example 343]

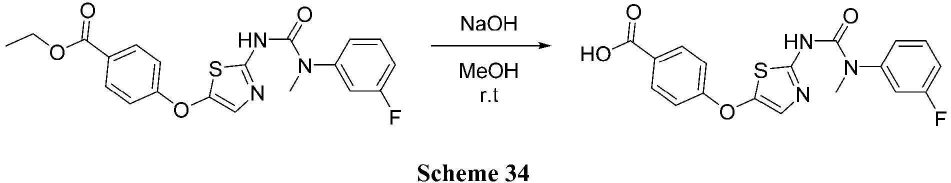

### Synthesis of 4-((2-(3-(3-fluorophenyl)-3-methylureido)thiazol-5-yl)oxy)benzoic acid (Example 343)

After dissolving 1 eq of ethyl 4-((2-(3-(3-fluorophenyl)-3-methylureido)thiazol-5-yl)oxy)benzoate, which is the **compound of Example 268,** in methanol (50 mL/g), 10 eq of sodium hydroxide was added thereto and the mixture was stirred at room temperature for 4 hours. After completion of the reaction, the organic layer was removed using ethyl acetate and water, and hydrochloric acid (1 N) was added to the water layer to obtain a solid, which was then filtered with water to obtain the title **compound of Example 343.**

**[Table 38]**

| **No** | **Structure** | **Analysis data** |
|---|---|---|
| **343** | 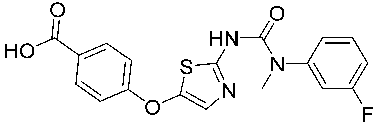 | ¹H NMR (400 MHz, DMSO-d6) δ 12.78 (s, 1H), 10.84 (s, 1H), 7.89 (dd, *J* = 9.0, 2.4 Hz, 2H), 7.36 (q, *J* = 7.8 Hz, 1H), 7.20 (dt, *J* = 10.9, 2.4 Hz, 1H), 7.17 (s, 1H), 7.14 - 7.09 (m, 3H), 7.05 - 7.00 (m, 1H), 3.25 (s, 3H). LCMS: m/z 388.18 [M+H]+ |

### [Example 344]

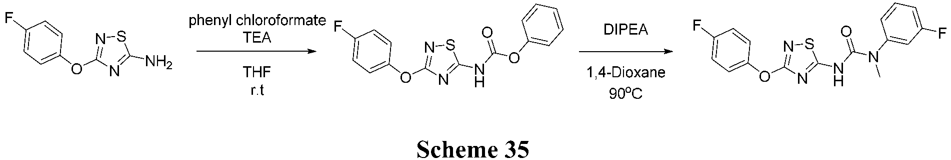

### Synthesis of phenyl (3-(4-fluorophenoxy)-1,2,4-thiadiazol-5-yl)carbamate

1 eq of 3-(4-fluorophenoxy)-1,2,4-thiadiazol-5-amine was dissolved as **reactant X1** in tetrahydrofuran (10 mL/g). 1.2 eq of phenyl chloroformate and 1.5 eq of triethylamine were added to the reaction mixture at 0°C. The mixture was stirred at room temperature for about 1 hour. After confirming the completion of the reaction, the mixture was extracted with ethyl acetate and water. The ethyl acetate layer was dried over magnesium sulfate, concentrated under reduced pressure, purified, and separated by column chromatography (hexane:ethyl acetate) to obtain phenyl (3-(4-fluorophenoxy)-1,2,4-thiadiazol-5-yl)carbamate.

### Synthesis of 3-(3-(4-fluorophenoxy)-1,2,4-thiadiazol-5-yl)-1-(3-fluorophenyl)-1-methylurea (Example 344)

1 eq of phenyl (3-(4-fluorophenoxy)-1,2,4-thiadiazol-5-yl)carbamate was dissolved in 1,4-dioxane (10 mL/g). 1.5 eq of 3-fluoro-N-methylaniline as **reactant X2** and 2 eq of N,N-diisopropylethylamine were added thereto, and the mixture was stirred at 90°C for about 1 hour. After confirming the completion of the reaction, the mixture was extracted with ethyl acetate and water. The ethyl acetate layer was dried over magnesium sulfate, concentrated under reduced pressure, purified, and separated by column chromatography (hexane:ethyl acetate) to obtain the title compound of **Example 344.**

### [Examples 345-350]

The **compounds of Examples 345-350** were synthesized in substantially the same manner as described in the method for preparing the compound of Example 344 **(Scheme 35)** by using **reactants X1 and X2 of Table 39** instead of reactants X1 and X2 in the method for preparing the compound of Example 344.

The structures of the compounds of Examples 344-350, reactants X1, X2, and analytical data are shown in Table 39.

**[Table 39]**

| **No** | **Structure** | **Reactant X1** | **Reactant X2** | **Analysis data** |
|---|---|---|---|---|
| **344** | 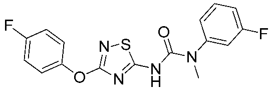 | 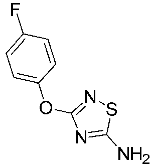 | 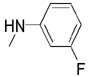 | ¹H NMR (400 MHz, DMSO-d6) δ 11.88 (s, 1H), 7.47 (dd, J = 14.9, 8.1 Hz, 1H), 7.35 - 7.15 (m, 7H), 3.34 (s, 3H). LCMS: m/z 363.18 [M+H]+ |
| **345** | 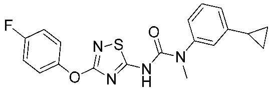 | 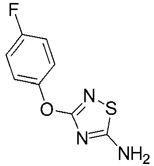 | 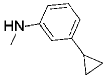 | ¹H NMR (400 MHz, DMSO-d6) δ 11.71 (s, 1H), 7.32 - 7.21 (m, 5H), 7.09 (d, J = 8.5 Hz, 2H), 7.03 (d, J = 7.9 Hz, 1H), 3.30 (s, 3H), 1.98 - 1.91 (m, 1H), 1.01 - 0.94 (m, 2H), 0.74 - 0.68 (m, 2H). LCMS: m/z 385.23 [M+H]+ |
| **346** | 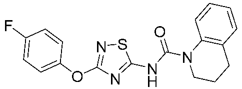 | 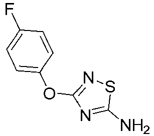 | 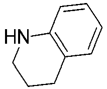 | ¹H NMR (400 MHz, DMSO-d6) δ 12.18 (s, 1H), 7.54 (d, J = 8.5 Hz, 1H), 7.34 - 7.23 (m, 4H), 7.20 -7.14 (m, 2H), 7.07 (t, J = 7.1 Hz, 1H), 3.82 - 3.77 (m, 2H), 2.74 (t, J = 6.5 Hz, 2H), 1.95 - 1.86 (m, 2H). LCMS: m/z 371.21 [M+H]+ |
| **347** | 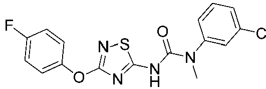 | 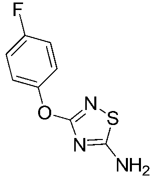 | 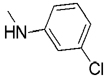 | ¹H NMR (400 MHz, DMSO-d6) δ 11.89 (s, 1H), 7.53 (t, J = 2.0 Hz, 1H), 7.46 (t, J = 7.9 Hz, 1H), 7.40 (d, J = 8.4 Hz, 1H), 7.35 (dd, J = 7.8, 1.3 Hz, 1H), 7.32 - 7.21 (m, 4H), 3.33 (s, 3H). LCMS: m/z 379.14 [M+H]+ |
| **348** | 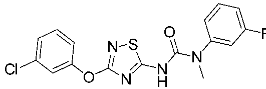 | 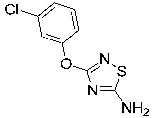 | 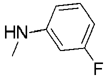 | ¹H NMR (400 MHz, DMSO-d6) δ 11.91 (s, 1H), 7.50 - 7.42 (m, 2H), 7.41 (t, J = 2.1 Hz, 1H), 7.35 - 7.29 (m, 2H), 7.23 (d, J = 8.1 Hz, 2H), 7.18 (td, J = 8.5, 2.2 Hz, 1H), 3.34 (s, 3H). LCMS: m/z 379.14 [M+H]+ |
| **349** | 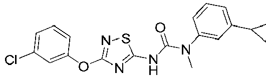 | 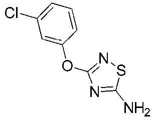 | 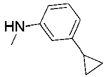 | ¹H NMR (400 MHz, DMSO-d6) δ 11.75 (s, 1H), 7.45 (t, J = 8.1 Hz, 1H), 7.41 (s, 1H), 7.30 (t, J = 7.8 Hz, 2H), 7.23 (dd, J = 8.2, 1.4 Hz, 1H), 7.12 - 7.06 (m, 2H), 7.03 (d, J = 7.8 Hz, 1H), 3.30 (s, 3H), 1.95 (ddd, J = 12.9, 8.4, 4.8 Hz, 1H), 1.01 - 0.93 (m, 2H), 0.71 (q, J = 4.9 Hz, 2H). LCMS: m/z 399.27 [M-H]- |
| **350** | 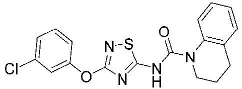 | 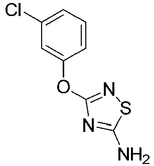 | 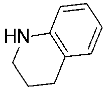 | ¹H NMR (400 MHz, DMSO-d6) δ 12.21 (s, 1H), 7.54 (d, J = 8.2 Hz, 1H), 7.48 (d, J = 8.1 Hz, 1H), 7.44 (dd, J = 4.3, 2.2 Hz, 1H), 7.33 (dd, J = 8.0, 1.1 Hz, 1H), 7.26 (dd, J = 8.2, 2.2 Hz, 1H), 7.18 (dd, J = 9.2, 4.9 Hz, 2H), 7.07 (t, J = 6.9 Hz, 1H), 3.83 - 3.76 (m, 2H), 2.74 (t, J = 6.5 Hz, 2H), 1.91 (dq, J = 12.7, 6.4 Hz, 2H). LCMS: m/z 387.20 [M+H]+ |

### [Example 351]

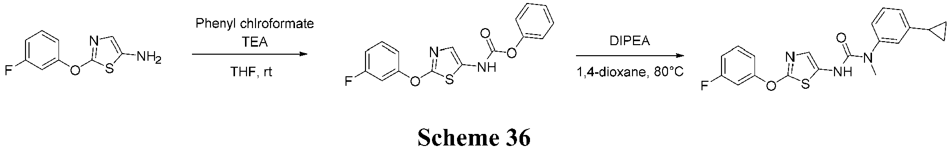

### Synthesis of phenyl (2-(3-fluorophenoxy)thiazol-5-yl)carbamate

After dissolving 1 eq of 2-(3-fluorophenoxy)thiazol-5-amine as **reactant Y1** in tetrahydrofuran (10 mL/g), 1.5 eq of triethylamine was added thereto, and the mixture was cooled to 0°C. After slowly adding 1.2 eq of phenyl chloroformate, the mixture was slowly warmed to room temperature and stirred for 4 hours. After completion of the reaction, the mixture was extracted with ethyl acetate and water, dried over magnesium sulfate, and then concentrated under reduced pressure. The concentrate was purified and separated by column chromatography (hexane:ethyl acetate) to obtain phenyl (2-(3-fluorophenoxy)thiazol-5-yl)carbamate.

### Synthesis of 1-(3-cyclopropylphenyl)-3-(2-(3-fluorophenoxy)thiazol-5-yl)-1-methylurea (Example 351)

1 eq of phenyl (2-(3-fluorophenoxy)thiazol-5-yl)carbamate was dissolved in 1,4-dioxane (10 mL/g). 1.5 eq of 3-cyclopropyl-N-methylaniline as **reactant Y2** and 2 eq of N,N-diisopropylethylamine were added thereto, and the mixture was heated to 80°C. After completion of the reaction, the mixture was extracted with ethyl acetate and water. The ethyl acetate layer was dried over magnesium sulfate, concentrated under reduced pressure, purified, and separated by column chromatography (hexane:ethyl acetate) to obtain the title **compound of Example 351.**

### [Examples 352-362]

The **compounds of Examples 352-362** were synthesized in substantially the same manner as described in the method for preparing the compound of Example 351 **(Scheme 36)** by using **reactants Y1 and Y2 of Table 40** instead of reactants Y1 and Y2 in the method for preparing the compound of Example 351.

The structures of the compounds of Examples 351-362, reactants Y1, Y2, and analytical data are shown in Table 40.

**[Table 40]**

| **No** | **Structure** | **Reactant Y1** | **Reactant Y2** | **Analysis data** |
|---|---|---|---|---|
| **351** | 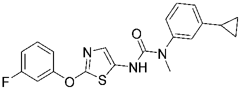 | 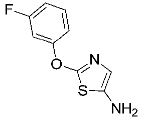 | 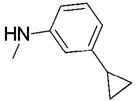 | ¹H NMR (400 MHz, CDCl3) δ 7.30 (dd, J = 13.1, 5.3 Hz, 1H), 7.28 - 7.21 (m, 1H), 7.01 (dd, J = 7.6, 1.9 Hz, 2H), 6.96 (dd, J = 8.4, 1.9 Hz, 2H), 6.94 - 6.89 (m, 1H), 6.87 - 6.80 (m, 1H), 6.55 (s, 1H), 6.46 (s, 1H), 3.25 (s, 3H), 1.87 (ddd, J = 13.3, 8.5, 5.0 Hz, 1H), 1.03 - 0.96 (m, 2H), 0.67 (dt, J = 6.6, 4.9 Hz, 2H). LCMS: m/z 384.1 [M+H]+ |
| **352** | 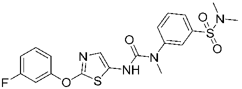 | 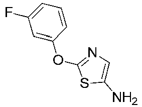 | 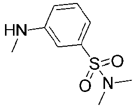 | ¹H NMR (400 MHz, CDCl3) δ 7.85 - 7.73 (m, 1H), 7.72 (d, J = 5.3 Hz, 1H), 7.61 (dd, J = 20.4, 9.5 Hz, 1H), 7.53 - 7.44 (m, 1H), 7.34 (d, J = 8.1 Hz, 1H), 7.11 - 6.90 (m, 2H), 6.73 (s, 1H), 6.56 (s, 1H), 3.42 (s, 3H), 2.81 (s, 6H). LCMS: m/z 451.1 [M+H]+ |
| **353** | 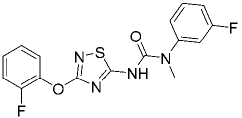 | 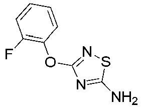 | 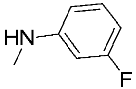 | ¹H NMR (400 MHz, CDCl3) δ 8.46 (s, 1H), 7.41 (td, J = 8.1, 6.3 Hz, 1H), 7.24 (td, J = 8.0, 1.6 Hz, 1H), 7.16 - 7.03 (m, 5H), 6.98 (dt, J = 9.0, 2.2 Hz, 1H), 3.31 (s, 3H). LCMS: m/z 363.0 [M+H]+ |
| **354** | 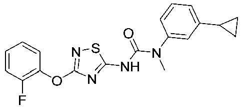 | 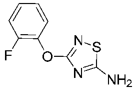 | 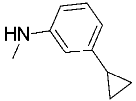 | ¹H NMR (400 MHz, CDCl3) δ 8.16 (s, 1H), 7.41 (t, J = 7.8 Hz, 1H), 7.37 - 7.30 (m, 1H), 7.20 (dd, J = 13.0, 5.1 Hz, 3H), 7.14 (d, J = 7.9 Hz, 1H), 7.09 (d, J = 8.9 Hz, 1H), 7.03 (t, J = 1.8 Hz, 1H), 3.38 (s, 3H), 2.00 - 1.91 (m, 1H), 1.12 - 1.05 (m, 2H), 0.80 - 0.74 (m, 2H). LCMS: m/z 385.1 [M+H]+ |
| **355** | 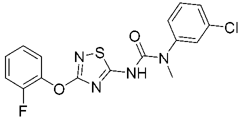 | 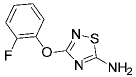 | 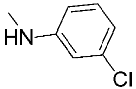 | ¹H NMR (400 MHz, CDCl3) δ 8.52 (s, 1H), 7.34 (tt, J = 4.6, 3.1 Hz, 2H), 7.29 (t, J = 1.9 Hz, 1H), 7.26 - 7.21 (m, 1H), 7.17 (dd, J = 5.5, 4.0 Hz, 1H), 7.16 - 7.04 (m, 3H), 3.34 - 3.28 (m, 3H). LCMS: m/z 379.0 [M+H]+ |
| **356** | 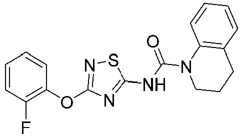 | 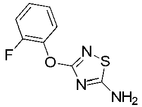 | 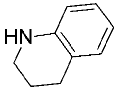 | ¹H NMR (400 MHz, CDCl3) δ 9.01 (s, 1H), 7.39 - 7.30 (m, 4H), 7.27 - 7.15 (m, 4H), 3.90 (t, J = 6.5 Hz, 2H), 2.82 (t, J = 6.7 Hz, 2H), 2.06 (p, J = 6.6 Hz, 2H). LCMS: m/z 371.1 [M+H]+ |
| **357** | 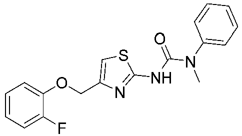 | 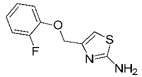 | 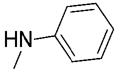 | ¹H NMR (400 MHz, CDCl3) δ 7.54 (t, J = 7.5 Hz, 2H), 7.46 (t, J = 7.4 Hz, 1H), 7.34 (d, J = 7.4 Hz, 2H), 7.13 - 6.98 (m, 3H), 6.97 - 6.93 (m, 1H), 6.91 (s, 1H), 5.05 (s, 2H), 3.42 (s, 3H). LCMS: m/z 358.10 [M+H]+ |
| **358** | 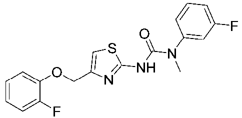 | 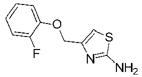 | 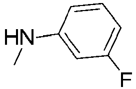 | ¹H NMR (400 MHz, CDCl3) δ 7.52 (dd, J = 14.3, 8.0 Hz, 1H), 7.22 - 7.11 (m, 2H), 7.11 - 7.03 (m, 4H), 6.99 - 6.95 (m, 1H), 6.94 (s, 1H), 5.07 (s, 2H), 3.43 (s, 3H). LCMS: m/z 376.09 [M+H]+ |
| **359** | 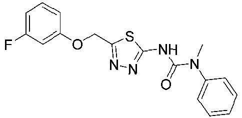 | 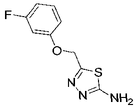 | 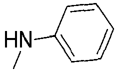 | ¹H NMR (400 MHz, CDCl3) δ 7.46 (t, J = 7.4 Hz, 2H), 7.39 (t, J = 7.3 Hz, 1H), 7.24 (d, J = 7.1 Hz, 2H), 7.19 - 7.13 (m, 1H), 6.70 (dd, J = 8.5, 1.7 Hz, 1H), 6.64 (ddd, J = 8.5, 4.7, 2.2 Hz, 2H), 5.30 (s, 2H), 3.32 (s, 3H). LCMS: m/z 359.09 [M+H]+ |
| **360** | 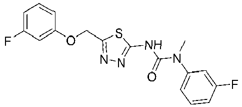 | 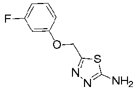 | 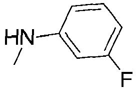 | ¹H NMR (400 MHz, CDCl3) δ 7.52 (dd, J = 14.5, 8.1 Hz, 1H), 7.28 - 7.23 (m, 1H), 7.20 (dd, J = 8.3, 2.4 Hz, 1H), 7.15 (d, J = 9.5 Hz, 1H), 7.08 (dt, J = 9.0, 2.2 Hz, 1H), 6.82 - 6.78 (m, 1H), 6.74 (dd, J = 8.0, 5.5 Hz, 2H), 5.39 (s, 2H), 3.41 (s, 3H). LCMS: m/z 377.08 [M+H]+ |
| **361** | 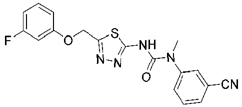 | 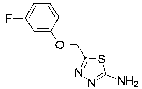 | 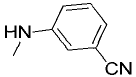 | ¹H NMR (400 MHz, CDCl3) δ 7.74 - 7.60 (m, 4H), 7.26 (d, J = 8.2 Hz, 1H), 6.77 (ddd, J = 19.4, 10.4, 2.2 Hz, 3H), 5.38 (s, 2H), 3.46 (s, 3H). LCMS: m/z 384.09 [M+H]+ |
| **362** | 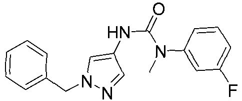 | 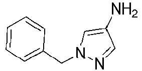 | 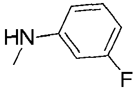 | ¹H NMR (400 MHz, DMSO-d6) *δ* 8.44 (s, 1H), 7.78 (s, 1H), 7.47 - 6.99 (m, 10H), 5.23 (s, 2H), 3.24 (s, 3H). LCMS: m/z 325.1 [M+H]+ |

### [Example 363]

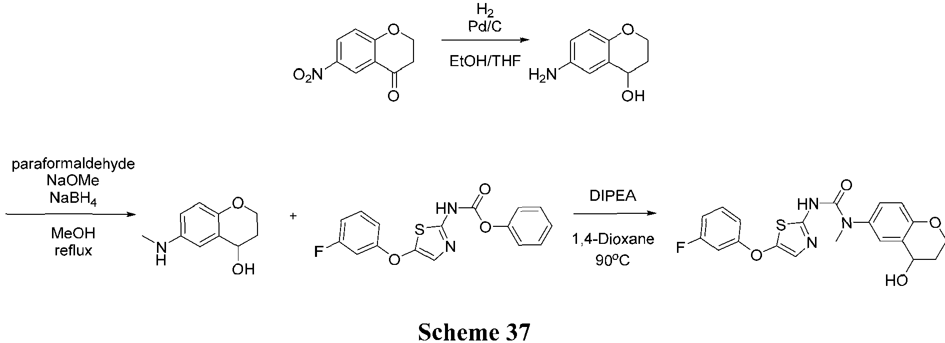

### Synthesis of 6-aminochroman-4-ol

1 eq of 6-nitrochroman-4-one was dissolved in ethanol (5 mL/g) and tetrahydrofuran (5 mL/g). 10 wt% of palladium/carbon was added thereto, and the mixture was hydrogenated under a hydrogen atmosphere to perform the reaction. The reaction mixture was stirred at room temperature for 6 hours. After confirming the completion of the reaction, the mixture was filtered through Celite. The filtrate was concentrated under reduced pressure to obtain a crude product, which was then purified by column chromatography and concentrated under vacuum to obtain 6-aminochroman-4-ol.

### Synthesis of 6-(methylamino)chroman-4-ol

1 eq of 6-aminochroman-4-ol was dissolved in methanol (10 mL/g). 1.5 eq of paraformaldehyde and 5 eq of sodium methoxide were added thereto, and the mixture was stirred under reflux for about 1 hour. 1 eq of sodium borohydride was added thereto at 0°C and the mixture was stirred for about 30 minutes. Water was added thereto, and then the organic layer was obtained by extraction using ethyl acetate and brine. Moisture remaining in the organic layer was completely removed with magnesium sulfate and then filtered. Moisture remaining in the organic layer was completely removed with magnesium sulfate and then filtered. The filtrate was concentrated under reduced pressure to obtain a crude product, which was then purified by column chromatography and concentrated under vacuum to obtain 6-(methylamino)chroman-4-ol.

### Synthesis of 3-(5-(3-fluorophenoxy)thiazol-2-yl)-1-(4-hydroxychroman-6-yl)-1-methylurea (Example 363)

1 eq of phenyl (5-(3-fluorophenoxy)thiazol-2-yl)carbamate was dissolved in 1,4-dioxane (10 mL/g). 1.5 eq of 6-(methylamino)chroman-4-ol and 2 eq of N,N-diisopropylethylamine were added thereto, and the mixture was stirred at 90°C for about 1 hour. After confirming the completion of the reaction, water was added thereto, and then the organic layer was obtained by extraction using ethyl acetate and brine. Moisture remaining in the organic layer was completely removed with magnesium sulfate and then filtered. The filtrate was concentrated under reduced pressure to obtain a crude product, which was then purified by column chromatography and concentrated under vacuum to obtain the title **compound of Example 363.**

**[Table 41]**

| **No** | **Structure** | **Analysis data** |
|---|---|---|
| **363** | 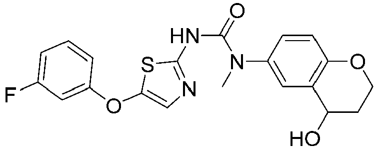 | ¹H NMR (400 MHz, DMSO-d6) δ 10.21 (s, 1H), 7.43 (dd, J = 15.8, 7.8 Hz, 1H), 7.24 (s, 1H), 7.17 (s, 1H), 7.10 (d, J = 8.5 Hz, 1H), 7.03 - 6.93 (m, 3H), 6.79 (d, J = 8.6 Hz, 1H), 5.44 (d, J = 5.0 Hz, 1H), 4.68 - 4.58 (m, 1H), 4.21 (t, J = 4.9 Hz, 2H), 3.23 (s, 3H), 2.06 - 1.97 (m, 1H), 1.93 - 1.83 (m, 1H). LC/MS m/z: 416.24 [M+H]+ |

### [Example 364]

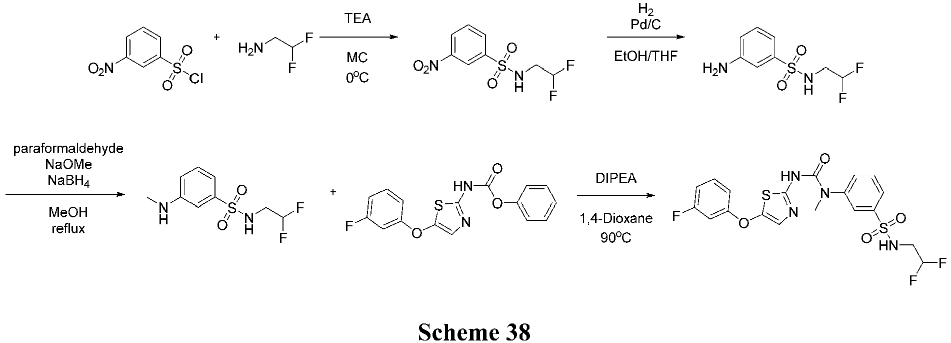

### Synthesis of N-(2,2-difluoroethyl)-3-nitrobenzenesulfonamide

1 eq of 3-nitrobenzenesulfonyl chloride was dissolved in methylene chloride (10 mL/g). 1.2 eq of 2,2-difluoroethan-1-amine and 3 eq of triethylamine were added thereto at 0°C, and the mixture was stirred. After confirming the completion of the reaction, water was added thereto, and then the organic layer was obtained by extraction using methylene chloride and brine. Moisture remaining in the organic layer was completely removed with magnesium sulfate and then filtered. The filtrate was concentrated under reduced pressure to obtain a crude product, which was then purified by column chromatography and concentrated under vacuum to obtain N-(2,2-difluoroethyl)-3-nitrobenzenesulfonamide.

### Synthesis of 3-amino-N-(2,2-difluoroethyl)benzenesulfonamide

1 eq of N-(2,2-difluoroethyl)-3-nitrobenzenesulfonamide was dissolved in ethanol (5 mL/g) and tetrahydrofuran (5 mL/g). 10 wt% of palladium/carbon was added thereto, and the mixture was hydrogenated under a hydrogen atmosphere to perform the reaction. The reaction mixture was stirred at room temperature for 8 hours. After confirming the completion of the reaction, the mixture was filtered through Celite. The filtrate was concentrated under reduced pressure to obtain a crude product, which was then purified by column chromatography and concentrated under vacuum to obtain 3-amino-N-(2,2-difluoroethyl)benzenesulfonamide.

### Synthesis of N-(2,2-difluoroethyl)-3-(methylamino)benzenesulfonamide

1 eq of 3-amino-N-(2,2-difluoroethyl)benzenesulfonamide was dissolved in methanol (10 mL/g). 1.5 eq of paraformaldehyde and 5 eq of sodium methoxide were added thereto, and the mixture was stirred under reflux for about 1 hour. 1 eq of sodium borohydride was added thereto at 0°C and the mixture was stirred for about 30 minutes. Water was added thereto, and then the organic layer was obtained by extraction using ethyl acetate and brine. Moisture remaining in the organic layer was completely removed with magnesium sulfate and then filtered. Moisture remaining in the organic layer was completely removed with magnesium sulfate and then filtered. The filtrate was concentrated under reduced pressure to obtain a crude product, which was then purified by column chromatography and concentrated under vacuum to obtain N-(2,2-difluoroethyl)-3-(methylamino)benzenesulfonamide.

### Synthesis of N-(2,2-difluoroethyl)-3-(3-(5-(3-fluorophenoxy)thiazol-2-yl)-1-methylureido)benzenesulfonamide (Example 364)

1 eq of phenyl (5-(3-fluorophenoxy)thiazol-2-yl)carbamate was dissolved in 1,4-dioxane (10 mL/g). 1.5 eq of N-(2,2-difluoroethyl)-3-(methylamino)benzenesulfonamide and 2 eq of N,N-diisopropylethylamine were added thereto, and the mixture was stirred at 90°C for about 1 hour. After confirming the completion of the reaction, water was added thereto, and then the organic layer was obtained by extraction using ethyl acetate and brine. Moisture remaining in the organic layer was completely removed with magnesium sulfate and then filtered. The filtrate was concentrated under reduced pressure to obtain a crude product, which was then purified by column chromatography and concentrated under vacuum to obtain the title **compound of Example 364.**

**[Table 42]**

| **No** | **Structure** | **Analysis data** |
|---|---|---|
| **364** | 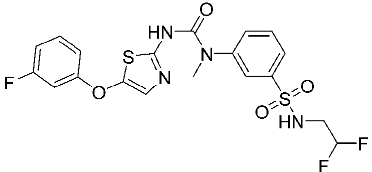 | ¹H NMR (400 MHz, DMSO-d6) δ 11.14 (s, 1H), 8.29 (s, 1H), 7.78 (s, 1H), 7.71 - 7.59 (m, 3H), 7.47 - 7.39 (m, 1H), 7.23 (s, 1H), 7.05 - 6.90 (m, 3H), 6.02 (t, J = 55.4 Hz, 1H), 3.37 (s, 3H), 3.25 (t, J = 14.1 Hz, 2H). LC/MS m/z: 486.94 [M+H]+ |

### <Experimental Example>

### Experimental Example 1. IP-one assay for confirming MrgprX2 activity

The activity of the present compound was evaluated using a HEK293 stable cell line (hereinafter, MrgprX2-HEK293) expressing human MrgprX2. The corresponding cells were cultured in a cell incubator under the conditions of 37°C and 5% CO₂ using a culture medium in which 10% FBS (Corning; 35-015-CV) and 1 µg/mL puromycin (Gibco; A1113802) were added to high glucose DMEM (Gibco; 10564029) containing GlutaMAX.

On the day before the experiment, MrgprX2-HEK293 cells were dispensed into a 96-well assay plate at a density of 10,000 cells in 100 µL of cell culture medium per well, and cultured in a cell incubator (n=3). On the day of the experiment, the cell culture medium was carefully removed, and then 100 µL of DPBS (Gibco; 14190-144) was dispensed into each well and removed to carry out washing. After that, 10 mM compound dissolved in dimethyl sulfoxide (DMSO) was serially diluted at a ratio of 1:3, and then diluted in an assay buffer included in an IP-one Gq kit (Cisbio; 62IPAPEB) to prepare diluents at a total of 10 concentrations (1 nM to 30 µM). The final DMSO concentration of each diluent was 0.3%, and the same amount of DMSO was added to the control. 100 µL of the prepared compound diluent was dispensed into each of the washed MrgprX2-HEK293 cells, and the cells were treated in a 37°C and 5% CO₂ cell incubator for 1 hour. After that, 200 nM cortistatin-14 (hereinafter, Cort14, MedchemExpress; HY-P1932A) was prepared in an assay buffer, and 100 µL thereof was dispensed into each well in order to stimulate MrgprX2. The final concentration of Cort14 was 100 nM. For the action of Cort14, the MrgprX2-HEK293 cells were treated in a 37°C and 5% CO₂ incubator for 1 hour. After that, the assay buffer was completely removed from each well, and the cell membrane was lysed by dispensing 30 µL of lysis buffer included in the IP-one Gq kit. 15 µL of the lysate was removed, and 6 µL of detection buffer included in the IP-one Gq kit was dispensed into the remaining half of the lysate, which was then reacted at room temperature for 1 hour under dark conditions. The plate was subjected to HTRF measurement using a Tecan Spark 10 m instrument. Graphs and IC₅₀ values were calculated from the measured HTRF ratio using GraphPad Prism 10.

The results of Experimental Example 1 are shown in Table 43 below, and A, B and C in Table 43 mean the following, respectively.

A: IC₅₀< 500 nM, B: 500 nM ≤ IC₅₀ < 1 uM, C: 1 uM ≤ IC₅₀ < 3 uM,

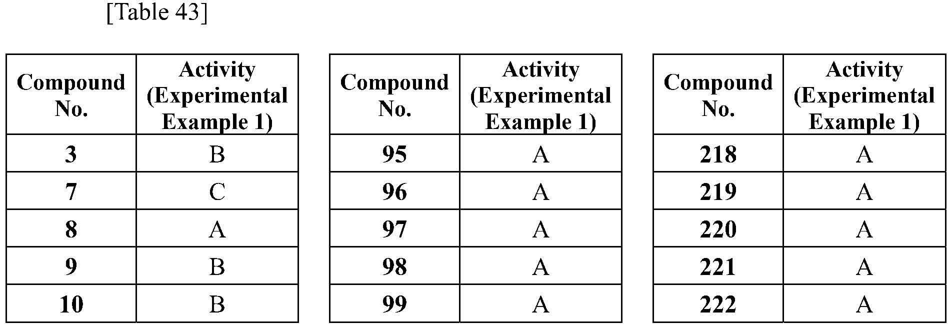

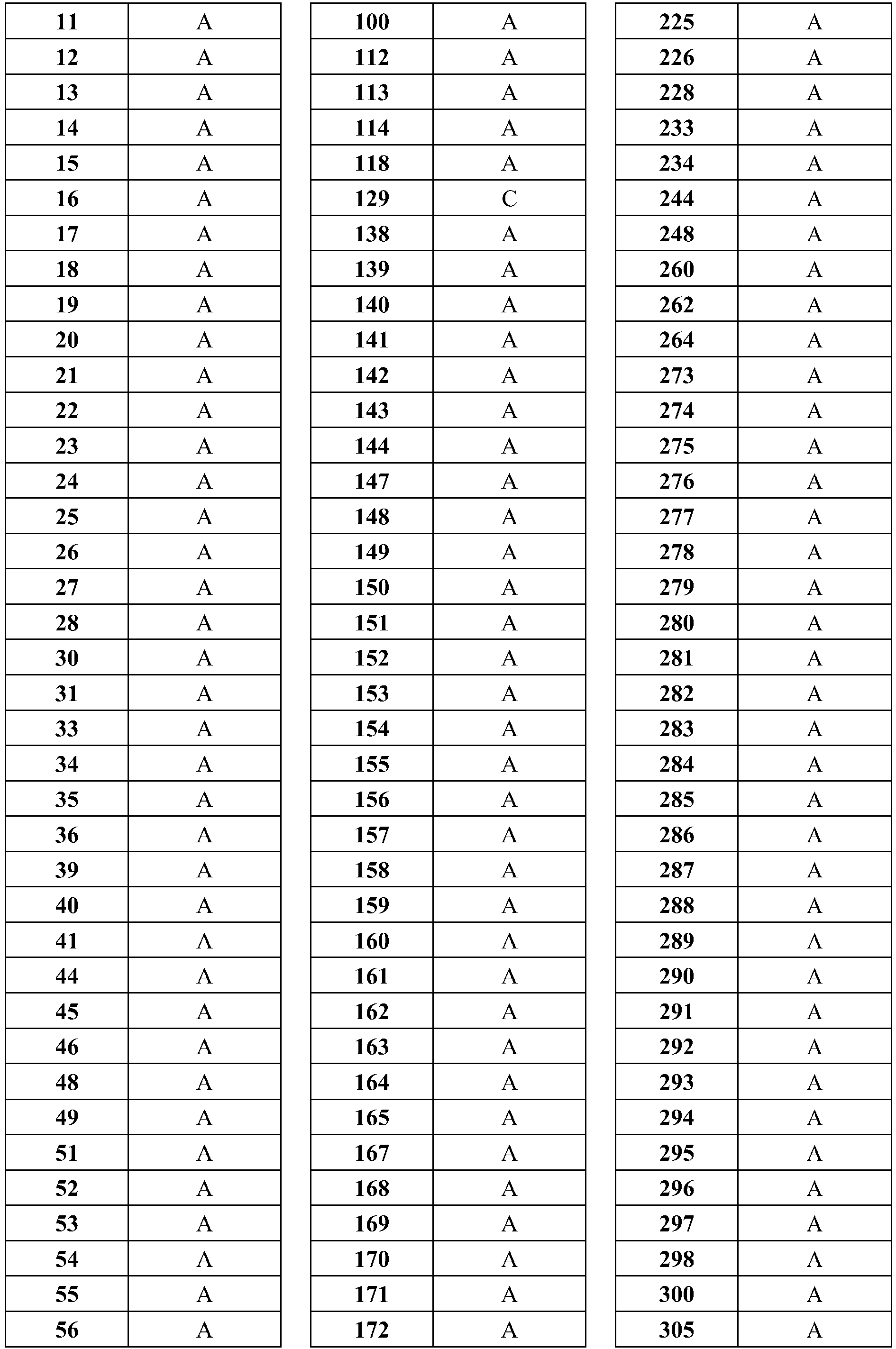

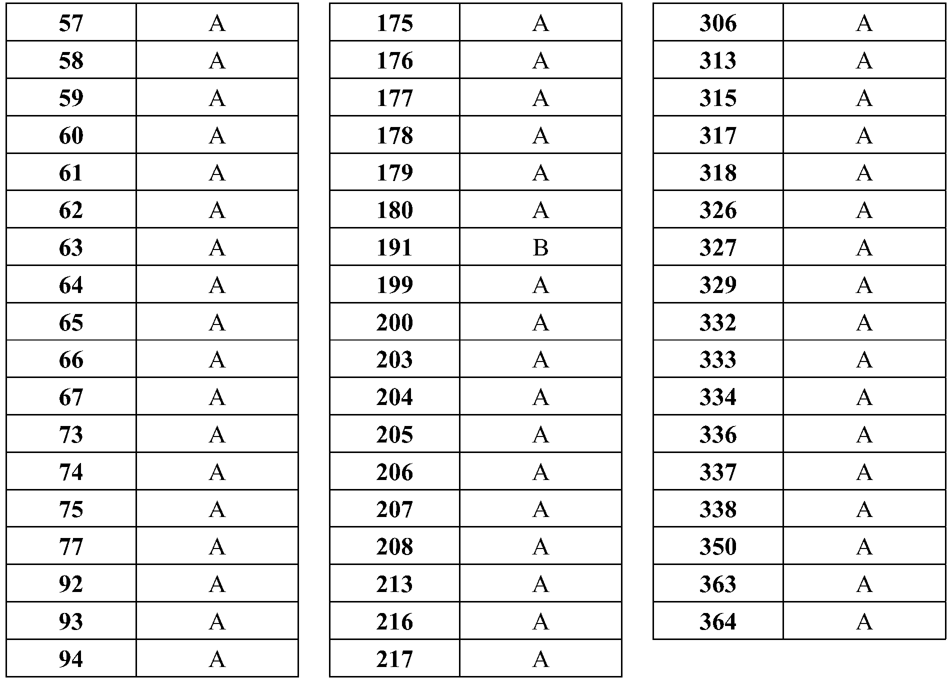

### Experimental Example 2. Calcium mobilization assay

Human MRGPRX2 stable cells (Creative-biogene) were cultured in a 37°C and 5% CO₂ cell incubator using a DMEM (Gibco; 10564029) cell culture medium containing 1 µg/mL puromycin (Gibco) and 10% FBS (Corning).

After that, on the day before the experiment, 10,000 cells/well were transferred to a 96-well black plate together with 100 µL of cell culture medium and cultured in a cell incubator. On the day of the experiment, a 2x loading buffer capable of treating a final concentration of 1 nM to 30 µM was prepared through 1:3 serial dilution of the compound dissolved in DMSO at 10 mM as an antagonist using a loading buffer of a Fura-2 QBT calcium kit (Molecular devices; R8197). Each well was treated with 100 µL of the 2x loading buffer to react the antagonist at 37°C for 1 hour. After that, cortistatin-14 (MedchemExpress), an agonist of the compound, was prepared as a 5x solution using a HBSS buffer, and then dispensed into a 96-well v-bottom plate as much as required. A cell plate prepared for calcium mobilization measurement was placed into Flexstation 3 (Molecular Devices) together with the plate prepared with the agonist, and the cells were treated with 50 µL of the agonist using an auto injector included in the instrument. By setting a flex mode, fluorescence at Em 340, 380 nm, and EX 510 nm wavelengths was measured for a total of 90 seconds at an interval of four seconds from 15 seconds before the injection of the agonist. The measured value was calculated as (Em 510 nm from Ex 340 nm)/(Em 510 nm from Ex 380 nm) to measure calcium mobilization, and then the calcium mobilization according to an antagonist concentration was used to calculate IC₅₀ values using GraphPad Prism.

The results of Experimental Example 2 are shown in Table 44 below, and A, B and C in Table 44 mean the following, respectively.

A: IC₅₀ < 500 nM, B: 500 nM ≤ IC₅₀ < 1 uM, C: 1 uM ≤ IC₅₀ < 3 uM

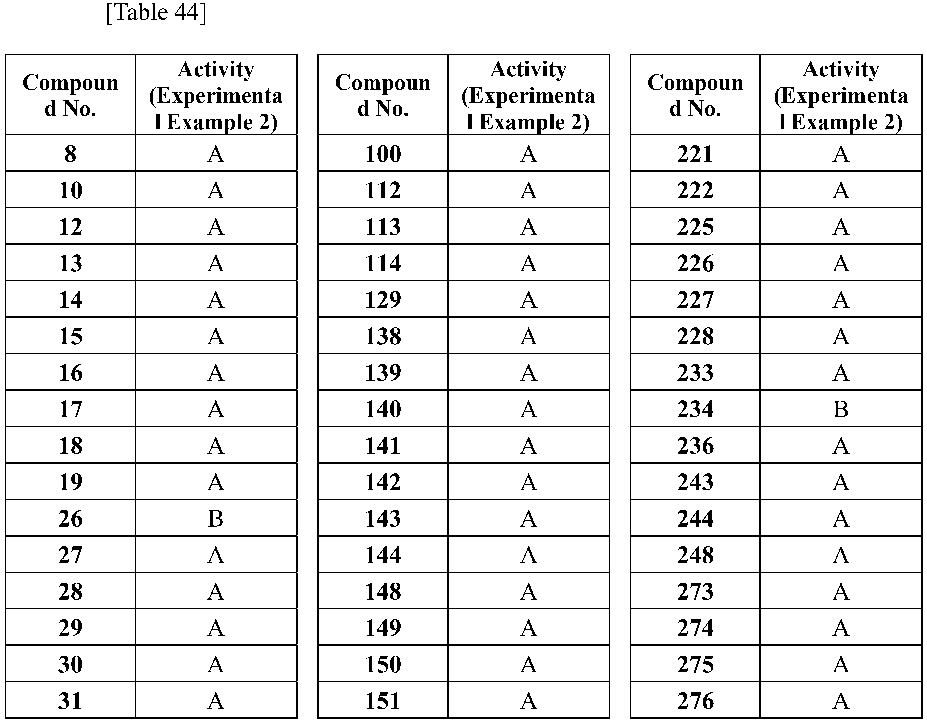

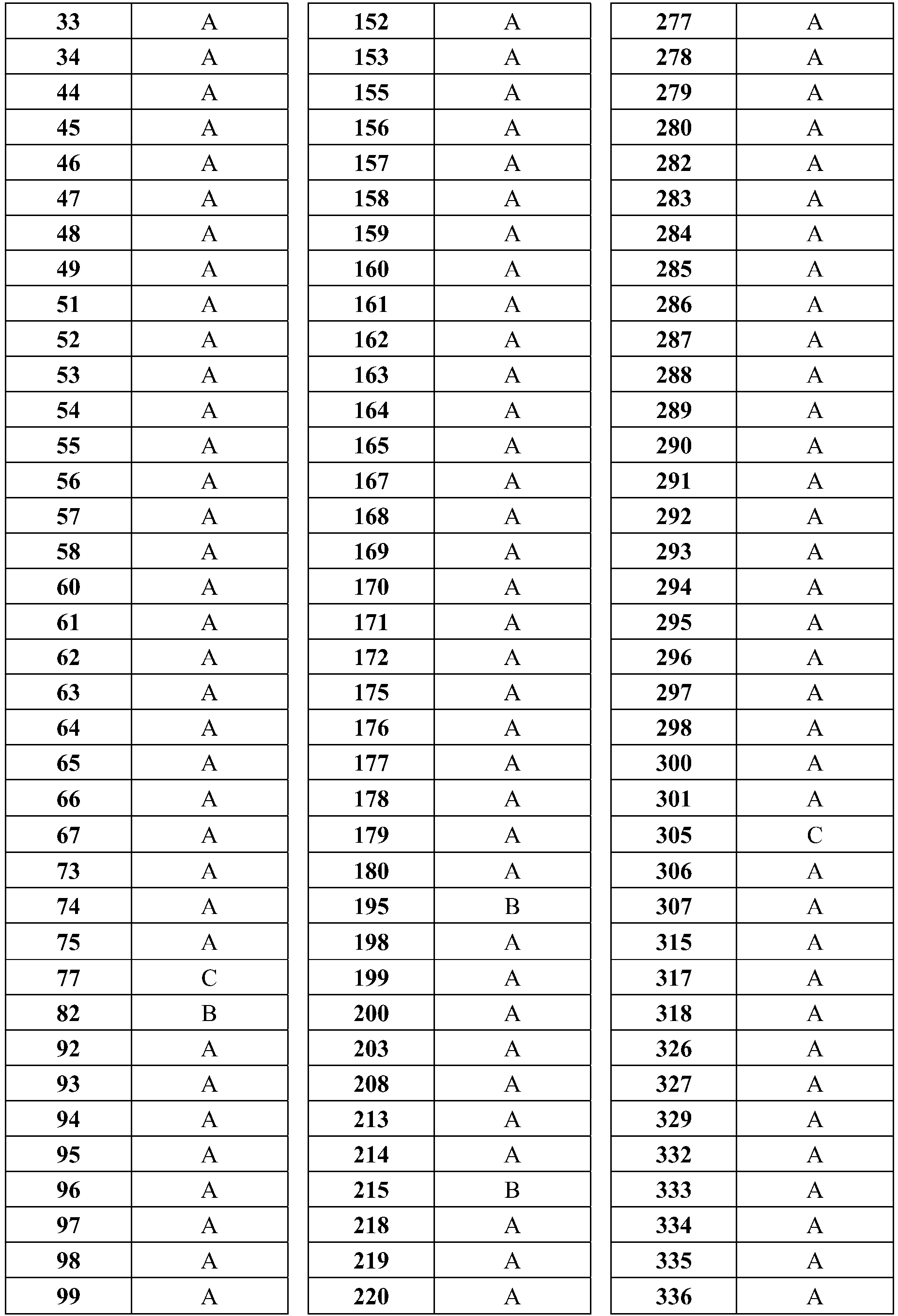

It was confirmed through the IP-one assay and the calcium mobilization assay of above Experimental Examples 1 and 2 that the activity of the present compound is excellent. In addition, it was confirmed that the present compound has excellent physical properties and low toxicity even in ADMET (metabolic stability, PK, CYP, hERG), and the like.

## Claims

1. A compound represented by chemical formula I or chemical formula II below, a stereoisomer thereof, or a pharmaceutically acceptable salt thereof: 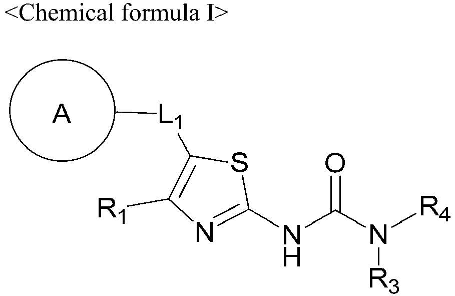 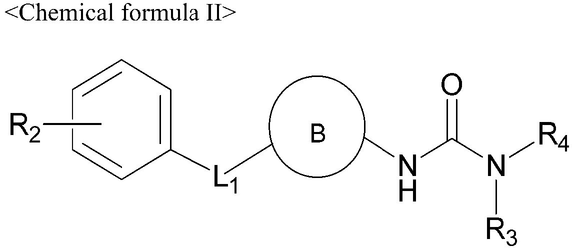 wherein in chemical formula I,
A is 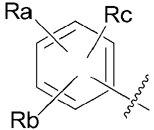 (Ra, Rb and Rc are each independently H, halogen, CN, -NO₂, C1-C6 alkyl, C1-C6 haloalkyl, -O-(C1-C6 alkyl), -C(=O)-OH, -C(=O)-(C1-C6 alkyl), -C(=O)-O-(C1-C6 alkyl), -N(C1-C6 alkyl)(C1-C6 alkyl) or C3-C12 cycloalkyl), 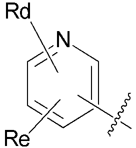 (Rd and Re are each independently H or halogen), 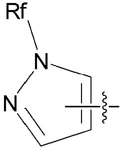 (Rf is H or C1-C6 alkyl), or 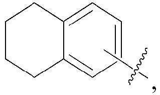
R₁ is H, C1-C6 alkyl or -C(=O)-O-(C1-C6 alkyl);
wherein in chemical formula II, B is 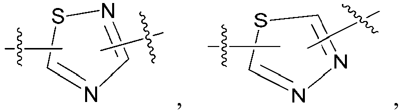 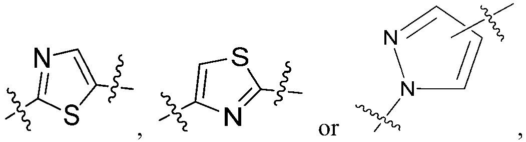
R₂ is H or halogen;
and wherein in each of chemical formulas I and II,
Li is -(CH₂)x- (x is an integer of 1 to 3), -O-, -N(Rg)- (Rg is H or C1-C6 alkyl) or -O-(CH₂)y- (y is an integer of 1 to 3),
R₃ and R₄ are each a functional group as defined below, or are linked to each other to form a cyclic group comprising at least one N,
R₃ is H, C1-C6 alkyl or 3- to 12-membered heterocycloalkyl comprising at least one heteroatom selected from N, O and S, in which at least one H of C1-C6 alkyl may be each independently substituted with OH, CN, -O-(C1-C6 alkyl) or C3-C12 cycloalkyl,
R₄ is 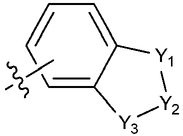 (Y₁ to Y₃ each independently represent CH₂, O, S or NH), 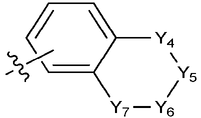 (Y₄ to Y₆ each independently represent CH₂, O, S, C(=O) or NH), 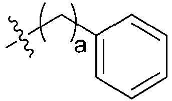 (a is an integer of 0 to 3), 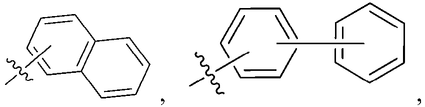 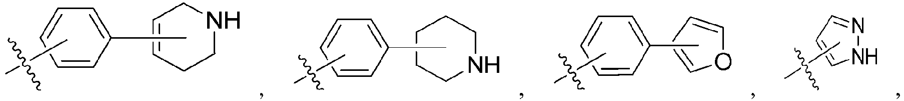 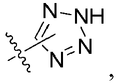 or 5- to 12-membered heteroaryl comprising at least one heteroatom selected from N, O and S,
in which at least one H of R₄ may be each independently substituted with halogen, - CN, -NO₂, -OH, C1-C6 alkyl (in which at least one H of C1-C6 alkyl may be each independently substituted with -O-(C1-C6 alkyl)), C1-C6 haloalkyl, C2-C6 alkynyl, C3-C6 cycloalkyl, -O-(C1-C6 alkyl), -O-(C1-C6 haloalkyl), -S-(C1-C6 haloalkyl), -NH₂, -N(C1-C6 alkyl)(C1-C6 alkyl), -C(=O)-Rg (Rg is C1-C6 alkyl, -NH₂, -O-(C1-C6 alkyl), -OH, 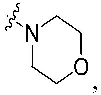 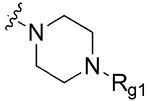 (R_{g1} is H or C1-C6 alkyl), or 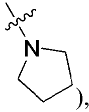 -[(C1-C6 alkylene)]z-NH-S(=O)₂-Rₕ (Rₕ is C1-C6 alkyl, C3-C6 cycloalkyl, -N(C1-C6 alkyl)(C1-C6 alkyl), and z is 0 or 1), -O-S(=O)₂-Rᵢ (Rᵢ is C1-C6 alkyl or C6-C12 aryl, in which at least one H of C6-C12 aryl may be each independently substituted with C1-C6 alkyl), -S(=O)₂-Rⱼ (Rⱼ is C1-C6 alkyl or 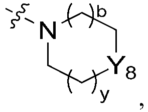 Y₈ is CH₂, O or NH, b is 0 or 1, and y is 0 or 1, in which at least one H of Rⱼ may be each independently substituted with halogen, C1-C6 alkyl or C1-C6 haloalkyl), or - S(=O)₂-N(Rₘ)Rₙ (Rm and Rn are each independently H, C1-C6 alkyl, C1-C6 haloalkyl, C3-C12 cycloalkyl, 3- to 12-membered heterocycloalkyl comprising at least one heteroatom selected from N, O and S, or C6-C12 aryl, and at least one H of each of Rm and Rn may be each independently substituted with OH or -O-(C1-C6 alkyl));
a cyclic group formed by linking R₃ and R₄ to each other is 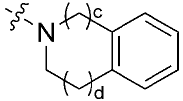 (c and d each independently represent any one integer of 0 to 3), 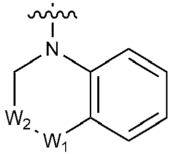 (W₁ and W₂ each represent CH₂, NH or O, and W₁ and W₂ are not simultaneously CH₂), 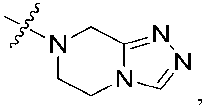 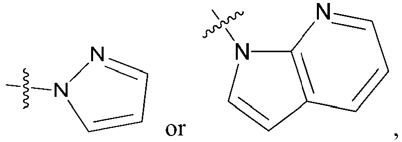 and
at least one H of the cyclic group may be each independently substituted with halogen, CN, OH, NO₂, C1-C6 alkyl, C1-C6 haloalkyl, C3-C12 cycloalkyl, 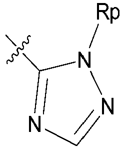 (Rp is H or C1-C6 alkyl) or -S(=O)₂-N(C1-C6 alkyl)(C1-C6 alkyl).

2. The compound, the stereoisomer thereof, or the pharmaceutically acceptable salt thereof of claim 1,
wherein in each of chemical formula I and chemical formula II, R₄ is 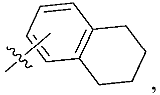 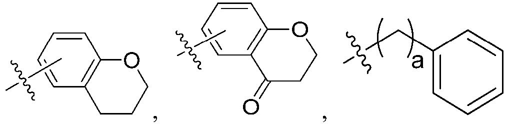 (a is an integer of 0 to 3), 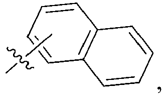 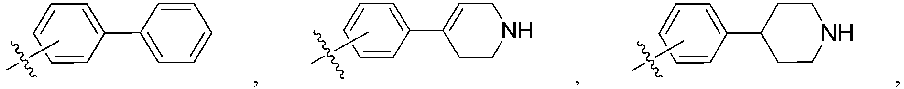 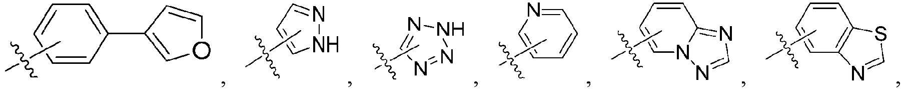 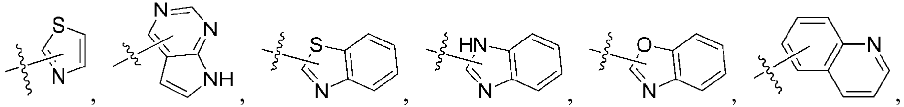 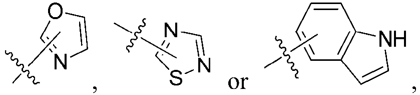
at least one H of R₄ is each independently halogen, -CN, -NO₂, -OH, C1-C6 alkyl (in which at least one H of C1-C6 alkyl may be each independently substituted with -O-(C1-C6 alkyl)), C1-C6 haloalkyl, C2-C6 alkynyl, C3-C6 cycloalkyl, -O-(C1-C6 alkyl), -O-(C1-C6 haloalkyl), -S-(C1-C6 haloalkyl), -NH₂, -N(C1-C6 alkyl)(C1-C6 alkyl), -C(=O)-Rg (Rg is C1-C6 alkyl, -NH₂, -O-(C1-C6 alkyl), -OH, 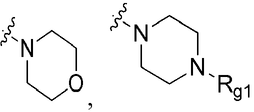 (R_{g1} is H or C1-C6 alkyl), or 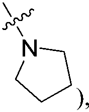 -[(C1-C6 alkylene)]z-NH-S(=O)₂-Rₕ (Rₕ is C1-C6 alkyl, C3-C6 cycloalkyl, - N(C1-C6 alkyl)(C1-C6 alkyl), and z is 0 or 1), -O-S(=O)₂-Rᵢ (Rᵢ is C1-C6 alkyl or C6-C12 aryl, in which at least one H of C6-C12 aryl may be each independently substituted with C1-C6 alkyl), -S(=O)₂-Rⱼ (Rⱼ is C1-C6 alkyl or 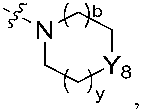 Y₈ is CH₂, O or NH, b is 0 or 1, and y is 0 or 1, in which at least one H of Rⱼ may be each independently substituted with halogen, C1-C6 alkyl or C1-C6 haloalkyl), or -S(=O)₂-N(Rₘ)Rₙ (Rm and Rn are each independently H, C1-C6 alkyl, C1-C6 haloalkyl, C3-C12 cycloalkyl, 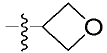
or C6-C12 aryl, and at least one H of each of Rm and Rn may be each independently substituted with OH or -O-(C1-C6 alkyl));
a cyclic group formed by linking R₃ and R₄ to each other is 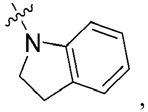 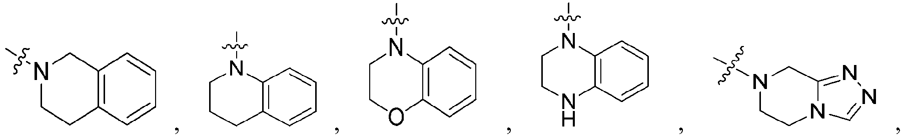 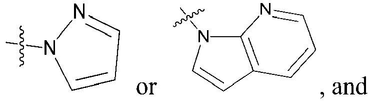
at least one H of the cyclic group may be each independently substituted with halogen, CN, OH, NO₂, C1-C6 alkyl, C1-C6 haloalkyl, C3-C12 cycloalkyl, 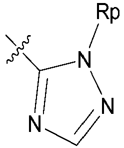 (Rp is H or C1-C6 alkyl) or -S(=O)₂-N(C1-C6 alkyl)(C1-C6 alkyl).

3. The compound, the stereoisomer thereof, or the pharmaceutically acceptable salt thereof of claim 1, comprising a compound represented by chemical formula Ia below: 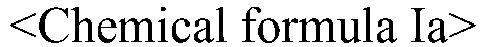 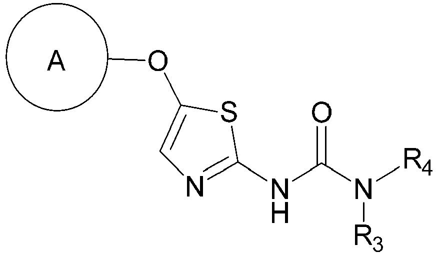 wherein in chemical formula Ia,
A is 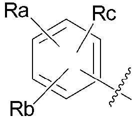
(Ra, Rb and Rc are each independently H, halogen, CN, C1-C6 alkyl, C1-C6 haloalkyl, -CF₃, -O-(C1-C6 alkyl), -C(=O)-OH, -C(=O)-(C1-C6 alkyl), -C(=O)-O-(C1-C6 alkyl), -N(C1-C6 alkyl)(C1-C6 alkyl) or C6-C12 cycloalkyl), 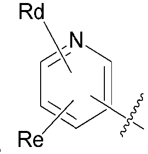 (Rd is H and Re is halogen), 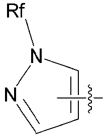 (Rf is C1-C6 alkyl), or 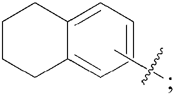
R₃ and R₄ are each a functional group as defined below, or are linked to each other to form a cyclic group comprising at least one N,
R₃ is H or C1-C6 alkyl, in which at least one H of C1-C6 alkyl may be each independently substituted with CN,
R₄ is 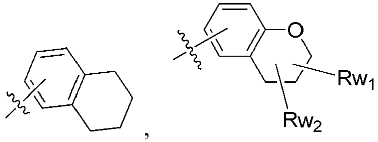 (Rw₁ and Rw₂ each independently represent H, halogen or OH), 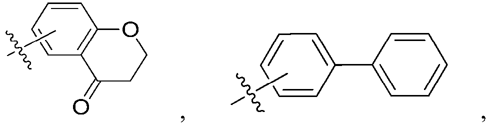 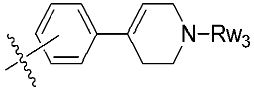 (Rw₃ represents H or C1-C6 alkyl), 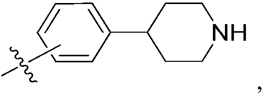 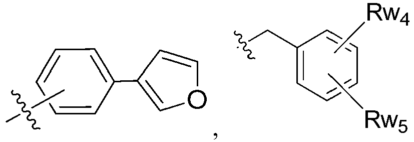 (Rw₄ and Rw₅ each independently represent H, C1-C6 haloalkyl or -CF₃), 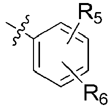 (R₅ and R₆ are each independently H, halogen, -CN, -NO₂, -OH, C1-C6 alkyl (in which at least one H of C1-C6 alkyl may be each independently substituted with -O-(C1-C6 alkyl)), -CH₃, -CH(CH₃)(CH₃), -CH(CH₃)-O-CH₃, C1-C6 haloalkyl, -CF₃, -C≡C-CH₃, C3-C6 cycloalkyl, -O-(C1-C6 alkyl), -O-CH₃, -O-(C1-C6 haloalkyl), -O-CF₃, -O-CHF₂, -S-(C1-C6 haloalkyl), -S-CF₃, -NH₂, -N(CH₃)(CH₃), or -C(=O)-Rg (Rg is C1-C6 alkyl, -NH₂, -O-(C1-C6 alkyl), -OH, 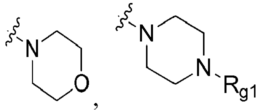 (R_{g1} is H or C1-C6 alkyl), or 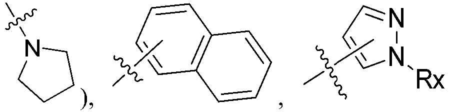 (Rx is H or C1-C6 alkyl), 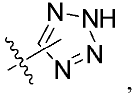 or heteroaryl, which is any one selected from 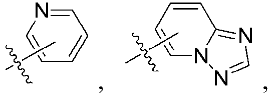 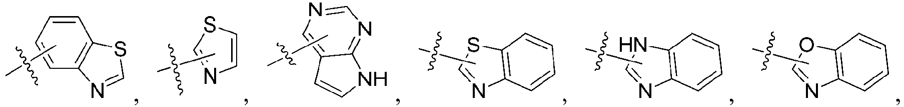 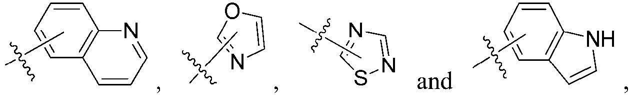 in which at least one H of heteroaryl may be substituted with C1-C6 alkyl, C1-C6 haloalkyl or -CF₃;
a cyclic group formed by linking R₃ and R₄ to each other is 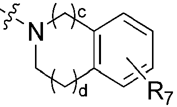 (c and d each independently represent any one integer of 0 to 3, and R₇ is H, halogen, OH, NO₂, C1-C6 alkyl, C1-C6 haloalkyl, -CF₃, or -S(=O)₂-N(C1-C6 alkyl)(C1-C6 alkyl), 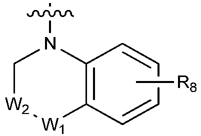 (W₁ and W₂ each independently represent CH₂, NH, N(C1-C6 alkyl) or O, and W₁ and W₂ are not simultaneously CH₂, and R₈ is H, halogen, CN, OH, NO₂, C1-C6 alkyl, C1-C6 haloalkyl or -CF₃), 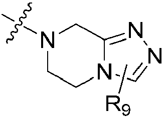 (R₉ is H, C1-C6 alkyl or C3-C12 cycloalkyl), 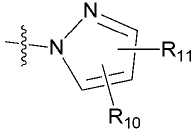 (R₁₀ and R₁₁ are each independently H, C1-C6 alkyl, C1-C6 haloalkyl, - CF₃ or 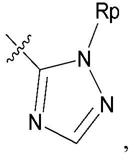 and Rp is H or C1-C6 alkyl), or

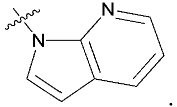

4. The compound, the stereoisomer thereof, or the pharmaceutically acceptable salt thereof of claim 1, comprising a compound represented by chemical formula Ib, chemical formula Ic or chemical formula Id below: 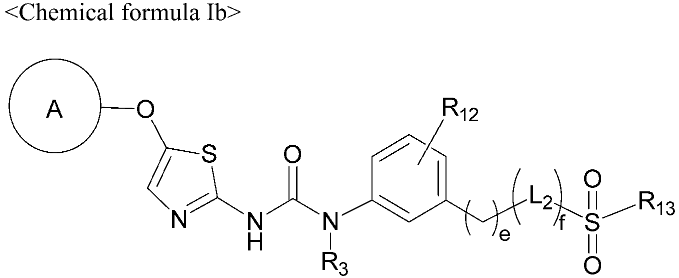 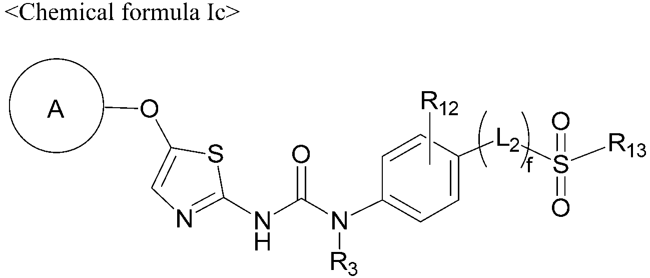 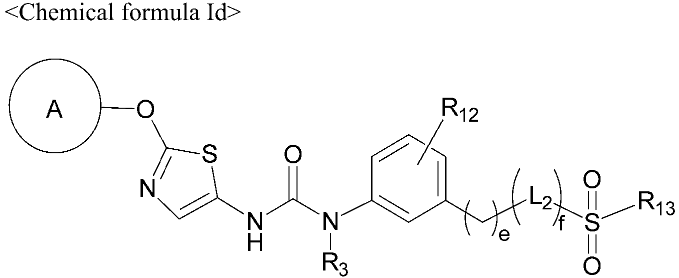
wherein in each of chemical formula Ib, chemical formula Ic and chemical formula Id,
A is 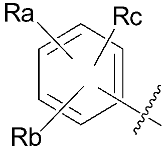
(Ra, Rb and Rc are each independently H, halogen, CN, NO₂, C1-C6 alkyl, C1-C6 haloalkyl, -CF₃, -O-(C1-C6 alkyl), -C(=O)-(C1-C6 alkyl) or -C(=O)-O-(C1-C6 alkyl), 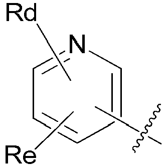 (Rd and Re are each independently H or halogen), or 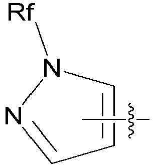 (in which Rf is H or C1-C6 alkyl);
R₃ is H or C1-C6 alkyl;
L₂ is -NH- or -O-, and e and f are each independently 0 or 1;
R₁₂ is H, halogen, C1-C6 alkyl or -O-(C1-C6 alkyl);
R₁₃ is C1-C6 alkyl, C3-C6 cycloalkyl, C6-C12 aryl (in which at least one H of C6-C12 aryl may be each independently substituted with C1-C6 alkyl), 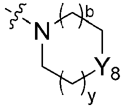 (Y₈ is CH₂ or O, b is 0 or 1, and y is 0 or 1, of which at least one H may be each independently substituted with halogen, C1-C6 alkyl, C1-C6 haloalkyl or -CH₂F) or -N(Rₘ)Rₙ (in which Rm and Rn are each independently H, C1-C6 alkyl (in which at least one H of C1-C6 alkyl may be each independently substituted with -O-(C1-C6 alkyl) or OH), -CH₃, -CH₂CH₃, -CH(CH₃)(CH₃), - CH₂CH₂-O-CH₃, -CH₂-C(CH₃)₂(OH), C1-C6 haloalkyl, -CH₂CH₂F, -CH₂-CF₂H, C3-C12 cycloalkyl, 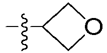 or C6-C12 aryl).

5. A compound shown in a table below, a stereoisomer thereof, or a pharmaceutically acceptable salt thereof:
| **Compound No.** | **Structure** | **IUPAC compound name (English)** |
|---|---|---|
| **1** | 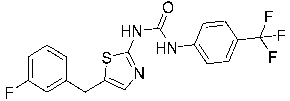 | 1-(5-(3-fluorobenzyl)thiazol-2-yl)-3-(4-(trifluoromethyl)phenyl)urea |
| **2** | 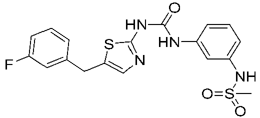 | N-(3-(3-(5-(3-fluorobenzyl)thiazol-2-yl)ureido)phenyl)methanesulfonamide |
| **3** | 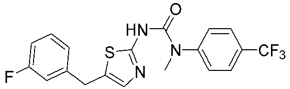 | 3-(5-(3-fluorobenzyl)thiazol-2-yl)-1-methyl-1-(4-(trifluoromethyl)phenyl)urea |
| **4** | 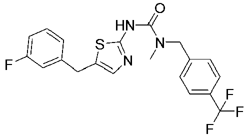 | 3-(5-(3-fluorobenzyl)thiazol-2-yl)-1-methyl-1-(4-(trifluoromethyl)benzyl)urea |
| **5** | 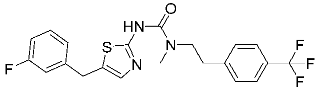 | 3-(5-(3-fluorobenzyl)thiazol-2-yl)-1-methyl-1-(4-(trifluoromethyl)phenethyl)urea |
| **6** | 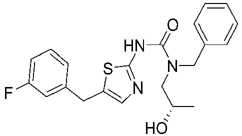 | (S)-1-benzyl-3-(5-(3-fluorobenzyl)thiazol-2-yl)-1-(2-hydroxypropyl)urea |
| **7** | 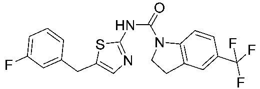 | N-(5-(3-fluorobenzyl)thiazol-2-yl)-5-(trifluoromethyl)indoline-1-carboxamide |
| **8** | 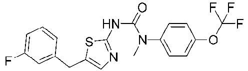 | 3-(5-(3-fluorobenzyl)thiazol-2-yl)-1-methyl-1-(4-(trifluoromethoxy)phenyl)urea |
| **9** | 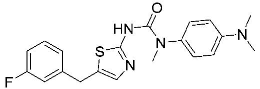 | 1-(4-(dimethylamino)phenyl)-3-(5-(3-fluorobenzyl)thiazol-2-yl)-1-methylurea |
| **10** | 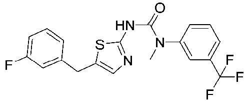 | 3-(5-(3-fluorobenzyl)thiazol-2-yl)-1-methyl-1-(3-(trifluoromethyl)phenyl)urea |
| **11** | 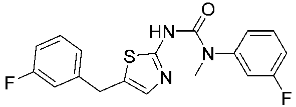 | 3-(5-(3-fluorobenzyl)thiazol-2-yl)-1-(3-fluorophenyl)-1-methylurea |
| **12** | 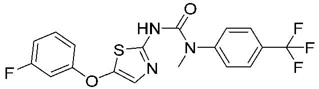 | 3-(5-(3-fluorophenoxy)thiazol-2-yl)-1-methyl-1-(4-(trifluoromethyl)phenyl)urea |
| **13** | 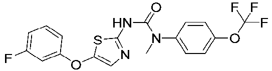 | 3-(5-(3-fluorophenoxy)thiazol-2-yl)-1-methyl-1-(4-(trifluoromethoxy)phenyl)urea |
| **14** | 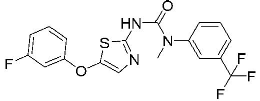 | 3-(5-(3-fluorophenoxy)thiazol-2-yl)-1-methyl-1-(3-(trifluoromethyl)phenyl)urea |
| **15** | 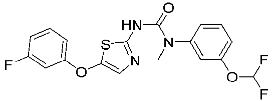 | 1-(3-(difluoromethoxy)phenyl)-3-(5-(3-fluorophenoxy)thiazol-2-yl)-1-methylurea |
| **16** | 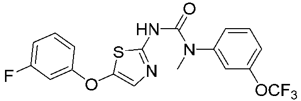 | 3-(5-(3-fluorophenoxy)thiazol-2-yl)-1-methyl-1-(3-(trifluoromethoxy)phenyl)urea |
| **17** | 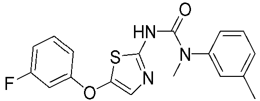 | 3-(5-(3-fluorophenoxy)thiazol-2-yl)-1-methyl-1-(m-tolyl)urea |
| **18** | 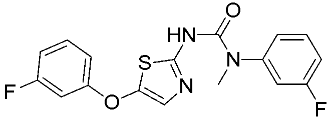 | 3-(5-(3-fluorophenoxy)thiazol-2-yl)-1-(3-fluorophenyl)-1-methylurea |
| **19** | 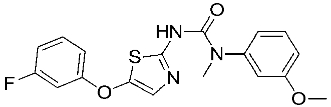 | 3-(5-(3-fluorophenoxy)thiazol-2-yl)-1-(3-methoxyphenyl)-1-methylurea |
| **20** | 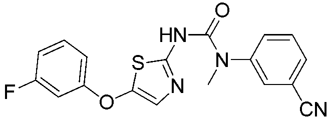 | 1-(3-cyanophenyl)-3-(5-(3-fluorophenoxy)thiazol-2-yl)-1-methylurea |
| **21** | 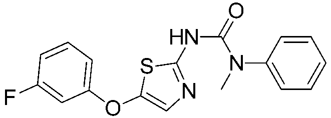 | 3-(5-(3-fluorophenoxy)thiazol-2-yl)-1-methyl-1-phenylurea |
| **22** | 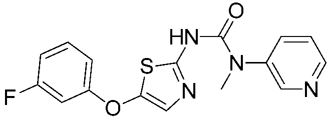 | 3-(5-(3-fluorophenoxy)thiazol-2-yl)-1-methyl-1-(pyridin-3-yl)urea |
| **23** | 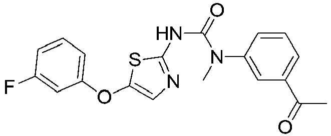 | 1-(3-acetylphenyl)-3-(5-(3-fluorophenoxy)thiazol-2-yl)-1-methylurea |
| **24** | 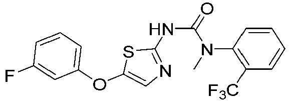 | 3-(5-(3-fluorophenoxy)thiazol-2-yl)-1-methyl-1-(2-(trifluoromethyl)phenyl)urea |
| **25** | 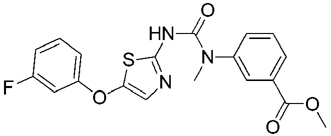 | Methyl 3-(3-(5-(3-fluorophenoxy)thiazol-2-yl)-1-methylureido)benzoate |
| **26** | 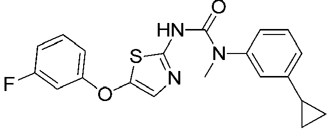 | 1-(3-cyclopropylphenyl)-3-(5-(3-fluorophenoxy)thiazol-2-yl)-1-methylurea |
| **27** | 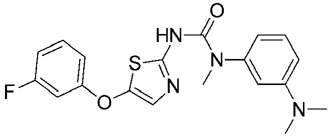 | 1-(3-(dimethylamino)phenyl)-3-(5-(3-fluorophenoxy)thiazol-2-yl)-1-methylurea |
| **28** | 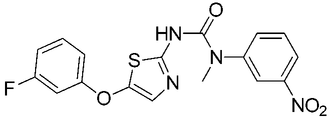 | 3-(5-(3-fluorophenoxy)thiazol-2-yl)-1-methyl-1-(3-nitrophenyl)urea |
| **29** | 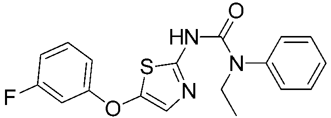 | 1-ethyl-3-(5-(3-fluorophenoxy)thiazol-2-yl)-1-phenylurea |
| **30** | 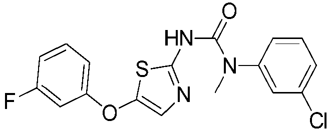 | 1-(3-chlorophenyl)-3-(5-(3-fluorophenoxy)thiazol-2-yl)-1-methylurea |
| **31** | 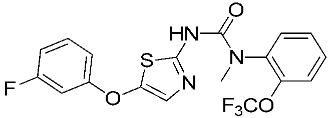 | 3-(5-(3-fluorophenoxy)thiazol-2-yl)-1-methyl-1-(2-(trifluoromethoxy)phenyl)urea |
| **32** | 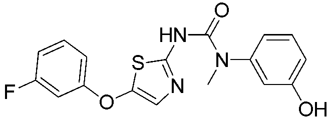 | 3-(5-(3-fluorophenoxy)thiazol-2-yl)-1-(3-hydroxyphenyl)-1-methylurea |
| **33** | 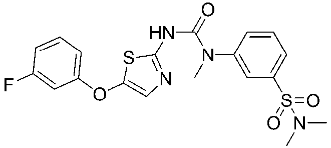 | 3-(3-(5-(3-fluorophenoxy)thiazol-2-yl)-1-methylureido)-N,N-dimethylbenzenesulfonamide |
| **34** | 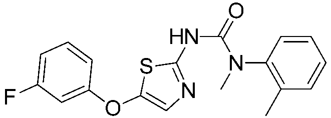 | 3-(5-(3-fluorophenoxy)thiazol-2-yl)-1-methyl-1-(o-tolyl)urea |
| **35** | 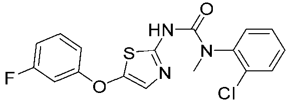 | 1-(2-chlorophenyl)-3-(5-(3-fluorophenoxy)thiazol-2-yl)-1-methylurea |
| **36** | 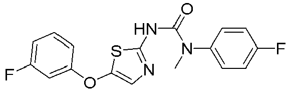 | 3-(5-(3-fluorophenoxy)thiazol-2-yl)-1-(4-fluorophenyl)-1-methylurea |
| **37** | 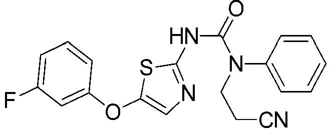 | 1-(2-cyanoethyl)-3-(5-(3-fluorophenoxy)thiazol-2-yl)-1-phenylurea |
| **38** | 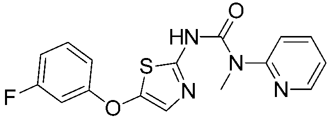 | 3-(5-(3-fluorophenoxy)thiazol-2-yl)-1-methyl-1-(pyridin-2-yl)urea |
| **39** | 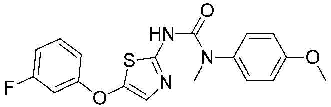 | 3-(5-(3-fluorophenoxy)thiazol-2-yl)-1-(4-methoxyphenyl)-1-methylurea |
| **40** | 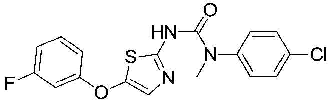 | 1-(4-chlorophenyl)-3-(5-(3-fluorophenoxy)thiazol-2-yl)-1-methylurea |
| **41** | 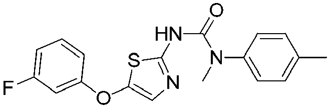 | 3-(5-(3-fluorophenoxy)thiazol-2-yl)-1-methyl-1-(p-tolyl)urea |
| **42** | 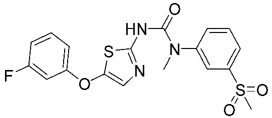 | 3-(5-(3-fluorophenoxy)thiazol-2-yl)-1-methyl-1-(3-(methylsulfonyl)phenyl)urea |
| **43** | 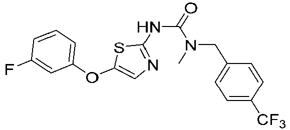 | 3-(5-(3-fluorophenoxy)thiazol-2-yl)-1-methyl-1-(4-(trifluoromethyl)benzyl)urea |
| **44** | 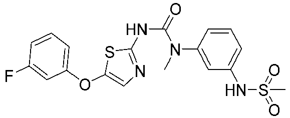 | N-(3-(3-(5-(3-fluorophenoxy)thiazol-2-yl)-1-methylureido)phenyl)methanesulfonamide |
| **45** | 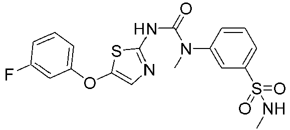 | 3-(3-(5-(3-fluorophenoxy)thiazol-2-yl)-1-methylureido)-N-methylbenzenesulfonamide |
| **46** | 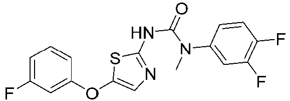 | 1-(3,4-difluorophenyl)-3-(5-(3-fluorophenoxy)thiazol-2-yl)-1-methylurea |
| **47** | 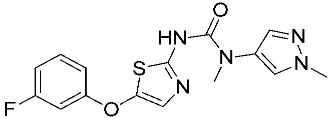 | 3-(5-(3-fluorophenoxy)thiazol-2-yl)-1-methyl-1-(1-methyl-1H-pyrazol-4-yl)urea |
| **48** | 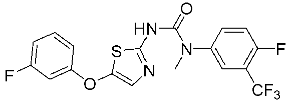 | 1-(4-fluoro-3-(trifluoromethyl)phenyl)-3-(5-(3-fluorophenoxy)thiazol-2-yl)-1-methylurea |
| **49** | 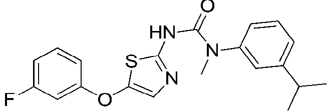 | 3-(5-(3-fluorophenoxy)thiazol-2-yl)-1-(3-isopropylphenyl)-1-methylurea |
| **50** | 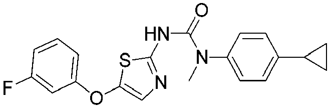 | 1-(4-cyclopropylphenyl)-3-(5-(3-fluorophenoxy)thiazol-2-yl)-1-methylurea |
| **51** | 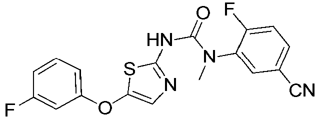 | 1-(5-cyano-2-fluorophenyl)-3-(5-(3-fluorophenoxy)thiazol-2-yl)-1-methylurea |
| **52** | 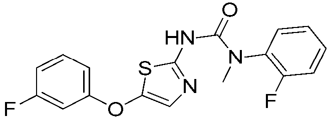 | 3-(5-(3-fluorophenoxy)thiazol-2-yl)-1-(2-fluorophenyl)-1-methylurea |
| **53** | 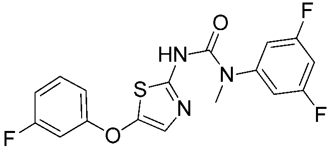 | 1-(3,5-difluorophenyl)-3-(5-(3-fluorophenoxy)thiazol-2-yl)-1-methylurea |
| **54** | 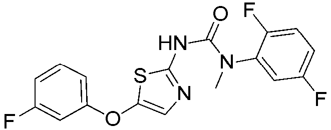 | 1-(2,5-difluorophenyl)-3-(5-(3-fluorophenoxy)thiazol-2-yl)-1-methylurea |
| **55** | 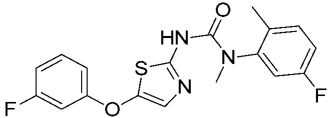 | 1-(5-fluoro-2-methylphenyl)-3-(5-(3-fluorophenoxy)thiazol-2-yl)-1-methylurea |
| **56** | 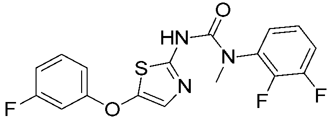 | 1-(2,3-difluorophenyl)-3-(5-(3-fluorophenoxy)thiazol-2-yl)-1-methylurea |
| **57** | 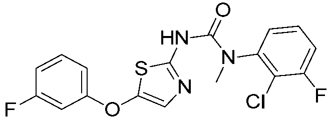 | 1-(2-chloro-3-fluorophenyl)-3-(5-(3-fluorophenoxy)thiazol-2-yl)-1-methylurea |
| **58** | 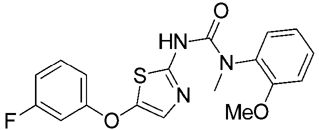 | 3-(5-(3-fluorophenoxy)thiazol-2-yl)-1-(2-methoxyphenyl)-1-methylurea |
| **59** | 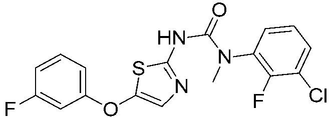 | 1-(3-chloro-2-fluorophenyl)-3-(5-(3-fluorophenoxy)thiazol-2-yl)-1-methylurea |
| **60** | 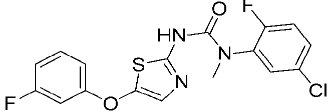 | 1-(5-chloro-2-fluorophenyl)-3-(5-(3-fluorophenoxy)thiazol-2-yl)-1-methylurea |
| **61** | 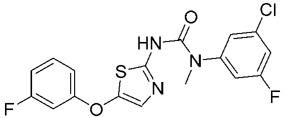 | 1-(3-chloro-5-fluorophenyl)-3-(5-(3-fluorophenoxy)thiazol-2-yl)-1-methylurea |
| **62** | 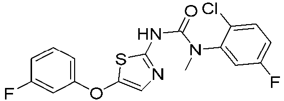 | 1-(2-chloro-5-fluorophenyl)-3-(5-(3-fluorophenoxy)thiazol-2-yl)-1-methylurea |
| **63** | 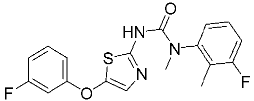 | 1-(3-fluoro-2-methylphenyl)-3-(5-(3-fluorophenoxy)thiazol-2-yl)-1-methylurea |
| **64** | 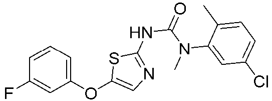 | 1-(5-chloro-2-methylphenyl)-3-(5-(3-fluorophenoxy)thiazol-2-yl)-1-methylurea |
| **65** | 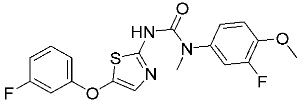 | 1-(3-fluoro-4-methoxyphenyl)-3-(5-(3-fluorophenoxy)thiazol-2-yl)-1-methylurea |
| **66** | 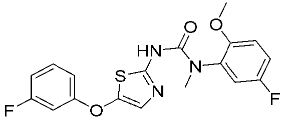 | 1-(5-fluoro-2-methoxyphenyl)-3-(5-(3-fluorophenoxy)thiazol-2-yl)-1-methylurea |
| **67** | 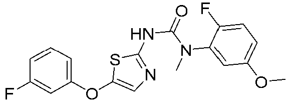 | 1-(2-fluoro-5-methoxyphenyl)-3-(5-(3-fluorophenoxy)thiazol-2-yl)-1-methylurea |
| **68** | 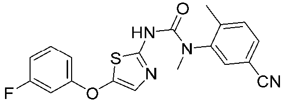 | 1-(5-cyano-2-methylphenyl)-3-(5-(3-fluorophenoxy)thiazol-2-yl)-1-methylurea |
| **69** | 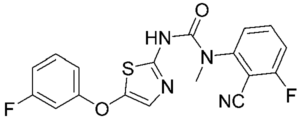 | 1-(2-cyano-3-fluorophenyl)-3-(5-(3-fluorophenoxy)thiazol-2-yl)-1-methylurea |
| **70** | 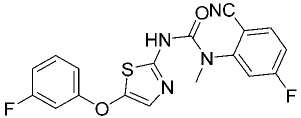 | 1-(2-cyano-5-fluorophenyl)-3-(5-(3-fluorophenoxy)thiazol-2-yl)-1-methylurea |
| **71** | 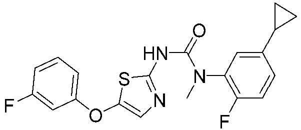 | 1-(5-cyclopropyl-2-fluorophenyl)-3-(5-(3-fluorophenoxy)thiazol-2-yl)-1-methylurea |
| **72** | 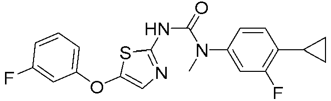 | 1-(4-cyclopropyl-3-fluorophenyl)-3-(5-(3-fluorophenoxy)thiazol-2-yl)-1-methylurea |
| **73** | 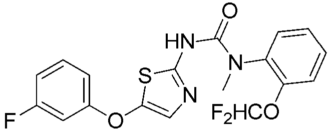 | 1-(2-(difluoromethoxy)phenyl)-3-(5-(3-fluorophenoxy)thiazol-2-yl)-1-methylurea |
| **74** | 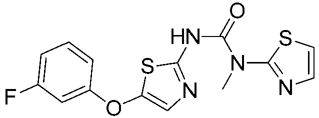 | 3-(5-(3-fluorophenoxy)thiazol-2-yl)-1-methyl-1-(thiazol-2-yl)urea |
| **75** | 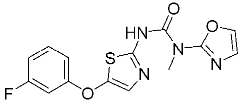 | 3-(5-(3-fluorophenoxy)thiazol-2-yl)-1-methyl-1-(oxazol-2-yl)urea |
| **76** | 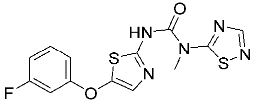 | 3-(5-(3-fluorophenoxy)thiazol-2-yl)-1-methyl-1-(1,2,4-thiadiazol-5-yl)urea |
| **77** | 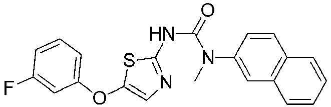 | 3-(5-(3-fluorophenoxy)thiazol-2-yl)-1-methyl-1-(naphthalen-2-yl)urea |
| **78** | 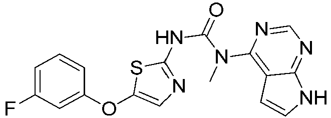 | 3-(5-(3-fluorophenoxy)thiazol-2-yl)-1-methyl-1-(7H-pyrrolo[2,3-d]pyrimidin-4-yl)urea |
| **79** | 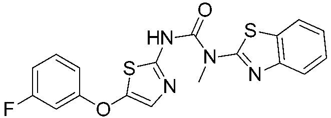 | 1-(benzo[d]thiazol-2-yl)-3-(5-(3-fluorophenoxy)thiazol-2-yl)-1-methylurea |
| **80** | 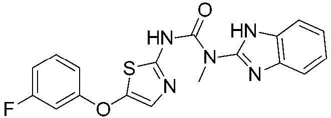 | 1-(1H-benzo[d]imidazol-2-yl)-3-(5-(3-fluorophenoxy)thiazol-2-yl)-1-methylurea |
| **81** | 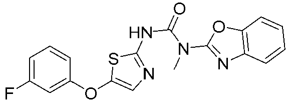 | 1-(benzo[d]oxazol-2-yl)-3-(5-(3-fluorophenoxy)thiazol-2-yl)-1-methylurea |
| **82** | 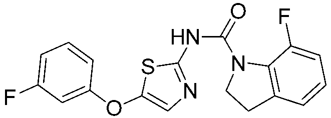 | 7-fluoro-N-(5-(3-fluorophenoxy)thiazol-2-yl)indoline-1-carboxamide |
| **83** | 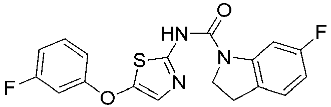 | 6-fluoro-N-(5-(3-fluorophenoxy)thiazol-2-yl)indoline-1-carboxamide |
| **84** | 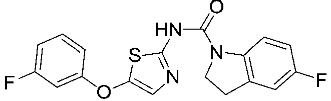 | 5-fluoro-N-(5-(3-fluorophenoxy)thiazol-2-yl)indoline-1-carboxamide |
| **85** | 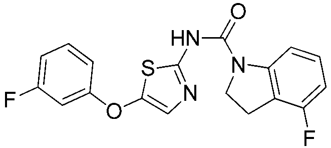 | 4-fluoro-N-(5-(3-fluorophenoxy)thiazol-2-yl)indoline-1-carboxamide |
| **86** | 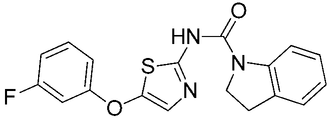 | N-(5-(3-fluorophenoxy)thiazol-2-yl)indoline-1-carboxamide |
| **87** | 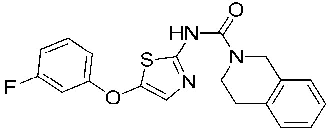 | N-(5-(3-fluorophenoxy)thiazol-2-yl)-3,4-dihydroisoquinoline-2(1H)-carboxamide |
| **88** | 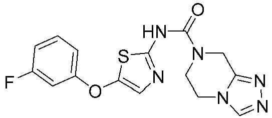 | N-(5-(3-fluorophenoxy)thiazol-2-yl)-5,6-dihydro-[1,2,4]triazolo[4,3-a]pyrazine-7(8H)-carboxamide |
| **89** | 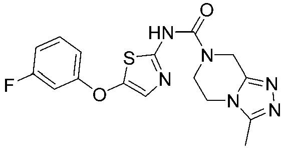 | N-(5-(3-fluorophenoxy)thiazol-2-yl)-3-methyl-5,6-dihydro-[1,2,4]triazolo[4,3-a]pyrazine-7(8H)-carboxamide |
| **90** | 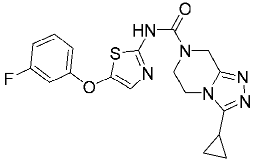 | 3-cyclopropyl-N-(5-(3-fluorophenoxy)thiazol-2-yl)-5,6-dihydro-[1,2,4]triazolo[4,3-a]pyrazine-7(8H)-carboxamide |
| **91** | 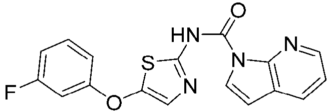 | N-(5-(3-fluorophenoxy)thiazol-2-yl)-1H-pyrrolo[2,3-b]pyridine-1-carboxamide |
| **92** | 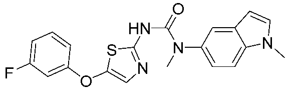 | 3-(5-(3-fluorophenoxy)thiazol-2-yl)-1-methyl-1-(1-methyl-1H-indol-5-yl)urea |
| **93** | 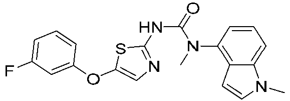 | 3-(5-(3-fluorophenoxy)thiazol-2-yl)-1-methyl-1-(1-methyl-1H-indol-4-yl)urea |
| **94** | 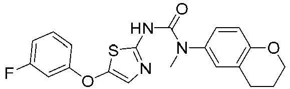 | 1-(chroman-6-yl)-3-(5-(3-fluorophenoxy)thiazol-2-yl)-1-methylurea |
| **95** | 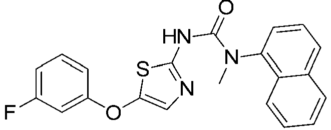 | 3-(5-(3-fluorophenoxy)thiazol-2-yl)-1-methyl-1-(naphthalen-1-yl)urea |
| **96** | 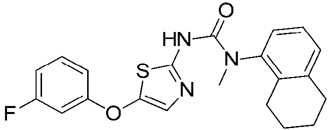 | 3-(5-(3-fluorophenoxy)thiazol-2-yl)-1-methyl-1-(5,6,7,8-tetrahydronaphthalen-1-yl)urea |
| **97** | 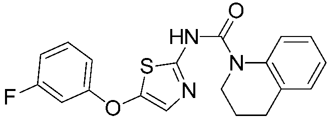 | N-(5-(3-fluorophenoxy)thiazol-2-yl)-3,4-dihydroquinoline-1 (2H)-carboxamide |
| **98** | 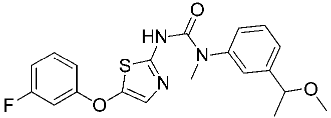 | 3-(5-(3-fluorophenoxy)thiazol-2-yl)-1-(3-(1-methoxyethyl)phenyl)-1-methylurea |
| **99** | 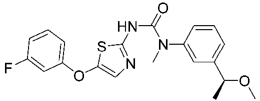 | (S)-3-(5-(3-fluorophenoxy)thiazol-2-yl)-1-(3-(1-methoxyethyl)phenyl)-1-methylurea |
| **100** | 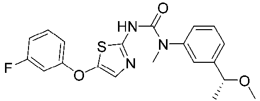 | (R)-3-(5-(3-fluorophenoxy)thiazol-2-yl)-1-(3-(1-methoxyethyl)phenyl)-1-methylurea |
| **101** | 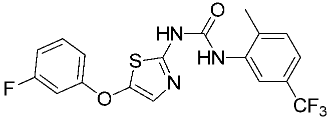 | 1-(5-(3-fluorophenoxy)thiazol-2-yl)-3-(2-methyl-5-(trifluoromethyl)phenyl)urea |
| **102** | 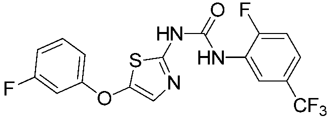 | 1-(2-fluoro-5-(trifluoromethyl)phenyl)-3-(5-(3-fluorophenoxy)thiazol-2-yl)urea |
| **103** | 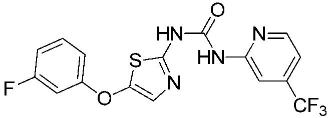 | 1-(5-(3-fluorophenoxy)thiazol-2-yl)-3-(4-(trifluoromethyl)pyridin-2-yl)urea |
| **104** | 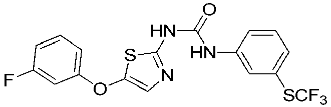 | 1-(5-(3-fluorophenoxy)thiazol-2-yl)-3-(3-((trifluoromethyl)thio)phenyl)urea |
| **105** | 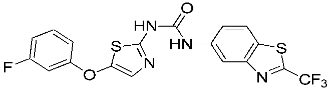 | 1-(5-(3-fluorophenoxy)thiazol-2-yl)-3-(2-(trifluoromethyl)benzo[d]thiazol-5-yl)urea |
| **106** | 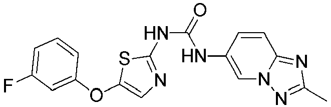 | 1-(5-(3-fluorophenoxy)thiazol-2-yl)-3-(2-methyl-[1,2,4]triazolo[1,5-a]pyridin-6-yl)urea |
| **107** | 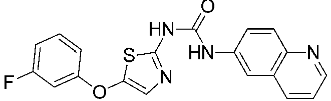 | 1-(5-(3-fluorophenoxy)thiazol-2-yl)-3-(quinolin-6-yl)urea |
| **108** | 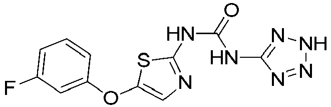 | 1-(5-(3-fluorophenoxy)thiazol-2-yl)-3-(2H-tetrazol-5-yl)urea |
| **109** | 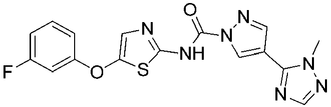 | N-(5-(3-fluorophenoxy)thiazol-2-yl)-4-(1-methyl-1H-1,2,4-triazol-5-yl)-1H-pyrazole-1-carboxamide |
| **110** | 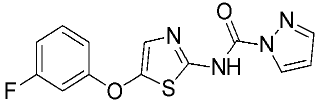 | N-(5-(3-fluorophenoxy)thiazol-2-yl)-1H-pyrazole-1-carboxamide |
| **111** | 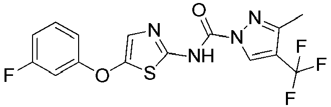 | N-(5-(3-fluorophenoxy)thiazol-2-yl)-3-methyl-4-(trifluoromethyl)-1H-pyrazole-1-carboxamide |
| **112** | 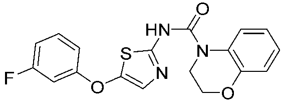 | N-(5-(3-fluorophenoxy)thiazol-2-yl)-2,3-dihydro-4H-benzo[b][1,4]oxazine-4-carboxamide |
| **113** | 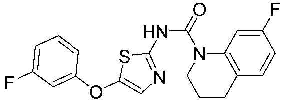 | 7-fluoro-N-(5-(3-fluorophenoxy)thiazol-2-yl)-3,4-dihydroquinoline-1 (2H)-carboxamide |
| **114** | 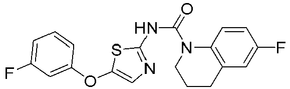 | 6-fluoro-N-(5-(3-fluorophenoxy)thiazol-2-yl)-3,4-dihydroquinoline-1 (2H)-carboxamide |
| **115** | 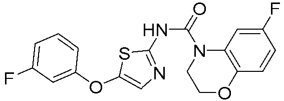 | 6-fluoro-N-(5-(3-fluorophenoxy)thiazol-2-yl)-2,3-dihydro-4H-benzo[b][1,4]oxazine-4-carboxamide |
| **116** | 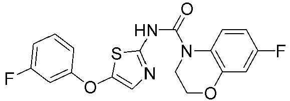 | 7-fluoro-N-(5-(3-fluorophenoxy)thiazol-2-yl)-2,3-dihydro-4H-benzo[b][1,4]oxazine-4-carboxamide |
| **117** | 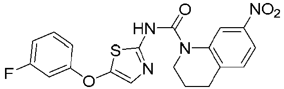 | N-(5-(3-fluorophenoxy)thiazol-2-yl)-7-nitro-3,4-dihydroquinoline-1 (2H)-carboxamide |
| **118** | 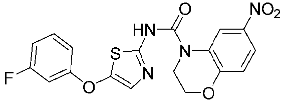 | N-(5-(3-fluorophenoxy)thiazol-2-yl)-6-nitro-2,3-dihydro-4H-benzo[b][1,4]oxazine-4-carboxamide |
| **119** | 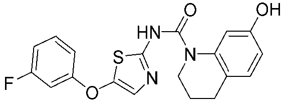 | N-(5-(3-fluorophenoxy)thiazol-2-yl)-7-hydroxy-3,4-dihydroquinoline-1 (2H)-carboxamide |
| **120** | 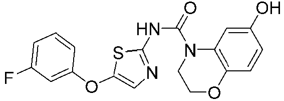 | N-(5-(3-fluorophenoxy)thiazol-2-yl)-6-hydroxy-2,3-dihydro-4H-benzo[b][1,4]oxazine-4-carboxamide |
| **121** | 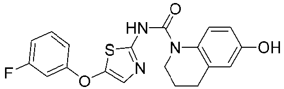 | N-(5-(3-fluorophenoxy)thiazol-2-yl)-6-hydroxy-3,4-dihydroquinoline-1(2H)-carboxamide |
| **122** | 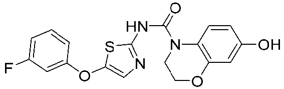 | N-(5-(3-fluorophenoxy)thiazol-2-yl)-7-hydroxy-2,3-dihydro-4H-benzo[b][1,4]oxazine-4-carboxamide |
| **123** | 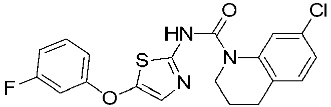 | 7-chloro-N-(5-(3-fluorophenoxy)thiazol-2-yl)-3,4-dihydroquinoline-1(2H)-carboxamide |
| **124** | 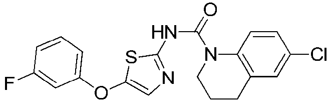 | 6-chloro-N-(5-(3-fluorophenoxy)thiazol-2-yl)-3,4-dihydroquinoline-1(2H)-carboxamide |
| **125** | 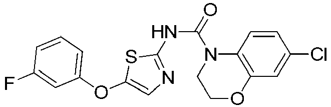 | 7-chloro-N-(5-(3-fluorophenoxy)thiazol-2-yl)-2,3-dihydro-4H-benzo[b][1,4]oxazine-4-carboxamide |
| **126** | 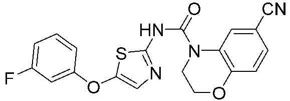 | 6-cyano-N-(5-(3-fluorophenoxy)thiazol-2-yl)-2,3-dihydro-4H-benzo[b][1,4]oxazine-4-carboxamide |
| **127** | 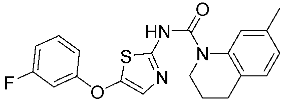 | N-(5-(3-fluorophenoxy)thiazol-2-yl)-7-methyl-3,4-dihydroquinoline-1(2H)-carboxamide |
| **128** | 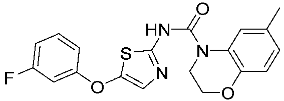 | N-(5-(3-fluorophenoxy)thiazol-2-yl)-6-methyl-2,3-dihydro-4H-benzo[b][1,4]oxazine-4-carboxamide |
| **129** | 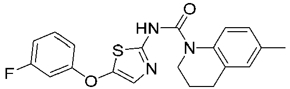 | N-(5-(3-fluorophenoxy)thiazol-2-yl)-6-methyl-3,4-dihydroquinoline-1(2H)-carboxamide |
| **130** | 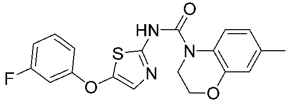 | N-(5-(3-fluorophenoxy)thiazol-2-yl)-7-methyl-2,3-dihydro-4H-benzo[b][1,4]oxazine-4-carboxamide |
| **131** | 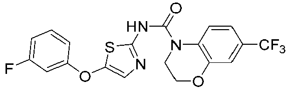 | N-(5-(3-fluorophenoxy)thiazol-2-yl)-7-(trifluoromethyl)-2,3-dihydro-4H-benzo[b][1,4]oxazine-4-carboxamide |
| **132** | 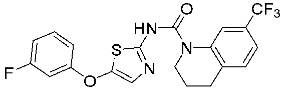 | N-(5-(3-fluorophenoxy)thiazol-2-yl)-7-(trifluoromethyl)-3,4-dihydroquinoline-1(2H)-carboxamide |
| **133** | 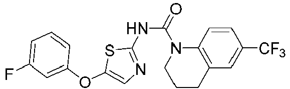 | N-(5-(3-fluorophenoxy)thiazol-2-yl)-6-(trifluoromethyl)-3,4-dihydroquinoline-1(2H)-carboxamide |
| **134** | 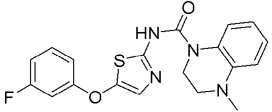 | N-(5-(3-fluorophenoxy)thiazol-2-yl)-4-methyl-3,4-dihydroquinoxaline-1(2H)-carboxamide |
| **135** | 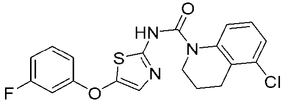 | 5-chloro-N-(5-(3-fluorophenoxy)thiazol-2-yl)-3,4-dihydroquinoline-1(2H)-carboxamide |
| **136** | 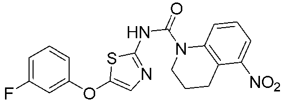 | N-(5-(3-fluorophenoxy)thiazol-2-yl)-5-nitro-3,4-dihydroquinoline-1(2H)-carboxamide |
| **137** | 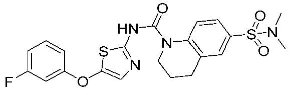 | 6-(N,N-dimethylsulfamoyl)-N-(5-(3-fluorophenoxy)thiazol-2-yl)-3,4-dihydroquinoline-1(2H)-carboxamide |
| **138** | 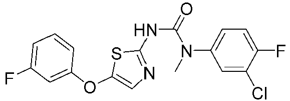 | 1-(3-chloro-4-fluorophenyl)-3-(5-(3-fluorophenoxy)thiazol-2-yl)-1-methylurea |
| **139** | 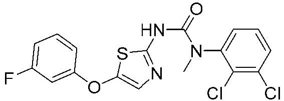 | 1-(2,3-dichlorophenyl)-3-(5-(3-fluorophenoxy)thiazol-2-yl)-1-methylurea |
| **140** | 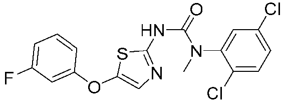 | 1-(2,5-dichlorophenyl)-3-(5-(3-fluorophenoxy)thiazol-2-yl)-1-methylurea |
| **141** | 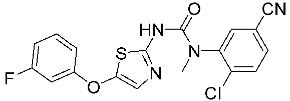 | 1-(2-chloro-5-cyanophenyl)-3-(5-(3-fluorophenoxy)thiazol-2-yl)-1-methylurea |
| **142** | 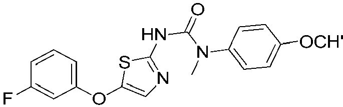 | 1-(4-(difluoromethoxy)phenyl)-3-(5-(3-fluorophenoxy)thiazol-2-yl)-1-methylurea |
| **143** | 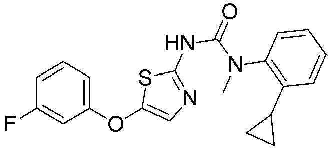 | 1-(2-cyclopropylphenyl)-3-(5-(3-fluorophenoxy)thiazol-2-yl)-1-methylurea |
| **144** | 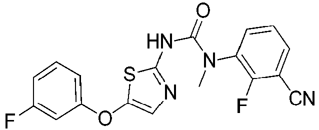 | 1-(3-cyano-2-fluorophenyl)-3-(5-(3-fluorophenoxy)thiazol-2-yl)-1-methylurea |
| **145** | 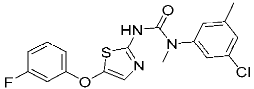 | 1-(3-chloro-5-methylphenyl)-3-(5-(3-fluorophenoxy)thiazol-2-yl)-1-methylurea |
| **146** | 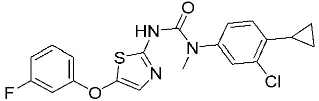 | 1-(3-chloro-4-cyclopropylphenyl)-3-(5-(3-fluorophenoxy)thiazol-2-yl)-1-methylurea |
| **147** | 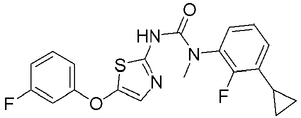 | 1-(3-cyclopropyl-2-fluorophenyl)-3-(5-(3-fluorophenoxy)thiazol-2-yl)-1-methylurea |
| **148** | 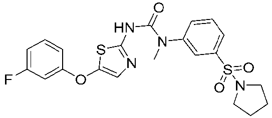 | 3-(5-(3-fluorophenoxy)thiazol-2-yl)-1-methyl-1-(3-(pyrrolidin-1-ylsulfonyl)phenyl)urea |
| **149** | 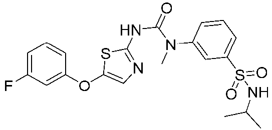 | 3-(3-(5-(3-fluorophenoxy)thiazol-2-yl)-1-methylureido)-N-isopropylbenzenesulfonamide |
| **150** | 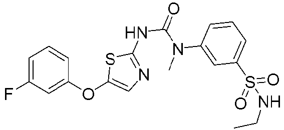 | N-ethyl-3-(3-(5-(3-fluorophenoxy)thiazol-2-yl)-1-methylureido)benzenesulfonamide |
| **151** | 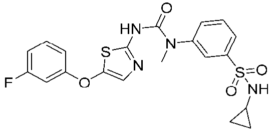 | N-cyclopropyl-3-(3-(5-(3-fluorophenoxy)thiazol-2-yl)-1-methylureido)benzenesulfonamide |
| **152** | 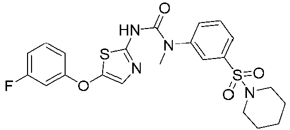 | 3-(5-(3-fluorophenoxy)thiazol-2-yl)-1-methyl-1-(3-(piperidin-1-ylsulfonyl)phenyl)urea |
| **153** | 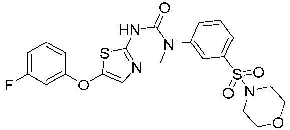 | 3-(5-(3-fluorophenoxy)thiazol-2-yl)-1-methyl-1-(3-(morpholinosulfonyl)phenyl)urea |
| **154** | 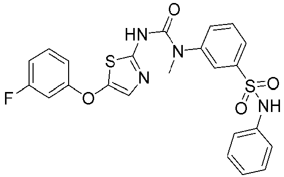 | 3-(3-(5-(3-fluorophenoxy)thiazol-2-yl)-1-methylureido)-N-phenylbenzenesulfonamide |
| **155** | 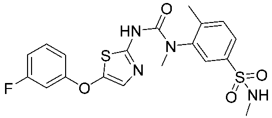 | 3-(3-(5-(3-fluorophenoxy)thiazol-2-yl)-1-methylureido)-N,4-dimethylbenzenesulfonamide |
| **156** | 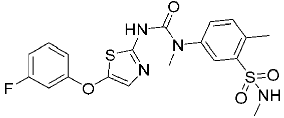 | 5-(3-(5-(3-fluorophenoxy)thiazol-2-yl)-1-methylureido)-N,2-dimethylbenzenesulfonamide |
| **157** | 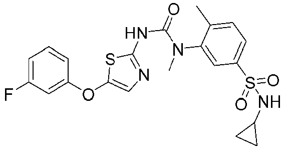 | N-cyclopropyl-3-(3-(5-(3-fluorophenoxy)thiazol-2-yl)-1-methylureido)-4-methylbenzenesulfonamide |
| **158** | 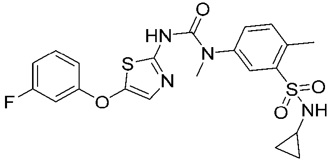 | N-cyclopropyl-5-(3-(5-(3-fluorophenoxy)thiazol-2-yl)-1-methylureido)-2-methylbenzenesulfonamide |
| **159** | 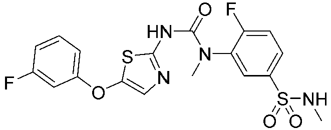 | 4-fluoro-3-(3-(5-(3-fluorophenoxy)thiazol-2-yl)-1-methylureido)-N-methylbenzenesulfonamide |
| **160** | 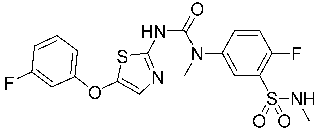 | 2-fluoro-5-(3-(5-(3-fluorophenoxy)thiazol-2-yl)-1-methylureido)-N-methylbenzenesulfonamide |
| **161** | 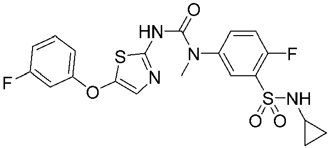 | N-cyclopropyl-2-fluoro-5-(3-(5-(3-fluorophenoxy)thiazol-2-yl)-1-methylureido)benzenesulfonamide |
| **162** | 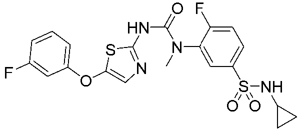 | N-cyclopropyl-4-fluoro-3-(3-(5-(3-fluorophenoxy)thiazol-2-yl)-1-methylureido)benzenesulfonamide |
| **163** | 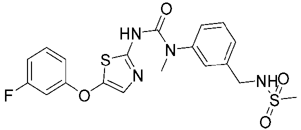 | N-(3-(3-(5-(3-fluorophenoxy)thiazol-2-yl)-1-methylureido)benzyl)methanesulfonamide |
| **164** | 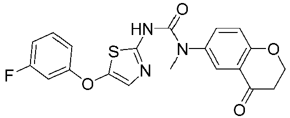 | 3-(5-(3-fluorophenoxy)thiazol-2-yl)-1-methyl-1-(4-oxochroman-6-yl)urea |
| **165** | 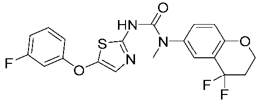 | 1-(4,4-difluorochroman-6-yl)-3-(5-(3-fluorophenoxy)thiazol-2-yl)-1-methylurea |
| **166** | 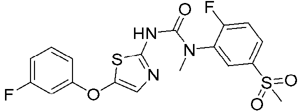 | 1-(2-fluoro-5-(methylsulfonyl)phenyl)-3-(5-(3-fluorophenoxy)thiazol-2-yl)-1-methylurea |
| **167** | 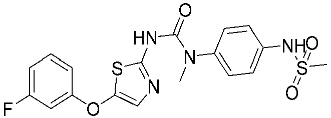 | N-(4-(3-(5-(3-fluorophenoxy)thiazol-2-yl)-1-methylureido)phenyl)methanesulfonamide |
| **168** | 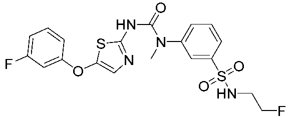 | N-(2-fluoroethyl)-3-(3-(5-(3-fluorophenoxy)thiazol-2-yl)-1-methylureido)benzenesulfonamide |
| **169** | 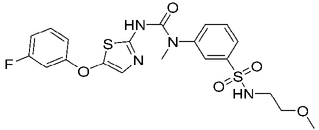 | 3-(3-(5-(3-fluorophenoxy)thiazol-2-yl)-1-methylureido)-N-(2-methoxyethyl)benzenesulfonamide |
| **170** | 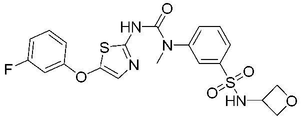 | 3-(3-(5-(3-fluorophenoxy)thiazol-2-yl)-1-methylureido)-N-(oxetan-3-yl)benzenesulfonamide |
| **171** | 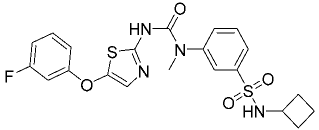 | N-cyclobutyl-3-(3-(5-(3-fluorophenoxy)thiazol-2-yl)-1-methylureido)benzenesulfonamide |
| **172** | 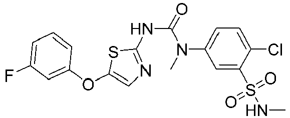 | 2-chloro-5-(3-(5-(3-fluorophenoxy)thiazol-2-yl)-1-methylureido)-N-methylbenzenesulfonamide |
| **173** | 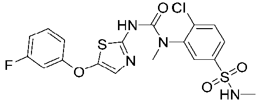 | 4-chloro-3-(3-(5-(3-fluorophenoxy)thiazol-2-yl)-1-methylureido)-N-methylbenzenesulfonamide |
| **174** | 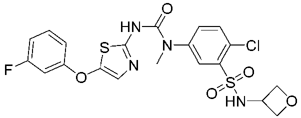 | 2-chloro-5-(3-(5-(3-fluorophenoxy)thiazol-2-yl)-1-methylureido)-N-(oxetan-3-yl)benzenesulfonamide |
| **175** | 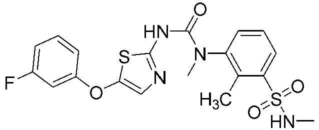 | 3-(3-(5-(3-fluorophenoxy)thiazol-2-yl)-1-methylureido)-N,2-dimethylbenzenesulfonamide |
| **176** | 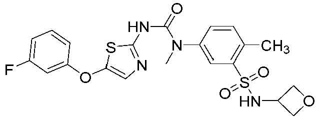 | 5-(3-(5-(3-fluorophenoxy)thiazol-2-yl)-1-methylureido)-2-methyl-N-(oxetan-3-yl)benzenesulfonamide |
| **177** | 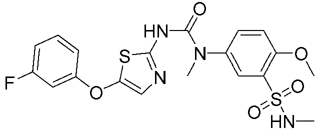 | 5-(3-(5-(3-fluorophenoxy)thiazol-2-yl)-1-methylureido)-2-methoxy-N-methylbenzenesulfonamide |
| **178** | 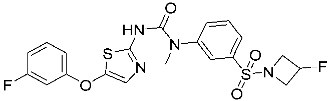 | 1-(3-((3-fluoroazetidin-1-yl)sulfonyl)phenyl)-3-(5-(3-fluorophenoxy)thiazol-2-yl)-1-methylurea |
| **179** | 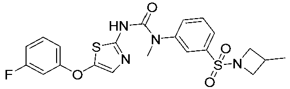 | 3-(5-(3-fluorophenoxy)thiazol-2-yl)-1-methyl-1-(3-((3-methylazetidin-1-yl)sulfonyl)phenyl)urea |
| **180** | 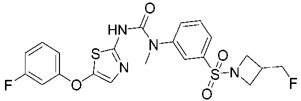 | 1-(3-((3-(fluoromethyl)azetidin-1-yl)sulfonyl)phenyl)-3-(5-(3-fluorophenoxy)thiazol-2-yl)-1-methylurea |
| **181** | 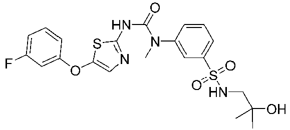 | 3-(3-(5-(3-fluorophenoxy)thiazol-2-yl)-1-methylureido)-N-(2-hydroxy-2-methylpropyl)benzenesulfonamide |
| **182** | 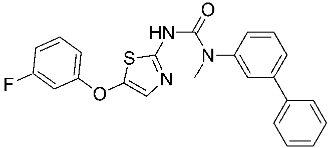 | 1-([1,1'-biphenyl]-3-yl)-3-(5-(3-fluorophenoxy)thiazol-2-yl)-1-methylurea |
| **183** | 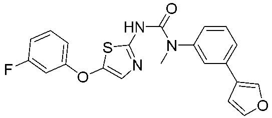 | 3-(5-(3-fluorophenoxy)thiazol-2-yl)-1-(3-(furan-3-yl)phenyl)-1-methylurea |
| **184** | 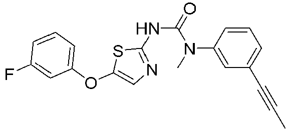 | 3-(5-(3-fluorophenoxy)thiazol-2-yl)-1-methyl-1-(3-(prop-1-yn-1-yl)phenyl)urea |
| **185** | 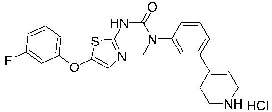 | 3-(5-(3-fluorophenoxy)thiazol-2-yl)-1-methyl-1-(3-(1,2,3,6-tetrahydropyridin-4-yl)phenyl)urea hydrochloride |
| **186** | 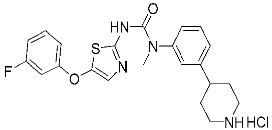 | 3-(5-(3-fluorophenoxy)thiazol-2-yl)-1-methyl-1-(3-(piperidin-4-yl)phenyl)urea hydrochloride |
| **187** | 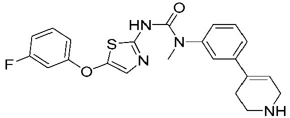 | 3-(5-(3-fluorophenoxy)thiazol-2-yl)-1-methyl-1-(3-(1,2,3,6-tetrahydropyridin-4-yl)phenyl)urea |
| **188** | 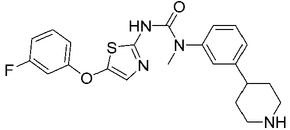 | 3-(5-(3-fluorophenoxy)thiazol-2-yl)-1-methyl-1-(3-(piperidin-4-yl)phenyl)urea |
| **189** | 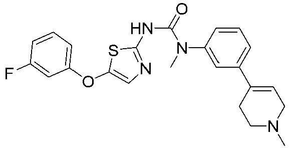 | 3-(5-(3-fluorophenoxy)thiazol-2-yl)-1-methyl-1-(3-(1-methyl-1,2,3,6-tetrahydropyridin-4-yl)phenyl)urea |
| **190** | 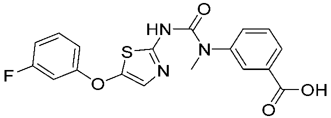 | 3-(3-(5-(3-fluorophenoxy)thiazol-2-yl)-1-methylureido)benzoic acid |
| **191** | 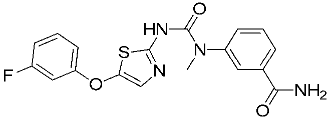 | 3-(3-(5-(3-fluorophenoxy)thiazol-2-yl)-1-methylureido)benzamide |
| **192** | 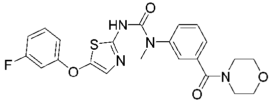 | 3-(5-(3-fluorophenoxy)thiazol-2-yl)-1-methyl-1-(3-(morpholine-4-carbonyl)phenyl)urea |
| **193** | 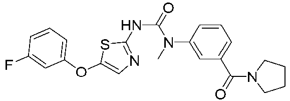 | 3-(5-(3-fluorophenoxy)thiazol-2-yl)-1-methyl-1-(3-(pyrrolidine-1-carbonyl)phenyl)urea |
| **194** | 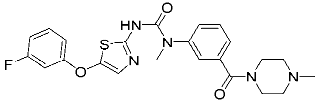 | 3-(5-(3-fluorophenoxy)thiazol-2-yl)-1-methyl-1-(3-(4-methylpiperazine-1-carbonyl)phenyl)urea |
| **195** | 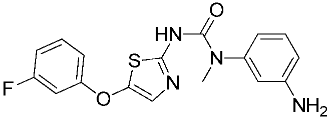 | 1-(3-aminophenyl)-3-(5-(3-fluorophenoxy)thiazol-2-yl)-1-methylurea |
| **196** | 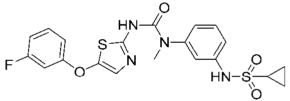 | N-(3-(3-(5-(3-fluorophenoxy)thiazol-2-yl)-1-methylureido)phenyl)cyclopropanesulfonamide |
| **197** | 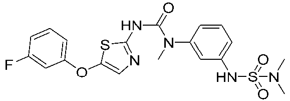 | N-(3-(3-(5-(3-fluorophenoxy)thiazol-2-yl)-1-methylureido)phenyl) N,N-dimethylsulfamide |
| **198** | 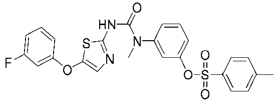 | 3-(3-(5-(3-fluorophenoxy)thiazol-2-yl)-1-methylureido)phenyl 4-methylbenzenesulfonate |
| **199** | 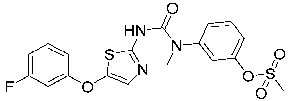 | 3-(3-(5-(3-fluorophenoxy)thiazol-2-yl)-1-methylureido)phenyl methanesulfonate |
| **200** | 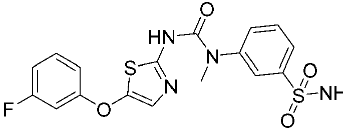 | 3-(3-(5-(3-fluorophenoxy)thiazol-2-yl)-1-methylureido)benzenesulfonamide |
| **201** | 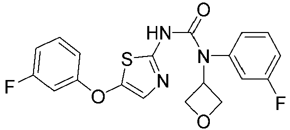 | 3-(5-(3-fluorophenoxy)thiazol-2-yl)-1-(3-fluorophenyl)-1-(oxetan-3-yl)urea |
| **202** | 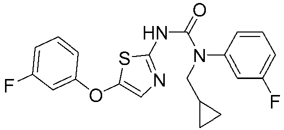 | 1-(cyclopropylmethyl)-3-(5-(3-fluorophenoxy)thiazol-2-yl)-1-(3-fluorophenyl)urea |
| **203** | 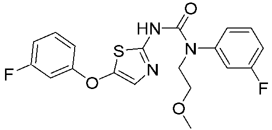 | 3-(5-(3-fluorophenoxy)thiazol-2-yl)-1-(3-fluorophenyl)-1-(2-methoxyethyl)urea |
| **204** | 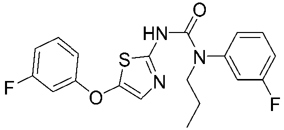 | 3-(5-(3-fluorophenoxy)thiazol-2-yl)-1-(3-fluorophenyl)-1-propylurea |
| **205** | 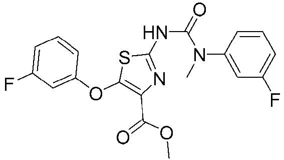 | Methyl 5-(3-fluorophenoxy)-2-(3-(3-fluorophenyl)-3-methylureido)thiazole-4-carboxylate |
| **206** | 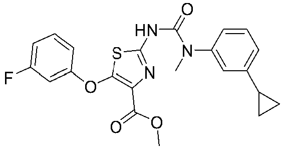 | Methyl 2-(3-(3-cyclopropylphenyl)-3-methylureido)-5-(3-fluorophenoxy)thiazole-4-carboxylate |
| **207** | 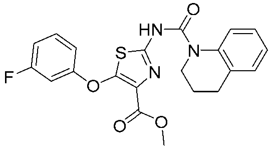 | Methyl 5-(3-fluorophenoxy)-2-(1,2,3,4-tetrahydroquinoline-1-carboxamido)thiazole-4-carboxylate |
| **208** | 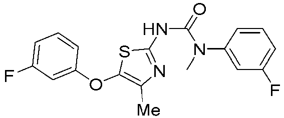 | 3-(5-(3-fluorophenoxy)-4-methylthiazol-2-yl)-1-(3-fluorophenyl)-1-methylurea |
| **209** | 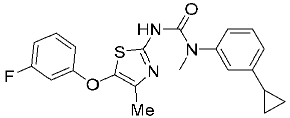 | 1-(3-cyclopropylphenyl)-3-(5-(3-fluorophenoxy)-4-methylthiazol-2-yl)-1-methylurea |
| **210** | 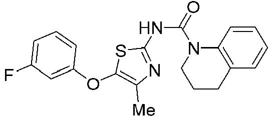 | N-(5-(3-fluorophenoxy)-4-methylthiazol-2-yl)-3,4-dihydroquinoline-1 (2H)-carboxamide |
| **211** | 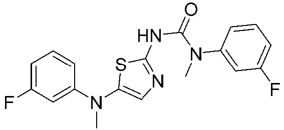 | 1-(3-fluorophenyl)-3-(5-((3-fluorophenyl)(methyl)amino)thiazol-2-yl)-1-methylurea |
| **212** | 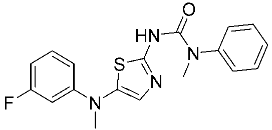 | 3-(5-((3-fluorophenyl)(methyl)amino)thiazol-2-yl)-1-methyl-1-phenylurea |
| **213** | 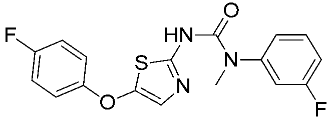 | 3-(5-(4-fluorophenoxy)thiazol-2-yl)-1-(3-fluorophenyl)-1-methylurea |
| **214** | 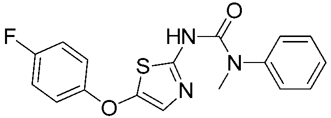 | 3-(5-(4-fluorophenoxy)thiazol-2-yl)-1-methyl-1-phenylurea |
| **215** | 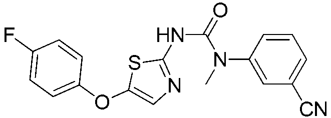 | 1-(3-cyanophenyl)-3-(5-(4-fluorophenoxy)thiazol-2-yl)-1-methylurea |
| **216** | 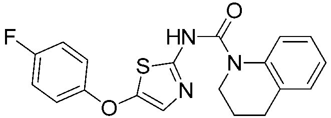 | N-(5-(4-fluorophenoxy)thiazol-2-yl)-3,4-dihydroquinoline-1 (2H)-carboxamide |
| **217** | 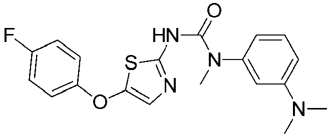 | 1-(3-(dimethylamino)phenyl)-3-(5-(4-fluorophenoxy)thiazol-2-yl)-1-methylurea |
| **218** | 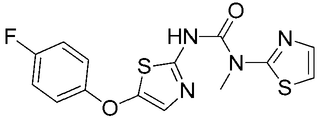 | 3-(5-(4-fluorophenoxy)thiazol-2-yl)-1-methyl-1-(thiazol-2-yl)urea |
| **219** | 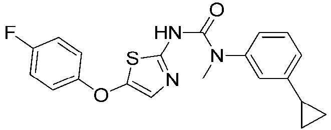 | 1-(3-cyclopropylphenyl)-3-(5-(4-fluorophenoxy)thiazol-2-yl)-1-methylurea |
| **220** | 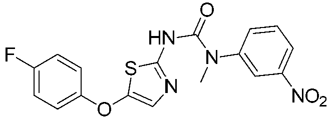 | 3-(5-(4-fluorophenoxy)thiazol-2-yl)-1-methyl-1-(3-nitrophenyl)urea |
| **221** | 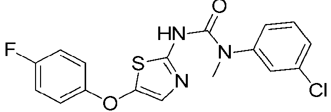 | 1-(3-chlorophenyl)-3-(5-(4-fluorophenoxy)thiazol-2-yl)-1-methylurea |
| **222** | 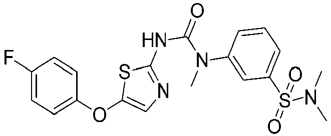 | 3-(3-(5-(4-fluorophenoxy)thiazol-2-yl)-1-methylureido)-N,N-dimethylbenzenesulfonamide |
| **223** | 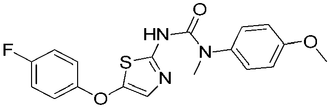 | 3-(5-(4-fluorophenoxy)thiazol-2-yl)-1-(4-methoxyphenyl)-1-methylurea |
| **224** | 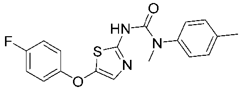 | 3 -(5-(4-fluorophenoxy)thiazol-2-yl)-1-methyl-1-(p-tolyl)urea |
| **225** | 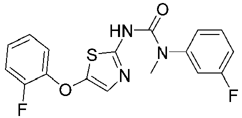 | 3-(5-(2-fluorophenoxy)thiazol-2-yl)-1-(3-fluorophenyl)-1-methylurea |
| **226** | 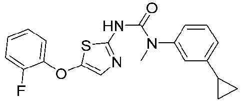 | 1-(3-cyclopropylphenyl)-3-(5-(2-fluorophenoxy)thiazol-2-yl)-1-methylurea |
| **227** | 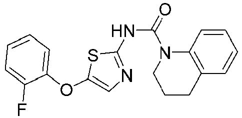 | N-(5-(2-fluorophenoxy)thiazol-2-yl)-3,4-dihydroquinoline-1(2H)-carboxamide |
| **228** | 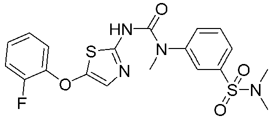 | 3-(3-(5-(2-fluorophenoxy)thiazol-2-yl)-1-methylureido)-N,N-dimethylbenzenesulfonamide |
| **229** | 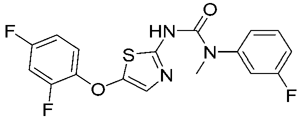 | 3-(5-(2,4-difluorophenoxy)thiazol-2-yl)-1-(3-fluorophenyl)-1-methylurea |
| **230** | 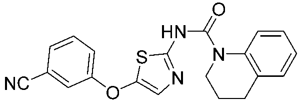 | N-(5-(3-cyanophenoxy)thiazol-2-yl)-3,4-dihydroquinoline-1(2H)-carboxamide |
| **231** | 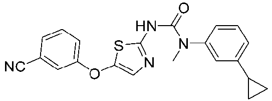 | 3-(5-(3-cyanophenoxy)thiazol-2-yl)-1-(3-cyclopropylphenyl)-1-methylurea |
| **232** | 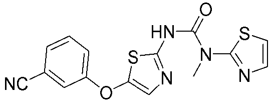 | 3-(5-(3-cyanophenoxy)thiazol-2-yl)-1-methyl-1-(thiazol-2-yl)urea |
| **233** | 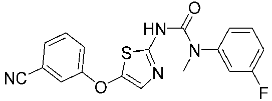 | 3-(5-(3-cyanophenoxy)thiazol-2-yl)-1-(3-fluorophenyl)-1-methylurea |
| **234** | 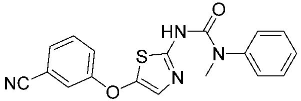 | 3-(5-(3-cyanophenoxy)thiazol-2-yl)-1-methyl-1-phenylurea |
| **235** | 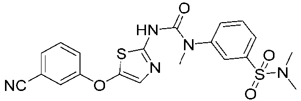 | 3-(3-(5-(3-cyanophenoxy)thiazol-2-yl)-1-methylureido)-N,N-dimethylbenzenesulfonamide |
| **236** | 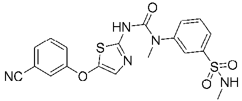 | 3-(3-(5-(3-cyanophenoxy)thiazol-2-yl)-1-methylureido)-N-methylbenzenesulfonamide |
| **237** | 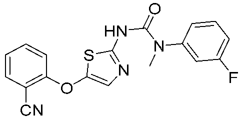 | 3-(5-(2-cyanophenoxy)thiazol-2-yl)-1-(3-fluorophenyl)-1-methylurea |
| **238** | 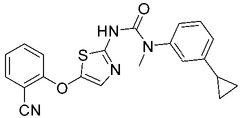 | 3-(5-(2-cyanophenoxy)thiazol-2-yl)-1-(3-cyclopropylphenyl)-1-methylurea |
| **239** | 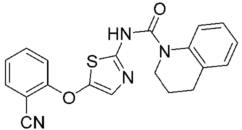 | N-(5-(2-cyanophenoxy)thiazol-2-yl)-3,4-dihydroquinoline-1(2H)-carboxamide |
| **240** | 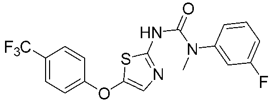 | 1-(3-fluorophenyl)-1-methyl-3-(5-(4-(trifluoromethyl)phenoxy)thiazol-2-yl)urea |
| **241** | 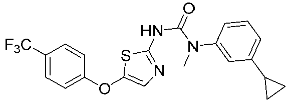 | 1-(3-cyclopropylphenyl)-1-methyl-3-(5-(4-(trifluoromethyl)phenoxy)thiazol-2-yl)urea |
| **242** | 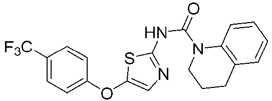 | N-(5-(3-fluorophenoxy)thiazol-2-yl)-1H-pyrrolo[2,3-b]pyridine-1-carboxamide |
| **243** | 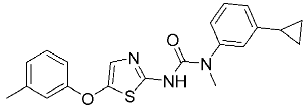 | 1-(3-cyclopropylphenyl)-1-methyl-3-(5-(m-tolyloxy)thiazol-2-yl)urea |
| **244** | 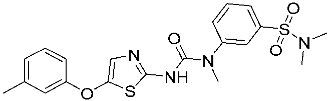 | N,N-dimethyl-3-(1-methyl-3-(5-(m-tolyloxy)thiazol-2-yl)ureido)benzenesulfonamide |
| **245** | 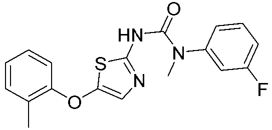 | 1-(3-fluorophenyl)-1-methyl-3-(5-(o-tolyloxy)thiazol-2-yl)urea |
| **246** | 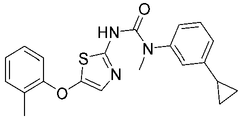 | 1-(3-cyclopropylphenyl)-1-methyl-3-(5-(o-tolyloxy)thiazol-2-yl)urea |
| **247** | 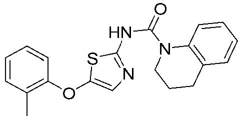 | N-(5-(o-tolyloxy)thiazol-2-yl)-3,4-dihydroquinoline-1(2H)-carboxamide |
| **248** | 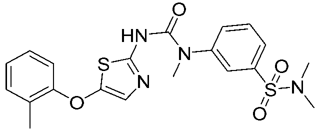 | N,N-dimethyl-3-(1-methyl-3-(5-(o-tolyloxy)thiazol-2-yl)ureido)benzenesulfonamide |
| **249** | 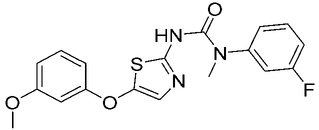 | 1-(3-fluorophenyl)-3-(5-(3-methoxyphenoxy)thiazol-2-yl)-1-methylurea |
| **250** | 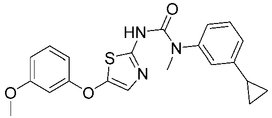 | 1-(3-cyclopropylphenyl)-3-(5-(3-methoxyphenoxy)thiazol-2-yl)-1-methylurea |
| **251** | 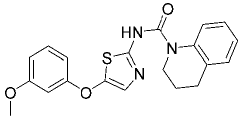 | N-(5-(3-methoxyphenoxy)thiazol-2-yl)-3,4-dihydroquinoline-1(2H)-carboxamide |
| **252** | 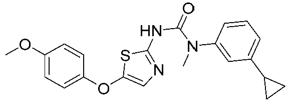 | 1-(3-cyclopropylphenyl)-3-(5-(4-methoxyphenoxy)thiazol-2-yl)-1-methylurea |
| **253** | 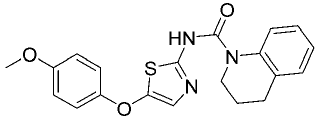 | N-(5-(4-methoxyphenoxy)thiazol-2-yl)-3,4-dihydroquinoline-1(2H)-carboxamide |
| **254** | 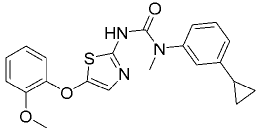 | 1-(3-cyclopropylphenyl)-3-(5-(2-methoxyphenoxy)thiazol-2-yl)-1-methylurea |
| **255** | 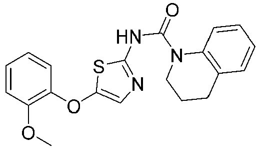 | N-(5-(2-methoxyphenoxy)thiazol-2-yl)-3,4-dihydroquinoline-1(2H)-carboxamide |
| **256** | 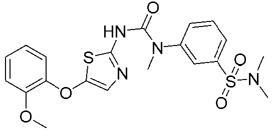 | 3-(3-(5-(2-methoxyphenoxy)thiazol-2-yl)-1-methylureido)-N,N-dimethylbenzenesulfonamide |
| **257** | 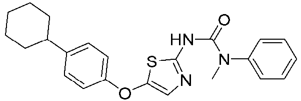 | 3-(5-(4-cyclohexylphenoxy)thiazol-2-yl)-1-methyl-1-phenylurea |
| **258** | 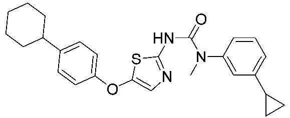 | 3-(5-(4-cyclohexylphenoxy)thiazol-2-yl)-1-(3-cyclopropylphenyl)-1-methylurea |
| **259** | 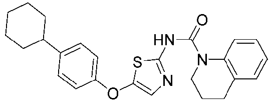 | N-(5-(4-cyclohexylphenoxy)thiazol-2-yl)-3,4-dihydroquinoline-1(2H)-carboxamide |
| **260** | 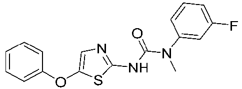 | 1-(3-fluorophenyl)-1-methyl-3-(5-phenoxythiazol-2-yl)urea |
| **261** | 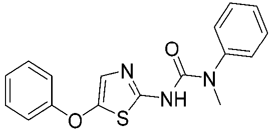 | 1-methyl-3-(5-phenoxythiazol-2-yl)-1-phenylurea |
| **262** | 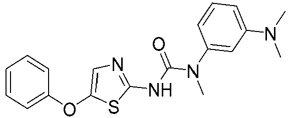 | 1-(3-(dimethylamino)phenyl)-1-methyl-3-(5-phenoxythiazol-2-yl)urea |
| **263** | 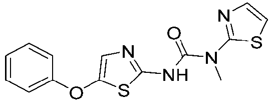 | 1-methyl-3-(5-phenoxythiazol-2-yl)-1-(thiazol-2-yl)urea |
| **264** | 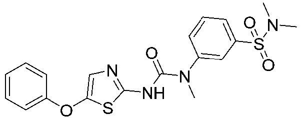 | N,N-dimethyl-3-(1-methyl-3-(5-phenoxythiazol-2-yl)ureido)benzenesulfonamide |
| **265** | 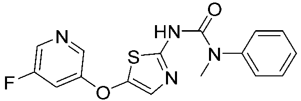 | 3-(5-((5-fluoropyridin-3-yl)oxy)thiazol-2-yl)-1-methyl-1-phenylurea |
| **266** | 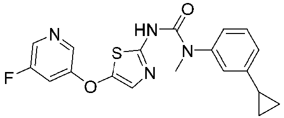 | 1-(3-cyclopropylphenyl)-3-(5-((5-fluoropyridin-3-yl)oxy)thiazol-2-yl)-1-methylurea |
| **267** | 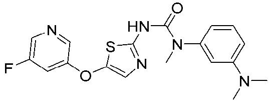 | 1-(3-(dimethylamino)phenyl)-3-(5-((5-fluoropyridin-3-yl)oxy)thiazol-2-yl)-1-methylurea |
| **268** | 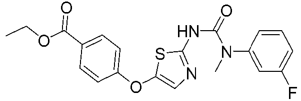 | Ethyl 4-((2-(3-(3-fluorophenyl)-3-methylureido)thiazol-5-yl)oxy)benzoate |
| **269** | 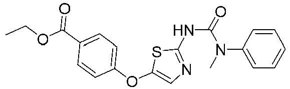 | Ethyl 4-((2-(3-methyl-3-phenylureido)thiazol-5-yl)oxy)benzoate |
| **270** | 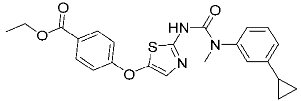 | Ethyl 4-((2-(3-(3-cyclopropylphenyl)-3-methylureido)thiazol-5-yl)oxy)benzoate |
| **271** | 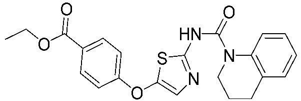 | Ethyl 4-((2-(1,2,3,4-tetrahydroquinoline-1-carboxamido)thiazol-5-yl)oxy)benzoate |
| **272** | 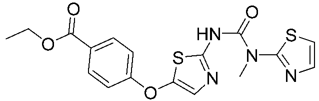 | Ethyl 4-((2-(3-methyl-3-(thiazol-2-yl)ureido)thiazol-5-yl)oxy)benzoate |
| **273** | 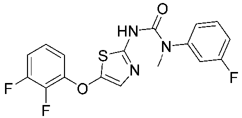 | 3-(5-(2,3-difluorophenoxy)thiazol-2-yl)-1-(3-fluorophenyl)-1-methylurea |
| **274** | 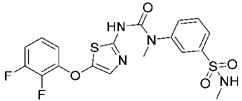 | 3-(3-(5-(2,3-difluorophenoxy)thiazol-2-yl)-1-methylureido)-N-methylbenzenesulfonamide |
| **275** | 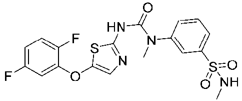 | 3-(3-(5-(2,5-difluorophenoxy)thiazol-2-yl)-1-methylureido)-N-methylbenzenesulfonamide |
| **276** | 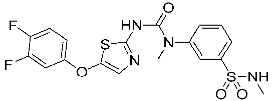 | 3-(3-(5-(3,4-difluorophenoxy)thiazol-2-yl)-1-methylureido)-N-methylbenzenesulfonamide |
| **277** | 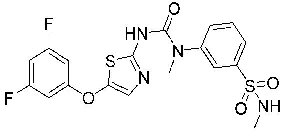 | 3-(3-(5-(3,5-difluorophenoxy)thiazol-2-yl)-1-methylureido)-N-methylbenzenesulfonamide |
| **278** | 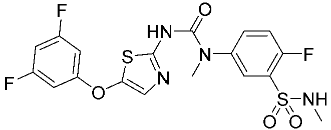 | 5-(3-(5-(3,5-difluorophenoxy)thiazol-2-yl)-1-methylureido)-2-fluoro-N-methylbenzenesulfonamide |
| **279** | 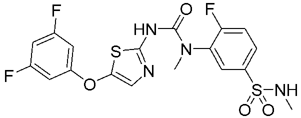 | 3-(3-(5-(3,5-difluorophenoxy)thiazol-2-yl)-1-methylureido)-4-fluoro-N-methylbenzenesulfonamide |
| **280** | 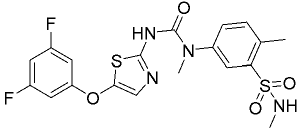 | 5-(3-(5-(3,5-difluorophenoxy)thiazol-2-yl)-1-methylureido)-N,2-dimethylbenzenesulfonamide |
| **281** | 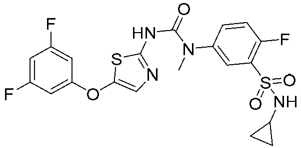 | N-cyclopropyl-5-(3-(5-(3,5-difluorophenoxy)thiazol-2-yl)-1-methylureido)-2-fluorobenzenesulfonamide |
| **282** | 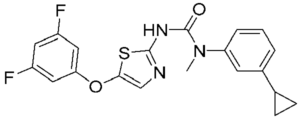 | 1-(3-cyclopropylphenyl)-3-(5-(3,5-difluorophenoxy)thiazol-2-yl)-1-methylurea |
| **283** | 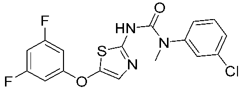 | 1-(3-chlorophenyl)-3-(5-(3,5-difluorophenoxy)thiazol-2-yl)-1-methylurea |
| **284** | 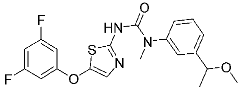 | 3-(5-(3,5-difluorophenoxy)thiazol-2-yl)-1-(3-(1-methoxyethyl)phenyl)-1-methylurea |
| **285** | 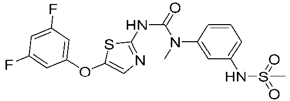 | N-(3-(3-(5-(3,5-difluorophenoxy)thiazol-2-yl)-1-methylureido)phenyl)methanesulfonamide |
| **286** | 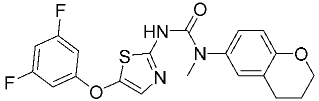 | 1-(chroman-6-yl)-3-(5-(3,5-difluorophenoxy)thiazol-2-yl)-1-methylurea |
| **287** | 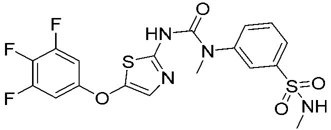 | N-methyl-3-(1-methyl-3-(5-(3,4,5-trifluorophenoxy)thiazol-2-yl)ureido)benzenesulfonamide |
| **288** | 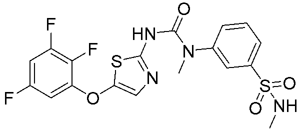 | N-methyl-3-(1-methyl-3-(5-(2,3,5-trifluorophenoxy)thiazol-2-yl)ureido)benzenesulfonamide |
| **289** | 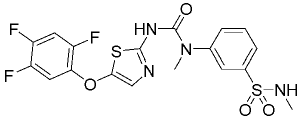 | N-methyl-3-(1-methyl-3-(5-(2,4,5-trifluorophenoxy)thiazol-2-yl)ureido)benzenesulfonamide |
| **290** | 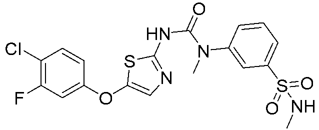 | 3-(3-(5-(4-chloro-3-fluorophenoxy)thiazol-2-yl)-1-methylureido)-N-methylbenzenesulfonamide |
| **291** | 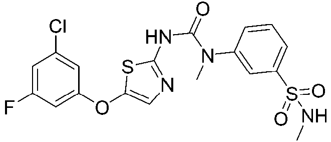 | 3-(3-(5-(3-chloro-5-fluorophenoxy)thiazol-2-yl)-1-methylureido)-N-methylbenzenesulfonamide |
| **292** | 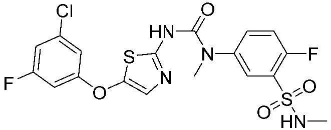 | 5-(3-(5-(3-chloro-5-fluorophenoxy)thiazol-2-yl)-1-methylureido)-2-fluoro-N-methylbenzenesulfonamide |
| **293** | 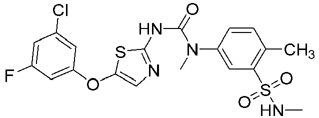 | 5-(3-(5-(3-chloro-5-fluorophenoxy)thiazol-2-yl)-1-methylureido)-N,2-dimethylbenzenesulfonamide |
| **294** | 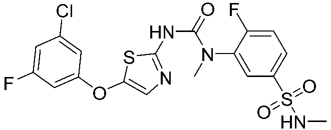 | 3-(3-(5-(3-chloro-5-fluorophenoxy)thiazol-2-yl)-1-methylureido)-4-fluoro-N-methylbenzenesulfonamide |
| **295** | 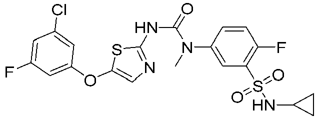 | 5-(3-(5-(3-chloro-5-fluorophenoxy)thiazol-2-yl)-1-methylureido)-N-cyclopropyl-2-fluorobenzenesulfonamide |
| **296** | 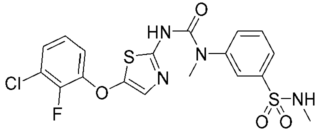 | 3-(3-(5-(3-chloro-2-fluorophenoxy)thiazol-2-yl)-1-methylureido)-N-methylbenzenesulfonamide |
| **297** | 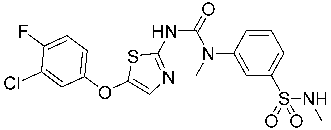 | 3-(3-(5-(3-chloro-4-fluorophenoxy)thiazol-2-yl)-1-methylureido)-N-methylbenzenesulfonamide |
| **298** | 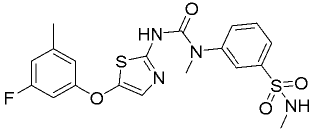 | 3-(3-(5-(3-fluoro-5-methylphenoxy)thiazol-2-yl)-1-methylureido)-N-methylbenzenesulfonamide |
| **299** | 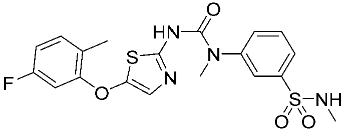 | 3-(3-(5-(5-fluoro-2-methylphenoxy)thiazol-2-yl)-1-methylureido)-N-methylbenzenesulfonamide |
| **300** | 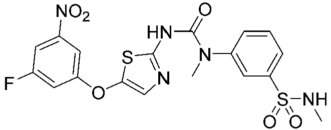 | 3-(3-(5-(3-fluoro-5-nitrophenoxy)thiazol-2-yl)-1-methylureido)-N-methylbenzenesulfonamide |
| **301** | 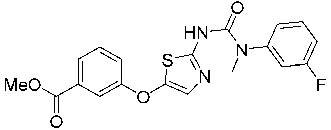 | Methyl 3-((2-(3-(3-fluorophenyl)-3-methylureido)thiazol-5-yl)oxy)benzoate |
| **302** | 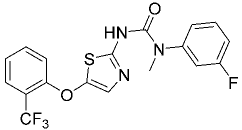 | 1-(3-fluorophenyl)-1-methyl-3-(5-(2-(trifluoromethyl)phenoxy)thiazol-2-yl)urea |
| **303** | 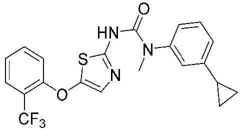 | 1-(3-cyclopropylphenyl)-1-methyl-3-(5-(2-(trifluoromethyl)phenoxy)thiazol-2-yl)urea |
| **304** | 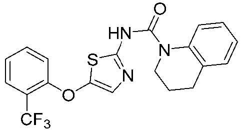 | N-(5-(2-(trifluoromethyl)phenoxy)thiazol-2-yl)-3,4-dihydroquinoline-1(2H)-carboxamide |
| 305 | 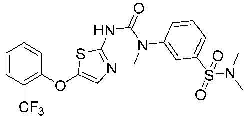 | N,N-dimethyl-3-(1-methyl-3-(5-(2-(trifluoromethyl)phenoxy)thiazol-2-yl)ureido)benzenesulfonamide |
| 306 | 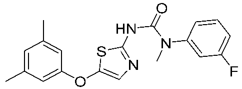 | 3-(5-(3,5-dimethylphenoxy)thiazol-2-yl)-1-(3-fluorophenyl)-1-methylurea |
| 307 | 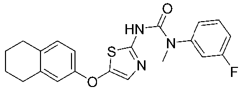 | 1-(3-fluorophenyl)-1-methyl-3-(5-((5,6,7,8-tetrahydronaphthalen-2-yl)oxy)thiazol-2-yl)urea |
| 308 | 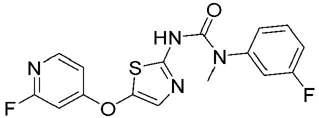 | 1-(3-fluorophenyl)-3-(5-((2-fluoropyridin-4-yl)oxy)thiazol-2-yl)-1-methylurea |
| **309** | 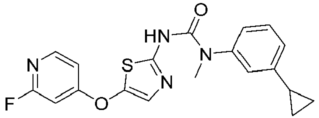 | 1-(3-cyclopropylphenyl)-3-(5-((2-fluoropyridin-4-yl)oxy)thiazol-2-yl)-1-methylurea |
| **310** | 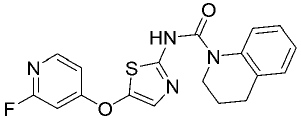 | N-(5-((2-fluoropyridin-4-yl)oxy)thiazol-2-yl)-3,4-dihydroquinoline-1(2H)-carboxamide |
| **311** | 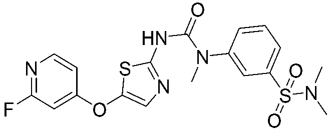 | 3-(3-(5-((2-fluoropyridin-4-yl)oxy)thiazol-2-yl)-1-methylureido)-N,N-dimethylbenzenesulfonamide |
| **312** | 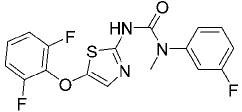 | 3-(5-(2,6-difluorophenoxy)thiazol-2-yl)-1-(3-fluorophenyl)-1-methylurea |
| **313** | 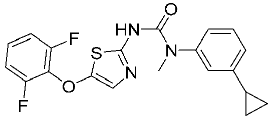 | 1-(3-cyclopropylphenyl)-3-(5-(2,6-difluorophenoxy)thiazol-2-yl)-1-methylurea |
| **314** | 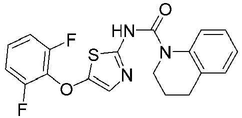 | N-(5-(2,6-difluorophenoxy)thiazol-2-yl)-3,4-dihydroquinoline-1(2H)-carboxamide |
| **315** | 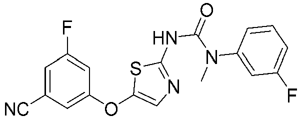 | 3-(5-(3-cyano-5-fluorophenoxy)thiazol-2-yl)-1-(3-fluorophenyl)-1-methylurea |
| **316** | 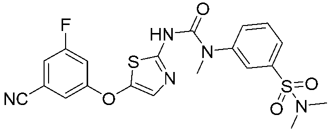 | 3-(3-(5-(3-cyano-5-fluorophenoxy)thiazol-2-yl)-1-methylureido)-N,N-dimethylbenzenesulfonamide |
| **317** | 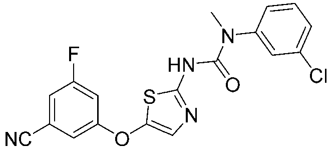 | 1-(3-chlorophenyl)-3-(5-(3-cyano-5-fluorophenoxy)thiazol-2-yl)-1-methylurea |
| **318** | 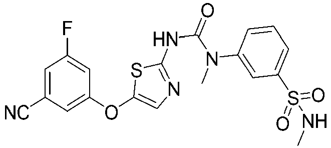 | 3-(3-(5-(3-cyano-5-fluorophenoxy)thiazol-2-yl)-1-methylureido)-N-methylbenzenesulfonamide |
| **319** | 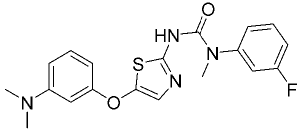 | 3-(5-(3-(dimethylamino)phenoxy)thiazol-2-yl)-1-(3-fluorophenyl)-1-methylurea |
| **320** | 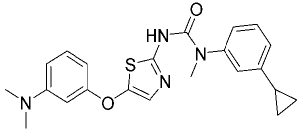 | 1-(3-cyclopropylphenyl)-3-(5-(3-(dimethylamino)phenoxy)thiazol-2-yl)-1-methylurea |
| **321** | 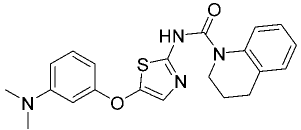 | N-(5-(3-(dimethylamino)phenoxy)thiazol-2-yl)-3,4-dihydroquinoline-1(2H)-carboxamide |
| **322** | 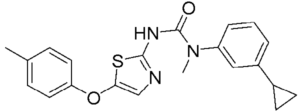 | 1-(3-cyclopropylphenyl)-1-methyl-3-(5-(p-tolyloxy)thiazol-2-yl)urea |
| **323** | 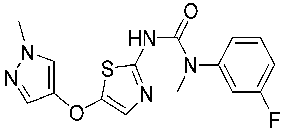 | 1-(3-fluorophenyl)-1-methyl-3-(5-((1-methyl-1H-pyrazol-4-yl)oxy)thiazol-2-yl)urea |
| **324** | 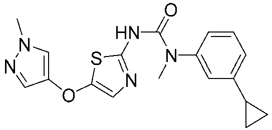 | 1-(3-cyclopropylphenyl)-1-methyl-3-(5-((1-methyl-1H-pyrazol-4-yl)oxy)thiazol-2-yl)urea |
| **325** | 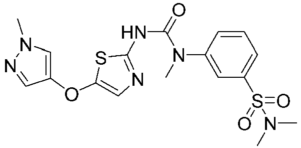 | N,N-dimethyl-3-(1-methyl-3-(5-((1-methyl-1H-pyrazol-4-yl)oxy)thiazol-2-yl)ureido)benzenesulfonamide |
| **326** | 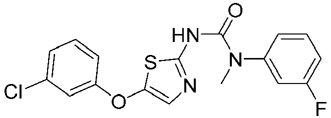 | 3-(5-(3-chlorophenoxy)thiazol-2-yl)-1-(3-fluorophenyl)-1-methylurea |
| **327** | 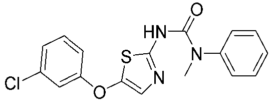 | 3-(5-(3-chlorophenoxy)thiazol-2-yl)-1-methyl-1-phenylurea |
| **328** | 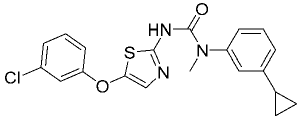 | 3-(5-(3-chlorophenoxy)thiazol-2-yl)-1-(3-cyclopropylphenyl)-1-methylurea |
| **329** | 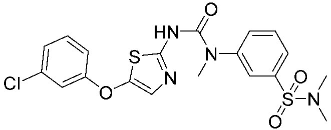 | 3-(3-(5-(3-chlorophenoxy)thiazol-2-yl)-1-methylureido)-N,N-dimethylbenzenesulfonamide |
| **330** | 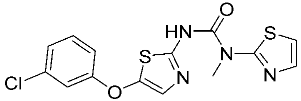 | 3-(5-(3-chlorophenoxy)thiazol-2-yl)-1-methyl-1-(thiazol-2-yl)urea |
| **331** | 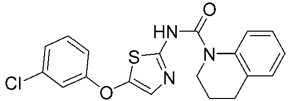 | N-(5-(3-chlorophenoxy)thiazol-2-yl)-3,4-dihydroquinoline-1(2H)-carboxamide |
| **332** | 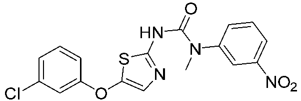 | 3-(5-(3-chlorophenoxy)thiazol-2-yl)-1-methyl-1-(3-nitrophenyl)urea |
| **333** | 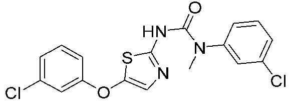 | 3-(5-(3-chlorophenoxy)thiazol-2-yl)-1-(3-chlorophenyl)-1-methylurea |
| **334** | 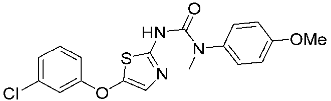 | 3-(5-(3-chlorophenoxy)thiazol-2-yl)-1-(4-methoxyphenyl)-1-methylurea |
| **335** | 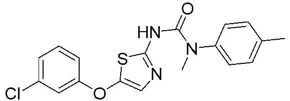 | 3-(5-(3-chlorophenoxy)thiazol-2-yl)-1-methyl-1-(p-tolyl)urea |
| **336** | 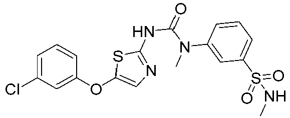 | 3-(3-(5-(3-chlorophenoxy)thiazol-2-yl)-1-methylureido)-N-methylbenzenesulfonamide |
| **337** | 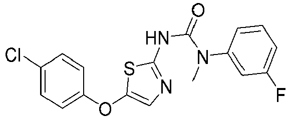 | 3-(5-(4-chlorophenoxy)thiazol-2-yl)-1-(3-fluorophenyl)-1-methylurea |
| **338** | 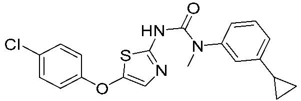 | 3-(5-(4-chlorophenoxy)thiazol-2-yl)-1-(3-cyclopropylphenyl)-1-methylurea |
| **339** | 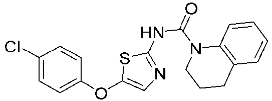 | N-(5-(4-chlorophenoxy)thiazol-2-yl)-3,4-dihydroquinoline-1(2H)-carboxamide |
| **340** | 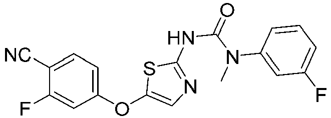 | 3-(5-(4-cyano-3-fluorophenoxy)thiazol-2-yl)-1-(3-fluorophenyl)-1-methylurea |
| **341** | 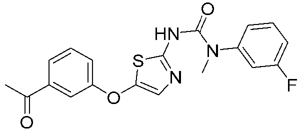 | 3-(5-(3-acetylphenoxy)thiazol-2-yl)-1-(3-fluorophenyl)-1-methylurea |
| **342** | 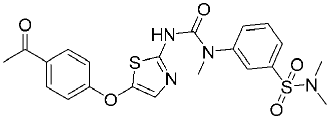 | 3-(3-(5-(4-acetylphenoxy)thiazol-2-yl)-1-methylureido)-N,N-dimethylbenzenesulfonamide |
| **343** | 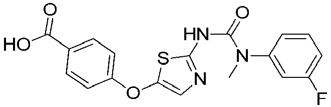 | 4-((2-(3-(3-fluorophenyl)-3-methylureido)thiazol-5-yl)oxy)benzoic acid |
| **344** | 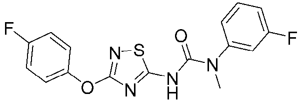 | 3-(3-(4-fluorophenoxy)-1,2,4-thiadiazol-5-yl)-1-(3-fluorophenyl)-1-methylurea |
| **345** | 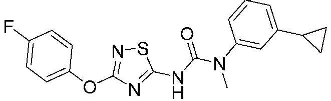 | 1-(3-cyclopropylphenyl)-3-(3-(4-fluorophenoxy)-1,2,4-thiadiazol-5-yl)-1-methylurea |
| **346** | 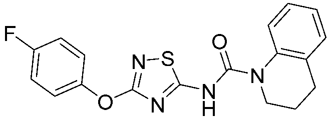 | N-(3-(4-fluorophenoxy)-1,2,4-thiadiazol-5-yl)-3,4-dihydroquinoline-1(2H)-carboxamide |
| **347** | 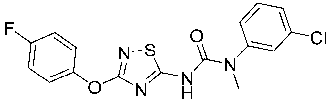 | 1-(3-chlorophenyl)-3-(3-(4-fluorophenoxy)-1,2,4-thiadiazol-5-yl)-1-methylurea |
| **348** | 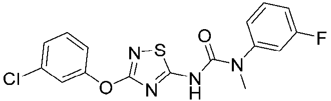 | 3-(3-(3-chlorophenoxy)-1,2,4-thiadiazol-5-yl)-1-(3-fluorophenyl)-1-methylurea |
| **349** | 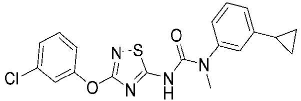 | 3-(3-(3-chlorophenoxy)-1,2,4-thiadiazol-5-yl)-1-(3-cyclopropylphenyl)-1-methylurea |
| **350** | 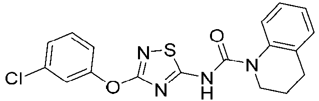 | N-(3-(3-chlorophenoxy)-1,2,4-thiadiazol-5-yl)-3,4-dihydroquinoline-1(2H)-carboxamide |
| **351** | 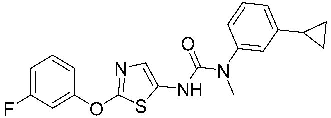 | 1-(3-cyclopropylphenyl)-3-(2-(3-fluorophenoxy)thiazol-5-yl)-1-methylurea |
| **352** | 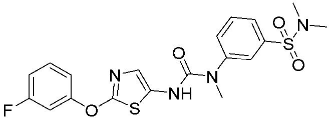 | 3-(3-(2-(3-fluorophenoxy)thiazol-5-yl)-1-methylureido)-N,N-dimethylbenzenesulfonamide |
| **353** | 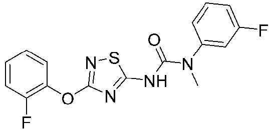 | 3-(3-(2-fluorophenoxy)-1,2,4-thiadiazol-5-yl)-1-(3-fluorophenyl)-1-methylurea |
| **354** | 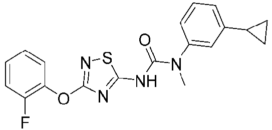 | 1-(3-cyclopropylphenyl)-3-(3-(2-fluorophenoxy)-1,2,4-thiadiazol-5-yl)-1-methylurea |
| **355** | 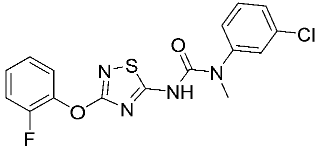 | 1-(3-chlorophenyl)-3-(3-(2-fluorophenoxy)-1,2,4-thiadiazol-5-yl)-1-methylurea |
| **356** | 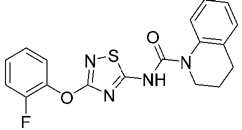 | N-(3-(2-fluorophenoxy)-1,2,4-thiadiazol-5-yl)-3,4-dihydroquinoline-1(2H)-carboxamide |
| **357** | 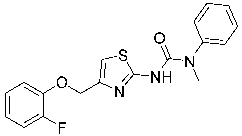 | 3-(4-((2-fluorophenoxy)methyl)thiazol-2-yl)-1-methyl-1-phenylurea |
| **358** | 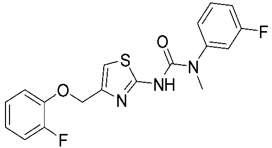 | 3-(4-((2-fluorophenoxy)methyl)thiazol-2-yl)-1-(3-fluorophenyl)-1-methylurea |
| **359** | 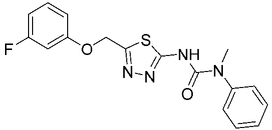 | 3-(5-((3-fluorophenoxy)methyl)-1,3,4-thiadiazol-2-yl)-1-methyl-1-phenylurea |
| **360** | 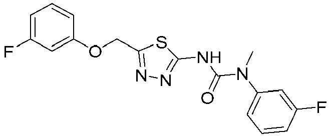 | 3-(5-((3-fluorophenoxy)methyl)-1,3,4-thiadiazol-2-yl)-1-(3-fluorophenyl)-1-methylurea |
| **361** | 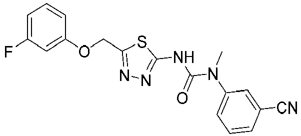 | 1-(3-cyanophenyl)-3-(5-((3-fluorophenoxy)methyl)-1,3,4-thiadiazol-2-yl)-1-methylurea |
| **362** | 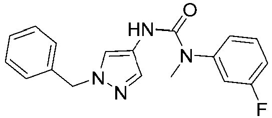 | 3-(1-benzyl-1H-pyrazol-4-yl)-1-(3-fluorophenyl)-1-methylurea |
| **363** | 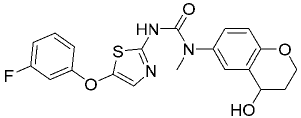 | 3-(5-(3-fluorophenoxy)thiazol-2-yl)-1-(4-hydroxychroman-6-yl)-1-methylurea |
| **364** | 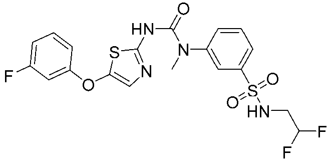 | N-(2,2-difluoroethyl)-3-(3-(5-(3-fluorophenoxy)thiazol-2-yl)-1-methylureido)benzenesulfonamide |

6. A pharmaceutical composition for preventing or treating diseases comprising at least one selected from allergic diseases comprising chronic urticaria, anaphylaxis, non-histaminergic itching, psoriasis, asthma, atopic dermatitis, or rosacea; inflammatory bowel disease; arthritis; and migraine, comprising the compound, the stereoisomer thereof, or the pharmaceutically acceptable salt thereof according to any one of claims 1 to 5 as an active ingredient.

7. A method for preventing or treating diseases comprising at least one selected from allergic diseases, inflammatory bowel disease, arthritis and migraine, the method comprising administering the compound, the stereoisomer thereof, or the pharmaceutically acceptable salt thereof according to any one of claims 1 to 5.

8. A use of the compound, the stereoisomer thereof, or the pharmaceutically acceptable salt thereof according to any one of claims 1 to 5 for preventing or treating diseases comprising at least one selected from allergic diseases, inflammatory bowel disease, arthritis, and migraine.

9. A use of the compound, the stereoisomer thereof or the pharmaceutically acceptable salt thereof according to any one of claims 1 to 5 in the manufacture of a medicament for preventing or treating diseases comprising at least one selected from allergic diseases, inflammatory bowel disease, arthritis, and migraine.
